(19) Europäisches Patentamt
European Patent Office
Office européen des brevets

(11) **EP 4 432 289 A1**

(12) **EUROPEAN PATENT APPLICATION**

(43) Date of publication:
**18.09.2024 Bulletin 2024/38**

(21) Application number: **24163981.4**

(22) Date of filing: **15.03.2024**

(51) International Patent Classification (IPC):
**G16B 20/00** (2019.01)     **G16B 40/20** (2019.01)

(52) Cooperative Patent Classification (CPC):
**G16B 40/20; G16B 20/00**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **15.03.2023 US 202363490435 P
07.06.2023 US 202363506770 P
20.06.2023 US 202363509254 P
29.06.2023 US 202363511138 P
25.09.2023 US 202363585119 P**

(71) Applicant: **Shape Therapeutics Inc.
Seattle, WA 98109 (US)**

(72) Inventors:
• **BANJANIN, Bora Srecko
Seattle, WA 98109 (US)**
• **HAUSE JR., Ronald James
Seattle, WA 98109 (US)**
• **JIANG, Yue
Seattle, WA 98109 (US)**
• **BOOTH, Brian
Seattle, WA 98109 (US)**
• **SAVVA, Yiannis
Seattle, WA 98109 (US)**
• **BAGEPALLI, Lina Rajili
Seattle, WA 98109 (US)**
• **STEIN, Kevin Christopher
Seattle, WA 98109 (US)**
• **BRIGGS, Adrian Wrangham
Seattle, WA 98109 (US)**
• **PACKARD, Thomas Alexander
Seattle, WA 98109 (US)**

(74) Representative: **Korenberg, Alexander Tal et al
Kilburn & Strode LLP
Lacon London
84 Theobalds Road
London WC1X 8NL (GB)**

Remarks:
The complete document including Reference
Table(s) and the Sequence Listing(s) can be
downloaded from the EPO website

(54) **GENERATIVE SEQUENCE SCREENING WITH CONDITIONAL GANS, DIFFUSION MODELS, AND DENOISING DIFFUSION CONDITIONAL GANS**

(57)    Systems and methods for generating a polymer sequence for a biological molecule having one or more target biological properties are provided. A plurality of target metric values for one or more target biological properties of a biological molecule and a seed for a nucleic acid or amino acid sequence for the biological molecule are inputted into a conditional generator model of a conditional generative adversarial network to obtain as output from the conditional generator model a nucleic acid or amino acid sequence for the biological molecule that is predicted by the conditional generator model to confer on the biological molecule the one or more target biological properties approximating the plurality of target metric values.

FIG. 23B

**Description**

**1. CROSS REFERENCE TO RELATED APPLICATIONS**

This application claim priority to U.S. Provisional Patent Application No. 63/490,435, filed March 15, 2023, U.S. Provisional Patent Application No. 63/506,770, filed June 7, 2023, U.S. Provisional Patent Application No. 63/509,254, filed June 20, 2023, U.S. Provisional Patent Application No. 63/5011,138, filed June 29, 2023, and U.S. Provisional Patent Application No. 63/585,119, filed September 25, 2023, the contents of which are hereby incorporated by reference herein, in their entireties, for all purposes.

**2. TECHNICAL FIELD**

This specification describes technologies generally relating to generating sequences using models, in particular generative adversarial networks (GANs), diffusion models, and denoising diffusion conditional GANs (ddGANs).

**3. SEQUENCE LISTING**

The instant application contains a Sequence Listing which has been submitted herewith and is hereby incorporated by reference in its entirety. Said .xml copy, created on March 13, 2024, is named 128604-5007-SEQ.xml, and is 27,879 bytes in size.

**4. BACKGROUND**

RNA editing is a post-transcriptional process that recodes hereditary information by changing the nucleotide sequence of RNA molecules (Rosenthal, J Exp Biol. 2015 June; 218(12): 1812-1821). One form of post-transcriptional RNA modification is the conversion of adenosine-to-inosine (A-to-I), mediated by adenosine deaminase acting on RNA (ADAR) enzymes. Adenosine-to-inosine (A-to-I) RNA editing alters genetic information at the transcript level and is a biological process commonly conserved in metazoans. A-to-I editing is catalyzed by RNP complexes formed between guide RNAs (gRNAs) and adenosine deaminase acting on RNA (ADAR) enzymes. Such an intracellular RNA-editing mechanism potentially provides a versatile RNA-mutagenesis method for transcriptome manipulation. Another form of post-transcriptional RNA modification is the conversion of cytidine to uracil (C to U), mediated by RNP complexes formed between guide RNAs and apolipoprotein B editing complex (APOBEC) enzymes.

Current systems used to edit RNA have limitations which, in some embodiments, lead to aberrant effector activity, have a delivery barrier, unintended transcriptomic modifications, or immunogenicity. Further methods and systems for improved efficiency, specificity, and safety of targeted RNA editing are needed.

Recombinant adeno-associated viruses (rAAV) provide the leading platform for in vivo delivery of gene therapies. Current clinical trials employ a limited number of AAV capsids, primarily from naturally occurring human or primate serotypes such as AAV1, AAV2, AAV5, AAV6, AAV8, AAV9, AAVrh.10, AAV4rh.74, and AAVhu.67. These capsids often provide suboptimal targeting to tissues of interest, both due to poor infectivity of the tissue of interest and competing liver tropism. Increasing the dose to ensure infection of desired tissues can lead to dose-dependent liver toxicity. In addition, use of naturally-occurring capsids presents an immunological memory challenge - pre-immune patient populations are excluded from treatment and repeat dosing in a previously immune naïve patient is often not possible. Thus, there is a need for additional AAV capsids for use in gene therapy, in particular capsids that confer upon the rAAV high infectivity for specific tissues, such as muscle tissue and tissues in the central nervous system, and low liver tropism.

Regulatory elements, including promoters, enhancers, insulators, and the like operate in a sequence-specific fashion to direct transcription and/or translation. Discovery of sequence determinants of these regulatory elements, including tissue-specific activities, is made difficult by the fact that the genome is repetitive and has evolved to perform multiple functions. Furthermore, the human genome is too short to encode all combinations, orientations and spacings of approximately 1,639 human transcription factors in multiple independent sequence contexts. Thus, despite the information generated by genome-scale experiments, most sequence determinants that drive the activity of regulatory elements, including tissue specific activity, remain unknown. This is further complicated by the intricacy of binding site (*e.g.*, transcription factor binding sites) grammar of individual regulatory elements. For instance, enhancers typically have clusters of such binding sites, the presence and arrangement of which is defined by a grammar that affects the overall ability of a given enhancer to promote gene expression and, in some instances, the tissue specificity of such gene expression.

## 5. SUMMARY

In general, there is a need to generate candidate sequences of biological polymers, such as DNA, RNA, and protein sequences, that are likely to have target properties for a given application, to provide a more efficient and focused search space for candidate molecules having target properties. Given the above background, there is a need in the art for improved methods and systems for determining polymer sequences, such as sequences for guide RNAs, regulatory elements, and/or AAV capsid proteins. Provided herein, among other aspects are machine learning approaches to evaluating, predicting, and/or designing polymer sequences using a model, *e.g.*, a generative adversarial network (GAN), a diffusion model, and/or a denoising diffusion conditional GAN (ddGAN).

One aspect of the present disclosure provides a method for generating a polymer sequence for a biological molecule having one or more target biological properties. In some embodiments, the method is performed at a computer system comprising at least one processor and a memory storing at least one program for execution by the at least one processor.

In some embodiments, the method includes inputting (i) a plurality of target metric values for one or more target biological properties of a biological molecule and (ii) a seed for a nucleic acid or amino acid sequence for the biological molecule into a conditional generator model of a conditional generative adversarial network to obtain as output from the conditional generator model a nucleic acid or amino acid sequence for the biological molecule that is predicted by the conditional generator model to confer on the biological molecule the one or more target biological properties approximating the plurality of target metric values (*e.g.*, where the generator model comprises at least 10,000 parameters).

Another aspect of the present disclosure provides a method for generating a polymer sequence for a biological molecule having one or more target biological properties. In some embodiments, the method is performed at a computer system comprising at least one processor and a memory storing at least one program for execution by the at least one processor.

In some embodiments, the method includes inputting (i) a plurality of target metrics for one or more target biological properties of a biological molecule and (ii) a seed for a nucleic acid or amino acid sequence for the biological molecule into an initial state $X_1$ in a plurality of consecutive states $X_N$ in a Markov chain of a generative diffusion model to obtain as output from the generative diffusion model a nucleic acid or amino acid sequence for the biological molecule that is predicted by the generative diffusion model to confer on the biological molecule the one or more target biological properties approximating the plurality of target metrics. For each respective consecutive state $X_n$ in the plurality of consecutive states $X_N$ in the Markov chain following the initial state Xi, the diffusion model generates a corresponding denoised seed for the nucleic acid or amino acid sequence for the biological molecule using a transition model, where the transitional model comprises a plurality of layers, that accounts for (*e.g.*, wherein the corresponding denoised seed accounts for) the plurality of target metrics for the one or more target biological properties using as input: the seed for the nucleic acid or amino acid sequence, when the respective state $X_n$ is the state immediately following the initial state Xi, and the corresponding denoised seed sequence from the respective state $X_{n-1}$ in the plurality of consecutive states $X_N$ in the Markov chain that immediately precedes the respective state $X_n$, when the respective state $X_n$ is not the state immediately following the initial state Xi.

In some embodiments, an indication of the position, in the Markov chain, of the transition from the state that immediately precedes the respective consecutive state $X_n$ is incorporated into one or more respective layers in the plurality of layers of the transition model; and the generative diffusion model generates, for each respective consecutive state $X_n$ in the plurality of consecutive states $X_N$ in the Markov chain following the initial state Xi, the corresponding denoised seed for the nucleic acid or amino acid sequence for the biological molecule. In some embodiments, the plurality of layers of the transition model comprises one or more attention layers that attend to an embedding of the indication of the position, in the Markov chain, of the transition. In some embodiments, the plurality of layers of the transition model comprises one or more temporal projection layers that project the indication of the position, in the Markov chain, of the transition on an output of a previous layer in the plurality of layers of the transition model using a mapping function between the indication of the position, in the Markov chain, of the transition and a corresponding set of weights for the temporal projection layer.

In some embodiments, the transition model comprises a U-Net neural network.

In some embodiments, the transition model is a conditional generator model of a conditional generative adversarial network that generates, for each respective consecutive state $X_n$ in the plurality of consecutive states $X_N$ in the Markov chain following the initial state Xi, a corresponding nucleic acid or amino acid sequence predicted by the conditional generator model to confer on the biological molecule the one or more target biological properties approximating the plurality of target metric vales using as input: the seed for the nucleic acid or amino acid sequence, when the respective state $X_n$ is the state immediately following the initial state Xi, and the corresponding denoised seed sequence from the respective state $X_{n-1}$ in the plurality of consecutive states $X_N$ in the Markov chain that immediately precedes the respective state $X_n$, when the respective state $X_n$ is not the state immediately following the initial state $X_1$; and for each respective consecutive state $X_n$ in the plurality of consecutive states $X_N$ in the Markov chain following the initial state Xi, other than a terminal state $X_{n=N}$ in the Markov chain, the diffusion model samples from a posterior distribution of seed values for the corresponding nucleic acid or amino acid sequence to generate the corresponding denoised seed for the nucleic acid or amino acid sequence.

In some embodiments, the plurality of target metrics for the one or more target biological properties of the biological molecule are incorporated into one or more respective layers in the plurality of layers of the transition model.

In some embodiments, the plurality of layers of the transition model comprises one or more attention layers that attend to an embedding of the plurality of target metrics for the one or more target biological properties of the biological molecule. In some embodiments, the plurality of layers of the transition model comprises one or more projection layers that project the plurality of target metrics for the one or more target biological properties on an output of a previous layer in the plurality of layers of the transition model using a mapping function between the plurality of target metrics for the one or more target biological properties of the biological molecule and a corresponding set of weights for the projection layer. In some embodiments, the transition model comprises a U-Net neural network having a first plurality of layer blocks and a second plurality of layer blocks, where: each respective layer block in the first plurality of layer blocks comprises a residual network layer and a down-sampling layer; each respective layer block in the second plurality of layer blocks comprises a residual network layer and an up-sampling layer; and at least one respective block layer in the first plurality of block layers or the second plurality of block layers further comprises a respective projection layer in the one or more projection layers. In some embodiments, at least one respective block layer in the first plurality of block layers further comprises a respective projection layer in the one or more projection layers; and at least one respective block layer in the second plurality of block layers further comprises a respective projection layer in the one or more projection layers. In some embodiments, each respective block layer in the first plurality of block layers further comprises a respective projection layer in the one or more projection layers; and each respective block layer in the second plurality of block layers further comprises a respective projection layer in the one or more projection layers.

In some embodiments, the generative diffusion model is a bit diffusion model.

For example, in some embodiments, a nucleotide sequence having $n$ nucleotides is represented as $n$ discrete bit tokens, *e.g.*, 2-bit tokens, 3-bit tokens, 4-bit tokens, *etc.*

In some embodiments, the biological molecule is a polynucleotide and the sequence of the polynucleotide is represented with a 2-bit encoding. That is, each character in the 2-bit encoding represents a different nucleotide, *e.g.*, 00 = adenine, 01 = cytosine, 10 = guanine, and 11 = thymine/uridine, or any other assignment of the four nucleic acids to the four characters of a 2-bit encoding. In some embodiments, the encoding of the polynucleotide sequence, *e.g.*, a seed for the sequence of the polynucleotide, is mapped from {0, 1} to {-1, 1}. That is, "0's" in the encoding are mapped to "-1's" and the value is allowed to float between -1 and 1, rather than between 0 and 1, during denoising.

In some embodiments, the biological molecule is a polynucleotide and the sequence of the polynucleotide is represented with a 4-bit encoding. For example, in some embodiments, each position of the 4-bit character represents a different nucleotide, *e.g.*, the first position (*e.g.*, character 1000) represents adenine, the second position (*e.g.*, character 0100) represents cytosine, the third position (*e.g.*, character 0010) represents guanine, and the fourth position (*e.g.*, character 0001) represents thymine/uridine, or any other assignment of the four natural nucleic acids to the sixteen characters of a 4-bit encoding. In some embodiments, the encoding of the polynucleotide sequence, *e.g.*, a seed for the sequence of the polynucleotide, is mapped from {0, 1} to {-1, 1}. That is, "0's" in the encoding are mapped to "-1's" and the value is allowed to float between -1 and 1, rather than between 0 and 1, during denoising.

In some embodiments, the biological molecule is a polypeptide and the sequence of the polypeptide is represented with a 5-bit encoding. That is, separate characters in the 5-bit encoding represent different amino acids. In some embodiments, the encoding of the polypeptide sequence, *e.g.*, a seed for the sequence of the polypeptide, is mapped from {0, 1} to {-1, 1}. That is, "0's" in the encoding are mapped to "-1's" and the value is allowed to float between -1 and 1, rather than between 0 and 1, during denoising.

In some embodiments, 5-bit encoding is established with distances between the 5-bit code for the 20 amino acids determined via dimensionality reduction based on their biophysical properties. In this fashion, amino acids with more similar biological properties are closer together in 5-bit space, facilitating easy sampling through similar amino acids during the diffusion process. For instance, as shown in the encoding schema exemplified in Table 2, leucine and isoleucine, which are biophysically very similar, are encoded by the closely related 5-bit characters 10100 (Ile) and 10101 (Lue). Conversely, the biophysical dissimilar amino acids alanine and phenylalanine are encoded by the distantly-related 5-bit characters 00001 (Ala) and 11000 (Phe).

In some embodiments, the transition model generates, for each respective consecutive state $X_n$ in the plurality of consecutive states $X_N$ in the Markov chain following the initial state $X_1$, a corresponding nucleic acid or amino acid sequence predicted by the conditional generator model to confer on the biological molecule the one or more target biological properties approximating the plurality of target metric vales using as input: the seed for the nucleic acid or amino acid sequence, when the respective state $X_n$ is the state immediately following the initial state Xi, and the corresponding denoised seed sequence from the respective state $X_{n-1}$ in the plurality of consecutive states $X_N$ in the Markov chain that immediately precedes the respective state $X_n$, when the respective state $X_n$ is not the state immediately following the initial state $X_1$; and for each respective consecutive state $X_n$ in the plurality of consecutive states $X_N$ in the Markov chain following the initial state Xi, other than a terminal state $X_{n=N}$ in the Markov chain, the diffusion model samples from a posterior distribution of seed values for the corresponding nucleic acid or amino acid sequence to generate the corresponding denoised seed for the nucleic acid or amino acid sequence.

In some embodiments, for a respective consecutive state $X_n$ in the plurality of consecutive states $X_N$ in the Markov chain, the corresponding nucleic acid or amino acid sequence predicted by the conditional generator model to confer on the biological molecule the one or more target biological properties approximating the plurality of target metric vales is self-conditioned on the corresponding nucleic acid or amino acid sequence predicted by the conditional generator model to confer on the biological molecule the one or more target biological properties from the respective state $X_{n-1}$ in the plurality of consecutive states $X_N$ in the Markov chain that immediately precedes the respective state $X_n$.

In some embodiments, the biological molecule is a nucleic acid.

In some embodiments, the nucleic acid is a transcriptional or translational regulatory element.

In some embodiments, the nucleic acid is a guide RNA (gRNA) that facilitates deamination of one or more target adenosines in a target RNA by an Adenosine Deaminases Acting on RNA (ADAR) protein.

In some embodiments, the nucleic acid is a gRNA that facilitates deamination of one or more target cytidines in a target RNA by an Apolipoprotein B mRNA Editing Catalytic Polypeptide-like (APOBEC) protein.

In some embodiments, the one or more target biological properties comprises a metric for the efficiency of deamination of the one or more target adenosines by a first ADAR protein or the one or more target cytidines by a first APOBEC protein.

In some embodiments, the metric for the efficiency of deamination is (i) a prevalence of deamination of the one or more target adenosines or the one or more target cytidines in a plurality of instances of the target mRNA or (ii) a prevalence of the absence of deamination of any nucleotide position in a respective instance of a target mRNA in a plurality of instances of the target mRNA.

In some embodiments, the one or more target biological properties comprises a metric for the specificity of deamination of the one or more target adenosines or the one or more target cytidines relative to one or more nucleotide positions, other than the nucleotide positions of the one or more target adenosines or the one or more target cytidines, in a target mRNA by a first ADAR protein or a first APOBEC protein.

In some embodiments, the metric for the specificity of deamination of the target nucleotide position relative to one or more nucleotide positions, other than the target nucleotide position, in the target mRNA by the first ADAR protein or the first APOBEC protein is: (i) a comparison of (a) a prevalence of deamination of the target nucleotide position in a plurality of instances of the target mRNA and (b) a prevalence of deamination of at least one nucleotide position, other than the target nucleotide position, in a respective instance of the target mRNA in a plurality of instances of the target mRNA, (ii) a prevalence of deamination of the target nucleotide position, without coincident deamination of one or more nucleotide positions other than the target nucleotide position, in a respective instance of the target mRNA in a plurality of instances of the target mRNA, or (iii) a prevalence of deamination of at least one nucleotide position, other than the target nucleotide position, in a respective instance of the target mRNA in a plurality of instances of the target mRNA.

In some embodiments, at the one or more nucleotide positions, other than the target nucleotide position, in the target mRNA, deamination results in a non-synonymous codon edit.

In some embodiments, a respective biological property in the one or more target biological properties is normalized by a metric for an efficiency or specificity of deamination of one or more nucleotide positions, other than the target nucleotide position, in a target mRNA by a first ADAR protein or a first APOBEC protein.

In some embodiments, the one or more target biological properties comprises a metric for an efficiency or specificity of deamination of one or more nucleotide positions, other than the target nucleotide position, in a target mRNA by a first ADAR protein or first APOBEC protein when facilitated by hybridization of the gRNA to a target mRNA.

In some embodiments, the first ADAR protein is human ADAR1 or human ADAR2.

In some embodiments, the one or more target biological properties comprises an estimation of a minimum free energy (MFE) for the gRNA.

In some embodiments, the one or more target biological properties comprises an estimation of a minimum free energy (MFE) for the guide-target RNA scaffold formed between the guide RNA (gRNA) and the target mRNA.

In some embodiments, a polynucleotide sequence for the target mRNA, encompassing the target nucleotide position and at least a region of the mRNA 5' of the target nucleotide position and a region of the mRNA 3' of the target nucleotide position, is incorporated into one or more respective layers in the plurality of layers of the transition model. In some embodiments, the plurality of layers of the transition model comprises one or more attention layers that attend to an embedding of the polynucleotide sequence for the target mRNA. In some embodiments, the plurality of layers of the transition model comprises one or more projection layers that project the polynucleotide sequence for the target mRNA on an output of a previous layer in the plurality of layers of the transition model using a mapping function between an embedding of polynucleotide sequence for the target mRNA and a corresponding set of weights for the projection layer. In some embodiments, the transition model comprises a U-Net neural network having a first plurality of layer blocks and a second plurality of layer blocks, where: each respective layer block in the first plurality of layer blocks comprises a residual network layer and a down-sampling layer; each respective layer block in the second plurality of layer blocks comprises a residual network layer and an up-sampling layer; and at least one respective block layer in the first plurality of block layers or the second plurality of block layers further comprises a respective projection layer in the one or more projection layers. In some embodiments, at least one respective block layer in the first plurality of block layers further comprises a respective projection layer in the one or more projection layers; and at least one respective block layer in the second plurality of block layers further comprises a respective projection layer in the one or more projection layers. In some embodiments, each respective block layer in the first plurality of block layers further comprises a respective projection layer in the one or more projection layers; and each respective block layer in the second plurality of block layers further comprises a respective projection layer in the one or more projection layers.

In some embodiments, the biological molecule is a polypeptide.

In some embodiments, the polypeptide is all or a portion of a capsid protein.

In some embodiments, the one or more target biological properties of the polypeptide comprise a measure of specificity of a recombinant Adeno Associated Virus (rAAV) comprising the capsid protein.

In some embodiments, the measure of specificity of the rAAV for a respective tissue type in the one or more tissue types is normalized by a measure of specificity of a wild type AAV of the same serotype as the rAAV for the respective tissue type.

In some embodiments, the measure of specificity of the rAAV for a respective tissue type in the one or more tissue types is normalized by a measure of specificity of the rAAV for one or more tissue types other than the respective tissue type.

Still another aspect of the present disclosure provides a computer system including one or more processors and a non-transitory computer-readable medium including computer-executable instructions that, when executed by the one or more processors, cause the processors to perform any of the methods and/or embodiments disclosed above.

Yet another aspect of the present disclosure provides a non-transitory computer-readable storage medium having stored thereon program code instructions that, when executed by a processor, cause the processor to perform any of the methods and/or embodiments disclosed above.

The systems, methods, and non-transitory computer readable storage medium of the present invention have other features and advantages that will be apparent from, or are set forth in more detail in, the accompanying drawings, which are incorporated herein, and the following Detailed Description, which together serve to explain certain principles of exemplary embodiments of the present invention.

## 6. BRIEF DESCRIPTION OF THE DRAWINGS

The patent or application file contains at least one drawing executed in color. Copies of this patent or patent application publication with color drawing(s) will be provided by the Office upon request and payment of the necessary fee.

FIGS. 1A, 1B, 1C, 1D, and 1E collectively illustrate example schematics for a conditional generative adversarial network (GAN), in accordance with some embodiments of the present disclosure. FIG. 1A illustrates an example schematic for training a conditional GAN. FIGS. 1B and 1C collectively illustrate example representations of inputs and outputs used for training a conditional GAN. FIG. 1D illustrates an example schematic for using a generator model from a conditional GAN. FIG. 1E illustrates example representations of inputs and outputs for use in a generator model from a conditional GAN.

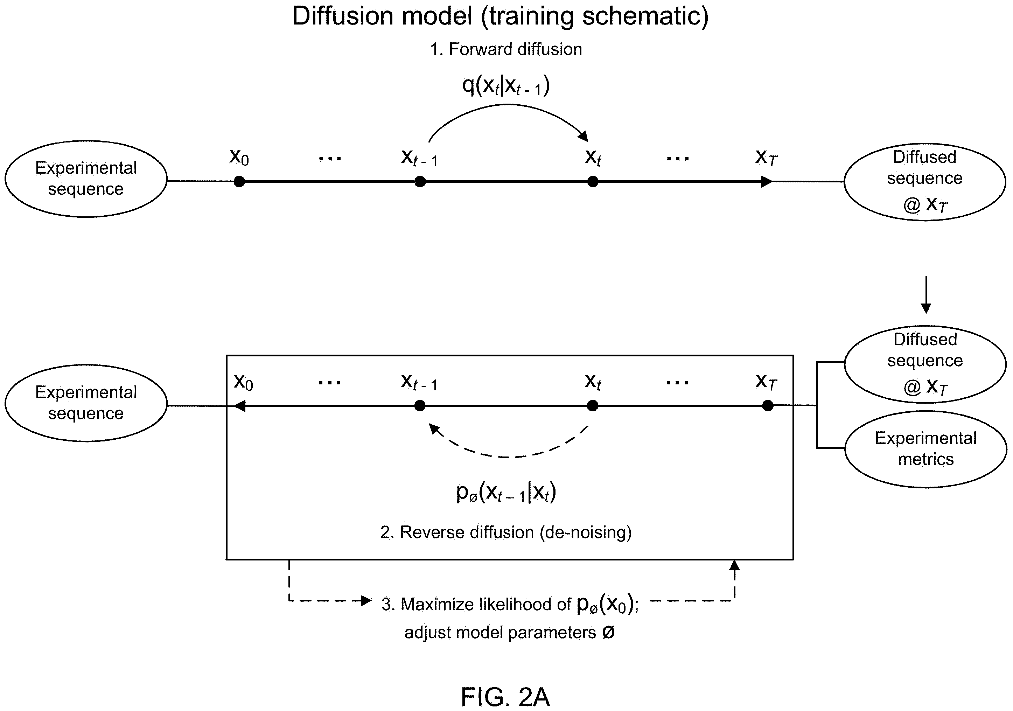

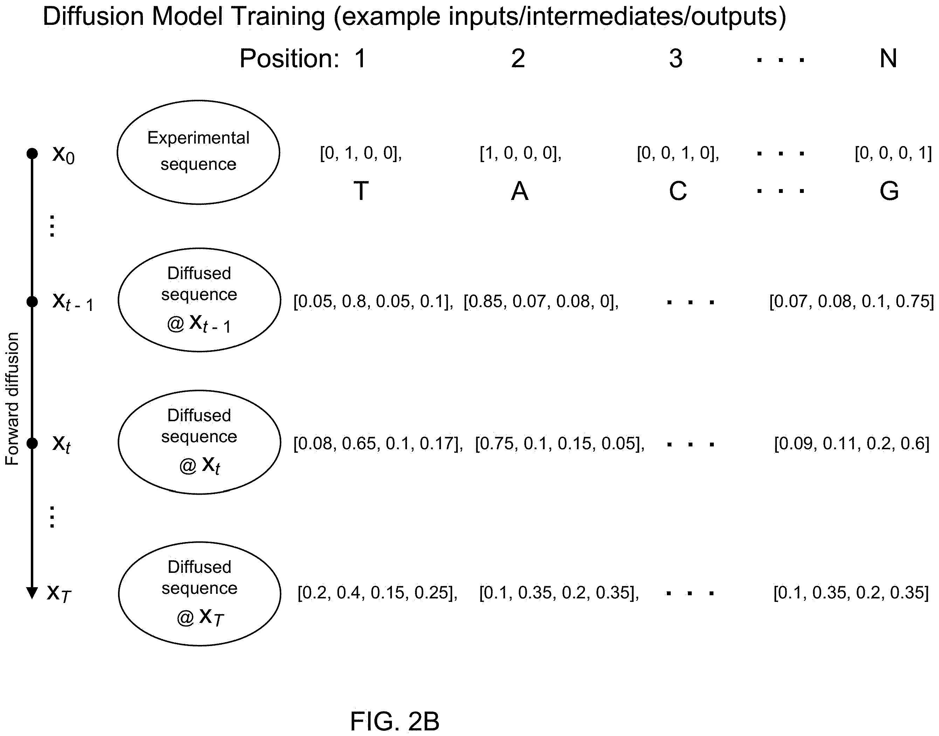

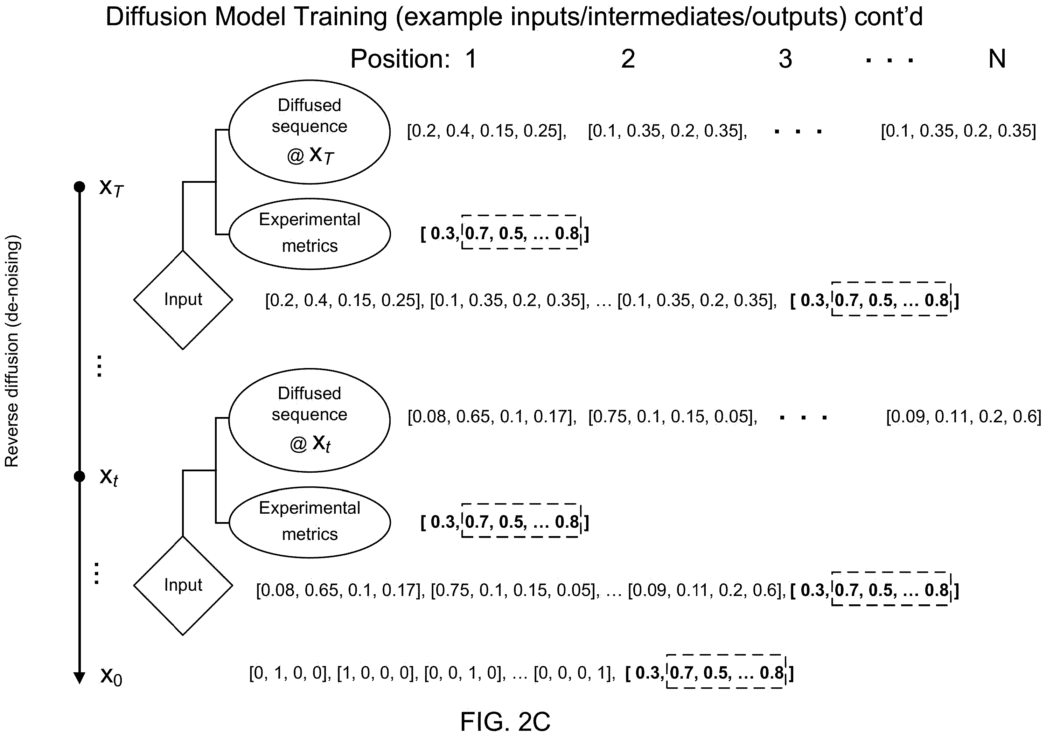

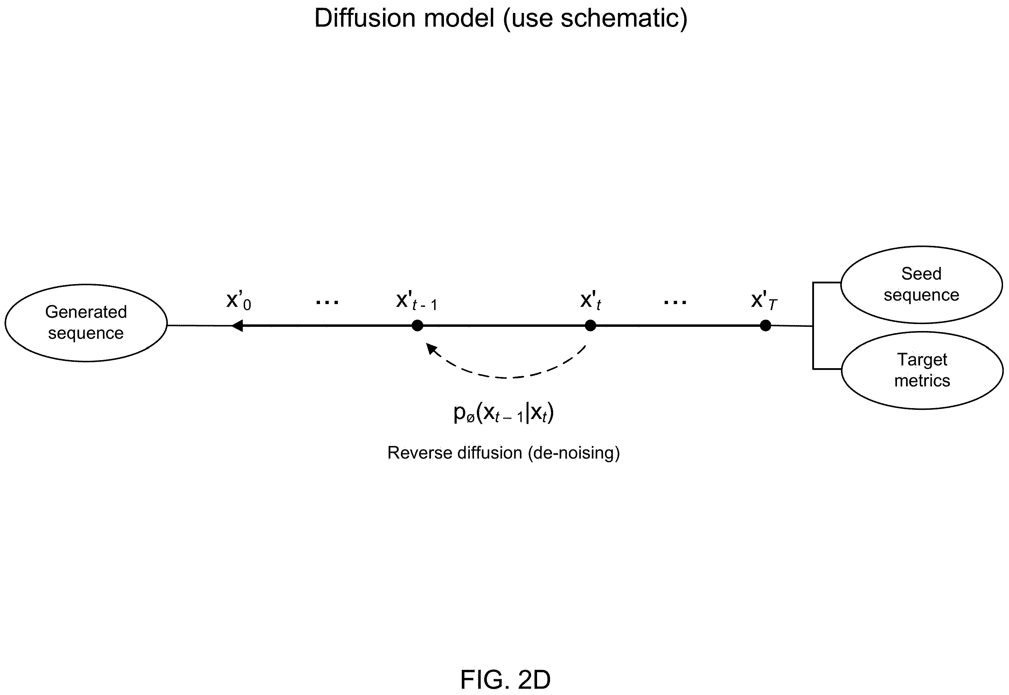

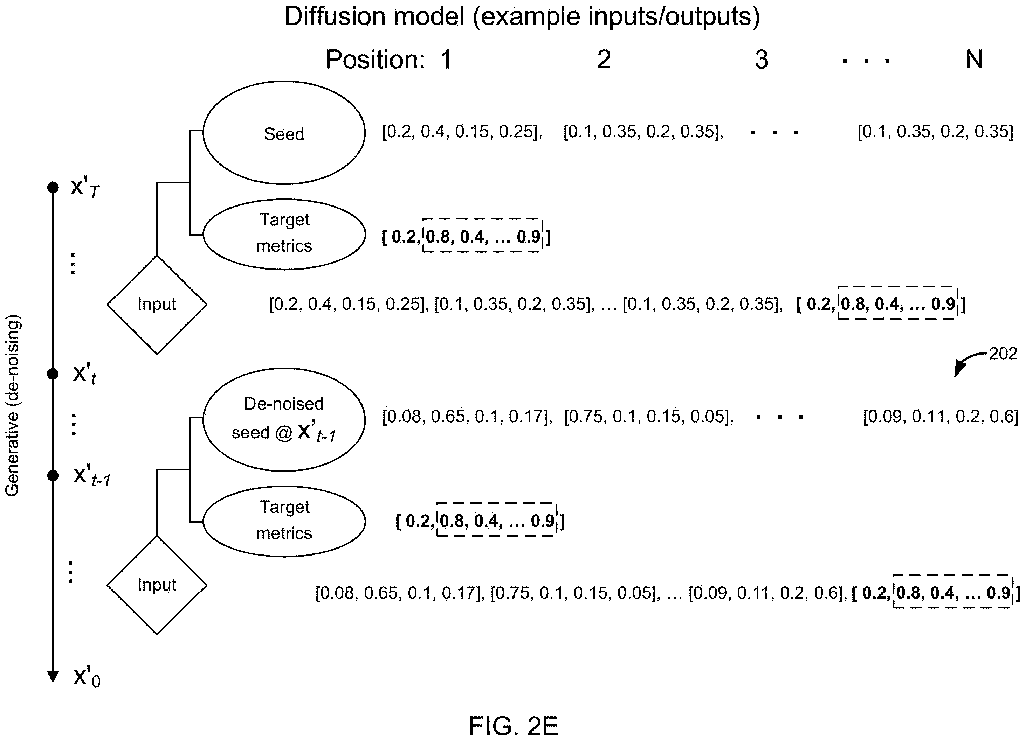

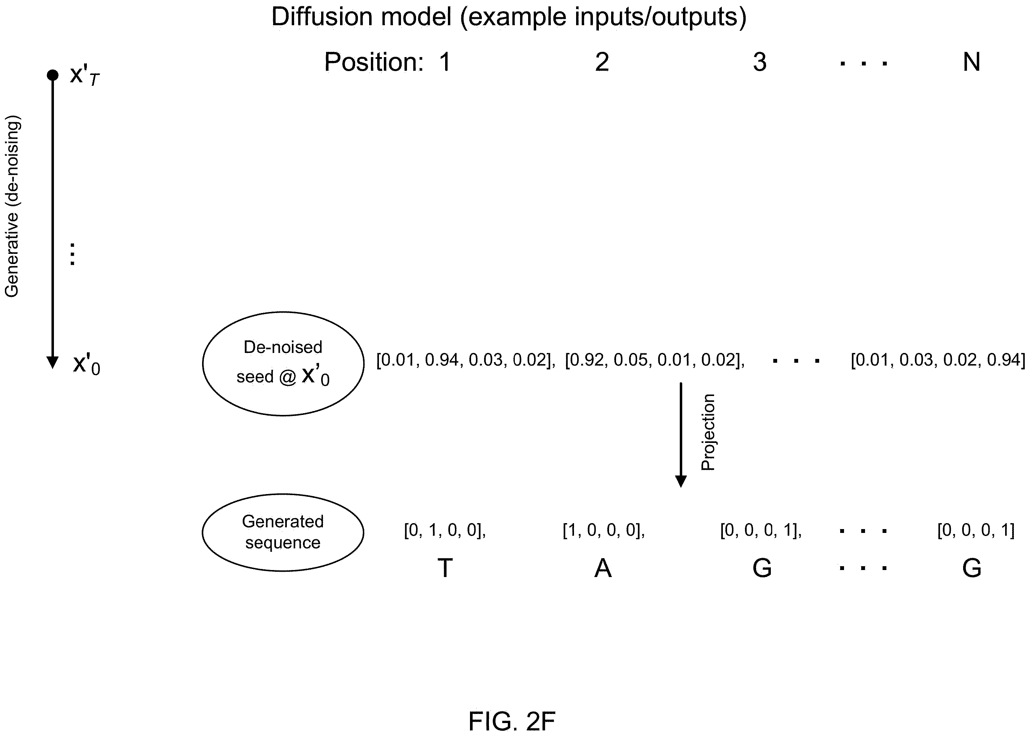

FIGS. 2A, 2B, 2C, 2D, 2E, and 2F collectively illustrate example schematics for a diffusion model, in accordance with some embodiments of the present disclosure. FIG. 2A illustrates an example schematic for training a diffusion model. FIGS. 2B and 2C collectively illustrate example representations of inputs and outputs used for training a diffusion model. FIG. 2D illustrates an example schematic for generating a polymer sequence using reverse diffusion or denoising via a diffusion model. FIGS. 2E and 2F collectively illustrate example representations of inputs used and outputs obtained from a diffusion model for generating a polymer sequence.

FIGS. 3A and 3B collectively illustrate example schematics for a denoising diffusion conditional GAN (ddGAN), in accordance with some embodiments of the present disclosure. FIG. 3A illustrates an example schematic for training a conditional ddGAN. FIG. 3B illustrates an example schematic for generating a polymer sequence using a conditional ddGAN.

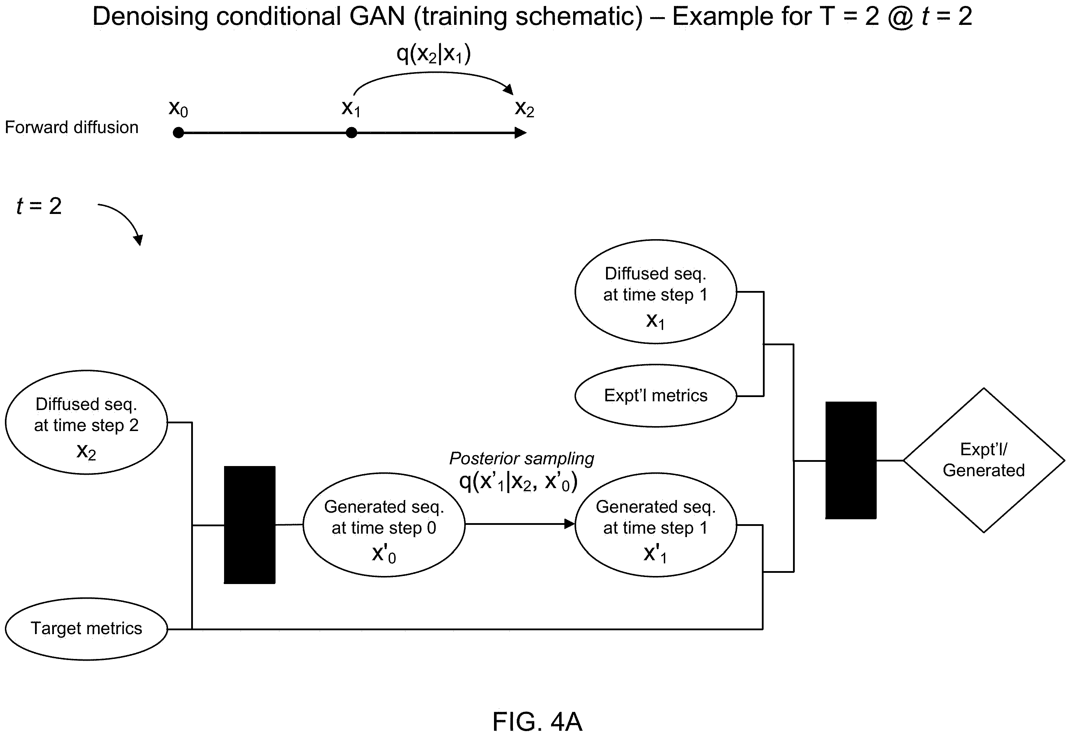

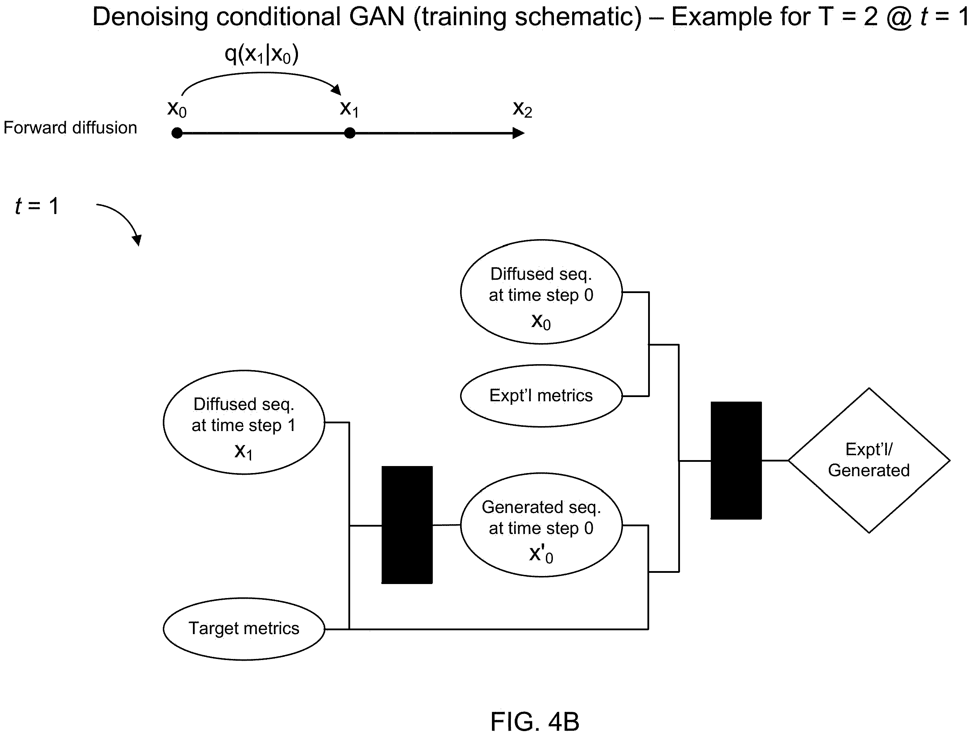

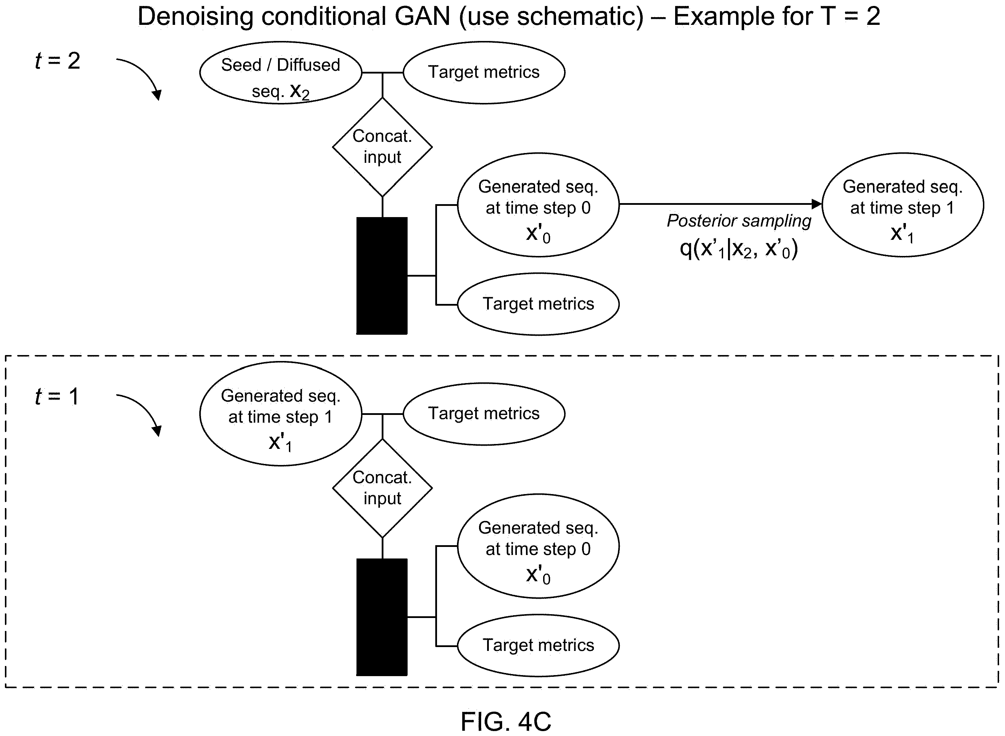

FIGS 4A, 4B, and 4C collectively provide an example illustrative implementation of a conditional ddGAN using a total of T = 2 consecutive states, or time steps, for diffusion and denoising, in accordance with some embodiments of the present disclosure. FIG. 4A illustrates a training process for the example implementation at an initial state indicated as t = 2. FIG. 4B illustrates the training process for the example implementation at a consecutive state indicated as t = 1. FIG. 4C illustrates a use schematic for the example implementation, over consecutive states t = 2 and t = 1, where the conditional ddGAN outputs a generated polymer sequence.

FIGS. 5A, 5B, and 5C collectively illustrate an example computer system for generating a polymer sequence for a biological molecule having one or more target biological properties, in which dashed boxes indicate optional features, in accordance with various embodiments of the present disclosure.

FIG. 6 provides a flowchart illustrating example methods for generating a polymer sequence (*e.g.*, a nucleic acid sequence or amino acid sequence) for a biological molecule having one or more target biological properties, in accordance with some embodiments of the present disclosure.

FIGS. 7A, 7B, 7C, 7D, 7E, 7F, 7G, and 7H collectively provide a flowchart illustrating example methods for generating a polymer sequence (*e.g.*, a nucleic acid sequence or amino acid sequence) for a biological molecule having one or more target biological properties, in which dashed boxes indicate optional features, in accordance with some embodiments of the present disclosure.

FIGS. 8A and 8B collectively illustrate an example generation of a polymer sequence for a biological molecule having one or more target biological properties, in accordance with an embodiment of the present disclosure. FIG. 8A illustrates a seed for a nucleic acid sequence. FIG. 8B illustrates a denoised guide RNA obtained from the seed shown in FIG. 8A.

FIGS. 9A, 9B, and 9C collectively illustrate a visual representation of denoising a nucleotide sequence from a seed using a generative bit diffusion model where nucleotide identity is one-hot encoded in a 4-bit schema, in accordance with an embodiment of the present disclosure.

FIG. 10 illustrates example nucleic acid sequences for a gRNA-target sequence scaffold generated by a bit diffusion model, in accordance with an embodiment of the present disclosure.

FIG. 11 illustrates example predicted performance metrics for nucleic acid sequences generated by a bit diffusion model, plotted against ADAR1-specific conditioning metrics, in accordance with an embodiment of the present disclosure.

FIG. 12 illustrates example performance of generative designs obtained from a bit diffusion model for previously unseen targets, in accordance with an embodiment of the present disclosure.

FIG. 13 illustrates example predicted performance metrics for nucleic acid sequences generated by a bit diffusion model, plotted against ADAR2-specific conditioning metrics, in accordance with an embodiment of the present disclosure.

FIG. 14A illustrates example predicted performance metrics for nucleic acid sequences generated by a bit diffusion model conditioned on ADAR1/2-specific metrics, in accordance with an embodiment of the present disclosure. FIG. 14B illustrates the distributional similarity between the generated and actual samples.

FIGS. 15A, 15B, 15C, 15D, 15E, 15F, 15G, 15H, and 15I collectively illustrate the predicted performance of sequences generated by bit diffusion models trained on a target-specific library (MAPT), in accordance with an embodiment of the present disclosure.

FIGS. 16A, 16B, 16C, 16D, 16E, 16F, 16G, 16H, and 16I collectively illustrate the predicted performance of sequences generated by bit diffusion models trained on a target-agnostic library (ML), in accordance with an embodiment of the present disclosure.

FIGS. 17A, 17B, 17C, 17D, 17E, 17F, 17G, 17H, and 17I collectively illustrate the predicted performance of sequences generated by input optimization models trained on a target-specific library, in accordance with an embodiment of the present disclosure.

FIGS. 18A, 18B, 18C, 18D, 18E, 18F, 18G, 18H, and 18I collectively illustrate the predicted performance of sequences generated by input optimization models trained on a target-agnostic library, in accordance with an embodiment of the present disclosure.

FIGS. 19A, 19B, 19C, 19D, 19E, 19F, 19G, 19H, and 19I collectively illustrate the predicted performance of sequences identified by structural sampling guide sequences having common secondary structure features by scoring each sequence using a target-agnostic predictive model of ADAR editing efficacy and specificity, in accordance with an embodiment of the present disclosure.

FIGS. 20A and 20B collectively illustrate example performance of a bit diffusion model for generating sequences for AAV5 variants having mutations in the 581-589 region, where performance is measured *in silico* using a correlation between the conditioning used for bit diffusion and a prediction using a separate model trained on the same dataset, in accordance with an embodiment of the present disclosure.

FIGS. 21A, 21B, and 21C collectively illustrate an example generation of a 5-bit encoded amino acid sequence from a seed sequence, in accordance with an embodiment of the present disclosure.

FIG. 22 illustrates example performance of a retrained bit diffusion model for generating enhancer sequences from seeds, where performance is measured *in silico* using a correlation between the conditioning used for bit diffusion and a prediction using a separate model trained on the same dataset, in accordance with an embodiment of the present disclosure.

FIGS. 23A and 23B collectively illustrate an example schematic of a conditioning denoising U-Net model, and methods of use thereof, in accordance with some embodiments of the present disclosure.

FIGS. 24A, 24B, and 24C collectively illustrate the results of ADAR gRNA identification by generative design and/or structural sampling for 42 novel targets using various generalized (target-independent) machine learning models as described in Example 5, and in accordance with some embodiments of the present disclosure.

FIGS. 25A, 25B, and 25C collectively illustrate performance of gradient boosted ensemble models trained using XGBoost, as described in Example 6.

FIG. 26 illustrates position-wise ADAR1 and ADAR2 editing of a target mRNA sequence mediated by a guide RNA containing a core footprint sequence, as described in Example 6.

FIGS. 27A, 27B, and 27C collectively illustrate predicted ADAR editing metrics for guide RNA incorporating a core footprint sequence and generated by various denoising diffusion techniques, as described in Example 6.

FIGS. 28A, 28B, and 28C collectively illustrate predicted ADAR editing metrics for guide RNA incorporating different spans of a core footprint sequence and generated by various denoising diffusion techniques, as described in Example 6.

It should be understood that the appended drawings are not necessarily to scale, presenting a somewhat simplified representation of various features illustrative of the basic principles of the invention.

## 7. DETAILED DESCRIPTION

### 7.1. Introduction

*Guide RNAs.* Personalized medicine for the treatment of monogenic diseases requires a rapid, cost-effective drug discovery process that is safe, programmable, and precise. The recruitment of endogenous adenosine deaminase acting on RNA (ADAR) enzymes by guide RNAs (gRNAs) antisense to a target transcript can allow for precise adenosine-to-inosine (A-to-I) editing at the RNA level, which is interpreted by the cellular machineries as an adenosine-to-guanosine substitution. This process, known as ADAR editing, plays a role in regulating the innate immune system by marking endogenous dsRNA structures as "self." However, its therapeutic potential has been limited due to two factors: ADAR's natural preference for certain primary and secondary structural dsRNA substrates; and its proclivity to edit multiple adenosines within a given dsRNA substrate. Here, we demonstrate the power of machine learning (ML) to engineer novel gRNAs for challenging targets and rapidly identify gRNAs de novo to any target of interest.

Natural RNA substrates of ADAR and apolipoprotein B editing complex (APOBEC) are edited with high selectivity and efficiency due to precise higher order structures, *e.g.*, secondary, tertiary, and quaternary structures formed between the RNA substrates, the gRNA, and the enzyme. In certain instances, guide RNA (gRNA) sequences can be designed such that they form gRNA-target scaffolds with the target mRNAs to be edited, which are double-stranded RNA (dsRNA) substrates that bear unique structural features that help guide ADAR or APOBEC-mediated editing of the target sequence. Such an intracellular RNA-editing mechanism can be exploited, *e.g.*, to edit mutations found in various genetic diseases at the mRNA level, and without modifying the genome of a patient. However, conventional systems used to edit RNA have limitations that can lead to aberrant effector activity, present delivery barriers, unintended transcriptomic modifications, and/or immunogenicity. In addition, the space from which such gRNA sequences can be selected is prohibitively large for conventional design and screening methodologies.

Therapeutic RNA editing using ADAR or APOBEC enzymes, *e.g.*, by redirecting natural ADAR or APOBEC enzymes or by delivering exogenous ADAR or APOBEC enzymes, offers promise as a safe alternative to gene therapies that operate by altering the subject's genome. For example, some gene therapies introduce DNA breaks in the host's genome, which are repaired to introduce a permanent change in the host's genome. Imprecise editing by these gene therapies, for example by introducing an unintended mutation at a target site or any alteration at an off-target site, can thereby permanently harm the host's genome. RNA editing, by contrast, transiently alters the flow of genetic information in the host by editing RNA, *e.g.*, messenger RNA (mRNA), without permanently altering the host's genome. Further, RNA editing strategies that redirect endogenous ADAR or APOBEC enzymes do not require introduction of exogenous proteins, which further complicates therapeutic delivery and risks further immunogenetic responses in the host.

However, ADAR and APOBEC enzymes possess inherent editing promiscuity. To date, sequence preferences and deterministic rules for how gRNA mediate result in various editing performances remain poorly understood. This is complicated by the fact the ADAR and APOBEC interactions with nucleic acids are influenced by tertiary nucleic acid structure and quaternary protein-nucleic acid structures, rather than just primary nucleic acid sequence.

For example, efforts to predict the editing preference of ADAR proteins for different dsRNA substrates have shown that ADAR editing activity, in some instances, not only tolerates various mismatches, bulges, loops, and other secondary and tertiary structural features, but also exhibits improved performance as a result of such deviations from perfect base-pairing. *See*, for instance, Liu et al., "Learning cis-regulatory principles of ADAR-based RNA editing from CRISPR-mediated mutagenesis." Nat Commun. 2021;12(1):2165, which is hereby incorporated herein by reference in its entirety. Moreover, gRNAs for ADAR editing can range from as small as about 20 nucleotides to about 151 nucleotides or more, and have further been shown, in certain instances, to tolerate mismatches at up to 50-60% of possible editing sites while still allowing recognition by the ADAR protein. *See*, for instance, Aquino-Jarquin, "Novel engineered programmable systems for ADAR-mediated RNA editing," Mol. Ther. Nucleic Acids, 19:1065-72 (2020); Eggington et al., "Predicting sites of ADAR editing in double-stranded RNA," Nat. Commun., 2(1):319 (2011), each of which is hereby incorporated herein by reference in its entirety.

Thus, for an example target mRNA having 150 nucleotides, a conservative estimate of the space from which a corresponding gRNA sequence can be selected would be on the order of $10^{27}$, where any 10% of the positions in the gRNA sequence of 150 nucleotides are substituted, and assuming only single-base mismatches (*e.g.*, A, C, G, or T) at each mutated position in the gRNA sequence. As another example, assuming only single-base mismatches over 10% of the gRNA sequence, the corresponding space for a target mRNA having only 50 nucleotides still includes more than half of a billion potential gRNAs. However, in practice, the space from which the corresponding gRNA sequence for a given target mRNA is selected is much larger than these estimates, given that the structural features that regulate ADAR editing specificity and efficiency are far more complex than simple base substitutions, including insertions and/or deletions, and considering that potential gRNA candidates include varying lengths that can be shorter or longer than the target mRNA or target mRNA region of interest. In some such cases, the space to be interrogated for a single gRNA corresponding to a single target mRNA is at least $10^{30}$, $10^{40}$, $10^{50}$, or greater. Conventional methods for *in vitro*, *in vivo*, and *in silico* gRNA screening cannot properly evaluate such large space to identify optimal gRNA sequences. As such, improved methods and systems for identifying and/or designing gRNA sequences are needed.

These problems are attractive computational challenges for machine learning (ML). The problem compounds when considering the similarly enormous number of possible RNA editing sites in animals, such as mammals. In particular, more than 100 million adenosine to inosine (A-to-I) editing sites are estimated to occur in humans, and a further 50,000 sites are estimated to occur in mice. *See*, for instance, Kim et al., "RNA editing at a limited number of sites is sufficient to prevent MDA5 activation in the mouse brain." PLOS Genetics. 2021;17(5):e1009516, which is hereby incorporated herein by reference in its entirety. Given the sheer number of potential candidate gRNAs for any given mRNA target, and the sheer number of potential mRNA targets that contain A-to-I editing sites, a large-scale design or optimization of potential gRNAs for ADAR-mediated editing would be impossible to perform with any breadth. Moreover, with such a large candidate space, it would be impossible to perform a sufficient number of *in vitro* screening assays to sample the space to even identify an optimal starting point for tuning gRNA performance. While machine learning models provide the ability to screen many more guides *in silico*, compared to *in vitro* approaches, even brute force *in silico* screening remains sub-optimal in such a large space. Thus, there is a need in the art for *a priori* design of gRNA sequences that enable specific and efficient editing of novel RNA targets. In particular, there is a need in the art for machine learning methods and systems that use generative processes for guide design and selection based on target properties, such as the models (*e.g.*, generative adversarial networks (GANs), diffusion models, and/or denoising diffusion conditional GANs (ddGANs)) described in this application.

Initial attempts to utilize antisense RNAs for ADAR mediated editing relied on exogenous or hyperactive ADAR enzymes. Subsequently, it was shown that for gene-encoded gRNAs, the antisense length needed to be greater than 60 nucleotides to recruit the endogenous enzyme. This long stretch of dsRNA led to bystander editing of the adjacent adenosines. To reduce the by bystander editing several approaches have been reported, including the use of A-G mismatches, U deletions, discontinuous hybridization arms, and repetitive bulges. However, the solutions may often result in reduced on target editing. The ability to explore a wider secondary structure landscape during the gRNA discovery process may yield novel solutions to provide both high efficiency and selectivity.

Previous approaches attempting to identify RNA primary and secondary features predictive of ADAR editing have focused predominantly on supervised learning of the proximal nucleotide context for specific targets. Early in vitro studies coupled with more recent co-crystal structures have identified ADAR's natural editing preference as 5'-UAG-3' but lack information on how to design gRNAs to circumvent this preference. High-throughput saturation mutagenesis screening of three natural RNA editing substrates for ADAR1 (NEIL1, TTYH2, and AJUBA1) identified several characteristics predictive of gRNA mediated editing outcomes within each target, but those models failed to accurately predict editing across targets. Furthermore, generative machine learning was not employed to engineer novel gRNAs with optimized on-target adenosine editing and specificity beyond the sequence space explored of singletons and doubletons. By broadening the primary sequence and secondary structure search space, more globally top solutions can be identified.

Described herein, among other aspects, are applications of machine learning, that may optionally be coupled with high throughput screening (HTS), for the identification and/or de novo generation of gRNA capable of facilitating ADAR or APOBEC-mediated editing of multiple target nucleotide sites in an RNA molecule. These approaches allow for the exploration of the enormous gRNA design space to propose highly efficient and specific novel gRNA designs that validate experimentally.

In some embodiments, the machine learning methods, systems, and platforms described herein generate gRNA sequences that facilitate RNA editing *in vivo.* For example, in some embodiments, gRNAs sequences are generated that direct ADAR-mediated deamination of adenosine to inosine in target mRNA. Inosine is then recognized by the translational machinery most frequently as guanine. In some embodiments, such targeted deamination is useful to correct G→A transitions found in genes linked to disorders, *e.g.*, where the G→A transition results in expression of a protein with a point mutation or truncation contributing to the etiology of a disorder. In some embodiments, such targeted deamination is useful to introduce A→G transitions, *e.g.*, to introduce a mutation in the amino acid sequence encoded by a target mRNA or to introduce a stop codon causing a truncation of a protein. In some embodiments, such targeted deamination is useful to modify a splicing pattern of a gene transcript, *e.g.*, where the A→G transition results in generation of a splice site (*e.g.*, restoration of a wild type splice site or generation of a novel splice site), abrogation of an existing splice site (*e.g.*, destruction of a mutant splice site or destruction of a wild type splice site), weakening of an existing splice site, or strengthening of an existing splice site. In some embodiments, such targeted deamination is useful to modify protein translation efficiency, *e.g.*, by strengthening or weakening a translational initiation signal or by strengthening or weakening translational elongation. In some embodiments, such generative guide design is performed by models (*e.g.*, generative adversarial networks (GANs), diffusion models, and/or denoising diffusion conditional GANs (ddGANs)) trained against one or more conditions (*e.g.*, ADAR performance metrics).

Similarly, in some embodiments, the machine learning methods, systems, and platforms described herein generate gRNA sequences that facilitate RNA editing by directing APOBEC-mediated deamination of cytosine to uracil in target mRNA. In some embodiments, such targeted deamination is useful to correct T→C transitions found in genes linked to disorders, *e.g.*, where the T→C transition results in expression of a protein with a point mutation or truncation contributing to the etiology of a disorder. In some embodiments, such targeted deamination is useful to introduce C→U transitions, *e.g.*, to introduce a mutation in the amino acid sequence encoded by a target mRNA or to introduce a stop codon causing a truncation of a protein. In some embodiments, such targeted deamination is useful to modify a splicing pattern of a gene transcript, *e.g.*, where the C→U transition results in generation of a splice site (*e.g.*, restoration of a wild type splice site or generation of a novel splice site), abrogation of an existing splice site (*e.g.*, destruction of a mutant splice site or destruction of a wild type splice site), weakening of an existing splice site, or strengthening of an existing splice site. In some embodiments, such targeted deamination is useful to modify protein translation efficiency, *e.g.*, by strengthening or weakening a translational initiation signal or by strengthening or weakening translational elongation. In some embodiments, such generative guide design is performed by models (*e.g.*, generative adversarial networks (GANs), diffusion models, and/or denoising diffusion conditional GANs (ddGANs)) trained against one or more conditions (*e.g.*, ADAR performance metrics).

In some embodiments, the generated gRNA designs facilitate ADAR or APOBEC editing with high selectivity and specificity for any custom target. In some implementations, the gRNA designs obtained using the systems and methods disclosed herein outperform the gRNA from HTS used, in part, to train the models. Advantageously, in some embodiments, the novel gRNA designs exhibit primary, secondary, and/or tertiary sequence diversity beyond that of the original HTS screen. Moreover, in some implementations, these models are leveraged to improve and accelerate the gRNA discovery process by reducing the amount of running time and computational resources needed to interrogate the potential candidate gRNA space, and to expand the state of knowledge of the relationship between RNA primary sequence, secondary structure, tertiary structure, and ADAR or APOBEC activity.

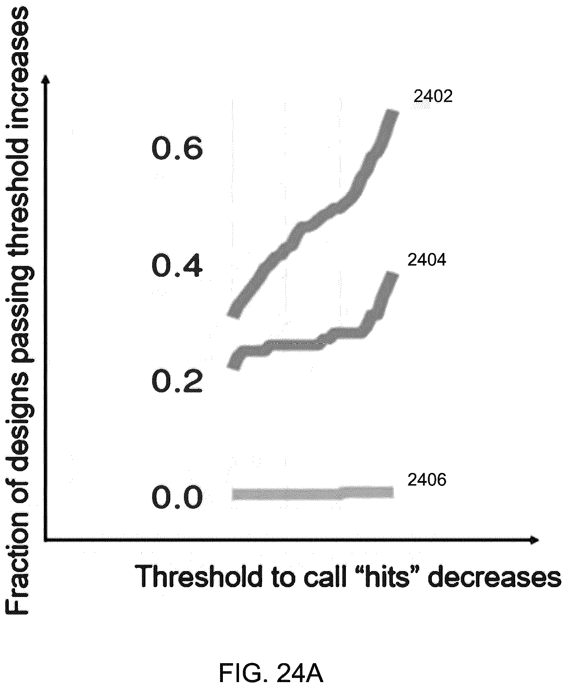

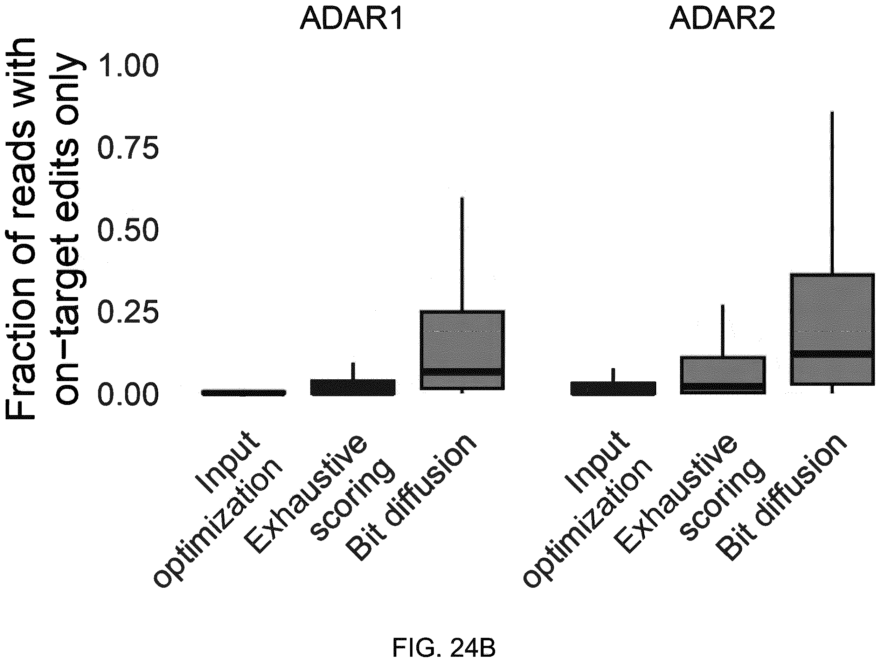

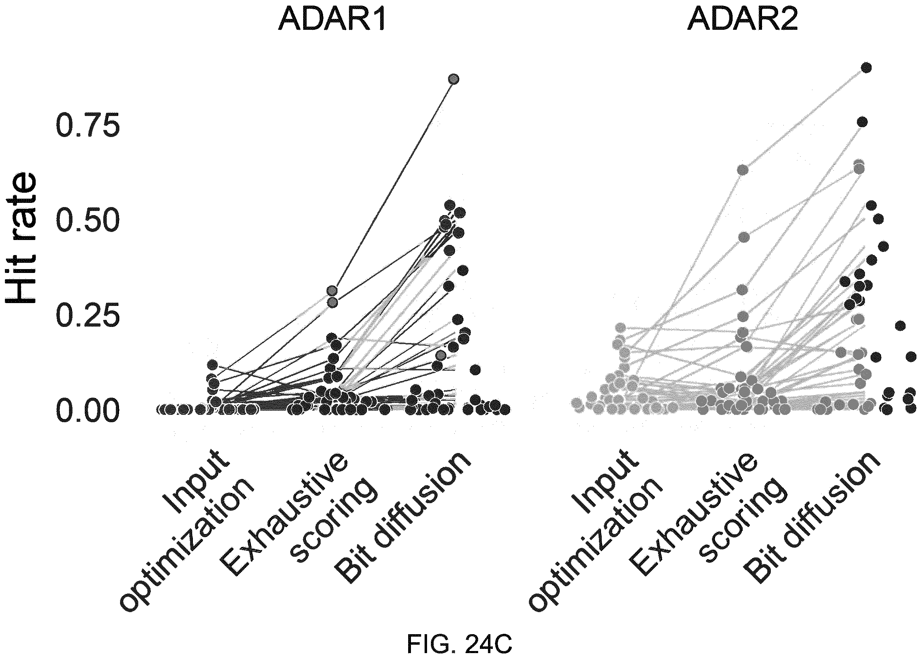

In some embodiments, the machine learning models, methods, and/or systems described herein improve ADAR or APOBEC gRNA identification. For instance, Example 5 compares generative design and structural sampling of gRNA against 42 novel mRNA target sequences not previously seen by the models by bit diffusion using a performance-conditioned U-Net transitional model, input optimization using a CNN ensemble model, and structural sampling of gRNA sequences by scoring ADAR editing metrics with a predictive target-agnostic XGBoost model. As described in Example 5, and illustrated in **FIG. 24,** generative design by bit diffusion outperformed generative design by input optimization and structural sampling of gRNA sequences using a predictive model. In **FIG. 24A,** 2402 represents the percentage of hits obtained by generative bit diffusion as the threshold is relaxed, 2404 represents the percentage of hits obtained by screening with the XGBoost model, and 2406 represents the percentage of hits obtained from input optimization. As such, the use of diffusion models, as described herein, improves machine-learning identification of ADAR or APOBEC gRNA by more efficiently providing gRNA sequences that satisfy target metrics. That is, because a higher percentage of the gRNA sequences generated using the denoising diffusion models described herein meet target editing efficacy and specificity, fewer guide sequences need to be generated using these methods as compared to methods using input optimization or structural sampling sequences using a predictive model. In fact, it is extremely difficult to effectively screen gRNA sequences in silico as the space is extremely large and choosing effective heuristics can be time consuming and ineffective.

Moreover, the diffusion processes used in the examples herein were approximately 10,000 times faster than sequence generation by input optimization. Thus, the methods, systems, and models described herein also improve the function of a computer system by improving the computational efficiency when generatively designing gRNA sequences for editing of a target sequence. Inference using reverse diffusion is a technique in which a model samples noise and is then iteratively applied a predetermined number of times. The solutions found through this process have been validated through experimental and computational methods, as exemplified in the Examples. When performing input optimization, a random seed of the guide sequence is initialized and then a nonlinear optimization is solved, e.g., for a predetermined amount of time. To reach solutions, the nonlinear optimization procedure requires substantially more forward passes through the network (e.g., 40 times more passes) in addition to backward passes, which the diffusion process doesn't have to do at all. Accordingly, relative to input optimization procedures for generative design of gRNA sequences, the denoising diffusion procedures herein improve computational efficiency. Accordingly, in some embodiments, the methods, systems, and machine learning models described herein reduce the computational resources required to generate novel ADAR and/or APOBEC gRNA sequences, e.g., by up to at least 4-orders of magnitude relative to conventional computational methods for generating novel ADAR and/or APOBEC gRNA sequences.

*Variant capsid proteins.* In some implementations, engineered capsids, engineered capsid polypeptides, and 581-589 regions of capsid polypeptides confer tissue tropism for specific tissues or a combination thereof (*e.g.,* liver, CNS (cortex forebrain, cortex occipital, cortex temporal, thalamus, hypothalamus, substantia nigra, hippocampus DG, hippocampus CA1, hippocampus CA3, cerebellum), skeletal muscle, heart, lung, spleen, lymph node, bone marrow, mammary gland, skin, adrenal gland, thyroid, colon, sciatic nerve, and/or and spinal cord tissues) to a viral capsid. Current gene therapies utilize AAV viruses with wild type AAV capsid polypeptides. These therapies suffer from a lack of tissue specific tropism and, as such, can exhibit poor biodistribution, non-specific tissue tropism, or both. Even upon accumulation in target tissues, wild type AAV, such as wild type AAV9, can exhibit poor tissue-specific transduction. The rAAVs disclosed herein, and the systems and methods for generating the same, having variant AAV5 viral protein capsid polypeptide sequences, can display tissue and cell-type specific tropism (*e.g.,* high transduction of specific tissue cells), decreased off-target tissue accumulation and infection (*e.g.,* de-targeting), reduced capacity to pre-existing immunity, or any combination thereof. These attributes allow for reduction in clinical dose and a concomitant decrease in dose-dependent toxic side effects as well as increased manufacturability.

For example, engineered capsids comprising engineered capsid polypeptides with 581-589 regions for tissue-specific delivery of a payload (*e.g.,* a polynucleotide, such as a transgene) encapsidated by the engineered capsid. Recombinant AAVs comprising VP capsid polypeptides with 581-589 regions engineered for tissue specificity can be used to specifically infect a target tissue. Using tissue-tropic rAAV viral capsids for payload delivery provides numerous advantages over using adeno-associated virus (AAV) viral capsids that lack tissue tropism including reduced toxicity, lower dose needed to produce a therapeutic effect, wider therapeutic window, and reduced immune response. Furthermore, tissue-specific payload delivery can enable targeted therapies even when administering systemically. For example, a target tissue-tropic AAV capsid can be systemically administered to specifically deliver a payload to the target tissue for treatment of a disease specific to the target tissue. In another example, a target tissue-tropic AAV capsid can be systemically administered to specifically deliver a payload to a specific organ for treatment of a target tissue disease. In some embodiments, a target tissue-tropic AAV capsid of the present disclosure can be systemically administered to specifically deliver a payload to target cell subtypes for treatment of a target tissue disease.

In some embodiments, a tissue-tropic capsid of the present disclosure is tissue-tropic for one or more tissues in a plurality of tissues including, but not limited to, liver, CNS (cortex forebrain, cortex occipital, cortex temporal, thalamus, hypothalamus, substantia nigra, hippocampus DG, hippocampus CA1, hippocampus CA3, cerebellum), skeletal muscle, heart, lung, spleen, lymph node, bone marrow, mammary gland, skin, adrenal gland, thyroid, colon, sciatic nerve, and/or and spinal cord tissues. Additionally or optionally, a tissue-tropic capsid further displays enhanced transduction of one or more cell subtypes for any one or more tissues in the plurality of tissues.

In an illustrative embodiment, variation is introduced into each of residues 581 to 589 of a variant capsid protein. Each of the 20 natural amino acids is introduced at each of the 9 positions of the 581-589 region, providing a theoretical library diversity of $20^9$ ($20\text{^}9$; approximately $5 \times 10^{11}$) unique sequence variants.

In some implementations, the 581-589 region targeted for engineering is the most likely to interact with target cell receptors, and relatively tolerant to changes without disrupting capsid assembly. Unlike earlier approaches that add unstructured peptides that protrude above the capsid 3-fold axis of symmetry, the approach introduces sequence diversity that alters the characteristics of the binding pocket. In addition, this approach may change the overall structure of the receptor-binding trimer, allowing for altered allosteric interactions outside the binding pocket (*e.g.*, AAVR PKD1). Introduced diversity is non-random, thereby reducing missense and frameshifts of randomized libraries.

*Regulatory elements.* In some embodiments, regulatory elements regulate (*e.g.*, modulate, coordinate, or otherwise impact) the expression of one or more sequences in a cell. In some embodiments, regulatory elements include nucleotide sequences, such as promoters, enhancers, terminators, polyadenylation sequences, and/or introns. In some embodiments, regulatory elements affect coding sequences in the cell. In some implementations, engineered regulatory elements are used to produce a therapeutic effect, such as to inhibit overexpression or enhance under-expression and/or to activate or silence gene expression for gene therapy applications.

## 7.2. Definitions

Unless defined otherwise, all technical and scientific terms used herein have the meaning commonly understood by one of ordinary skill in the art to which the invention pertains.

As used herein, the term "engineered guide RNA" can be used interchangeably with "guide RNA" and refers to a designed polynucleotide that is at least partially complementary to a target RNA. An engineered guide RNA of the present disclosure can be used to facilitate modification of the target RNA. Modification of the target RNA includes alteration of RNA splicing, reduction or enhancement of protein translation, target RNA knockdown, target RNA degradation, and/or ADAR mediated RNA editing of the target RNA. In some cases, guide RNAs facilitate ADAR mediated RNA editing for the purpose of target mRNA knockdown, downstream protein translation reduction or inhibition, downstream protein translation enhancement, correction of mutations (including correction of any G to A mutation, such as missense or nonsense mutations), introduction of mutations (*e.g.*, introduction of an A to I (read as a G by cellular machinery) substitution), or alter the function of any adenosine containing a regulatory motif (*e.g.*, polyadenylation signal, miRNA binding site, *etc.*). In some cases, a guide RNA can effect a functional outcome (*e.g.*, target RNA modulation, downstream protein translation) via a combination of mechanisms, for example, ADAR-mediated RNA editing and binding and/or degrading target RNA. In some cases, a guide RNA can facilitate introduction of mutations at sites targeted by enzymes in order to modify the affinity of such enzymes for targeting and cleaving such sites. The guide RNAs of this disclosure can contain one or more structural features. A structural feature can be formed from latent structure in latent (unbound) guide RNA upon hybridization of the engineered latent guide RNA to a target RNA. Latent structure refers to a structural feature that forms or substantially forms only upon hybridization of a guide RNA to a target RNA. For example, upon hybridization of the guide RNA to the target RNA, the latent structural feature is formed in the resulting double stranded RNA (also referred herein as guide-target RNA scaffold). In such cases, a structural feature can include, but is not limited to, a mismatch, a wobble base pair, a symmetric internal loop, an asymmetric internal loop, a symmetric bulge, or an asymmetric bulge. In other instances, a structural feature can be a pre-formed structure (*e.g.*, a GluR2 recruitment hairpin, or a hairpin from U7 snRNA).

As used herein, the term "double-stranded RNA substrate" or "dsRNA substrate" refers to a guide-target RNA scaffold formed upon hybridization of an engineered guide RNA to a target RNA. The resulting double stranded substrate is referred as a "guide target RNA scaffold." Such guide-target RNA scaffolds can form various secondary, tertiary, and quaternary structures, which may or may not be present in in the gRNA or target RNA prior to hybridization. Accordingly, in some instances, such secondary structures of a guide-target RNA scaffold that are not present in the gRNA prior to hybridization to the target RNA molecule are said to arise from "latent features" of the gRNA molecule. Non-limiting examples of such structural features include mismatches, bulges (*e.g.*, symmetrical bulges or asymmetrical bulges), internal loops (*e.g.*, symmetrical internal loops or asymmetrical internal loops), and hairpins (*e.g.*, recruiting hairpins or a non-recruiting hairpins). Other such structures are further described herein.

In some embodiments, a gRNA described herein has a plurality of structural features, *e.g.*, a combination of latent and actual features. For example, in some embodiments, the gRNA has from 1 to 50 structural features. In some embodiments, the gRNA has from 1 to 5, from 5 to 10, from 10 to 15, from 15 to 20, from 20 to 25, from 25 to 30, from 30 to 35, from 35 to 40, from 40 to 45, from 45 to 50, from 5 to 20, from 1 to 3, from 4 to 5, from 2 to 10, from 20 to 40, from 10 to 40, from 20 to 50, from 30 to 50, from 4 to 7, or from 8 to 10 features. In some embodiments, the plurality of structural features includes one or more latent structures capable of forming a different structural feature of a guide-target RNA scaffold upon hybridization of the gRNA to a target RNA. In some embodiments, the plurality of structural features includes a structural feature formed prior to hybridization of the gRNA to the target RNA, *e.g.*, a GluR2 recruitment hairpin or a hairpin from U7 snRNA.

Similarly, in some embodiments, a guide-target RNA scaffold described herein has a plurality of structural features. For example, in some embodiments, the guide-target RNA scaffold has from 1 to 50 structural features. In some embodiments, the guide-target RNA scaffold has from 1 to 5, from 5 to 10, from 10 to 15, from 15 to 20, from 20 to 25, from 25 to 30, from 30 to 35, from 35 to 40, from 40 to 45, from 45 to 50, from 5 to 20, from 1 to 3, from 4 to 5, from 2 to 10, from 20 to 40, from 10 to 40, from 20 to 50, from 30 to 50, from 4 to 7, or from 8 to 10 features. In some embodiments, the plurality of structural features includes one or more structural features formed, at least in part from a latent structure of the gRNA. In some embodiments, the plurality of structural features includes one or more structural feature formed in the gRNA prior to hybridization to the target RNA, *e.g.*, a GluR2 recruitment hairpin or a hairpin from U7 snRNA. In some embodiments, the plurality of structural features includes one or more structural feature formed in the target RNA prior to hybridization of the gRNA to the target RNA.

As used herein, the term "targeting sequence" can be used interchangeably with "targeting domain" or "targeting region" and refers to a polynucleotide sequence within an engineered guide RNA sequence that is at least partially complementary to a target polynucleotide. The target polynucleotide (*e.g.*, a target RNA or a target DNA) may be a region of a polynucleotide of interest, such as a gene or a messenger RNA. As used herein, a "complementary" sequence refers to a sequence that is a reverse complement relative to a second sequence. A targeting sequence of an engineered guide RNA allows the engineered guide RNA to hybridize to a target polynucleotide (*e.g.*, a target RNA) through base pairing, such as Watson Crick base pairing. A targeting sequence can be located at either the N-terminus or C-terminus of the engineered guide RNA, or both, or the targeting sequence can be within the engineered guide RNA. The targeting sequence can be of any length sufficient to hybridize with the target polynucleotide.

As used herein, "messenger RNA" or "mRNA" are RNA molecules comprising a sequence that encodes a polypeptide or protein. In general, RNA can be transcribed from DNA. In some cases, precursor mRNA containing non-protein coding regions in the sequence can be transcribed from DNA and then processed to remove all or a portion of the non-coding regions (introns) to produce mature mRNA. As used herein, the term "pre-mRNA" can refer to the RNA molecule transcribed from DNA before undergoing processing to remove the non-protein coding regions.

As used herein, unless otherwise dictated by context "nucleotide" or "nt" refers to ribonucleotide.

As used herein, the terms "patient" and "subject" are used interchangeably, and may be taken to mean any living organism which may be treated with compounds found using the present disclosure. As such, the terms "patient" and "subject" include, but are not limited to, any non-human mammal, primate and human.

The term "stop codon" can refer to a three-nucleotide contiguous sequence within messenger RNA that signals a termination of translation. Non-limiting examples include in RNA, UAG (amber), UAA (ochre), UGA (umber, also known as opal) and in DNA TAG, TAA or TGA. Unless otherwise noted, the term can also include nonsense mutations within DNA or RNA that introduce a premature stop codon, causing any resulting protein to be abnormally shortened.

A "therapeutically effective amount" of a composition is an amount sufficient to achieve a desired therapeutic effect, and does not require cure or complete remission.

The terms "treat," "treated," "treating", or "treatment" as used herein have the meanings commonly understood in the medical arts, and therefore does not require cure or complete remission, and therefore includes any beneficial or desired clinical results. Treatment includes eliciting a clinically significant response without excessive levels of side effects. Treatment also includes prolonging survival as compared to expected survival if not receiving treatment.

As used herein, "preventing" a disease refers to inhibiting the full development of a disease.

As used herein, the term "latent structure" refers to a structural feature that substantially forms only upon hybridization of a guide RNA to a target RNA. For example, the sequence of a guide RNA provides one or more structural features, but these structural features substantially form only upon hybridization to the target RNA, and thus the one or more latent structural features manifest as structural features upon hybridization to the target RNA. Upon hybridization of the guide RNA to the target RNA, the structural feature is formed, and the latent structure provided in the guide RNA is, thus, unmasked. The formation and structure of a latent structural feature upon binding to the target RNA depends on the guide RNA sequence. For example, formation and structure of the latent structural feature may depend on a pattern of complementary and mismatched residues in the guide RNA sequence relative to the target RNA. The guide RNA sequence may be engineered to have a latent structural feature that forms upon binding to the target RNA.

As used herein, the term "engineered latent guide RNA" refers to an engineered guide RNA that comprises a portion of sequence that, upon hybridization or only upon hybridization to a target RNA, substantially forms at least a portion of a structural feature, other than a single A/C mismatch feature at the target adenosine to be edited.

As used herein, the term "guide-target RNA scaffold" refers to the resulting double-stranded RNA formed upon hybridization of a guide RNA, with latent structure, to a target RNA. A guide-target RNA scaffold has one or more structural features formed within the double-stranded RNA duplex upon hybridization. For example, the guide-target RNA scaffold can have one or more structural features selected from a bulge, mismatch, internal loop, hairpin, or wobble base pair.

As used herein, the term "structured motif" refers to two or more structural features in a guide-target RNA scaffold.

As used herein, the term "mismatch" refers to a single nucleotide in a guide RNA that is unpaired to an opposing single nucleotide in a target RNA within the guide-target RNA scaffold. A mismatch can comprise any two single nucleotides that do not base pair. Where the number of participating nucleotides on the guide RNA side and the target RNA side exceeds 1, the resulting structure is no longer considered a mismatch, but rather, is considered a bulge or an internal loop, depending on the size of the structural feature. In some embodiments, a mismatch is an A/C mismatch. An A/C mismatch can comprise a C in an engineered guide RNA of the present disclosure opposite an A in a target RNA. An A/C mismatch can comprise an A in an engineered guide RNA of the present disclosure opposite a C in a target RNA. A G/G mismatch can comprise a G in an engineered guide RNA of the present disclosure opposite a G in a target RNA. In some embodiments, a mismatch positioned 5' of the edit site can facilitate base-flipping of the target A to be edited. A mismatch can also help confer sequence specificity. Thus, a mismatch can be a structural feature formed from latent structure provided by an engineered latent guide RNA.

As used herein, the term "bulge" refers to a structure, substantially formed only upon formation of the guide-target RNA scaffold, where contiguous nucleotides in either the engineered guide RNA or the target RNA are not complementary to their positional counterparts on the opposite strand. A bulge can change the secondary or tertiary structure of the guide-target RNA scaffold. A bulge can have from 0 to 4 contiguous nucleotides on the guide RNA side of the guide-target RNA scaffold and 1 to 4 contiguous nucleotides on the target RNA side of the guide-target RNA scaffold or a bulge can have from 0 to 4 nucleotides on the target RNA side of the guide-target RNA scaffold and 1 to 4 contiguous nucleotides on the guide RNA side of the guide-target RNA scaffold. However, a bulge, as used herein, does not refer to a structure where a single participating nucleotide of the engineered guide RNA and a single participating nucleotide of the target RNA do not base pair - a single participating nucleotide of the engineered guide RNA and a single participating nucleotide of the target RNA that do not base pair is referred to herein as a mismatch. Further, where the number of participating nucleotides on either the guide RNA side or the target RNA side exceeds 4, the resulting structure is no longer considered a bulge, but rather, is considered an internal loop.

As used herein, the term "symmetrical bulge" refers to a structure formed when the same number of nucleotides is present on each side of the bulge.

As used herein, the term "asymmetrical bulge" refers to a structure formed when a different number of nucleotides is present on each side of the bulge.

As used herein, the term "internal loop" refers to the structure, substantially formed only upon formation of the guide-target RNA scaffold, where nucleotides in either the engineered guide RNA or the target RNA are not complementary to their positional counterparts on the opposite strand and where one side of the internal loop, either on the target RNA side or the engineered guide RNA side of the guide-target RNA scaffold, has 5 nucleotides or more. Where the number of participating nucleotides on both the guide RNA side and the target RNA side drops below 5, the resulting structure is no longer considered an internal loop, but rather, is considered a bulge or a mismatch, depending on the size of the structural feature. An internal loop can be a symmetrical internal loop or an asymmetrical internal loop.

As used herein, the term "symmetrical internal loop" refers to a structure formed when the same number of nucleotides is present on each side of the internal loop.

As used herein, the term "asymmetrical internal loop" refers to a structure formed when a different number of nucleotides is present on each side of the internal loop.

As used herein, the term "hairpin" refers to an RNA duplex wherein a portion of a single RNA strand has folded in upon itself to form the RNA duplex. The portion of the single RNA strand folds upon itself due to having nucleotide sequences that base pair to each other, where the nucleotide sequences are separated by an intervening sequence that does not base pair with itself, thus forming a base-paired portion and non-base paired, intervening loop portion.

As used herein, the term "recruitment hairpin" refers to a hairpin structure capable of recruiting, at least in part, an RNA editing entity, such as ADAR. In some cases, a recruitment hairpin can be formed and present in the absence of binding to a target RNA. In some embodiments, a recruitment hairpin is a GluR2 domain or portion thereof. In some embodiments, a recruitment hairpin is an Alu domain or portion thereof. A recruitment hairpin, as defined herein, can include a naturally occurring ADAR substrate or truncations thereof. Thus, a recruitment hairpin such as GluR2 is a pre-formed structural feature that may be present in constructs comprising an engineered guide RNA, not a structural feature formed by latent structure provided in an engineered latent guide RNA.

As used herein, the term "non-recruitment hairpin" refers to a hairpin structure that does not have a primary function of recruiting an RNA editing entity, *e.g.*, that is not capable of recruiting an RNA editing entity. A non-recruitment hairpin, in some instances, does not recruit an RNA editing entity. In some instances, a non-recruitment hairpin has a dissociation constant for binding to an RNA editing entity under physiological conditions that is insufficient for binding. For example, a non-recruitment hairpin has a dissociation constant for binding an RNA editing entity at 25 °C that is greater than about 1 mM, 10 mM, 100 mM, or 1 M, as determined in an *in vitro* assay. A non-recruitment hairpin can exhibit functionality that improves localization of the engineered guide RNA to the target RNA. In some embodiments, the non-recruitment hairpin improves nuclear retention. In some embodiments, the non-recruitment hairpin comprises a hairpin from U7 snRNA. Thus, a non-recruitment hairpin such as a hairpin from U7 snRNA is a pre-formed structural feature that can be present in constructs comprising engineered guide RNA constructs, not a structural feature formed by latent structure provided in an engineered latent guide RNA.

As used herein, the term "wobble base pair" refers to two bases that weakly base pair. For example, a wobble base pair of the present disclosure can refer to a G paired with a U. Thus, a wobble base pair can be a structural feature formed from latent structure provided by an engineered latent guide RNA.

As used herein, the term "macro-footprint" refers to an over-arching structure of a guide RNA. In some embodiments, a macro-footprint flanks a micro-footprint. Further, while a macro-footprint sequence can flank a micro-footprint sequence, additional latent structures can be incorporated that flank either end of the macro-footprint as well. In some embodiments, such additional latent structures are included as part of the macro-footprint. In some embodiments, such additional latent structures are separate, distinct, or both separate and distinct from the macro-footprint.

As used herein, the term "micro-footprint" refers to a guide structure with latent structures that, when manifested, facilitate editing of the adenosine of a target RNA via an adenosine deaminase enzyme. A macro-footprint can serve to guide an RNA editing entity (*e.g.*, ADAR) and direct its activity towards a micro-footprint. In some embodiments, included within the micro-footprint sequence is a nucleotide that is positioned such that, when the guide RNA is hybridized to the target RNA, the nucleotide opposes the adenosine to be edited by the adenosine deaminase and does not base pair with the adenosine to be edited. This nucleotide is referred to herein as the "mismatched position" or "mismatch" and can be a cytosine. Micro-footprint sequences as described herein have upon hybridization of the engineered guide RNA and target RNA, at least one structural feature selected from the group consisting of: a bulge, an internal loop, a mismatch, a hairpin, and any combination thereof. Engineered guide RNAs with superior micro-footprint sequences can be selected based on their ability to facilitate editing of a specific target RNA. Engineered guide RNAs selected for their ability to facilitate editing of a specific target are capable of adopting various micro-footprint latent structures, which can vary on a target-by-target basis.

As used herein, the term "barbell" refers to a guide macro-footprint having a pair of internal loop latent structures that manifest upon hybridization of the guide RNA to the target RNA.

As used herein, the term "dumbbell" refers to a macro-footprint having two symmetrical internal loops, wherein the target A to be edited is positioned between the two symmetrical loops for selective editing of the target A. The two symmetrical internal loops are each formed by 6 nucleotides on the guide RNA side of the guide-target RNA scaffold and 6 nucleotides on the target RNA side of the guide-target RNA scaffold. Thus, a dumbbell can be a structural feature formed from latent structure provided by an engineered latent guide RNA.

As used herein, the term "U-deletion" refers to a type of asymmetrical bulge. In some embodiments, a U-deletion is an asymmetrical bulge formed upon binding of an engineered guide RNA to an mRNA transcribed from a target gene. In some embodiments, a U-deletion is formed by 0 nucleotides on the engineered guide RNA side of the guide-target RNA scaffold and 1 nucleotide on the target RNA side of the guide-target RNA scaffold. For instance, in some implementations, a U-deletion is formed by an "A" on the target RNA side of the guide-target RNA scaffold and a deletion of a "U" on the engineered guide RNA side of the guide-target RNA scaffold. In some embodiments, U-deletions are used opposite of a local off-target nucleotide position (*e.g.*, an off-target adenosine) to reduces off-target editing.

As used herein, the term "base paired region" or "bp region" refers to a region of the guide-target RNA scaffold in which bases in the guide RNA are paired with opposing bases in the target RNA. Base paired regions can extend from one end or proximal to one end of the guide-target RNA scaffold to or proximal to the other end of the guide-target RNA scaffold. Base paired regions can extend between two structural features. Base paired regions can extend from one end or proximal to one end of the guide-target RNA scaffold to or proximal to a structural feature. Base paired regions can extend between two structural features. Base paired regions can extend from one end or proximal to one end of the guide-target RNA scaffold to or proximal to a structural feature. Base paired regions can extend from a structural feature to the other end of the guide-target RNA scaffold.

The term percent "identity," in the context of two or more nucleic acid or polypeptide sequences, refers to two or more sequences or subsequences that have a specified percentage of nucleotides or amino acid residues that are the same, when compared and aligned for maximum correspondence, as measured using one of the sequence comparison algorithms described below (*e.g.*, BLASTP and BLASTN or other algorithms available to persons of skill) or by visual inspection. Depending on the application, the percent "identity" can exist over a region of the sequence being compared, *e.g.*, over a functional domain, or, alternatively, exist over the full length of the two sequences to be compared.

For sequence comparison, typically one sequence acts as a reference sequence (also called the subject sequence) to which test sequences (also called query sequences) are compared. The percent sequence identity is defined as a test sequence's percent identity to a reference sequence. For example, when stated "Sequence A having a sequence identity of 50% to Sequence B," Sequence A is the test sequence and Sequence B is the reference sequence. When using a sequence comparison algorithm, test and reference sequences are input into a computer program, subsequence coordinates are designated, if necessary, and sequence algorithm program parameters are designated. The sequence comparison algorithm then aligns the sequences to achieve the maximum alignment, based on the designated program parameters, introducing gaps in the alignment if necessary. The percent sequence identity for the test sequence(s) relative to the reference sequence can then be determined from the alignment of the test sequence to the reference sequence. The equation for percent sequence identity from the aligned sequence is as follows:

$$[(\text{Number of Identical Positions})/(\text{Total Number of Positions in the Test Sequence})] \times 100\%$$

For purposes herein, percent identity and sequence similarity calculations are performed using the BLAST algorithm for sequence alignment, which is described in Altschul et al., J. Mol. Biol. 215:403-410 (1990). Software for performing BLAST analyses is publicly available through the National Center for Biotechnology Information (ncbi.nlm.nih.gov). The BLAST algorithm uses a test sequence (also called a query sequence) and a reference sequence (also called a subject sequence) to search against, or in some cases, a database of multiple reference sequences to search against. The BLAST algorithm performs sequence alignment by finding high-scoring alignment regions between the test and the reference sequences by scoring alignment of short regions of the test sequence (termed "words") to the reference sequence. The scoring of each alignment is determined by the BLAST algorithm and takes factors into account, such as the number of aligned positions, as well as whether introduction of gaps between the test and the reference sequences would improve the alignment. The alignment scores for nucleic acids can be scored by set match/mismatch scores. For protein sequences, the alignment scores can be scored using a substitution matrix to evaluate the significance of the sequence alignment, for example, the similarity between aligned amino acids based on their evolutionary probability of substitution. For purposes herein, the substitution matrix used is the BLOSUM62 matrix. For purposes herein, the public default values of April 6, 2023 are used when using the BLASTN and BLASTP algorithms. The BLASTN and BLASTP algorithms then output a "Percent Identity" output value and a "Query Coverage" output value. The overall percent sequence identity as used herein can then be calculated from the BLASTN or BLASTP output values as follows:

Percent Sequence Identity = ("Percent Identity" output value) × ("Query Coverage" output value)

The following non-limiting examples illustrate the calculation of percent identity between two nucleic acids sequences. The percent identity is calculated as follows: [(number of identical nucleotide positions)/(total number of nucleotides in the test sequence)] × 100%. Percent identity is calculated to compare test sequence 1: AAAAAGGGGG (length = 10 nucleotides) to reference sequence 2: AAAAAAAAAA (length = 10 nucleotides). The percent identity between test sequence 1 and reference sequence 2 would be [(5)/(10)] × 100% = 50%. Test sequence 1 has 50% sequence identity to reference sequence 2. In another example, percent identity is calculated to compare test sequence 3: CCCCCG-GGGGGGGGGGCCCCC (length = 20 nucleotides) to reference sequence 4: GGGGGGGGGG (length = 10 nucleotides). The percent identity between test sequence 3 and reference sequence 4 would be [(10)/(20)] ×100% = 50%. Test sequence 3 has 50% sequence identity to reference sequence 4. In another example, percent identity is calculated to compare test sequence 5: GGGGGGGGGG (length = 10 nucleotides) to reference sequence 6: CCCCCG-GGGGGGGGGGCCCCC (length = 20 nucleotides). The percent identity between test sequence 5 and reference sequence 6 would be [(10)/(10)] ×100% = 100%. Test sequence 5 has 100% sequence identity to reference sequence 6.

The following non-limiting examples illustrate the calculation of percent identity between two protein sequences. The percent identity is calculated as follows: [(number of identical amino acid positions)/(total number of amino acids in the test sequence)] × 100%. Percent identity is calculated to compare test sequence 7: FFFFFYYYYY (length = 10 amino acids) to reference sequence 8: YYYYYYYYYY (length = 10 amino acids). The percent identity between test sequence 7 and reference sequence 8 would be [(5)/(10)] ×100% = 50%. Test sequence 7 has 50% sequence identity to reference sequence 8. In another example, percent identity is calculated to compare test sequence 9: LLLLLFFFFF-FYYYYYLLLLL (length = 20 amino acids) to reference sequence 10: FFFFFYYYYY (length = 10 amino acids). The percent identity between test sequence 9 and reference sequence 10 would be [(10)/(20)] ×100% = 50%. Test sequence 9 has 50% sequence identity to reference sequence 10. In another example, percent identity is calculated to compare test sequence 11: FFFFFYYYYY (length = 10 amino acids) to reference sequence 12: LLLLLFFFFFYYYYYLLLLL (length = 20 amino acids). The percent identity between test sequence 11 and reference sequence 12 would be [(10)/(10)] ×100% = 100%. Test sequence 11 has 100% sequence identity to reference sequence 12.

As used herein, "tropism" of a recombinant adeno-associated virus (rAAV), such as a rAAV5, for a tissue refers to the ability of a given rAAV to preferentially infect a given cell type or tissue. A degree of tropism may be determined by a ratio of an infection rate in a targeted tissue to an infection rate in a different, non-targeted tissue. As used herein, increased tropism for a given cell type or tissue, such as increased tropism conferred by a 581-589 region, is determined relative to a wild type AAV5 capsid. As used herein, "detargeting" of a rAAV to a tissue may refer to the ability of a given rAAV to avoid infecting a detargeted tissue or cell type while infecting one or more other tissues or cell types. A degree of detargeting may be determined by a ratio of an infection rate in a detargeted tissue to an infection rate of a different, non-detargeted tissue. As used herein, increased detargeting for a given cell type or tissue, such as increased detargeting conferred by a 581-589 region, is determined relative to a wild type AAV5 capsid.

As used herein, "tissue tropism" refers to a preference of a virus having an engineered VP capsid polypeptide of the present disclosure to infect a given tissue or be enriched in or accumulate in a given tissue. A "tissue-tropic" rAAV may specifically target or infect a first tissue or set of tissues (*e.g.*, CNS, cardiac, liver, skeletal muscle, skin, bone, eye, and/or other tissues) and may not target or infect a second tissue or set of tissues. Alternatively or additionally, in some embodiments, a respective tissue target includes, but is not limited to, adrenal gland, aorta, bone with bone marrow, brain (cerebellum), brain (hippocampus, dentate gyrus), brain (hippocampus, CA1), brain (hippocampus, CA3), brain (hypothalamus), brain (cortex, temporal), brain (cortex, forebrain), brain (cortex, occipital), brain (substantia nigra), brain (thalamus), cecum, colon, duodenum, epididymis, esophagus, eye, gallbladder, heart, ileum, jejunum, kidney, liver, lung, lymph node(s), mammary gland, ovary, pancreas, parathyroid gland, peripheral nerve (sciatic), pituitary, prostate, salivary gland, seminal vesicle, skeletal muscle, skin, spinal cord, spleen, stomach, testis, thymus, thyroid gland, trachea, urinary bladder, uterus, and vagina. For example, a "CNS-tropic" rAAV may specifically target or infect CNS tissue and may not target or infect liver, muscle, skin, bone, eye, or other tissues. In another example a "CNS and cardiac-tropic" rAAV may specifically target or infect CNS and cardiac tissues and may not target or infect liver, skeletal muscle, skin, bone, eye, or other tissues. A "tissue-detargeted" rAAV may specifically avoid targeting or avoid infection of the detargeted tissue or set of tissues while infecting a second tissue or set of tissues. For example, a "liver-detargeted" rAAV may not target or infect liver tissue but may infect one or more other tissues, such as CNS or cardiac tissue. Tissue tropism or tissue detargeting, when used as a relative term and depending on the context in which it is described herein, refers to an increase or decrease in tissue tropism of a given rAAV virion having a first capsid polypeptide in a first tissue as compared to a second tissue and/or refers to an increase or decrease in tissue tropism of a given rAAV virion having a first capsid polypeptide to an rAAV virion having a second capsid polypeptide. In some embodiments, the first tissue can be a group of tissues. In some embodiments, the second tissue can be a group of tissues. For example, the first tissue may be CNS or cardiac tissues and the second tissue may be a non-CNS or non-cardiac tissue consisting collectively of kidney, liver, skeletal muscle, lung, spleen, lymph node, bone marrow, mammary gland, skin, adrenal gland, thyroid, colon, sciatic nerve, and spinal cord tissues.

As used herein, the term "VP" refers to a viral capsid protein. For simplicity throughout this disclosure, viral capsid protein is generally referred to as "VP." Viral capsid protein is referred to as VP1 when referencing AAV5 VP1 positional notation. In all cases, viral capsid sequences and mutations disclosed herein should be understood as pertaining to all isoforms of the capsid protein (VP1, VP2, and VP3), as a mixture of these isoforms assemble to form virions. The positional amino acid residue designations "581 to 589" are relative to the translational start of the VP1 polypeptide and should be adjusted accordingly to the relative start sites of VP2 and VP3. It should be understood that the present disclosure, when describing any particular VP1 sequence with mutations at particular amino acid residue positions, necessarily also encompasses corresponding mutations in VP2 and VP3. For example, any consensus sequence or specific sequence of a VP1 capsid protein having one or more mutations in the 581-589 region, corresponding to amino acid residues 581 to 589 of VP1, also encompasses VP2 and VP3 capsid proteins having said one or more mutations in an amino acid residue region in VP2 and VP3 corresponding to the amino acid residues of the VP1 581 to 589 region.

As used herein, "581-589 region" refers to a region or fragment of VP1 corresponding to amino acid residues 581 to 589 relative to the translational start of the VP1 polypeptide. The 581-589 region corresponds to amino acid residues 445 to 453 of VP2 and to amino acid residues 389 to 397 of VP3. The 581-589 region may confer tissue tropism to an AAV, and defined variants may be engineered to confer tissue tropism to an rAAV formed from viral capsid polypeptides (VP1, VP2, and VP3) comprising the 581-589 region.

It should be understood that the present disclosure includes polynucleotide sequences encoding for any sequence disclosed herein. For example, if an amino acid sequence is provided, the present disclosure also encompasses a polynucleotide sequence encoding for said amino acid sequence. It should be understood that further embodiments include mutations in VP1, VP2, VP3, or any combination thereof that do not alter the desired properties (*e.g.*, a particular tissue tropism) or affect viral assembly, as described herein. In some embodiments, an rAAV virion is made of a capsid that may include the engineered AAV5 VP capsid polypeptides disclosed herein (*e.g.*, engineered VP1, VP2, and VP3 capsid polypeptides comprising a variant 581-589 region).

As used herein, the term "model" refers to a machine learning model or algorithm.

In some embodiments, a model includes an unsupervised learning algorithm. One example of an unsupervised learning algorithm is cluster analysis. In some embodiments, a model includes a supervised machine learning algorithm. Nonlimiting examples of supervised learning algorithms include, but are not limited to, logistic regression, neural networks, support vector machines, Naive Bayes algorithms, nearest neighbor algorithms, random forest algorithms, decision tree algorithms, boosted trees algorithms, multinomial logistic regression algorithms, linear models, linear regression, Gradient Boosting, mixture models, hidden Markov models, Gaussian NB algorithms, linear discriminant analysis, or any combinations thereof. In some embodiments, a model is a multinomial classifier algorithm. In some embodiments, a model is a 2-stage stochastic gradient descent (SGD) model. In some embodiments, a model is a deep neural network (*e.g.*, a deep-and-wide sample-level model).

*Neural networks.* In some embodiments, the model is a neural network (*e.g.*, a convolutional neural network and/or a residual neural network). Neural network algorithms, also known as artificial neural networks (ANNs), include convolutional and/or residual neural network algorithms (deep learning algorithms). In some embodiments, neural networks are machine learning algorithms that are trained to map an input dataset to an output dataset, where the neural network includes an interconnected group of nodes organized into multiple layers of nodes. For example, in some embodiments, the neural network architecture includes at least an input layer, one or more hidden layers, and an output layer. In some embodiments, the neural network includes any total number of layers, and any number of hidden layers, where the hidden layers function as trainable feature extractors that allow mapping of a set of input data to an output value or set of output values. In some embodiments, a deep learning algorithm is a neural network including a plurality of hidden layers, *e.g.*, two or more hidden layers. In some instances, each layer of the neural network includes a number of nodes (or "neurons"). In some embodiments, a node receives input that comes either directly from the input data or the output of nodes in previous layers, and performs a specific operation, *e.g.*, a summation operation. In some embodiments, a connection from an input to a node is associated with a parameter (*e.g.*, a weight and/or weighting factor). In some embodiments, the node sums up the products of all pairs of inputs, $x_i$, and their associated parameters. In some embodiments, the weighted sum is offset with a bias, b. In some embodiments, the output of a node or neuron is gated using a threshold or activation function, f, which, in some instances, is a linear or non-linear function. In some embodiments, the activation function is, for example, a rectified linear unit (ReLU) activation function, a Leaky ReLU activation function, or other function such as a saturating hyperbolic tangent, identity, binary step, logistic, arcTan, softsign, parametric rectified linear unit, exponential linear unit, softPlus, bent identity, softExponential, Sinusoid, Sine, Gaussian, or sigmoid function, or any combination thereof.

In some implementations, the weighting factors, bias values, and threshold values, or other computational parameters of the neural network, are "taught" or "learned" in a training phase using one or more sets of training data. For example, in some implementations, the parameters are trained using the input data from a training dataset and a gradient descent, for example, back-propagation, method so that the output value(s) that the ANN computes are consistent with the examples included in the training dataset. In some embodiments, the parameters are obtained from a back propagation neural network training process.

Any of a variety of neural networks are suitable for use in accordance with the present disclosure. Examples include, but are not limited to, feedforward neural networks, radial basis function networks, recurrent neural networks, residual neural networks, convolutional neural networks, residual convolutional neural networks, and the like, or any combination thereof. In some embodiments, the machine learning makes use of a pre-trained and/or transfer-learned ANN or deep learning architecture. In some implementations, convolutional and/or residual neural networks are used, in accordance with the present disclosure.

For instance, a deep neural network model includes an input layer, a plurality of individually parameterized (*e.g.*, weighted) convolutional layers, and an output scorer. The parameters (*e.g.*, weights) of each of the convolutional layers as well as the input layer contribute to the plurality of parameters (*e.g.*, weights) associated with the deep neural network model. In some embodiments, at least 50 parameters, at least 100 parameters, at least 1000 parameters, at least 2000 parameters, at least 5000 parameters, at least $1 \times 10^4$ parameters, at least $1 \times 10^5$ parameters, at least $1 \times 10^6$ parameters, at least $1 \times 10^7$ parameters, or at least $1 \times 10^8$ parameters are associated with the deep neural network model. As such, deep neural network models require a computer to be used because they cannot be mentally solved. In other words, given an input to the model, the model output needs to be determined using a computer rather than mentally in such embodiments. *See*, for example, Krizhevsky et al., 2012, "Imagenet classification with deep convolutional neural networks," in Advances in Neural Information Processing Systems 2, Pereira, Burges, Bottou, Weinberger, eds., pp. 1097-1105, Curran Associates, Inc.; Zeiler, 2012 "ADADELTA: an adaptive learning rate method," CoRR, vol. abs/1212.5701; and Rumelhart et al., 1988, "Neurocomputing: Foundations of research," ch. Learning Representations by Back-propagating Errors, pp. 696-699, Cambridge, MA, USA: MIT Press, each of which is hereby incorporated by reference.

Neural network algorithms, including convolutional neural network algorithms, suitable for use as models are disclosed in, for example, Vincent et al., 2010, "Stacked denoising autoencoders: Learning useful representations in a deep network with a local denoising criterion," J Mach Learn Res 11, pp. 3371-3408; Larochelle et al., 2009, "Exploring strategies for training deep neural networks," J Mach Learn Res 10, pp. 1-40; and Hassoun, 1995, Fundamentals of Artificial Neural Networks, Massachusetts Institute of Technology, each of which is hereby incorporated by reference. Additional example neural networks suitable for use as models are disclosed in Duda et al., 2001, Pattern Classification, Second Edition, John Wiley & Sons, Inc., New York; and Hastie et al., 2001, The Elements of Statistical Learning, Springer-Verlag, New York, each of which is hereby incorporated by reference in its entirety. Additional example neural networks suitable for use as models are also described in Draghici, 2003, Data Analysis Tools for DNA Microarrays, Chapman & Hall/CRC; and Mount, 2001, Bioinformatics: sequence and genome analysis, Cold Spring Harbor Laboratory Press, Cold Spring Harbor, New York, each of which is hereby incorporated by reference in its entirety.

*Support vector machines.* In some embodiments, the model is a support vector machine (SVM). SVM algorithms suitable for use as models are described in, for example, Cristianini and Shawe-Taylor, 2000, "An Introduction to Support Vector Machines," Cambridge University Press, Cambridge; Boser et al., 1992, "A training algorithm for optimal margin classifiers," in Proceedings of the 5th Annual ACM Workshop on Computational Learning Theory, ACM Press, Pittsburgh, Pa., pp. 142-152; Vapnik, 1998, Statistical Learning Theory, Wiley, New York; Mount, 2001, Bioinformatics: sequence and genome analysis, Cold Spring Harbor Laboratory Press, Cold Spring Harbor, N.Y.; Duda, Pattern Classification, Second Edition, 2001, John Wiley & Sons, Inc., pp. 259, 262-265; and Hastie, 2001, The Elements of Statistical Learning, Springer, New York; and Furey et al., 2000, Bioinformatics 16, 906-914, each of which is hereby incorporated by reference in its entirety. When used for classification, SVMs separate a given set of binary labeled data with a hyper-plane that is maximally distant from the labeled data. For certain cases in which no linear separation is possible, SVMs work in combination with the technique of 'kernels', which automatically realizes a non-linear mapping to a feature space. The hyper-plane found by the SVM in feature space corresponds, in some instances, to a non-linear decision boundary in the input space. In some embodiments, the plurality of parameters (*e.g.*, weights) associated with the SVM define the hyper-plane. In some embodiments, the hyper-plane is defined by at least 10, at least 20, at least 50, or at least 100 parameters and the SVM model requires a computer to calculate because it cannot be mentally solved.

*Naive Bayes algorithms.* In some embodiments, the model is a Naive Bayes algorithm. Naive Bayes models suitable for use as models are disclosed, for example, in Ng et al., 2002, "On discriminative vs. generative classifiers: A comparison of logistic regression and naive Bayes," Advances in Neural Information Processing Systems, 14, which is hereby incorporated by reference. A Naive Bayes model is any model in a family of "probabilistic models" based on applying Bayes' theorem with strong (naive) independence assumptions between the features. In some embodiments, they are coupled with Kernel density estimation. *See,* for example, Hastie et al., 2001, The elements of statistical learning: data mining, inference, and prediction, eds. Tibshirani and Friedman, Springer, New York, which is hereby incorporated by reference.

*Nearest neighbor algorithms.* In some embodiments, a model is a nearest neighbor algorithm. In some implementations, nearest neighbor models are memory-based and include no model to be fit. For nearest neighbors, given a query point $x_0$ (a test subject), the k training points $x_{(r)}$, r, ... , k (here the training subjects) closest in distance to $x_0$ are identified and then the point $x_0$ is classified using the k nearest neighbors. In some embodiments, Euclidean distance in feature space is used to determine distance as $d_{(i)} = \|x_{(i)} - x_{(o)}\|$. Typically, when the nearest neighbor algorithm is used, the abundance data used to compute the linear discriminant is standardized to have mean zero and variance 1. In some embodiments, the nearest neighbor rule is refined to address issues of unequal class priors, differential misclassification costs, and feature selection. Many of these refinements involve some form of weighted voting for the neighbors. For more information on nearest neighbor analysis, *see* Duda, Pattern Classification, Second Edition, 2001, John Wiley & Sons, Inc; and Hastie, 2001, The Elements of Statistical Learning, Springer, New York, each of which is hereby incorporated by reference.

A k-nearest neighbor model is a non-parametric machine learning method in which the input consists of the k closest training examples in feature space. In some embodiments, the output is a class membership. An object is classified by a plurality vote of its neighbors, with the object being assigned to the class most common among its k nearest neighbors (k is a positive integer, typically small). If k = 1, then the object is simply assigned to the class of that single nearest neighbor. *See*, Duda et al., 2001, Pattern Classification, Second Edition, John Wiley & Sons, which is hereby incorporated by reference. In some embodiments, the k-nearest neighbor model is used for regression and the output is a prediction of a property value of the object determined as an average of the values of the k nearest neighbors. In some embodiments, the number of distance calculations needed to solve the k-nearest neighbor model is such that a computer is used to solve the model for a given input because it cannot be mentally performed.

*Random forest, decision tree, and boosted tree algorithms.* In some embodiments, the model is a decision tree. Decision trees suitable for use as models are described generally by Duda, 2001, Pattern Classification, John Wiley & Sons, Inc., New York, pp. 395-396, which is hereby incorporated by reference. Tree-based methods partition the feature space into a set of rectangles, and then fit a model (like a constant) in each one. In some embodiments, the decision tree is random forest regression. For example, one specific algorithm is a classification and regression tree (CART). Other specific decision tree algorithms include, but are not limited to, ID3, C4.5, MART, and Random Forests. CART, ID3, and C4.5 are described in Duda, 2001, Pattern Classification, John Wiley & Sons, Inc., New York, pp. 396-408 and pp. 411-412, which is hereby incorporated by reference. CART, MART, and C4.5 are described in Hastie et al., 2001, The Elements of Statistical Learning, Springer-Verlag, New York, Chapter 9, which is hereby incorporated by reference in its entirety. Random Forests are described in Breiman, 1999, "Random Forests--Random Features," Technical Report 567, Statistics Department, U.C. Berkeley, September 1999, which is hereby incorporated by reference in its entirety. In some embodiments, the decision tree model includes at least 10, at least 20, at least 50, or at least 100 parameters (*e.g.*, weights and/or decisions) and requires a computer to calculate because it cannot be mentally solved.

*Regression.* In some embodiments, the model uses a regression algorithm. In some embodiments, a regression algorithm is any type of regression. For example, in some embodiments, the regression algorithm is logistic regression. In some embodiments, the regression algorithm is logistic regression with lasso, L2 or elastic net regularization. In some embodiments, those extracted features that have a corresponding regression coefficient that fails to satisfy a threshold value are pruned (removed from) consideration. In some embodiments, a generalization of the logistic regression model that handles multi category responses is used as the model. Logistic regression algorithms are disclosed in Agresti, An Introduction to Categorical Data Analysis, 1996, Chapter 5, pp. 103-144, John Wiley & Son, New York, which is hereby incorporated by reference. In some embodiments, the model makes use of a regression model disclosed in Hastie et al., 2001, The Elements of Statistical Learning, Springer-Verlag, New York. In some embodiments, the logistic regression model includes at least 10, at least 20, at least 50, at least 100, or at least 1000 parameters (*e.g.*, weights) and requires a computer to calculate because it cannot be mentally solved.

*Linear discriminant analysis algorithms.* In some embodiments, linear discriminant analysis (LDA), normal discriminant analysis (NDA), or discriminant function analysis is a generalization of Fisher's linear discriminant, a method used in statistics, pattern recognition, and machine learning to find a linear combination of features that characterizes or separates two or more classes of objects or events. In some embodiments, the resulting combination is used as the model (linear model) in some embodiments of the present disclosure.

*Mixture model and Hidden Markov model.* In some embodiments, the model is a mixture model, such as that described in McLachlan et al., Bioinformatics 18(3):413-422, 2002. In some embodiments, in particular, those embodiments including a temporal component, the model is a hidden Markov model such as described by Schliep et al., 2003, Bioinformatics 19(1):i255-i263.

*Clustering.* In some embodiments, the model is an unsupervised clustering model. In some embodiments, the model is a supervised clustering model. Clustering algorithms suitable for use as models are described, for example, at pages 211-256 of Duda and Hart, Pattern Classification and Scene Analysis, 1973, John Wiley & Sons, Inc., New York, (hereinafter "Duda 1973") which is hereby incorporated by reference in its entirety. As an illustrative example, in some embodiments, the clustering problem is described as one of finding natural groupings in a dataset. To identify natural groupings, two issues are addressed. First, a way to measure similarity (or dissimilarity) between two samples is determined. This metric (*e.g.*, similarity measure) is used to ensure that the samples in one cluster are more like one another than they are to samples in other clusters. Second, a mechanism for partitioning the data into clusters using the similarity measure is determined. One way to begin a clustering investigation is to define a distance function and to compute the matrix of distances between all pairs of samples in the training set. If distance is a good measure of similarity, then the distance between reference entities in the same cluster is significantly less than the distance between the reference entities in different clusters. However, in some implementations, clustering does not use a distance metric. For example, in some embodiments, a nonmetric similarity function s(x, x') is used to compare two vectors x and x'. In some such embodiments, s(x, x') is a symmetric function whose value is large when x and x' are somehow "similar." Once a method for measuring "similarity" or "dissimilarity" between points in a dataset has been selected, clustering uses a criterion function that measures the clustering quality of any partition of the data. Partitions of the dataset that extremize the criterion function are used to cluster the data. Particular exemplary clustering techniques contemplated for use in the present disclosure include, but are not limited to, hierarchical clustering (agglomerative clustering using a nearest-neighbor algorithm, farthest-neighbor algorithm, the average linkage algorithm, the centroid algorithm, or the sum-of-squares algorithm), k-means clustering, fuzzy k-means clustering algorithm, and Jarvis-Patrick clustering. In some embodiments, the clustering includes unsupervised clustering (*e.g.*, with no preconceived number of clusters and/or no predetermination of cluster assignments).

*Ensembles of models and boosting.* In some embodiments, an ensemble (two or more) of models is used. In some embodiments, a boosting technique such as AdaBoost is used in conjunction with many other types of learning algorithms to improve the performance of the model. In this approach, the output of any of the models disclosed herein, or their equivalents, is combined into a weighted sum that represents the final output of the boosted model. In some embodiments, the plurality of outputs from the models is combined using any measure of central tendency known in the art, including but not limited to a mean, median, mode, a weighted mean, weighted median, weighted mode, *etc.* In some embodiments, the plurality of outputs is combined using a voting method. In some embodiments, a respective model in the ensemble of models is weighted or unweighted.

As used herein, the term "parameter" refers to any coefficient or, similarly, any value of an internal or external element (*e.g.*, a weight and/or a hyperparameter) in an algorithm, model, regressor, and/or classifier that can affect (*e.g.*,

modify, tailor, and/or adjust) one or more inputs, outputs, and/or functions in the algorithm, model, regressor and/or classifier. For example, in some embodiments, a parameter refers to any coefficient, weight, and/or hyperparameter that can be used to control, modify, tailor, and/or adjust the behavior, learning, and/or performance of an algorithm, model, regressor, and/or classifier. In some instances, a parameter is used to increase or decrease the influence of an input (*e.g.*, a feature) to an algorithm, model, regressor, and/or classifier. As a nonlimiting example, in some embodiments, a parameter is used to increase or decrease the influence of a node (*e.g.*, of a neural network), where the node includes one or more activation functions. Assignment of parameters to specific inputs, outputs, and/or functions is not limited to any one paradigm for a given algorithm, model, regressor, and/or classifier but can be used in any suitable algorithm, model, regressor, and/or classifier architecture for a desired performance. In some embodiments, a parameter has a fixed value. In some embodiments, a value of a parameter is manually and/or automatically adjustable. In some embodiments, a value of a parameter is modified by a validation and/or training process for an algorithm, model, regressor, and/or classifier (*e.g.*, by error minimization and/or backpropagation methods). In some embodiments, an algorithm, model, regressor, and/or classifier of the present disclosure includes a plurality of parameters. In some embodiments, the plurality of parameters is n parameters, where: $n \geq 2$; $n \geq 5$; $n \geq 10$; $n \geq 25$; $n \geq 40$; $n \geq 50$; $n \geq 75$; $n \geq 100$; $n \geq 125$; $n \geq 150$; $n \geq 200$; $n \geq 225$; $n \geq 250$; $n \geq 350$; $n \geq 500$; $n \geq 600$; $n \geq 750$; $n \geq 1,000$; $n \geq 2,000$; $n \geq 4,000$; $n \geq 5,000$; $n \geq 7,500$; $n \geq 10,000$; $n \geq 20,000$; $n \geq 40,000$; $n \geq 75,000$; $n \geq 100,000$; $n \geq 200,000$; $n \geq 500,000$, $n \geq 1 \times 10^6$, $n \geq 5 \times 10^6$, $n \geq 1 \times 10^7$, $n \geq 1 \times 10^8$, or $\geq 1 \times 10^9$. In some embodiments, the plurality of parameters comprises no more than $1 \times 10^{10}$, no more than $1 \times 10^9$, no more than $1 \times 10^8$, no more than $1 \times 10^7$, no more than $1 \times 10^6$, no more than $1 \times 10^5$, no more than $1 \times 10^4$, or no more than $1 \times 10^3$. As such, the algorithms, models, regressors, and/or classifiers of the present disclosure cannot be mentally performed. In some embodiments n is between 10,000 and $1 \times 10^7$, between 100,000 and $5 \times 10^6$, or between 500,000 and $1 \times 10^6$. In some embodiments, the plurality of parameters falls within another range starting no lower than 2 parameters and ending no higher than $1 \times 10^{10}$ parameters. In some embodiments, the algorithms, models, regressors, and/or classifier of the present disclosure operate in a k-dimensional space, where k is a positive integer of 5 or greater (*e.g.*, 5, 6, 7, 8, 9, 10, *etc.*). As such, the algorithms, models, regressors, and/or classifiers of the present disclosure cannot be mentally performed.

As used herein, the term "untrained model" refers to a machine learning model or algorithm, such as a classifier or a neural network, that has not been trained on a target dataset. In some embodiments, "training a model" (*e.g.*, "training a neural network") refers to the process of training an untrained or partially trained model (*e.g.*, "an untrained or partially trained neural network"). Moreover, it will be appreciated that the term "untrained model" does not exclude the possibility that transfer learning techniques are used in such training of the untrained or partially trained model. For instance, Fernandes et al., 2017, "Transfer Learning with Partial Observability Applied to Cervical Cancer Screening," Pattern Recognition and Image Analysis: 8th Iberian Conference Proceedings, 243-250, which is hereby incorporated by reference, provides non-limiting examples of such transfer learning. In instances where transfer learning is used, the untrained model described above is provided with additional data over and beyond that of the primary training dataset. Typically, this additional data is in the form of parameters (*e.g.*, coefficients, weights, and/or hyperparameters) that were learned from another, auxiliary training dataset. Moreover, while a description of a single auxiliary training dataset has been disclosed, it will be appreciated that there is no limit on the number of auxiliary training datasets that can be used to complement the primary training dataset in training the untrained model in the present disclosure. For instance, in some embodiments, two or more auxiliary training datasets, three or more auxiliary training datasets, four or more auxiliary training datasets or five or more auxiliary training datasets are used to complement the primary training dataset through transfer learning, where each such auxiliary dataset is different than the primary training dataset. Any manner of transfer learning is used, in some such embodiments. For instance, consider the case where there is a first auxiliary training dataset and a second auxiliary training dataset in addition to the primary training dataset. In such a case, the parameters learned from the first auxiliary training dataset (by application of a first model to the first auxiliary training dataset) are applied to the second auxiliary training dataset using transfer learning techniques (*e.g.*, a second model that is the same or different from the first model), which in turn results in a trained intermediate model whose parameters are then applied to the primary training dataset and this, in conjunction with the primary training dataset itself, is applied to the untrained model. Alternatively, in another example embodiment, a first set of parameters learned from the first auxiliary training dataset (by application of a first model to the first auxiliary training dataset) and a second set of parameters learned from the second auxiliary training dataset (by application of a second model that is the same or different from the first model to the second auxiliary training dataset) are each individually applied to a separate instance of the primary training dataset (*e.g.*, by separate independent matrix multiplications) and both such applications of the parameters to separate instances of the primary training dataset in conjunction with the primary training dataset itself (or some reduced form of the primary training dataset such as principal components or regression coefficients learned from the primary training set) are then applied to the untrained model in order to train the untrained model.

Reference will now be made in detail to embodiments, examples of which are illustrated in the accompanying drawings. In the following detailed description, numerous specific details are set forth in order to provide a thorough understanding of the present disclosure. However, it will be apparent to one of ordinary skill in the art that the present disclosure may be practiced without these specific details. In other instances, well-known methods, procedures, and components have not been described in detail so as not to unnecessarily obscure aspects of the embodiments.

It will also be understood that, although the terms first, second, *etc.* may be used herein to describe various elements, these elements should not be limited by these terms. These terms are only used to distinguish one element from another. For instance, a first subject could be termed a second subject, and, similarly, a second subject could be termed a first subject, without departing from the scope of the present disclosure. The first subject and the second subject are both subjects, but they are not the same subject.

The terminology used in the present disclosure is for the purpose of describing particular embodiments only and is not intended to be limiting of the invention. As used in the description of the invention and the appended claims, the singular forms "a," "an," and "the" are intended to include the plural forms as well, unless the context clearly indicates otherwise. It will also be understood that the term "and/or" as used herein refers to and encompasses any and all possible combinations of one or more of the associated listed items. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

The present description includes example systems, methods, techniques, instruction sequences, and computing machine program products that embody illustrative implementations. For purposes of explanation, numerous specific details are set forth in order to provide an understanding of various implementations of the inventive subject matter. It will be evident, however, to those skilled in the art that implementations of the inventive subject matter may be practiced without these specific details. In general, well-known instruction instances, protocols, structures, and techniques have not been shown in detail.

The present description, for purpose of explanation, is described with reference to specific implementations. However, the illustrative discussions below are not intended to be exhaustive or to limit the implementations to the precise forms disclosed. Many modifications and variations are possible in view of the above teachings. The implementations are chosen and described in order to best explain the principles and their practical applications, to thereby enable others skilled in the art to best utilize the implementations and various implementations with various modifications as are suited to the particular use contemplated.

In the interest of clarity, not all of the routine features of the implementations described herein are shown and described. It will be appreciated that, in the development of any such actual implementation, numerous implementation-specific decisions are made in order to achieve the designer's specific goals, such as compliance with use case- and business-related constraints, and that these specific goals will vary from one implementation to another and from one designer to another. Moreover, it will be appreciated that such a design effort might be complex and time-consuming, but nevertheless be a routine undertaking of engineering for those of ordering skill in the art having the benefit of the present disclosure.

As used herein, the term "if" may be construed to mean "when" or "upon" or "in response to determining" or "in response to detecting," depending on the context. Similarly, the phrase "if it is determined" or "if [a stated condition or event] is detected" may be construed to mean "upon determining" or "in response to determining" or "upon detecting [the stated condition or event]" or "in response to detecting [the stated condition or event]," depending on the context.

As used herein, the term "about" or "approximately" can mean within an acceptable error range for the particular value as determined by one of ordinary skill in the art, which can depend in part on how the value is measured or determined, *e.g.*, the limitations of the measurement system. For example, "about" can mean within 1 or more than 1 standard deviation, per the practice in the art. "About" can mean a range of $\pm$ 20%, $\pm$ 10%, $\pm$ 5%, or $\pm$ 1% of a given value. Where particular values are described in the application and claims, unless otherwise stated, the term "about" means within an acceptable error range for the particular value. The term "about" can have the meaning as commonly understood by one of ordinary skill in the art. The term "about" can refer to $\pm$ 10%. The term "about" can refer to $\pm$ 5%.

## 7.3. Example System Embodiments

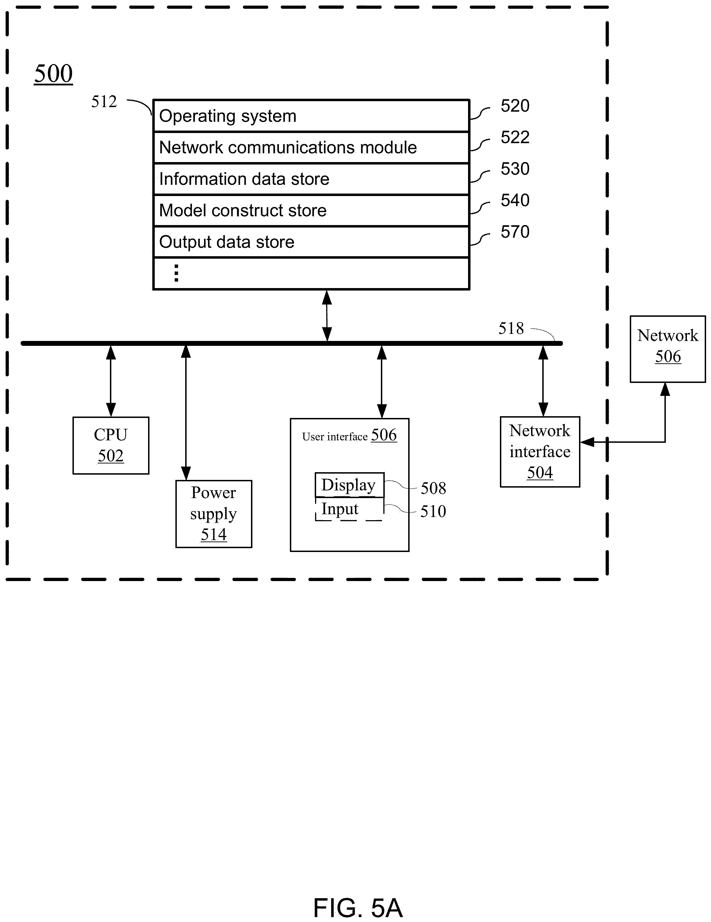

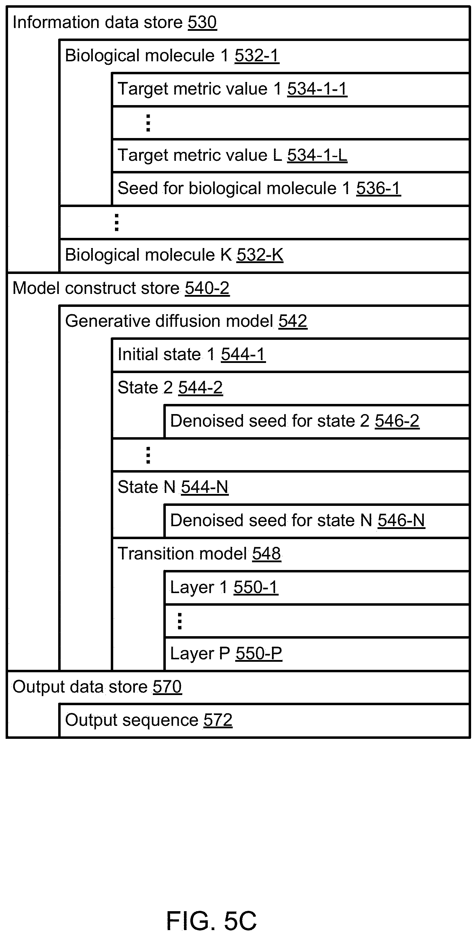

In the present disclosure, unless expressly stated otherwise, descriptions of devices and systems will include implementations of one or more computers. For instance, and for purposes of illustration in FIGS. 5A-C, a computer system 500 is represented as single device that includes all the functionality of the computer system 500. However, the present disclosure is not limited thereto. For instance, in some embodiments, the functionality of the computer system 500 is spread across any number of networked computers and/or reside on each of several networked computers and/or by hosted on one or more virtual machines and/or containers at a remote location accessible across a communications network (*e.g.*, communications network 506). One of skill in the art will appreciate that a wide array of different computer topologies is possible for the computer system 500, and other devices and systems of the preset disclosure, and that all such topologies are within the scope of the present disclosure. Moreover, rather than relying on a physical communications network 506, the illustrated devices and systems may wirelessly transmit information between each other. As such, the exemplary topology shown in FIGS. 5A-D merely serves to describe the features of an embodiment of the present disclosure in a manner that will be readily understood to one of skill in the art.

FIG. 5A depicts a block diagram of a computer system 500 according to some embodiments of the present disclosure. The computer system 500 at least facilitates predicting a deamination efficiency or specificity and/or generating a candidate sequence for a guide RNA (gRNA).

In some embodiments, the prediction of the deamination efficiency or specificity and/or the generation of the candidate sequence for the gRNA is prepared at the computer system 500. In some embodiments, the prediction of the deamination efficiency or specificity and/or the generation of the candidate sequence for the gRNA is then provided (*e.g.*, communicated through communication network 506) to a subject through a display of a respective client device. However, the present disclosure is not limited thereto.

In some embodiments, the communication network 506 optionally includes the Internet, one or more local area networks (LANs), one or more wide area networks (WANs), other types of networks, or a combination of such networks.

Examples of communication networks 506 include the World Wide Web (WWW), an intranet and/or a wireless network, such as a cellular telephone network, a wireless local area network (LAN) and/or a metropolitan area network (MAN), and other devices by wireless communication. The wireless communication optionally uses any of a plurality of communications standards, protocols and technologies, including Global System for Mobile Communications (GSM), Enhanced Data GSM Environment (EDGE), high-speed downlink packet access (HSDPA), high-speed uplink packet access (HSUPA), Evolution, Data-Only (EV-DO), HSPA, HSPA+, Dual-Cell HSPA (DC-HSPDA), long term evolution (LTE), near field communication (NFC), wideband code division multiple access (W-CDMA), code division multiple access (CDMA), time division multiple access (TDMA), Bluetooth, Wireless Fidelity (Wi-Fi) (*e.g.*, IEEE 802.11a, IEEE 802.11ac, IEEE 802.11ax, IEEE 802.11b, IEEE 802.11g and/or IEEE 802.11n), voice over Internet Protocol (VoIP), Wi-MAX, a protocol for e-mail (*e.g.*, Internet message access protocol (IMAP) and/or post office protocol (POP)), instant messaging (*e.g.*, extensible messaging and presence protocol (XMPP), Session Initiation Protocol for Instant Messaging and Presence Leveraging Extensions (SIMPLE), Instant Messaging and Presence Service (IMPS)), and/or Short Message Service (SMS), or any other suitable communication protocol, including communication protocols not yet developed as of the filing date of this document.

In various embodiments, the computer system 500 includes one or more processing units (CPUs, processing cores, *etc.*) 502, a network or other communications interface 504, and memory 512. In some embodiments, the computer system 500 includes a power supply 514 configured to provide a current to one or more components and/or hardware devices of the computer system 500 or a remote device.

In some embodiments, the computer system 500 includes a user interface 506. The user interface 506 typically includes a display 508 for presenting media, such as an output from a model of the present disclosure. In some embodiments, the display 508 is integrated within the computer system (*e.g.*, housed in the same chassis as the CPU 502 and memory 512). In some embodiments, the computer system 500 includes one or more input device(s) 510, which allow a subject to interact with the computer system 500. In some embodiments, the one or more input devices 510 include a keyboard, a mouse, and/or other input mechanisms. Alternatively, or in addition, in some embodiments, the display 508 includes a touch-sensitive surface (*e.g.*, where display 508 is a touch-sensitive display or computer system 500 includes a touch pad).

In some embodiments, the computer system 500 presents media to a user through the display 508. Examples of media presented by the display 508 include a prediction of a deamination efficiency or specificity, a generation of the candidate sequence for the gRNA, an output from a model, or a combination thereof. In typical embodiments, the media is presented by the display 508 through a client application.

In some embodiments, memory 512 includes high-speed random access memory, such as DRAM, SRAM, DDR RAM, or other random access solid state memory devices, and optionally also includes non-volatile memory, such as one or more magnetic disk storage devices, optical disk storage devices, flash memory devices, or other non-volatile solid state storage devices. In some embodiments, memory 512 optionally includes one or more storage devices remotely located from the CPU(s) 502. In some embodiments, memory 512, or alternatively the non-volatile memory device(s) within memory 512, includes a non-transitory computer readable storage medium. Access to memory 512 by other components of the computer system 500, such as the CPU(s) 502, is, optionally, controlled by a controller. In some embodiments, the memory 512 include mass storage that is remotely located with respect to the CPU(s) 502. In other words, some data stored in memory 512 is in fact hosted on devices that are external to the computer system 500, but that can be electronically accessed by the computer system 500 over an Internet, intranet, or other form of network 506 or electronic cable using communication interface 504.

In some embodiments, the memory 512 of the computer system 500 for predicting a deamination efficiency or specificity and/or generating a candidate sequence for a gRNA stores:

- an optional operating system 520 (*e.g.*, ANDROID, iOS, DARWIN, RTXC, LINUX, UNIX, OS X, WINDOWS, or an embedded operating system such as VxWorks) that includes procedures for handling various basic system services;

- an optional network communications module 522 associated with the computer system 500 that identifies the computer system 500 (*e.g.*, within the communication network 506);

- an information data store 530;

- a model construct store 540; and

- an output data store 570.

In some embodiments, the computer system 500 includes an operating system 520 that includes procedures for handling various basic system services. The operating system 520 (*e.g.*, iOS, DARWIN, RTXC, LINUX, UNIX, OS X, WINDOWS, or an embedded operating system such as VxWorks) includes various software components and/or drivers for controlling and managing general system tasks (*e.g.*, memory management, storage device control, power management, *etc.*) and facilitates communication between various hardware and software components of the computer system.

In some embodiments, an optional network communications module 522 is associated with the computer system 500. The optional network communications module 522 is utilized to, at least, uniquely identify the computer system 500 from other devices and components (*e.g.*, uniquely identify computer system 500 from a first client device, *etc.*). For instance, in some embodiments, the optional network communications module is utilized to receive information from a client device.

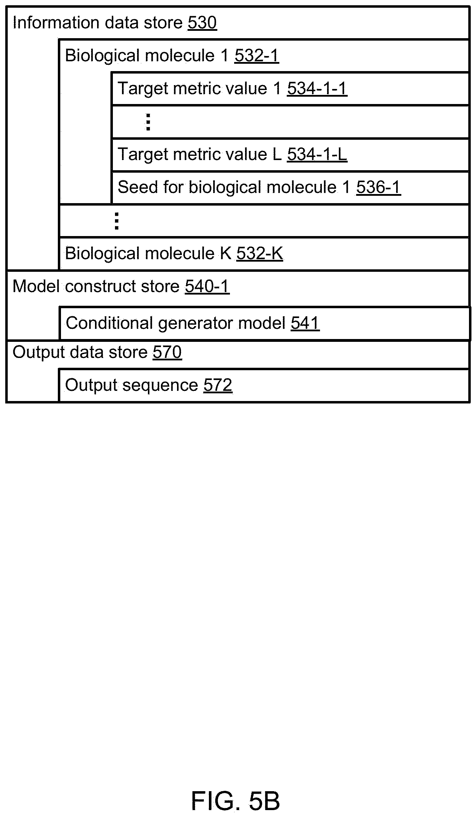

Referring to FIGS. 5B-C, in some embodiments, the system 500 at least includes instructions for generating a polymer sequence for a biological molecule having one or more target biological properties.

In some embodiments, the information data store 530 includes, for a respective biological molecule 532 (*e.g.*, 532-1,... 532-K), (i) a plurality of target metric values 534 (*e.g.*, 534-1-1,...534-1-L) for one or more target biological properties of a biological molecule and (ii) a seed 536 (*e.g.*, 536-1) for a nucleic acid or amino acid sequence for the biological molecule.

In some embodiments, the model construct store 540 (*e.g.*, 540-1) accepts, as input, the (i) plurality of target metric values 534 (*e.g.*, 534-1-1,...534-1-L) for one or more target biological properties of a biological molecule and (ii) seed 536 (*e.g.*, 536-1) for a nucleic acid or amino acid sequence for the biological molecule. In some embodiments, the model construct store 540-1 includes a conditional generator model 541 from a conditional generative adversarial network.

In some embodiments, the model construct store 540 (*e.g.*, 540-2) accepts, as input, the (i) plurality of target metric values 534 (*e.g.*, 534-1-1,...534-1-L) for one or more target biological properties of a biological molecule and (ii) seed 536 (*e.g.*, 536-1) for a nucleic acid or amino acid sequence for the biological molecule into an initial state $X_1$ 544-1 in a plurality of consecutive states $X_N$ 544 (*e.g.*, 544-2,...544-N) in a Markov chain of a generative diffusion model 542. In some embodiments, for each respective consecutive state $X_n$ 544 in the plurality of consecutive states $X_N$ in the Markov chain following the initial state Xi, 544-1 the diffusion model 542 generates a corresponding denoised seed 546 (*e.g.*, 546-2,...546-N) for the nucleic acid or amino acid sequence for the biological molecule using a transition model 548, wherein the transitional model comprises a plurality of layers 550 (*e.g.*, 550-1,...550-P), that accounts for (*e.g.*, wherein the corresponding denoised seed accounts for) the plurality of target metrics 534 for the one or more target biological properties using as input: the seed 536 for the nucleic acid or amino acid sequence, when the respective state $X_n$ is the state immediately following the initial state Xi, and the corresponding denoised seed sequence 546 from the respective state $X_{n-1}$ in the plurality of consecutive states $X_N$ in the Markov chain that immediately precedes the respective state $X_n$, when the respective state $X_n$ is not the state immediately following the initial state Xi.

In some embodiments, the output data store 570 (*e.g.*, 570) includes, as output from the conditional generator model 541 a nucleic acid or amino acid sequence 572 for the biological molecule 532 that is predicted by the conditional generator model to confer on the biological molecule the one or more target biological properties approximating the plurality of target metric values 534.

**7.4. Generating a Polymer Sequence for a Biological Molecule having Target Biological Properties**

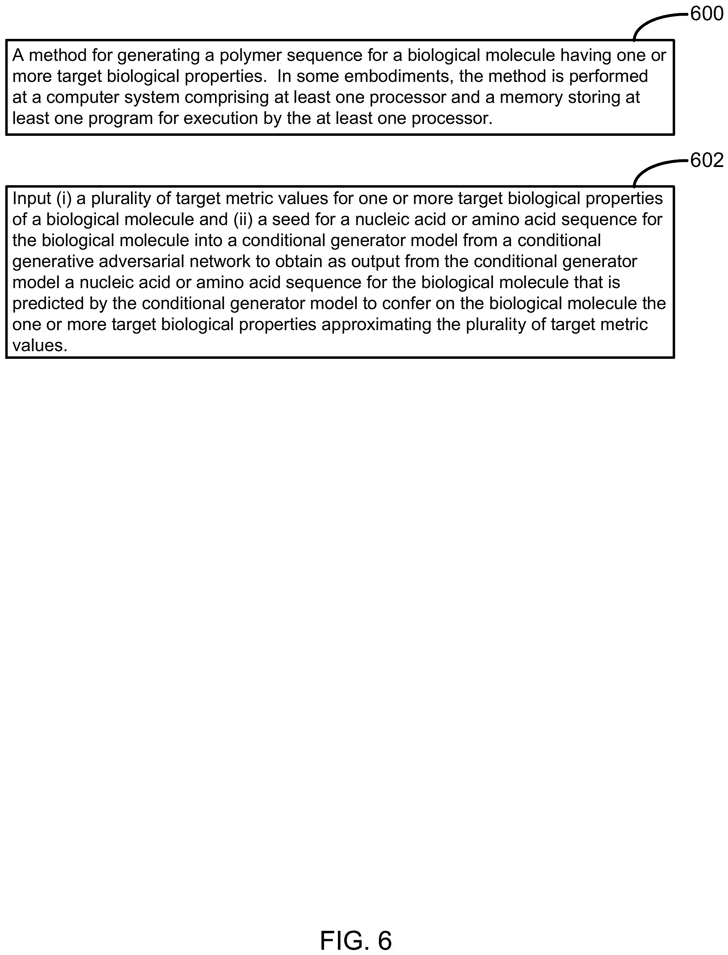

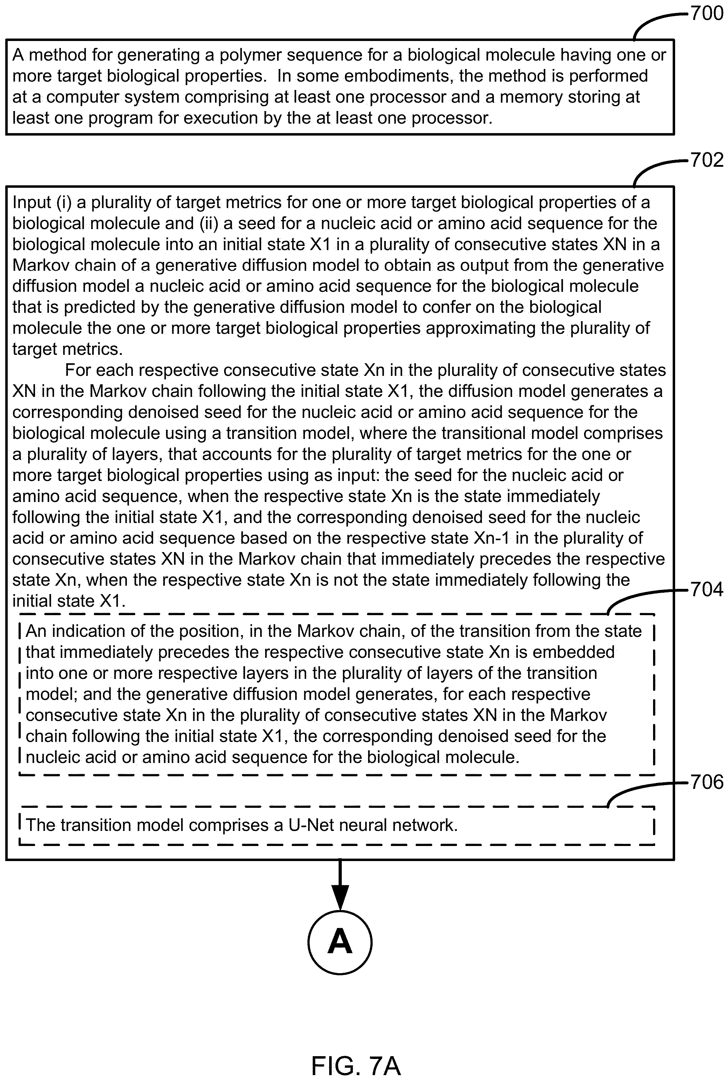

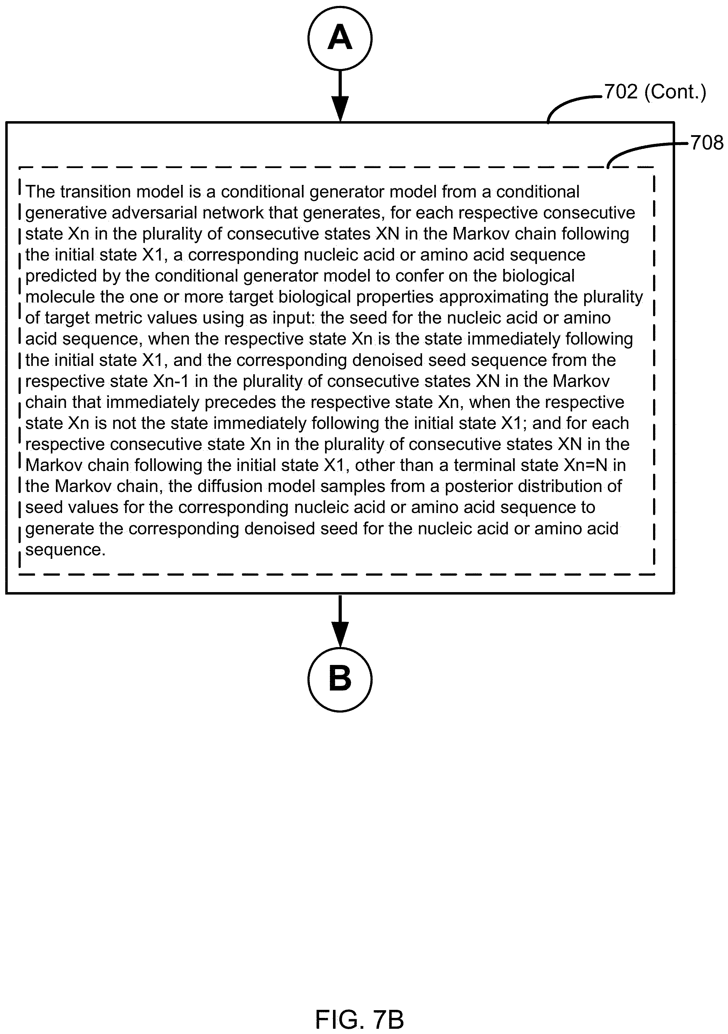

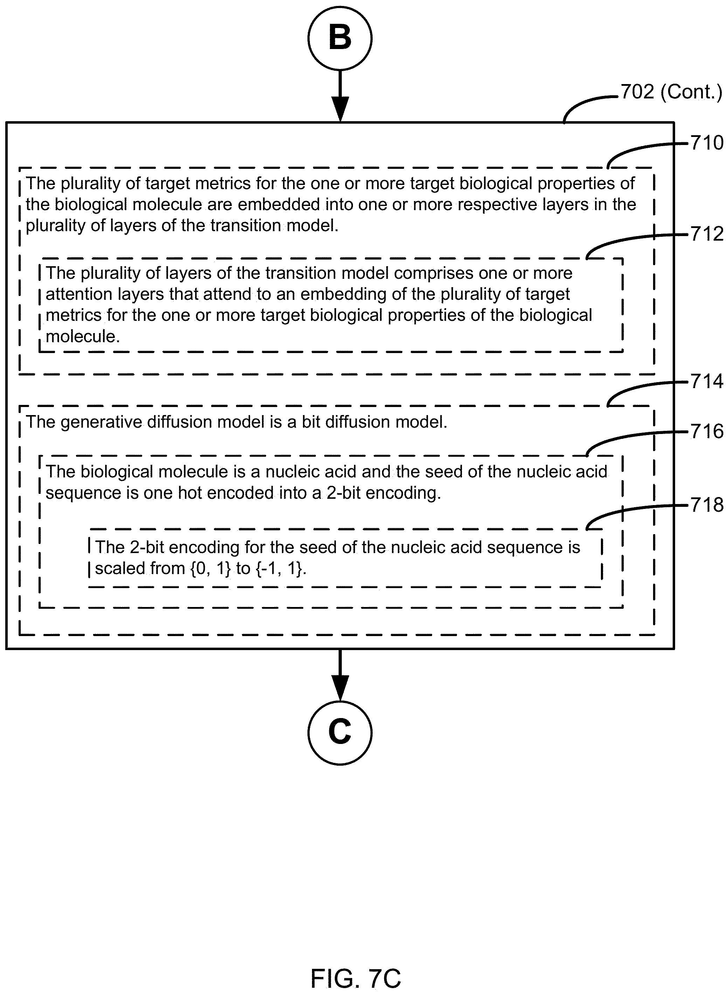

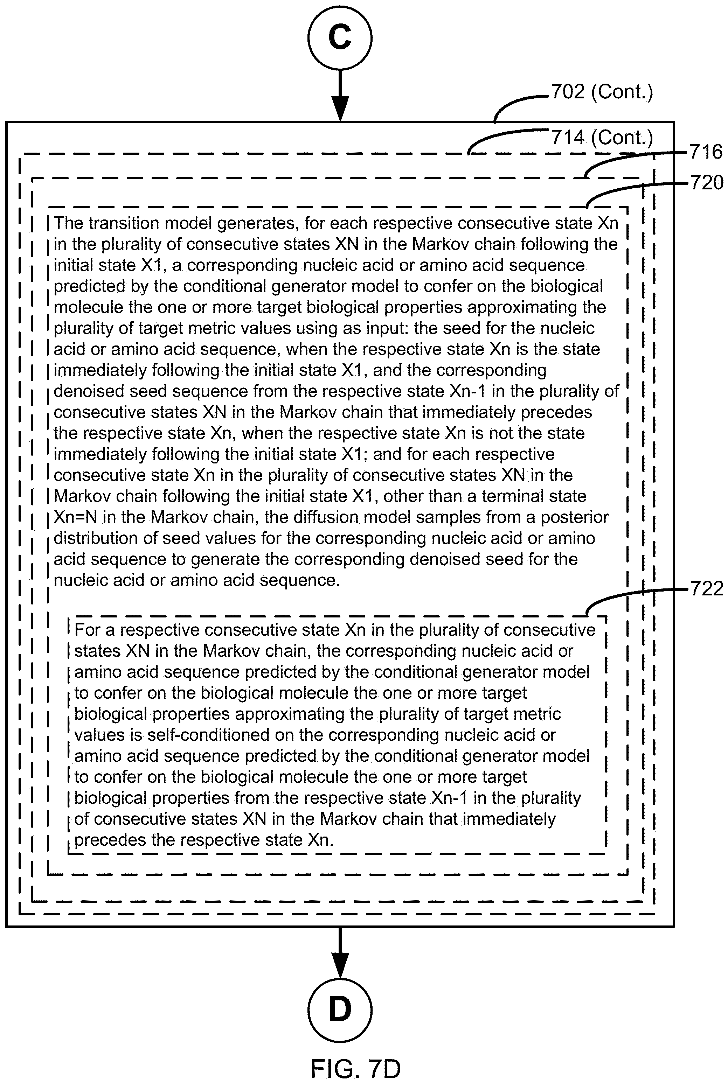

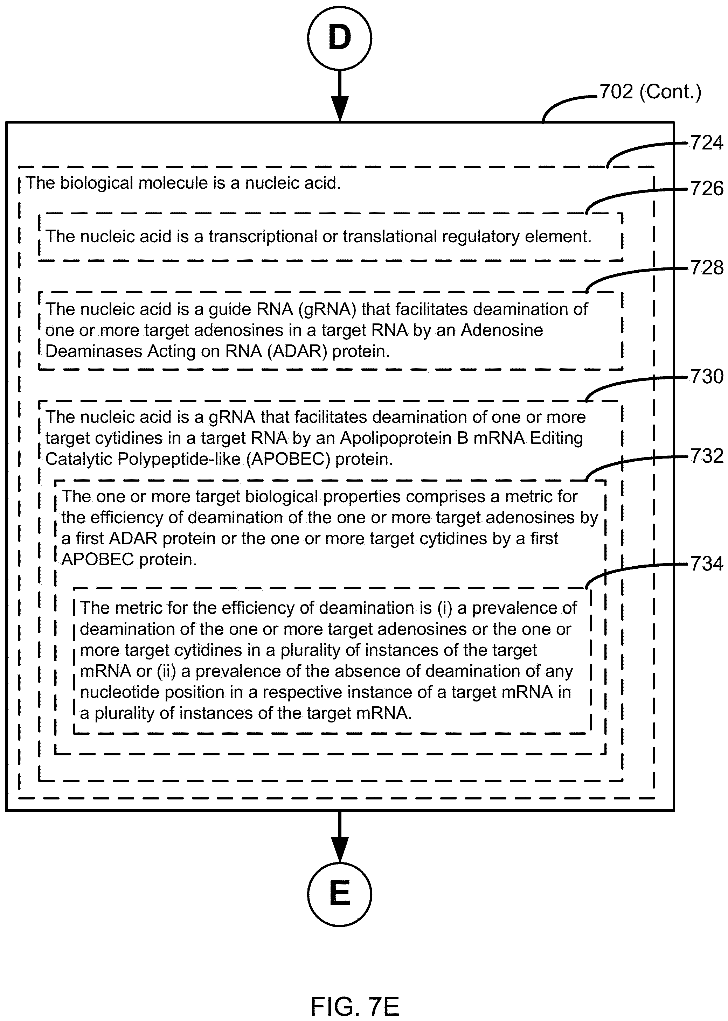

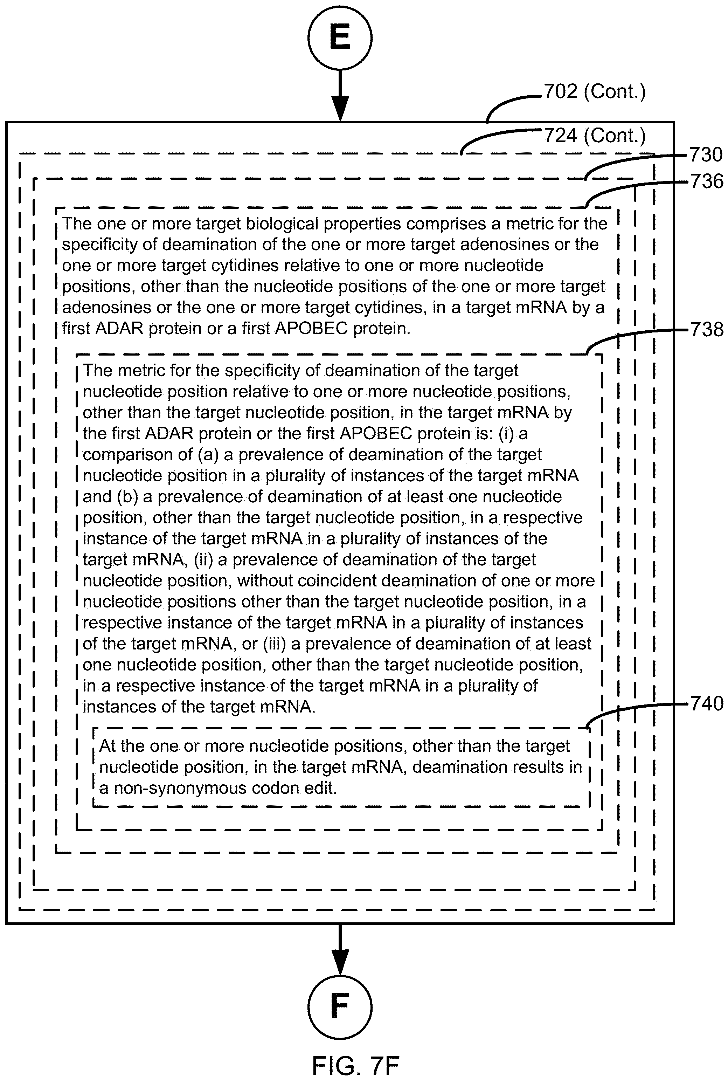

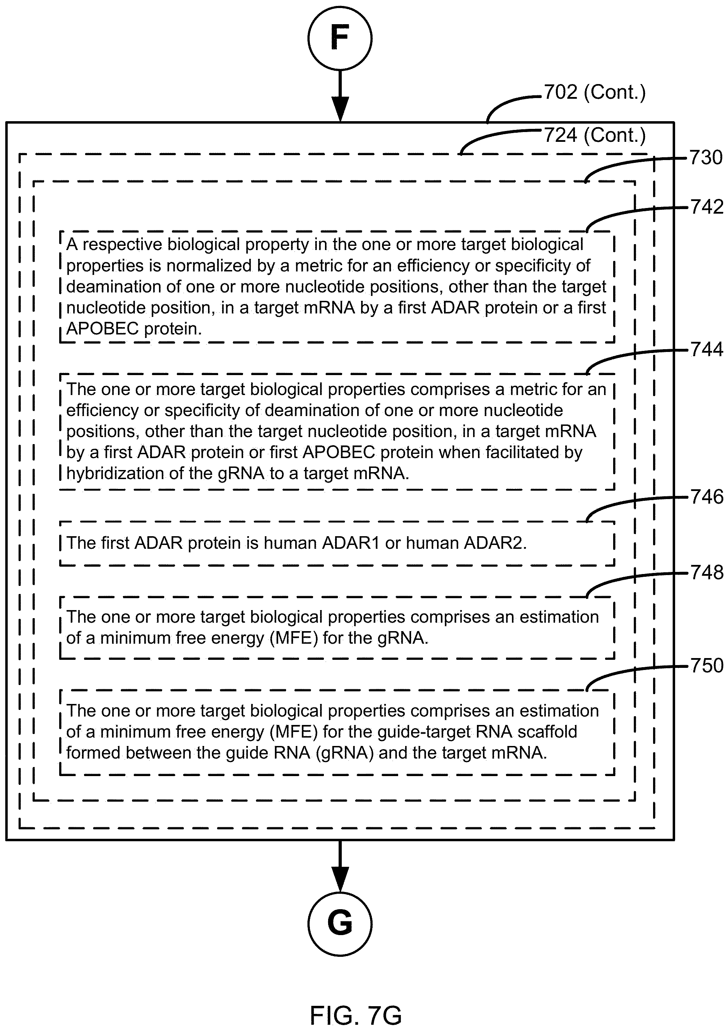

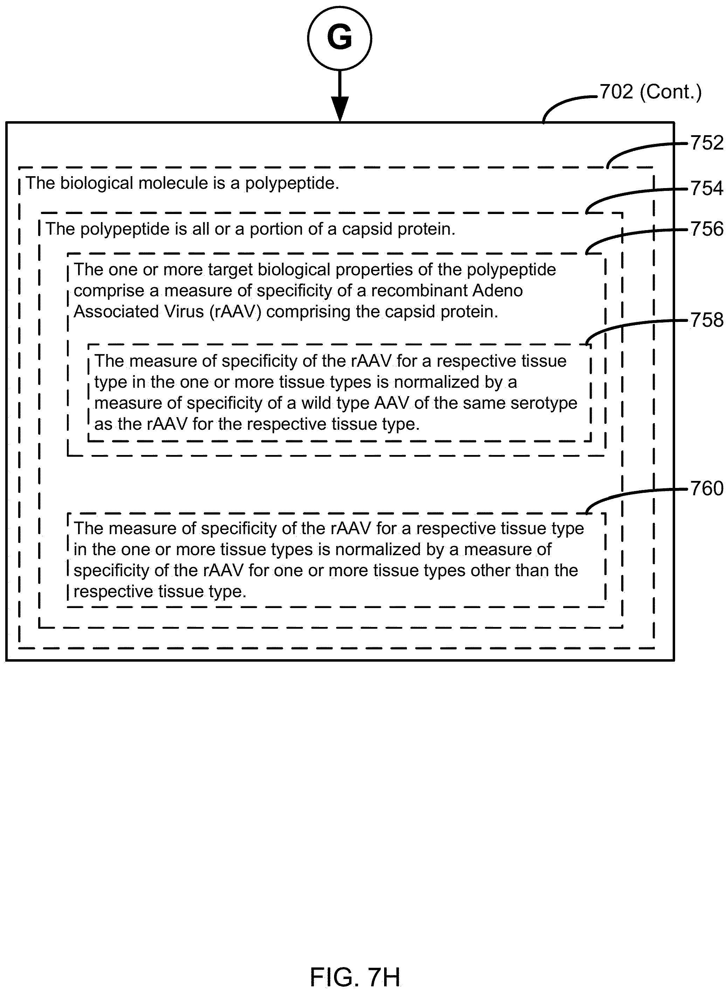

Now that a general topology of a system 500 has been described in accordance with various embodiments of the present disclosures, details regarding some processes in accordance with FIGS. 6 and 7A-H will be described.

FIGS. 6 and 7A-H illustrates flowcharts of methods (*e.g.*, method 600, method 700) for generating a polymer sequence for a biological molecule having one or more target biological properties, in accordance with embodiments of the present disclosure. Specifically, exemplary methods 600 and 700 for generating a polymer sequence for a biological molecule having one or more target biological properties are provided, in accordance with some embodiments of the present disclosure. In the flowcharts, the preferred parts of the methods are shown in solid line boxes, whereas optional variants of the methods, or optional equipment used by the methods, are shown in dashed line boxes. As such, FIGS.

6 and 7A-H illustrates methods for predicting a deamination efficiency or specificity.

Various modules in a memory of a computer system and/or a memory of a client device perform certain processes of the methods described in FIGS. 6 and 7A-H, unless expressly stated otherwise. Furthermore, it will be appreciated that the processes in FIGS. 6 and 7A-H can be encoded in a single module or any combination of modules.

In some aspects, the present disclosure harnesses the power of deep learning to create polymer sequences (*e.g.*, RNA sequences), for example for use in safe and efficient editing of a transcriptome of a subject (*e.g.*, a human subject). In some implementations, the generated polymer sequences, such as guide RNAs, can be or are used to treat, ameliorate, or fix genetic mutations in a subject. In some embodiments, a generated polymer sequence obtained as disclosed herein can be or is administered to a subject for use in gene therapy. For instance, as described above, delivery of DNA-encoded guide RNAs (gRNA) to recruit ADAR protein allows for programmable and precise RNA editing. ADAR is naturally a promiscuous enzyme with certain sequence editing preferences, but by screening millions of gRNAs, it is possible to learn the patterns and structures in RNA that specifically hone ADAR to edit a single site.

In some embodiments, the systems and methods disclosed herein utilize diffusion models and/or deep learning. For instance, generative models have been used to create entirely new images based on vast amounts of annotated data (*e.g.*, DALL-E2). Without being limited to any one theory of operation, in some embodiments, similar approaches utilize gRNA editing data to train generative deep learning models, treating RNA strands as tensors (*e.g.*, vectors, matrices, *etc.*) to design novel RNA "images." This approach allows for the exploration of a larger landscape of polymer sequences (*e.g.*, RNA sequences) and structural possibilities than ever before.

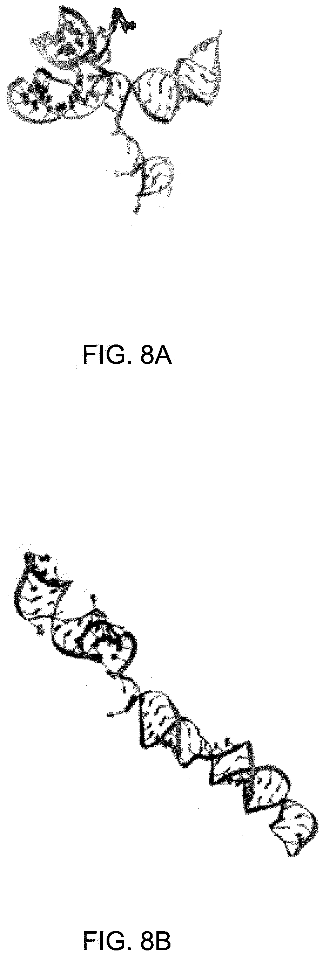

However, in contrast to many image generation tasks, the task of generating polymer sequences such as guide RNAs is often constrained by biological plausibility. For instance, many polymer sequence generation tasks focus on generating sequences that achieve highly efficient and specific performance (*e.g.*, RNA editing). Consider, for example, the denoising process illustrated in FIGS. 8A-B for a candidate gRNA targeting the LRRK2*G2019S mutation, which is causal for a subset of patients with Parkinson's disease. In some such implementations, a generative model is first trained on the relevant gRNA properties required to recruit ADAR to specifically correct that pathogenic mutation by randomly adding noise to the gRNAs thereby generating a seed (forward diffusion process). The model then performs a reverse diffusion process to remove the noise from the gRNA "image" conditioned on those optimal editing outcomes. To generate novel gRNAs, the reverse diffusion process is run on randomly generated "images" (*e.g.*, seeds). The performance of the generated polymer sequences is then experimentally tested, and such performance measures and/or generated polymer sequences can be fed back into the model for iterative training until a target performance is achieved.

With the overwhelming amount of high-throughput genomics data being generated today and the power of large language models for generative deep learning, the presently disclosed systems and methods provide avenues for major breakthroughs and improvements in the field of gene therapy.

Referring to block 600 in FIG. 6, one aspect of the present disclosure provides an example method for generating a polymer sequence for a biological molecule having one or more target biological properties. In some embodiments, the method is performed at a computer system comprising at least one processor and a memory storing at least one program for execution by the at least one processor.

Referring to block 602, in some embodiments, the method includes inputting (i) a plurality of target metric values for one or more target biological properties of a biological molecule and (ii) a seed for a nucleic acid or amino acid sequence for the biological molecule into a conditional generator model of a conditional generative adversarial network to obtain as output from the conditional generator model a nucleic acid or amino acid sequence for the biological molecule that is predicted by the conditional generator model to confer on the biological molecule the one or more target biological properties approximating the plurality of target metric values.

In some embodiments, the generator model comprises at least 10,000 parameters. In some embodiments, the generator model includes at least 1000, at least 10,000, at least 100,000, at least $1 \times 10^6$, at least $5 \times 10^6$, at least $1 \times 10^7$, at least $1 \times 10^8$, at least $1 \times 10^9$, or at least $1 \times 10^{10}$ parameters. In some embodiments, the generator model includes no more than $1 \times 10^{11}$, no more than $1 \times 10^{10}$, no more than $1 \times 10^9$, no more than $1 \times 10^8$, no more than $1 \times 10^7$, no more than $1 \times 10^6$, no more than 100,000, or no more than 10,000 parameters. In some embodiments, the generator model includes from 1000 to 100,000, from 50,000 to 500,000, from 100,000 to $5 \times 10^6$, from $1 \times 10^6$ to $1 \times 10^7$, from $1 \times 10^7$ to $1 \times 10^8$, from $1 \times 10^8$ to $1 \times 10^{10}$, or from $1 \times 10^7$ to $1 \times 10^{11}$ parameters. In some embodiments, the generator model includes another range of parameters starting no lower than 1000 parameters and ending no higher than $1 \times 10^{11}$ parameters.

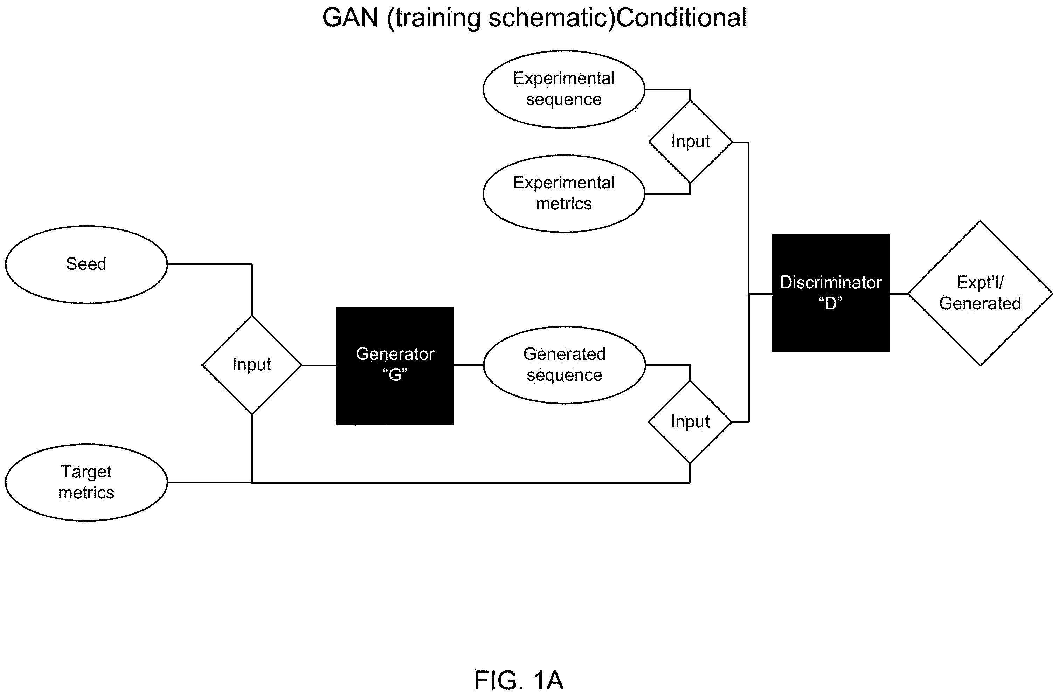

Conditional GANs are illustrated, for example, in FIGS. 1A-E.

In particular, as shown in FIG. 1A, the example schematic includes a conditional GAN model architecture used for training a generator "G" to generate novel gRNAs from seeds and target metric values for a biological molecule. Typically, during training, the generator G is inputted with (i) a seed for a nucleic acid or amino acid sequence ("seed") and (ii) target performance metrics ("target metrics"), and outputs a "generated" polymer sequence. The addition of target metrics is referred to as "conditioning" and applies additional constraints on the generator beyond simply generating sequences.

The "generated" sequence and its target metrics, along with an observed ("experimental") polymer sequence and its corresponding observed metrics, are inputted into the discriminator "D". In some embodiments, the discriminator is inputted with any number of experimental and/or generated samples to classify. In some embodiments, the discriminator is inputting with one experimental sample and one generated sample for classification. The discriminator attempts to accurately determine whether a given sample is experimental or generated.

Training is performed in an adversarial manner, for instance, as a zero-sum game. In other words, when the discriminator successfully identifies real (*e.g.*, experimental) and fake (*e.g.*, generated) samples, it is rewarded or no change is needed to the model parameters, whereas the generator is penalized with large updates to model parameters. Alternately, when the generator succeeds in generating samples that the discriminator cannot distinguish from experimental samples, it is rewarded, or no change is needed to the model parameters, but the discriminator is penalized and its model parameters are updated. At a limit, the generator generates perfect replicas from the input domain every time, and the discriminator cannot tell the difference and predicts "unsure" (*e.g.*, 50% for experimental and generated) in every case. While this is an example of an idealized case, in some embodiments, the conditional GAN is trained to a less than 50% classification performance for experimental and/or generated samples. *See,* for example, Brownlee, "A Gentle Introduction to Generative Adversarial Networks (GANs)," 2019, available on the Internet at machinelearningmastery.com/what-are-generative-adversarial-networks-gans.

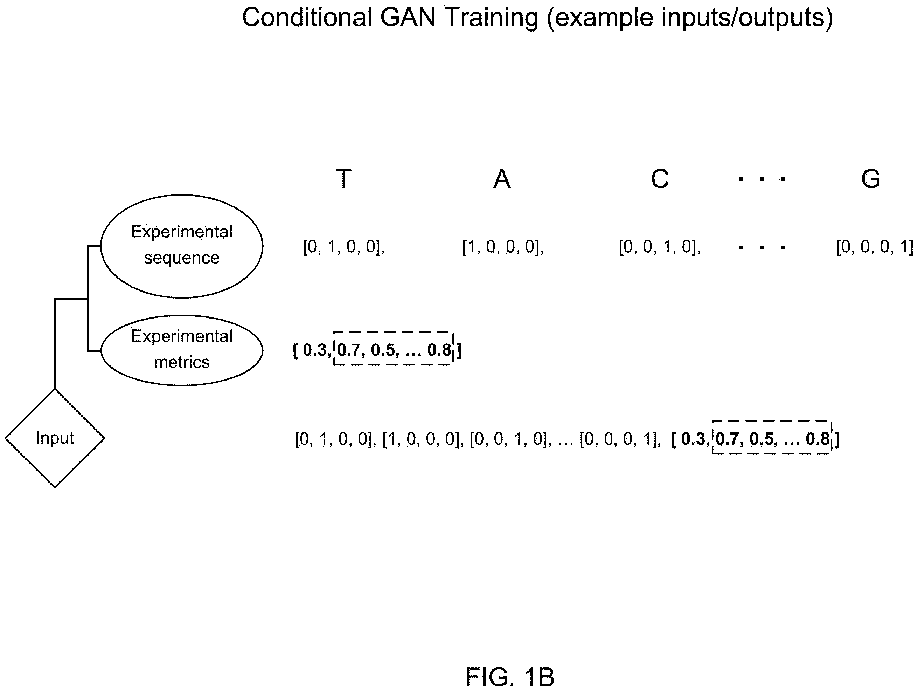

In some embodiments, the inputted sequences (whether as a seed, a generated sequence, or an experimental sequence), in some embodiments, are provided as a tensor (*e.g.*, vector, matrix, *etc.*) representation, optionally with noise added. In some embodiments, the inputted sequences are encoded or dimension reduced, as illustrated in FIGS. 1B-C.

For instance, in some embodiments, sequences are converted to one hot-encoded tensors (*e.g.*, vectors, matrices, *etc.*). Particularly, as an example, an experimental or generated sequence can be represented as one hot-encoded, in which each residue (*e.g.*, nucleic acid and/or amino acid) position is represented as a matrix having hard-coded values that indicate the identity of the residue at the position (*e.g.*, if the residue identity at the respective position is a nucleotide identity of A, then the matrix will have a "1" value at an matrix position for A, and a "0" value at all other matrix positions for T, C, and G). A seed can be represented at any level of complexity, including but not limited to (i) a tensor containing matrices and/or vectors representing randomized one hot-encoded residues (*e.g.*, TACG, AAAA, *etc.,* for nucleic acid sequences), (ii) a tensor representing a diffused sequence, containing matrices and/or vectors having decimal values adding up to 1, or (iii) a single value that the generator expands out to a tensor representing each position of a generated guide. In the example input/output figure of FIGS. 1B-C, the seed is shown as (ii) a tensor representing a diffused sequence, in which each nucleotide position is represented as a matrix and/or vector containing decimal values adding up to 1, rather than one hot-encoded residue identities at each position. Other implementations are possible, as will be apparent to one skilled in the art. For example, in some embodiments, each residue is represented by a vector with an element for each of A, T, C and G and with each element, indicating the presence (one) or absence (zero) of the respective residue type. In some such embodiments, a sequence of residue positions is represented as a matrix in which the vectors for each residue are stacked, so that one dimension of the matrix indicates residue type, and the other dimension indicates residue position.

In some embodiments, noise is added to a polymer sequence, *e.g.,* a known gRNA or variant capsid protein sequence, a random nucleic acid or amino acid sequence, or a consensus-generated nucleic acid or amino acid sequence, to form a seed for input. For instance, in some embodiments, the seed is generated by adding Gaussian noise to a polymer sequence. In some embodiments, the seed is a partially diffused, one-hot encoded polymer sequence, where each respective residue (*e.g.*, nucleotide and/or amino acid) position is a represented as a tensor (*e.g.*, a matrix or vector) having a corresponding partial value for each possible residue, *e.g.*, "A," "C," "G," and "T/U." While in nature, a single residue position cannot have the partial properties of different residue identities, it is possible in silico to model the contributions of different residue at the same position such that a single residue position can be represented by such partial properties provided by different residues.

In some embodiments, the target metrics shown in the training and use schematics are for an efficiency or specificity of deamination. For example, in some embodiments, the target metrics comprise one or more of on-target editing, specificity, target only, normalized specificity, no editing, ADAR preference, *etc.,* for one or more ADAR proteins, tissue tropism, and/or biochemical properties. In FIGS. 1B-C, the target metrics are shown as a vector including values for the one or more metrics.

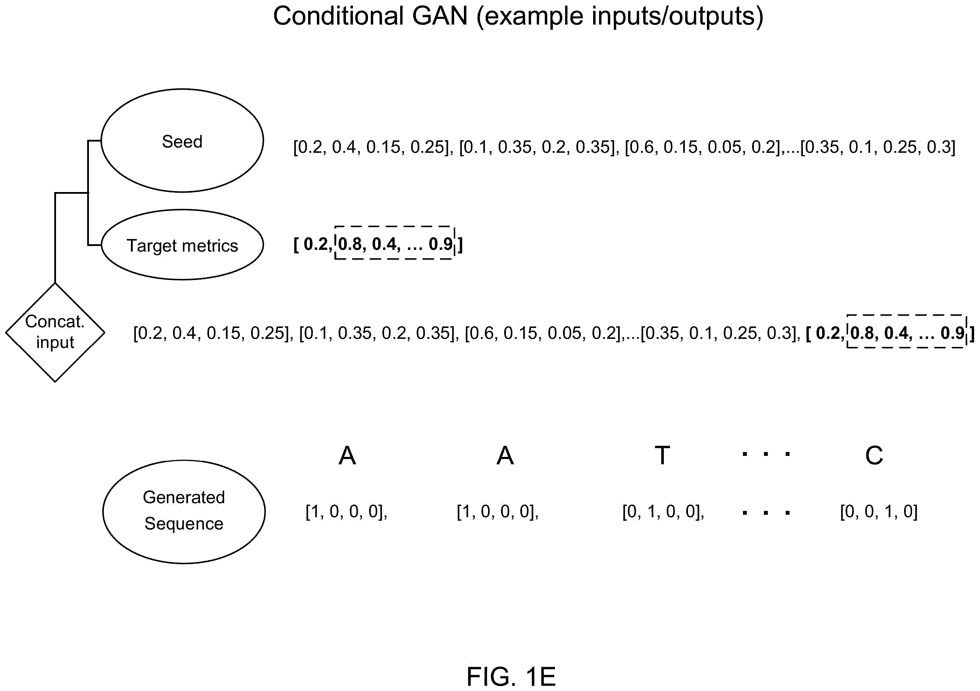

In some cases, the seed and the target metrics are concatenated prior to input to the generator ("Concat. input"), as shown in FIG. 1E. This includes, in some implementations, the tensor that represents the seed as a vector of values, to which the vector of target metrics is concatenated.

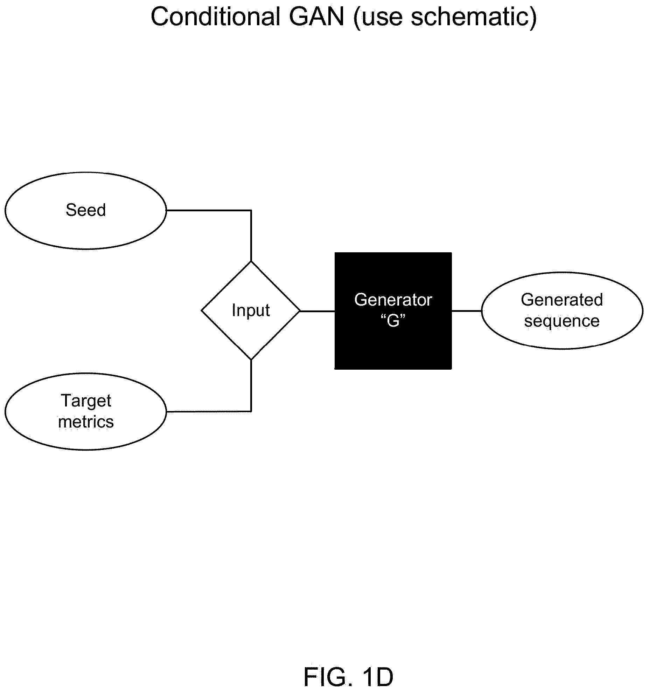

Returning to the schematic in FIG. 1D, once a target performance (*e.g.*, 50% or less classification) is met, the discriminator is discarded, and the trained generator is retained for generation of novel polymer sequences from seeds (*e.g.*, for a nucleic acid or amino acid sequence) and target metrics. In an example use schematic of the trained generator shown in FIG. 1D, a novel seed and target metrics is inputted (optionally in concatenated form, as described above), and the trained generator outputs a generated polymer sequence.

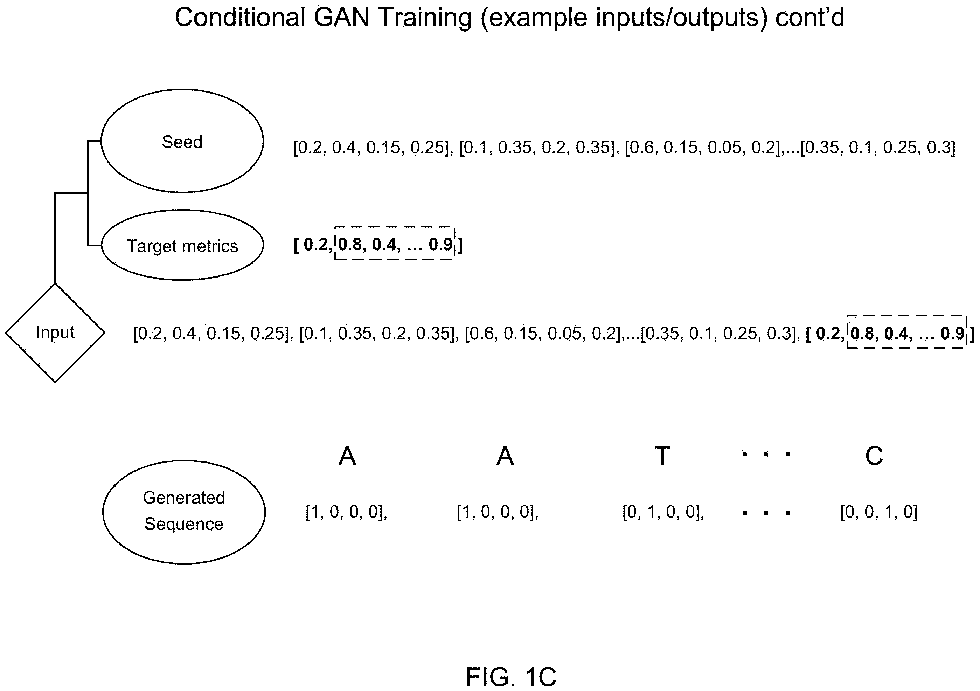

In some embodiments, a generative model described herein, e.g., models described in conjunction with methods 600 and 700 and/or with respect to **FIGs. 1-5** and/or **23,** considers only natural nucleotides at each position of a polynucleotide sequence, *e.g.*, a gRNA. For example, in some embodiments, a generative model described herein only allows for adenine (A), cytosine (C), guanine (G), and thymine (T) / uracil (U). Accordingly, in some embodiments, a generative model described herein allows for any of these four nucleotides to be present at any position in the generated polynucleotide sequence.

In some embodiments, the generative model is applied such that one or more nucleotide positions in a polynucleotide sequence is limited to only a subset of possible nucleotides. For example, in some embodiments, one or more position in a polynucleotide sequence being generated is fixed as a predetermined nucleotide. In some embodiments, one or more nucleotide positions in a polynucleotide is limited to 1, 2, or 3 possible nucleotides. Said another way, in some embodiments, one or more possible nucleotides is excluded as a possibility at one or more positions of the amino acid sequence being generated. For example, in some embodiments, uracil is excluded as a possible nucleotide at a position of an ADAR gRNA across from a target adenosine residue.

In some embodiments, a generative model described herein also allows for modified nucleotides to be present at one or more (*e.g.*, all) position of the generated polynucleotide sequence. For example, in some embodiments, a generative model allows for a 2'-O-methyl (2'-O-Me) base in place of, or in addition to, an unmodified nucleotide of the same base at one or more positions in the polynucleotide sequence.

Exemplary chemical modifications comprise any one of: 5' adenylate, 5' guanosine-triphosphate cap, 5' N7-Methylguanosine-triphosphate cap, 5' triphosphate cap, 3' phosphate, 3'thiophosphate, 5 phosphate, 5 thiophosphate, Cis-Syn thymidine dimer, trimers, C12 spacer, C3 spacer, C6 spacer, dSpacer, PC spacer, rSpacer, Spacer 18, Spacer 9,3'-3' modifications, 5'-5' modifications, abasic, acridine, azobenzene, biotin, biotin BB, biotin TEG, cholesteryl TEG, desthiobiotin TEG, DNP TEG, DNP-X, DOTA, dT-Biotin, dual biotin, PC biotin, psoralen C2, psoralen C6, TINA, 3'DABCYL, black hole quencher 1, black hole quencher 2, DABCYL SE, dT-DABCYL, IRDye QC-1, QSY-21, QSY-35, QSY-7, QSY-9, carboxyl linker, thiol linkers, 2'deoxyribonucleoside analog purine, 2'deoxyribonucleoside analog pyrimidine, ribonucleoside analog, 2'-O-methyl ribonucleoside analog, sugar modified analogs, wobble/universal bases, fluorescent dye label, 2'fluoro RNA, 2'O-methyl RNA, methylphosphonate, phosphodiester DNA, phosphodiester RNA, phosphothioate DNA, phosphorothioate RNA, UNA, pseudouridine-5'-triphosphate, 5-methylcytidine-5'-triphosphate, 2-O-methyl 3phosphorothioate or any combinations thereof.

A chemical modification can be made at any location of the engineered guide RNA. In some cases, a modification may be located in a 5' or 3' end. In some cases, a polynucleotide comprises a modification at a base selected from: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, or 150. More than one modification can be made to the engineered guide RNA. In some cases, a modification can be permanent. In other cases, a modification can be transient. In some cases, multiple modifications may be made to the engineered guide RNA. the engineered guide RNA modification can alter physio-chemical properties of a nucleotide, such as their conformation, polarity, hydrophobicity, chemical reactivity, base-pairing interactions, or any combination thereof.

A chemical modification can also be a phosphorothioate substitute. In some cases, a natural phosphodiester bond can be susceptible to rapid degradation by cellular nucleases and; a modification of internucleotide linkage using phosphorothioate (PS) bond substitutes can be more stable towards hydrolysis by cellular degradation. A modification can increase stability in a polynucleic acid. A modification can also enhance biological activity. In some cases, a phosphorothioate enhanced RNA polynucleic acid can inhibit RNase A, RNase T1, calf serum nucleases, or any combinations thereof. These properties can allow the use of PS-RNA polynucleic acids to be used in applications where exposure to nucleases may be of high probability in vivo or in vitro. For example, phosphorothioate (PS) bonds can be introduced between the last 3-5 nucleotides at the 5'-or 3'-end of a polynucleic acid which can inhibit exonuclease degradation. In some cases, phosphorothioate bonds can be added throughout an entire polynucleic acid to reduce attack by endonucleases.

In some embodiments, chemical modification can occur at 3'OH, group, 5'OH group, at the backbone, at the sugar component, or at the nucleotide base. Chemical modification can include non-naturally occurring linker molecules of interstrand or intrastrand cross links. In one aspect, the chemically modified nucleic acid comprises modification of one or more of the 3'OH or 5'OH group, the backbone, the sugar component, or the nucleotide base, or addition of non-naturally occurring linker molecules. In some embodiments, chemically modified backbone comprises a backbone other than a phosphodiester backbone. In some embodiments, a modified sugar comprises a sugar other than deoxyribose (in modified DNA) or other than ribose (modified RNA). In some embodiments, a modified base comprises a base other than adenine, guanine, cytosine, thymine or uracil. In some embodiments, the engineered guide RNA comprises at least one chemically modified base. In some instances, the engineered guide RNA comprises 2, 3, 4, 5, 6, 7, 8, 9, 10, 15, 20, or more modified bases. In some cases, chemical modifications to the base moiety include natural and synthetic modifications of adenine, guanine, cytosine, thymine, or uracil, and purine or pyrimidine bases.

In some embodiments, the at least one chemical modification of the engineered guide RNA comprises a modification of any one of or any combination of: modification of one or both of the non-linking phosphate oxygens in the phosphodiester backbone linkage; modification of one or more of the linking phosphate oxygens in the phosphodiester backbone linkage; modification of a constituent of the ribose sugar; Replacement of the phosphate moiety with "dephospho" linkers; modification or replacement of a naturally occurring nucleobase; modification of the ribose-phosphate backbone; modification of 5' end of polynucleotide; modification of 3' end of polynucleotide; modification of the deoxyribose phosphate backbone; substitution of the phosphate group; modification of the ribophosphate backbone; modifications to the sugar of a nucleotide; modifications to the base of a nucleotide; or stereopure of nucleotide. Chemical modifications to the engineered guide RNA include any modification contained herein, while some exemplary modifications are recited in **Table 2.**

**Table 2. Exemplary Chemical Modification**

| Modification of engineered guide RNA | Examples |
|---|---|
| Modification of one or both of the non-linking phosphate oxygens in the phosphodiester backbone linkage | sulfur (S), selenium (Se), BR3 (wherein R can be, *e.g.*, hydrogen, alkyl, or aryl), C (*e.g.*, an alkyl group, an aryl group, and the like), H, NR2, wherein R can be, *e.g.*, hydrogen, alkyl, or aryl, or wherein R can be, *e.g.*, alkyl or aryl |
| Modification of one or more of the linking phosphate oxygens in the phosphodiester backbone linkage | sulfur (S), selenium (Se), BR3 (wherein R can be, *e.g.*, hydrogen, alkyl, or aryl), C (*e.g.*, an alkyl group, an aryl group, and the like), H, NR2, wherein R can be, *e.g.*, hydrogen, alkyl, or aryl, or wherein R can be, *e.g.*, alkyl or aryl |
| Replacement of the phosphate moiety with "dephospho" linkers | methyl phosphonate, hydroxylamino, siloxane, carbonate, carboxymethyl, carbamate, amide, thioether, ethylene oxide linker, sulfonate, sulfonamide, thioformacetal, formacetal, oxime, methyleneimino, methylenemethylimino, methylenehydrazo, methylenedimethylhydrazo, or methyleneoxymethylimino |
| Modification or replacement of a naturally occurring nucleobase | Nucleic acid analog (examples of nucleotide analogs can be found in PCT/US2015/025175, PCT/US2014/050423, PCT/US2016/067353, PCT/US2018/041503, PCT/US18/041509, PCT/US2004/011786, or PCT/US2004/011833, all of which are expressly incorporated by reference in their entireties |
| Modification of the ribose-phosphate backbone | phosphorothioate, phosphonothioacetate, phosphoroselenates, boranophosphates, borano phosphate esters, hydrogen phosphonates, phosphonocarboxylate, phosphoroamidates, alkyl or aryl phosphonates, phosphonoacetate, or phosphotriesters |
| Modification of 5' end of polynucleotide | 5' cap or modification of 5' cap -OH |
| Modification of 3' end of polynucleotide | 3' tail or modification of 3' end -OH |
| Modification of the deoxyribose phosphate backbone | phosphorothioate, phosphonothioacetate, phosphoroselenates, borano phosphates, borano phosphate esters, hydrogen phosphonates, phosphoroamidates, alkyl or aryl phosphonates, or phosphotriesters |
| Substitution of the phosphate group | methyl phosphonate, hydroxylamino, siloxane, carbonate, carboxymethyl, carbamate, amide, thioether, ethylene oxide linker, sulfonate, sulfonamide, thioformacetal, formacetal, oxime, methyleneimino, methylenemethylimino, methylenehydrazo, methylenedimethylhydrazo, or methyleneoxymethylimino. |
| Modification of the ribophosphate backbone | morpholino, cyclobutyl, pyrrolidine, or peptide nucleic acid (PNA) nucleoside surrogates |
| Modifications to the sugar of a nucleotide | Locked nucleic acid (LNA), unlocked nucleic acid (UNA), or bridged nucleic acid (BNA) |
| Modification of a constituent of the ribose sugar | 2'-O-methyl, 2'-O-methoxy-ethyl (2'-MOE), 2'-fluoro, 2'-aminoethyl, 2'-deoxy-2'-fuloarabinou-cleic acid, 2'-deoxy, 2'-O-methyl, 3'-phosphorothioate, 3'-phosphonoacetate (PACE), or 3'-phosphonothioacetate (thioPACE) |
| Modifications to the base of a nucleotide | Modification of A, T, C, G, or U |
| Stereopure of nucleotide | S conformation of phosphorothioate or R conformation of phosphorothioate |

In some embodiments, one or more nucleotide modifications are used to enhance the properties of a polynucleotide. For example, certain 2'-O modifications are known to stabilize polynucleotides in vivo. Moreover, specific patterns of nucleotide modifications are used in certain therapeutic polynucleotides, *e.g.*, to stabilize the polynucleotide in vivo. Accordingly, in some embodiments, the model allows for a predetermined nucleotide modification pattern in the generated nucleic acid sequence. In some embodiments, the model restricts to generated nucleic acid sequence to a predetermined nucleotide modification pattern. Examples of nucleotide modifications patterns that have been used in conjunction with therapeutic polynucleotides, such as ADAR gRNA, are disclosed in US10988763; US10941402; EP3507366; US20200199586; EP3712269; WO2021071858; WO2021243023; EP2852668; EP3103872; US9340784; and US9796976, the contents of which are hereby incorporated by reference herein, in their entireties.

In some embodiments, the methods and systems described herein relate to an amino acid sequence corresponding to amino acids 581-589 of the wild type AAV5 VP1 protein (ATGTYNLQE: SEQ ID NO:31), and having at least one amino acid substitution compared to SEQ ID NO: 31, that confers increased CNS tropism to a recombinant AAV when the amino acid sequence is integrated into a capsid protein as compared the CNS tropism of an AAV when amino acids 581-589 of the wild type AAV5 VP1 protein (SEQ ID NO: 31) are integrated into the same capsid protein. In some embodiments, the methods and systems described herein relate to an amino acid sequence corresponding to amino acids 581-589 of the wild type AAV5 VP1 protein (SEQ ID NO:31), and having at least one amino acid substitution compared to SEQ ID NO: 31, that confers increased detargeting of a non-CNS tissue (*e.g.*, liver) to a recombinant AAV when the amino acid sequence is integrated into a capsid protein as compared to the detargeting of the non-CNS tissue by an AAV when amino acids 581-589 of the wild type AAV5 VP1 protein (SEQ ID NO:31) are integrated into the same capsid protein. In some embodiments, the methods and systems described herein relate to an amino acid sequence corresponding to amino acids 581-589 of the wild type AAV5 VP1 protein (SEQ ID NO:31), and having at least one amino acid substitution compared to SEQ ID NO: 31, that confers both increased CNS tropism and increased detargeting of a non-CNS tissue (*e.g.*, liver) to a recombinant AAV when the amino acid sequence is integrated into a capsid protein as compared the CNS tropism and detargeting of an AAV when amino acids 581-589 of the wild type AAV5 VP1 protein (SEQ ID NO:31) are integrated into the same capsid protein.

**Table 1.** Example amino acid sequences corresponding to amino acids 581-589 of the wild type AAV5 VP1 protein and having at least one amino acid substitution compared to SEQ ID NO: 31.

| Capsid | SEQ ID NO | Criteria |
|---|---|---|
| **TANVYRSGQ** | SEQ ID NO: 1 | abundance_enriched |
| **QTAEFKRHQ** | SEQ ID NO: 2 | abundance_enriched |
| **DAWCFISSY** | SEQ ID NO: 3 | ML_observed |
| **DAWLQLKDN** | SEQ ID NO: 4 | ML_observed |
| **EAWMYHQFH** | SEQ ID NO: 5 | ML_observed |
| **DAWSYQCYH** | SEQ ID NO: 6 | ML_observed |
| **DAWQMLSGN** | SEQ ID NO: 7 | ML_observed |
| **ESWMKLEWQ** | SEQ ID NO: 8 | ML_observed |
| **DAWSVLQDK** | SEQ ID NO: 9 | ML_observed |
| **ICMSRTQMK** | SEQ ID NO: 10 | ML_observed |
| **YWQSMLWQG** | SEQ ID NO: 11 | ML_observed |
| **MDDICEFYA** | SEQ ID NO: 12 | multiple_nhp |
| **TSHYITFTP** | SEQ ID NO: 13 | multiple_nhp |
| **CATRFNIGG** | SEQ ID NO: 14 | multiple_nhp |
| **AEDYWDLGA** | SEQ ID NO: 15 | multiple_nhp |
| **DAWMMMWGS** | SEQ NO: 16 | multiple_nhp |
| **TAAFAYKYE** | SEQ ID NO: 17 | multiple_samples |
| **LRTPHNHGL** | SEQ ID NO: 18 | multiple_samples |
| **YSGVRVTGY** | SEQ ID NO: 19 | multiple_samples |

(continued)

| Capsid | SEQ ID NO | Criteria |
|---|---|---|
| AAGRFNYFG | SEQ ID NO: 20 | sequence_similarity |
| EAWSKLEQP | SEQ ID NO: 21 | sequence_similarity |
| LWGWTLQHQ | SEQ ID NO: 22 | sequence_similarity |
| LVGWTLQHV | SEQ ID NO: 23 | sequence_similarity |
| TAGRFNYFD | SEQ ID NO: 24 | sequence_similarity |
| QKGPHNMSE | SEQ ID NO: 25 | sequence_similarity |
| DSASPIMGM | SEQ ID NO: 26 | sequence_similarity |
| EEEWLNGWQ | SEQ ID NO: 27 | sequence_similarity |
| DARVYRALD | SEQ ID NO: 28 | tissue_enriched |
| QALMLTRQQ | SEQ ID NO: 29 | tissue_enriched |
| ITLASKSMR | SEQ ID NO: 30 | tissue_enriched |

In some embodiments, the partial amino acid sequence of the variant capsid protein is selected from those sequences listed in Table 1. In some embodiments, the partial amino acid sequence of the variant capsid protein is selected from those sequences listed in Table 1 having the abundance_enriched criteria, wherein the abundance_enriched criteria are met by capsids that are observed at a greater frequency within CNS versus non-CNS tissues as defined by read count data. In some embodiments, the partial amino acid sequence of the variant capsid protein is selected from those sequences listed in Table 1 having the ML_observed criteria, wherein the ML_observed criteria is met by capsids that have the highest likelihood of targeting the CNS as predicted by supervised learning models described herein. In some embodiments, the partial amino acid sequence of the variant capsid protein is selected from those sequences listed in Table 1 having the multiple_nhp criteria, wherein the multiple_nhp criteria is met by capsids that are observed within the CNS of more than one non-human primate (NHP), in which the capsids are only seen in CNS (not seen in non-CNS tissue). In some embodiments, the partial amino acid sequence of the variant capsid protein is selected from those sequences listed in Table 1 having the multiple_samples criteria, wherein the multiple_samples criteria are met by capsids that are observed in multiple samples (samples within same NHP or samples between different NHPs) and only seen in CNS (not seen in non-CNS tissue). In some embodiments, the partial amino acid sequence of the variant capsid protein is selected from those sequences listed in Table 1 having the sequence_similarity criteria, wherein the sequence_similarity criteria are met by capsids that have a low edit distance between their variant sequence. In some embodiments, the partial amino acid sequence of the variant capsid protein is selected from those sequences listed in Table 1 having the tissue_enriched criteria, wherein the tissue_enriched criteria are met by capsids that are observed in a greater fraction of CNS tissues versus non-CNS tissues.

In some embodiments, the seed for the amino acid sequence has at least 70% sequence identity to a sequence listed in Table 1. In some embodiments, the seed for the amino acid sequence has at least 85% sequence identity to a sequence listed in Table 1. In some embodiments, the seed for the amino acid sequence has at least 70% or at least 85% sequence identity to a sequence listed in Table 1 having the abundance_enriched criteria. In some embodiments, the seed for the amino acid sequence has at least 70% or at least 85% sequence identity to a sequence listed in Table 1 having the ML_observed criteria. In some embodiments, the seed for the amino acid sequence has at least 70% or at least 85% sequence identity to a sequence listed in Table 1 having the multiple_nhp criteria. In some embodiments, the seed for the amino acid sequence has at least 70% or at least 85% sequence identity to a sequence listed in Table 1 having the multiple_samples criteria. In some embodiments, the seed for the amino acid sequence has at least 70% or at least 85% sequence identity to a sequence listed in Table 1 having the sequence_similarity criteria. In some embodiments, the seed for the amino acid sequence has at least 70% or at least 85% sequence identity to a sequence listed in Table 1 having the tissue_enriched criteria.

In some embodiments, the seed for the amino acid sequence is generated by modifying an amino acid sequence selected from those sequences listed in Table 1. In some embodiments, the seed for the amino acid sequence is generated by modifying an amino acid sequence selected from those sequences listed in Table 1. In some embodiments, the seed for the amino acid sequence is generated by modifying an amino acid sequence selected from those sequences listed in Table 1 having the ML_observed criteria. In some embodiments, the seed for the amino acid sequence is generated by modifying an amino acid sequence selected from those sequences listed in Table 1 having the multiple_nhp criteria. In some embodiments, the seed for the amino acid sequence is generated by modifying an amino acid sequence selected from those sequences listed in Table 1 having the multiple_samples criteria. In some embodiments, the seed for the amino acid sequence is generated by modifying an amino acid sequence selected from those sequences listed in Table 1 having the sequence_similarity criteria. In some embodiments, the seed for the amino acid sequence is generated by modifying an amino acid sequence selected from those sequences listed in Table 1 having the tissue_enriched criteria.

In some embodiments, the modifying comprises manually inputting a change to the amino acid sequence. In some embodiments, the modifying comprises systematically changing the amino acid sequence. In some embodiments, the modifying comprises randomly changing the amino acid sequence.

In some embodiments, the modifying comprises applying a gaussian noise filter to the amino acid sequence to generate a distribution of probabilities for the identity of one or more amino acid residues in the amino acid sequence.

In some embodiments, the seed for the amino acid sequence comprises, for each respective amino acid position in the amino acid sequence, a corresponding probability, for each respective amino acid in a plurality of amino acids, of the respective amino acid being present at the respective amino acid position.

In some embodiments, the amino acid sequence generated using the model is selected from those sequences listed in Table 1. In some embodiments, the amino acid sequence generated using the model is selected from those sequences listed in Table 1 having the abundance_enriched criteria. In some embodiments, the amino acid sequence generated using the model is selected from those sequences listed in Table 1 having the ML_observed criteria. In some embodiments, the amino acid sequence generated using the model is selected from those sequences listed in Table 1 having the multiple_nhp criteria. In some embodiments, the amino acid sequence generated using the model is selected from those sequences listed in Table 1 having the multiple_samples criteria. In some embodiments, the amino acid sequence generated using the model is selected from those sequences listed in Table 1 having the sequence_similarity criteria. In some embodiments, the amino acid sequence generated using the model is selected from those sequences listed in Table 1 having the tissue_enriched criteria.

In some embodiments, the amino acid sequence generated using the model has at least 70% sequence identity to a sequence listed in Table 1. In some embodiments, the amino acid sequence generated using the model has at least 85% sequence identity to a sequence listed in Table 1. In some embodiments, the amino acid sequence generated using the model has at least 70% or at least 85% sequence identity to a sequence listed in Table 1 having the abundance_enriched criteria. In some embodiments, the amino acid sequence generated using the model has at least 70% or at least 85% sequence identity to a sequence listed in Table 1 having the ML_observed criteria. In some embodiments, the amino acid sequence generated using the model has at least 70% or at least 85% sequence identity to a sequence listed in Table 1 having the multiple_nhp criteria. In some embodiments, the amino acid sequence generated using the model has at least 70% or at least 85% sequence identity to a sequence listed in Table 1 having the multiple_samples criteria. In some embodiments, the amino acid sequence generated using the model has at least 70% or at least 85% sequence identity to a sequence listed in Table 1 having the sequence_similarity criteria. In some embodiments, the amino acid sequence generated using the model has at least 70% or at least 85% sequence identity to a sequence listed in Table 1 having the tissue_enriched criteria.

In some embodiments, the method further comprises assembling a rAAV comprising the respective variant capsid protein having the candidate sequence and a nucleic acid.

In some embodiments, the method further comprises treating a disorder in a subject in need thereof by administering a rAAV comprising the respective variant capsid protein having the candidate sequence and a therapeutic nucleic acid to the subject.

In some embodiments, the biological molecule is a polynucleotide and the seed of the amino acid sequence is binary encoded. In some embodiments, the seed of the amino acid sequence is one hot encoded. Alternatively or additionally, in some embodiments, the seed of the amino acid sequence is encoded as discrete values. In some embodiments, the seed of the amino acid sequence is encoded using continuous values (*e.g.*, as a probability, ratio, fraction, or likelihood).

In some embodiments, the seed of the amino acid sequence is bit encoded for input into the model. In some embodiments, the encoding is a 5-bit encoding. In some embodiments, the encoding is a 6-bit encoding, 7-bit encoding, 8-bit encoding, or higher bit encoding. For example, 5-bit encoding allows for binary encoding with 32 different characters, {0,0,0,0,0} through {1, 1, 1, 1, 1}. As such, each natural amino acid can be encoded with a different character. In some embodiments, the amino acids are assigned to different characters without any particular considerations, *e.g.*, randomly assigned or assigned alphabetically. In some embodiments, one or more characteristics are considered when assigning amino acids to characters. For example, in some embodiments, amino acids having similar biophysical properties are assigned to characters sharing positional values. For example, the encoding shown in Table 2 takes biophysical characteristics of the natural amino acids into consideration.

Table 2 - Example 5-bit encoding of the
natural amino acids.

| Amino Acid | 5-bit Character |
|---|---|
| Ala | 00001 |
| Arg | 10000 |
| Asn | 01000 |
| Asp | 01001 |
| Cys | 00100 |
| Gln | 01100 |
| Glu | 01101 |
| Gly | 00010 |
| His | 10010 |
| Ile | 10100 |
| Lue | 10101 |
| Lys | 10001 |
| Met | 10110 |
| Phe | 11000 |
| Pro | 00011 |
| Ser | 00110 |
| Thr | 00111 |
| Trp | 11001 |
| Tyr | 11010 |
| Val | 10111 |

In some embodiments, the seed of the amino acid sequence comprises a plurality of positions, each respective position in the plurality of positions corresponding to a respective amino acid identity, and, for each respective position in the plurality of positions, the amino acid sequence comprises a corresponding encoding of the respective amino acid identity. In some embodiments, the encoding is one hot encoding of the respective amino acid identity.

In some implementations, the seed for the amino acid sequence is a tensor, each element in the tensor is a vector corresponding to a different amino acid position in the amino acid sequence, and each vector comprises, for each respective possible amino acid identity in a plurality of possible amino acid identities, a corresponding value for the respective amino acid identity. In some embodiments, the value comprises a one hot encoding of each of the plurality of possible amino acid identities such that the value indicates a presence or absence of the respective amino acid identity at the respective position. In some implementations, for example, the plurality of possible amino acid identities comprises at least 20 amino acid identities. In some embodiments, the plurality of possible amino acid identities comprises at least 22 amino acid identities. In an example embodiment, for a first position in the amino acid sequence, the corresponding vector is [1,0,0,0,0,0,0,0,0,0,0,0,0,0,0,0,0,0,0,0], where 1 indicates a presence for a first amino acid identity and 0 indicates an absence of all other amino acid identities.

In some embodiments, the seed for the amino acid sequence is not one hot encoded, and the seed for the amino acid sequence comprises, for each respective amino acid identity in a plurality of amino acid identities, a corresponding probability of the respective amino acid identity being present at the respective amino acid position.

In some embodiments, the seed for the amino acid sequence is 20-bit or 22-bit encoded.

In some embodiments, the amino acid comprises one or more feature properties and, for each respective feature property in the one or more feature properties, the seed for the amino acid sequence comprises a corresponding encoding of the respective feature property. In some embodiments, the encoding is one hot encoding of the respective feature property.

In some embodiments, the seed for the amino acid sequence is a vector, each element in the vector comprises a corresponding value for a different feature property of the amino acid in one or more feature properties. In some embodiments, the value comprises a one hot encoding of each of the one or more feature properties such that the value indicates that the amino acid comprises or does not comprise the respective feature property. For instance, in an example embodiment, the corresponding vector for a seed for an amino acid sequence is [1,0,0,0,0] for an amino acid comprising five feature properties, where 1 indicates that the amino acid comprises (*e.g.*, is characterized by) a first feature property (*e.g.*, negative charge) and 0 indicates that the amino acid does not comprise all other feature properties (*e.g.*, nonpolar aliphatic, nonpolar aromatic, polar, positive charge). Yet another example encoding for a seed for an amino acid sequence includes [0,0,0,0,1], where 1 indicates that the amino acid comprises the fifth feature property and 0 indicates that the amino acid does not comprise the first, second, third, and fourth feature properties. Various combinations of feature properties that characterize an amino acid are possible, as in the following examples: [01000], [01001], [01100], [01101], [00010], [10010], and so on.

In some embodiments, the seed for the amino acid sequence is not one hot encoded, and the seed for the amino acid sequence comprises, for each respective feature property in one or more feature properties, a corresponding probability that the respective amino acid comprises (*e.g.*, is characterized by) the respective feature property.

In some embodiments, the one or more feature properties comprises at least 3, at least 5, or at least 10 feature properties. In some embodiments, the one or more feature properties comprises at least 1, at least 3, at least 5, at least 10, at least 20, at least 50, at least 100, or at least 500 feature properties. In some embodiments, the one or more feature properties comprises no more than 1000, no more than 500, no more than 100, no more than 50, no more than 20, no more than 10, no more than 5, or no more than 3 feature properties. In some embodiments, the one or more feature properties consists of from 1 to 5, from 3 to 10, from 8 to 30, from 20 to 100, from 50 to 300, or from 200 to 1000 feature properties. In some embodiments, the one or more feature properties falls within another range starting no lower than 1 feature property and ending no higher than 1000 feature properties.

In some embodiments, the one or more feature properties are selected from the group consisting of negative charge, positive charge, nonpolar aliphatic, nonpolar aromatic, and polar.

In some embodiments, the one or more feature properties are selected from the group consisting of high expression, low expression, moderate expression, tissue-specific expression, target tissue expression, and peripheral tissue expression.

In some embodiments, the one or more feature properties are selected from the group consisting of a charge characteristic, a hydropathy value, a solubility value, a phosphorylation characteristic, a flexibility value, a ionic bonding characteristic, a hydrogen bonding characteristic, a hydrophilicity value, a surface accessibility value, a mutability value, a hydrogen bonding donor characteristic, a hydrogen bonding acceptor characteristic, an aggregate hydrogen bonding characteristic, a molecular mass value, a volume value, and a hydrophobicity value.

In some embodiments, charge refers to the electrostatic property of the amino acid side chain as an acid or base, having a positive or negative charge in an aqueous solvent at neutral pH. In some embodiments, phosphorylation refers to whether the functional group of an amino acid residue can have a phosphate group added as a post-translational modification. In some embodiments, ionic bond refers to the capacity of an amino acid residue side chain to participate in electrostatic interactions. In some embodiments, hydrogen bond refers to the capacity of an amino acid residue side chain to participate in hydrogen bond(s), hydrogen bond donor refers to the number of amino acid residue side chain atoms that can donate a hydrogen atom to a hydrogen bond under neutral pH conditions, and hydrogen bond acceptor refers to the number of amino acid residue side chain atoms that can accept a donor hydrogen atom in a hydrogen bond under neutral pH conditions. In some embodiments, aggregate hydrogen bond refers to the number of amino acid residue side chain atoms that can participate in a hydrogen bond. In some embodiments, molecular mass is the predicted molecular weight of an amino acid residue in unit Daltons. In some embodiments, volume refers to the predicted volume of a given amino acid residue in aqueous solution (*see, e.g.,* Zamyatnin, A.A., Protein volume in solution, Prog. Biophys. Mol. Biol., 24:107-123 (1972)). In some embodiments, hydropathy represents the hydrophobic (repels water) or hydrophilic (attracts water) properties of the side chain of a given amino acid residue (*see, e.g.,* Kyte and Doolittle, J. Mol. Biol., 157:105-132 (1982)). In some embodiments, the one or more features is hydrophobicity, where hydrophobicity is measured using Goldman Engelman Steitz, referring to the free energy transfer from amino acid residues in an alpha-helix from non-aqueous condition to water (*see, e.g.,* Engelman et al., Annu. Rev. Biophys. Chem., 15:321-353 (1986)). In some embodiments, flexibility refers to the symmetric or asymmetric distribution of amino acid residues in polypeptides (*see, e.g.,* Bhaskaran, R. & Ponnuswamy, P.R., Int. J. Peptide and Protein Res., 32:4:241-255 (1988)). In some embodiments, mutability refers to the probability that a given amino acid residue would change in across an evolutionary interval, and is calculated by the relative frequency at which a residue is replaced with another (*see, e.g.,* Dayhoff et al., Atlas of Protein Sequence and Structure, Vol. 5, Suppl.3 (1978)).

In some embodiments, the seed for the amino acid sequence is at least 4-bit, at least 5-bit, or at least 6-bit encoded. In some embodiments, the seed for the amino acid sequence is at least 2-bit, at least 3-bit, at least 4-bit, at least 5-bit, at least 6-bit, at least 7-bit, at least 8-bit, at least 10-bit, at least 20-bit, at least 50-bit, at least 100-bit, or at least 500-bit encoded. In some embodiments, the seed for the amino acid sequence is no more than 1000-bit, no more than 500-bit, no more than 100-bit, no more than 50-bit, no more than 20-bit, or no more than 10-bit encoded. In some embodiments, the seed for the amino acid sequence is from 2-bit to 10-bit, from 5-bit to 100-bit, from 50-bit to 500-bit, or from 200-bit to 1000-bit encoded. In some embodiments, the encoding for the seed for the amino acid sequence falls within another range starting no lower than 2-bit and ending no higher than 1000-bit.

In some embodiments, the seed for the amino acid sequence is randomly encoded. For instance, in some embodiments, the seed for the amino acid sequence is randomly bit encoded (binary) and/or randomly encoded as continuous or discrete variables.

In some embodiments, the seed for the amino acid sequence is binary encoded and a scale of each respective value in a plurality of values for the seed is transformed from {0, 1} to {-1, 1}. In some embodiments, the seed for the amino acid sequence is analog bit encoded.

For instance, in an example embodiment, a seed for an amino acid sequence comprises a corresponding vector [0,1,0,0,1] having a scale of {0, 1}, and the transformation transforms the respective vector to [-1, 1, -1, -1, 1].

In some embodiments, a generative model described herein considers only natural amino acids at each position of the amino acid sequence. For example, in some embodiments, a generative model described herein only allows for proteinogenic amino acids at each position of the amino acid sequence being generated. The standard eukaryotic genetic code includes twenty standard amino acids: L-Alanine (Ala / A), L-Arginine (Arg / R), L-Asparagine (Asn / N), L-Aspartic acid (Asp / D), L-Cysteine (Cys / C), L-Glutamic acid (Glu / E), L-Glutamine (Gln / Q), Glycine (Gly / G), L-Histidine (His / H), L-Isoleucine (Ile / I), L-Leucine (Leu / L), L-Lysine (Lys / K), L-Methionine (Met / M), L-Phenylalanine (Phe / F), L-Proline (Pro / P), L-Serine (Ser / S), L-Threonine (Thr / T), L-Tryptophan (Trp / W), L-Tyrosine (Tyr / Y), and L-Valine (Val / V). Accordingly, in some embodiments, a generative model described herein allows for any of these 20 amino acids to be present at any position in the generated amino acid sequence. In some embodiments, a generative model described herein also allows for another natural amino acid to be present at one or more (e.g., all) position of the generated amino acid sequence. For example, in some embodiments, a generative model allows for L-Selenocysteine (Sec / U) in addition to, or as an alternative to, L-Cysteine (Cys / C). Similarly, in some embodiments, a generative model allows for L-Pyrrolysine (Pyl / O) in addition to, or as an alternative to, L-Lysine (Lys / K).

In some embodiments, the generative model is applied such that one or more amino acid positions in an amino acid sequence is limited to only a subset of possible amino acids. For example, in some embodiments, one or more positions in an amino acid sequence being generated is fixed as a predetermined amino acid. In some embodiments, one or more amino acid positions in an amino acid sequence is limited to 2, 3, 4, 5, 6, 7, 8, 9, 10, 11, 12, 13, 14, 15, 16, 17, 18, or 19 possible amino acids. Said another way, in some embodiments, one or more possible amino acid is excluded as a possibility at one or more positions of the amino acid sequence being generated.

In some embodiments, a generative model described herein considers one or more modified or non-standard amino acids at one or more positions. For example, in some embodiments, in addition to the twenty standard amino acids, the model allows for one or more modified or non-standard amino acids at all positions. In some embodiments, the model allows for one or more modified or non-standard amino acid rather than a standard amino acid at one or more positions. Allowing the model to consider one or more modified or non-standard amino acid at one or more positions in the amino acid sequence is useful, for example, when a polypeptide having the amino acid sequence being generated will be synthetically produced, rather than expressed in a cell, *e.g.*, cell culture.

Non-limiting examples of modified and non-standard amino acids include L-Cysteine S-sulfate, Phospho-L-tyrosine, L-Cystine, L-desmosine, L-isodesmosine, L-Hydroxyproline, L-hydroxylysine, L-Gamma-carboxyglutamate, L-Phosphoserine, L-phosphothreonine, L-phosphotyrosine, L-N-acetyl lysine, L-Methyllysine, lanthionine, 2-aminoisobutyric acid, dehydroalanine, gamma-aminobutyric acid, ornithine, citrulline, and 3-aminopropanoic acid. In some embodiments, a generative model described herein allows for an R-amino acid at one or more position of the amino acid sequence being generated.

In some embodiments, an output for a respective model (*e.g.*, a generative and/or diffusion model) is an amino acid sequence that is binary (*e.g.*, bit) encoded, one hot encoded, and/or analog bit encoded. In some embodiments, the method further includes decoding the outputted amino acid sequence from the respective model.

Referring to block 700 in FIGS. 7A-H, another aspect of the present disclosure provides a method for generating a polymer sequence for a biological molecule having one or more target biological properties. In some embodiments, the method is performed at a computer system comprising at least one processor and a memory storing at least one program for execution by the at least one processor.

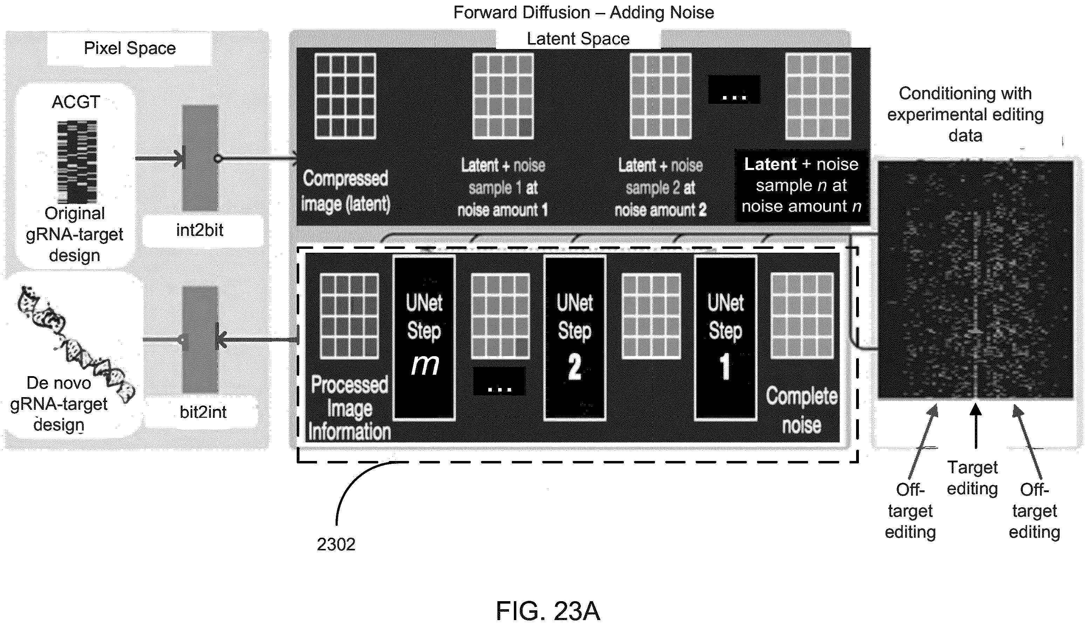

Referring to block 702, in some embodiments, the method includes inputting (i) a plurality of target metrics for one or more target biological properties of a biological molecule and (ii) a seed for a nucleic acid or amino acid sequence for the biological molecule into an initial state $X_1$ in a plurality of consecutive states $X_N$ in a Markov chain of a generative diffusion model to obtain as output from the generative diffusion model a nucleic acid or amino acid sequence for the biological molecule that is predicted by the generative diffusion model to confer on the biological molecule the one or more target biological properties approximating the plurality of target metrics. For each respective consecutive state $X_n$ in the plurality of consecutive states $X_N$ in the Markov chain following the initial state $X_1$, the diffusion model generates a corresponding denoised seed for the nucleic acid or amino acid sequence for the biological molecule using a transition model, where the transition model comprises a plurality of layers, that accounts for (*e.g.*, wherein the corresponding denoised seed accounts for) the plurality of target metrics for the one or more target biological properties using as input: the seed for the nucleic acid or amino acid sequence, when the respective state $X_n$ is the state immediately following the initial state $X_1$, and the corresponding denoised seed for the nucleic acid or amino acid sequence based on the respective state $X_{n-1}$ in the plurality of consecutive states $X_N$ in the Markov chain that immediately precedes the respective state $X_n$, when the respective state $X_n$ is not the state immediately following the initial state $X_1$. In some embodiments, when the respective state $X_n$ is the state immediately following the initial state $X_1$, the transition model accounts for the plurality of target metrics by conditioning the initial state $X_1$ using the plurality of target metrics or a representation thereof. In some embodiments, when the respective state $X_n$ is not the state immediately following the initial state $X_1$, the transition model accounts for the plurality of target metrics by conditioning the respective state $X_{n-1}$ using the plurality of target metrics or a representation thereof. Example methods for conditioning contemplated for use in the present disclosure are described in further detail elsewhere herein, for instance, with reference to FIGS. 23A-B below.

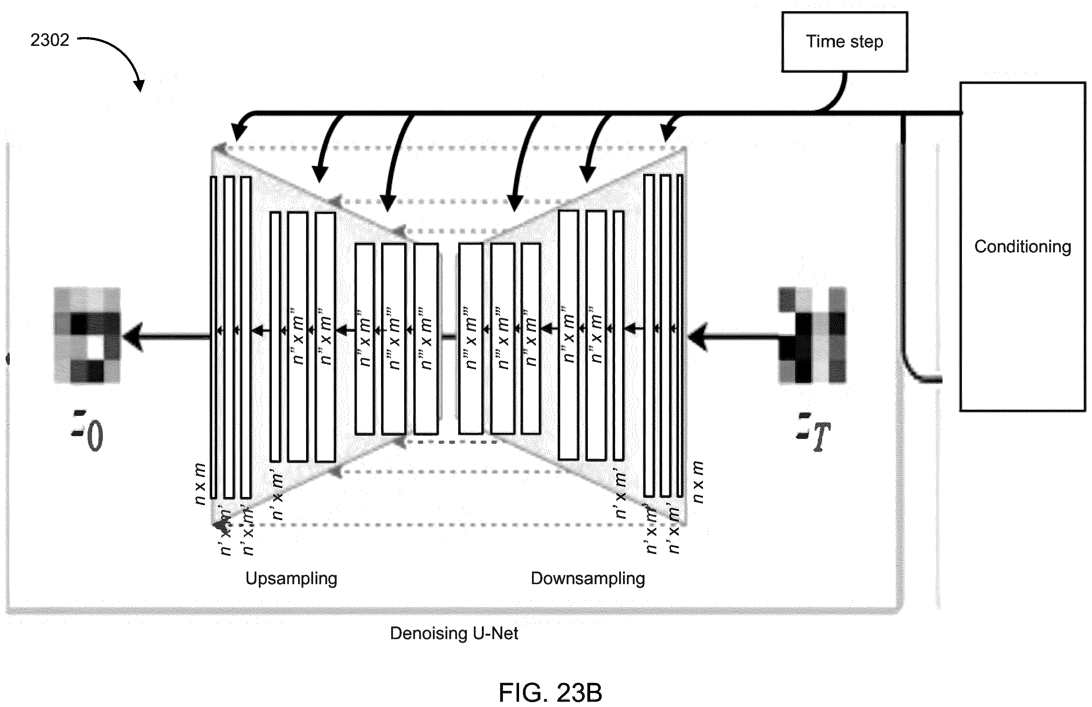

In some embodiments, the transition model uses as input the corresponding denoised seed for the nucleic acid or amino acid sequence from the respective state $X_{n-1}$ in the plurality of consecutive states $X_N$ in the Markov chain that immediately precedes the respective state $X_n$, when the respective state $X_n$ is not the state immediately following the initial state $X_1$ That is, in some embodiments, the seed resulting from one round of denoising is used as the seed for the next round of diffusion. E.g., referring to **FIG. 23B,** seed $Z_0$ output from the model 2302 during one round of denoising is used as seed $Z_1$ input to the model for the next round of denoising.

In other embodiments, the transition model uses as input a modified version of the corresponding denoised seed for the nucleic acid or amino acid sequence from the respective state $X_{n-1}$ in the plurality of consecutive states $X_N$ in the Markov chain that immediately precedes the respective state $X_n$, when the respective state $X_n$ is not the state immediately following the initial state Xi, wherein the corresponding denoised seed for the nucleic acid or amino acid sequence from the respective state $X_{n-1}$ in the plurality of consecutive states $X_N$ in the Markov chain that immediately precedes the respective state $X_n$ is modified to replace a denoised representation of one or more nucleotide or amino acid residues with a representation for a defined one or more nucleotide or amino acid residues.

In some embodiments, the modification is made in a partial diffusion process for generating the sequence. In some embodiments, a mask of fixed partial sequence at a certain position is generated and at each denoising (e.g., sampling) time step, current denoised sample is modified with that mask. Effectively, the next time step will denoise from this partially fixed previous time step.

In some embodiments, the partial diffusion process includes replacing a denoised representation of one or more nucleotide or amino acid residues prior to one or more rounds of denoising. For example, referring to **FIG. 2E,** the partially denoised seed 202 used as input to the model at time $X'_{t-1}$, is the output from the diffusion transition model following time $X'_t$. However, consider a case where it is known that the presence of an adenine at position 2 confers a target property upon a nucleic acid (e.g., ADAR-mediated or APOBEC-mediated editing efficacy and/or specificity). In a partial diffusion process, the seed representation for position 2 (e.g., [0.75, 0.1, 0.15, 0.05] at time $X'_{t-1}$) of the nucleic acid is replaced with a representation for adenine (e.g., [1, 0, 0, 0]) prior to inputting the seed into the transition model. In some embodiments, the representation for the defined one or more nucleotide or amino acid residues is introduced into the seed prior to every denoising step. In some embodiments, the representation for the defined one or more nucleotide or amino acid residues is introduced into the seed prior to less than every denoising step, e.g., every two, three, four, five, six, seven, eight, nine, ten, or more rounds.

In some embodiments, the biological molecule is a guide RNA and the defined one or more nucleotide residues is a footprint sequence conferring editing efficacy or specificity for a target sequence. In some embodiments, the footprint sequence is a single nucleotide. In some embodiments, the footprint sequence is 2, 3, 4, 5, 6, 7, 8, 9, 10, 11, 12, 13, 14, 15, 16, 17, 18, 19, 20, or more nucleotides in length. In some embodiments, the footprint sequence is at least 2 nucleotides, at least 3 nucleotides, at least 4 nucleotides, at least 5 nucleotides, at least 6 nucleotides, at least 7 nucleotides, at least 8 nucleotides, at least 9 nucleotides, at least 10 nucleotides, at least 11 nucleotides, at least 12 nucleotides, at least 13 nucleotides, at least 14 nucleotides, at least 15 nucleotides, at least 16 nucleotides, at least 17 nucleotides, at least 18 nucleotides, at least 19 nucleotides, at least 20 nucleotides, or more nucleotides in length. In some embodiments, the footprint sequence is no more than 30 nucleotides, no more than 25 nucleotides, no more than 20 nucleotides, no more than 15 nucleotides, no more than 14 nucleotides, no more than 13 nucleotides, no more than 12 nucleotides, no more than 11 nucleotides, no more than 10 nucleotides, no more than 9 nucleotides, no more than 8 nucleotides, no more than 7 nucleotides, no more than 6 nucleotides, no more than 5 nucleotides, or fewer nucleotides in length.

In some embodiments, the footprint is between 1 and 30 nucleotides, between 1 and 25 nucleotides, between 1 and 20 nucleotides, between 1 and 15 nucleotides, between 1 and 14 nucleotides, between 1 and 13 nucleotides, between 1 and 12 nucleotides, between 1 and 11 nucleotides, between 1 and 10 nucleotides, between 1 and 9 nucleotides, between 1 and 8 nucleotides, between 1 and 7 nucleotides, between 1 and 6 nucleotides, or between 1 and 5 nucleotides in length. In some embodiments, the footprint is between 5 and 30 nucleotides, between 5 and 25 nucleotides, between 5 and 20 nucleotides, between 5 and 15 nucleotides, between 5 and 14 nucleotides, between 5 and 13 nucleotides, between 5 and 12 nucleotides, between 5 and 11 nucleotides, between 5 and 10 nucleotides, between 5 and 9 nucleotides, between 5 and 8 nucleotides, between 5 and 7 nucleotides, or between 5 and 6 nucleotides in length. In some embodiments, the footprint is between 10 and 30 nucleotides, between 10 and 25 nucleotides, between 10 and 20 nucleotides, between 10 and 15 nucleotides, between 10 and 14 nucleotides, between 10 and 13 nucleotides, between 10 and 12 nucleotides, or between 10 and 11 nucleotides in length.

In some embodiments, a representation for the defined one or more nucleotide or amino acid residues are introduced into the sequence at the end of the process, also referred to herein as hardcoding the sequence. For example, in some embodiments, the denoising diffusion process is performed without biasing the seed for the nucleic acid or polypeptide and the defined one or more nucleotide or amino acid residues are used to replace residues following projection of the final seed into sequence space.

In some embodiments, the transition model comprises at least 10,000 parameters. In some embodiments, the transition model includes at least 1000, at least 10,000, at least 100,000, at least $1 \times 10^6$, at least $5 \times 10^6$, at least $1 \times 10^7$, at least $1 \times 10^8$, at least $1 \times 10^9$, or at least $1 \times 10^{10}$ parameters. In some embodiments, the transition model includes no more than $1 \times 10^{11}$, no more than $1 \times 10^{10}$, no more than $1 \times 10^9$, no more than $1 \times 10^8$, no more than $1 \times 10^7$, no more than $1 \times 10^6$, no more than 100,000, or no more than 10,000 parameters. In some embodiments, the transition model includes from 1000 to 100,000, from 50,000 to 500,000, from 100,000 to $5 \times 10^6$, from $1 \times 10^6$ to $1 \times 10^7$, from $1 \times 10^7$ to $1 \times 10^8$, from $1 \times 10^8$ to $1 \times 10^{10}$, or from $1 \times 10^7$ to $1 \times 10^{11}$ parameters. In some embodiments, the transition model includes another range of parameters starting no lower than 1000 parameters and ending no higher than $1 \times 10^{11}$ parameters.

In some embodiments, each respective consecutive state $X_n$ in the plurality of consecutive states $X_N$ in the Markov chain corresponds to a respective position in a plurality of positions in the Markov chain. For instance, in some embodiments, the respective position is a time step in a plurality of time steps. In some embodiments, each respective position in the plurality of positions corresponds to a value of $n$ in $N$. Referring to block 704, in some embodiments, an indication of the position, in the Markov chain, of the transition from the state that immediately precedes the respective consecutive state $X_n$ is incorporated into one or more respective layers in the plurality of layers of the transition model; and the generative diffusion model generates, for each respective consecutive state $X_n$ in the plurality of consecutive states $X_N$ in the Markov chain following the initial state $X_1$, the corresponding denoised seed for the nucleic acid or amino acid sequence for the biological molecule. In some embodiments, the plurality of layers of the transition model comprises one or more attention layers that attend to an embedding of the indication of the position, in the Markov chain, of the transition. In some embodiments, the plurality of layers of the transition model comprises one or more temporal projection layers that project the indication of the position, in the Markov chain, of the transition on an output of a previous layer in the plurality of layers of the transition model using a mapping function between the indication of the position, in the Markov chain, of the transition and a corresponding set of weights for the temporal projection layer. In some embodiments, a plurality of conditions are used to condition the model. In some embodiments, for each respective condition in the plurality of conditions, the model comprises, for each respective layer in the plurality of layers, a respective conditioning layer that generates an embedding of the respective condition and projects it onto an output of a previous layer. For instance, in some embodiments, the plurality of conditions comprises a time step, a target sequence, a target metric, and/or a tissue specificity. In some such embodiments, each respective condition in the plurality of conditions is separately embedded using a separate conditioning layer. In some implementations, one or more embeddings are combined (*e.g.*, added, concatenated, matrix multiplied, *etc.*) prior to projecting the embedding onto the output of the previous layer. For instance, in some embodiments, for a respective layer in the plurality of layers, a time step embedding is added to a target metrics embedding prior to projection onto the output of the previous layer.

FIGS. 2A-F collectively illustrate an illustrative schematic of an example diffusion model architecture. In particular, FIG. 2A illustrates an example schematic for training a generator "G" to generate polymer sequences from seeds. The example training process is divided into three stages for ease of illustration in the following discussion.

Stage 1: During training, an experimental sequence (original and undiffused experimental sequence "$x_0$") is diffused a predetermined number (T) of steps, as shown in FIG. 2B. The forward diffusion is based on a Markov chain, in which the probability density of each subsequent step is determined at time step t given the prior state at time step t-1. This determination can be represented as $q(x_t|x_{t-1})$ to generate each subsequent version of the sequence "$x_t$" from the prior version "$x_{t-1}$." The diffusion process generates a diffused sequence at time step T, or "$x_T$."

Stage 2: A reverse diffusion, or de-noising, process is performed in which the model attempts to estimate the probability density of the sequence at time step t-1, given the state at time step t. This estimation is represented as $p_\emptyset(x_{t-1}|x_t)$ and uses learned parameters $\emptyset$. The denoising process generates a "generated" denoised sequence, or "$x'_0$." In some implementations, as illustrated in FIG. 2C, the denoising process is further "conditioned" using one or more conditions (*e.g.*, target metric values such as "experimental metrics"). In some embodiments, the sequence (*e.g.*, to be denoised, such as a seed sequence or an intermediate denoised sequence thereof) is conditioned at each step.

In some embodiments, the conditioning comprises concatenating the one or more conditions (*e.g.*, target metric values), or a representation thereof, to the sequence to be denoised. In some embodiments, the conditioning comprises adding, multiplying, or scaling the one or more conditions, or a representation thereof, to the sequence to be denoised. Other methods for conditioning contemplated for use herein are described in further detail below, for instance with reference to FIGS. 23A-B, as will be apparent to one skilled in the art.

Stage 3: Referring again to FIG. 2A, the goal of training is generally to maximize the likelihood of observing $p_\emptyset(x_0)$ in the training (experimental) data, by maximizing the evidence lower bound (ELBO). The ELBO can be written as matching the true denoising distribution $q(x_{t-1}|x_t)$ with the parameterized denoising model $p_\emptyset(x_{t-1}|x_t)$. Generally, the likelihood can be written in terms of Kullback-Leibler (KL) Divergence, where the KL Divergence is an asymmetric statistical distance measure of how much one probability distribution P differs from a reference distribution Q. *See, e.g.,* O'Connor, 2022, "Introduction to Diffusion Models for Machine Learning," available on the Internet at assemblyai.com/blog/diffusion-models-for-machine-learning-introduction; and Xiao et al., "Tackling the Generative Learning Trilemma with Denoising Diffusion GANs," ICLR 2022, each of which is hereby incorporated herein by reference in its entirety.

Once trained, the trained model with adjusted parameters Ø is used for generation of polymer sequences from seeds (*e.g.*, one-hot encoded sequences, fully and/or partially diffused sequences obtained from experimental sequences, and/or randomly generated seed sequences that approximate diffused sequences). A use schematic of the trained diffusion model is shown in FIG. 2D and is similar to the denoising process decoupled from the diffusion process shown in FIG. 2A. Experimental, seed/diffused sequences, and generated sequences, in tensor format, for use at an initial and various consecutive states of the generative diffusion model are illustrated in FIGS. 2D-E. These sequences are similar to those previously depicted in FIG. 2C. In particular, as shown in FIG. 2E, in some embodiments, a generated sequence is obtained by determining a projection of a denoised sequence (*e.g.*, determining a residue identity based on the residue position in the denoised sequence having the highest likelihood or value at the respective position).

In some embodiments, the transition model comprises a neural network. Referring to block 706, in some embodiments, the transition model comprises a U-Net neural network.

As illustrated in FIGS. 23A-B, in some embodiments, the transition model is a conditional denoising U-Net model.

Generally, a U-Net model comprises a U structure with downsampling (contracting) and upsampling (expansive) paths. The U structure is such that the expansive path is relatively symmetric to the contracting path. Such structure further allows the model to propagate information from lower to higher resolution layers. In particular, high-resolution features from the downsampling path are combined with upsampled output in the upsampling path to increase the resolution of the output and assemble a more precise output. An exemplary structure of a U-Net is illustrated, for instance, in the denoising U-Net schematic 2302 in FIG. 23B. See, for example, Ronneberger, "U-Net: Convolutional Networks for Biomedical Image Segmentation," 2015, arXiv:1505.04597v1, available on the Internet at arxiv.org/pdf/1505.04597.pdf, which is hereby incorporated herein by reference in its entirety.

In some implementations, as illustrated in FIG. 23B, the U-Net architecture comprises a plurality of layer blocks (illustrated in blocks of three), each including at least a residual network layer and a down-sampling or up-sampling layer. In some implementations corresponding residual network layers having the same spatial resolutions (*e.g.*, n x m) are connected by skip connections (dashed gray arrows). Respective layer blocks further include conditioning layers projecting embeddings of corresponding conditions, as illustrated by solid curved arrows (*e.g.*, mRNA target sequence, target metrics, and/or a time step for the reverse diffusion process (*e.g.*, indicating where in the Markov chain the diffusion process is)), to condition the model.

In some embodiments, the diffusion and/or denoising process is performed in a latent space. In some embodiments, the method further includes obtaining a latent representation of an input to the model (*e.g.*, a seed). In some embodiments, the latent representation is obtained using all or a portion of an auto-encoder (*e.g.*, an encoder). In some embodiments, the method further includes determining the nucleic acid or amino acid sequence from a latent representation of the nucleic acid or amino acid sequence. In some embodiments, the nucleic acid or amino acid sequence is obtained using all or a portion of an auto-encoder (*e.g.*, a decoder).. In some embodiments, the latent representation comprises compressed information relative to the seed and/or the nucleic acid or amino acid sequence.

In some implementations, a U-Net model further comprises an attention mechanism. Generally, attention mechanisms apply weights to features in an input (*e.g.*, words in a sequence of words), such as via a dot product of an input tensor (*e.g.*, encodings of a sequence) and a weight tensor. In some implementations, differential weighting highlights features of high relevance relative to features of low relevance which can be used to extract information at each level of downsampling and/or upsampling. See, for example, Cristina, "The Attention Mechanism from Scratch," 2022 Machine Learning Mastery, available on the Internet at machinelearningmastery.com/the-attention-mechanism-from-scratch, which is hereby incorporated herein by reference in its entirety. In some embodiments, the U-Net model comprises a self-attention mechanism. Alternatively or additionally, in some embodiments, the U-Net model comprises a cross-attention mechanism. In some embodiments, the cross-attention and/or self-attention mechanisms are utilized in the forward diffusion process. Alternatively or additionally, in some embodiments, the cross-attention and/or self-attention mechanisms are utilized in the reverse diffusion process. In some embodiments, the U-Net model does not comprise an attention mechanism.

In some embodiments, as described above with reference to FIG. 23B, the U-Net model is a conditional diffusion model. Accordingly, in some embodiments, the upsampling and/or downsampling layer blocks comprise conditioning layers projecting embeddings of the corresponding conditions. Generally, embeddings comprise a representation (*e.g.*, in tensor form) of an object, such as a nucleic acid or amino acid sequence, a condition, and/or a representation thereof. In some implementations, embeddings capture semantic relationships or context between elements of the representation (*e.g.*, positions in a sequence). This is useful, for instance, for preserving position-specific information and/or inter-position interactions and dependencies in polymer sequences. Methods, models, and algorithms for embedding suitable for use in the present disclosure are known in the art, including but not limited to such examples as principal component analysis (PCA), singular value decomposition (SVD), Word2Vec, Sequence2Vec, Gene2Vec, kmer2vec, seq2seq, and/or BERT. See, for example, Mokhtarani, "Embeddings in Machine Learning: Everything You Need to Know," 2021, available on the Internet at featureform.com/post/the-definitive-guide-to-embeddings. In some embodiments, the conditions are incorporated into each downsampling or upsampling function. See, for example, Capelle, "How to Train a Conditional Diffusion Model from Scratch," 2023, Weights and Biases, available on the Internet at wandb.ai/capecape/train_sd/reports/How-To-Train-a-Conditional-Diffusion-Model-From-Scratch--VmlldzoyNzIzNTQ1, which is hereby incorporated herein by reference in its entirety. For instance, in some embodiments, each respective layer block (*e.g.*, each respective downsampling layer block and each respective upsampling layer block) in the plurality of layer blocks receives a conditioning signal (*e.g.*, an embedding of one or more corresponding conditions). In some embodiments, the conditioning signal is incorporated into each respective layer block at each respective level of downsampling or upsampling. Advantageously, in some embodiments, such repeated embedding and incorporation of the conditions prevents "drift" (*e.g.*, alteration or corruption) of the conditioning signal from the downsampling or upsampling process.

In some embodiments, a respective condition is selected from the group consisting of a target sequence, a target metric, and a time step for the reverse diffusion process. In some embodiments, a respective condition is one or more of a target sequence, a target metric, and a time step for the reverse diffusion process.

Generally, conditioning allows a model to account for one or more conditions when generating an output (*e.g.*, a nucleic acid or amino acid sequence). In some embodiments, conditioning transforms, alters, and/or otherwise modifies one or more elements of a sequence to be denoised (*e.g.*, a seed or a partially denoised sequence), or a representation thereof.

Consider an illustrative example where the sequence to be denoised is represented as a tensor of embeddings comprising a plurality of values. In some embodiments, the model performs a function (*e.g.*, reverse diffusion or denoising) on one or more elements of the tensor. In some such embodiments, a first representation of the sequence to be denoised is used by the model to generate a second representation of the sequence with all or a portion of noise removed relative to the first representation (see, for instance, the description of the diffusion model architecture with reference to FIGS. 2A-F, above).

In some implementations, this process is condition-agnostic. In other words, in some embodiments, the model generates the second representation of the sequence based on the first representation of the sequence, where the first representation of the sequence does not further incorporate one or more conditions.

Alternatively, in some implementations, the denoising process is conditioned. In some embodiments, at each respective layer in one or more layers (*e.g.*, each layer) of the model, one or more conditions, or representations thereof, are incorporated into the model. In some embodiments, the incorporating one or more conditions or representations thereof transforms, alters, and/or modifies the sequence to be denoised, or the representation thereof, such as by transforming, altering, and/or modifying one or more elements of a tensor. Such elements can correspond to different positions in the sequence, different residue identities, and/or other chemical or biological characteristics of the nucleic acid or amino acid sequence represented by the tensor. In some embodiments, the model generates the second representation of the sequence based on the first representation of the sequence, where the first representation of the sequence incorporates the one or more conditions or representations thereof. This allows for a more informed or context-aware denoising process.

In some embodiments, the one or more conditions are embedded or encoded, thereby obtaining one or more representations of the one or more conditions. In some embodiments, a respective condition is embedded or encoded using any of the methods for embedding or encoding disclosed herein (*e.g.*, one-hot encoding, bit-encoding, *etc.*). In some embodiments, a plurality of conditions is combined into a single representation. In some embodiments, two or more conditions are obtained as separate representations.

In some embodiments, at a respective layer in the one or more layers of the model, the one or more conditions are incorporated by projecting an embedding of the one or more conditions into the respective layer. In some embodiments, the projecting comprises applying one or more transformations to the representation of the sequence to be denoised. In some implementations, the one or more transformations comprises a linear transformation or a nonlinear transformation. In some embodiments, the one or more transformations comprises one or more tensor operations (*e.g.*,

addition, subtraction, multiplication, and/or division). In some embodiments, the one or more transformations comprises tensor multiplication (*e.g.*, matrix multiplication, vector multiplication, and/or scalar multiplication). Returning again to the illustrative example above, in some implementations, the sequence to be denoised is represented as a first tensor, the one or more conditions are represented as a second tensor, and the conditioning at a respective layer in the one or more layers comprises performing a tensor multiplication using the first tensor and the second tensor. In some embodiments, the one or more transformations comprises tensor concatenation. In some implementations, the sequence to be denoised is represented as a first tensor, the one or more conditions are represented as a second tensor, and the conditioning at a respective layer in the one or more layers comprises padding the first tensor using the second tensor. In some embodiments, the first tensor is not concatenated. Such conditioning can be viewed as a "weighting" of one or more elements (*e.g.*, values) of the sequence to be denoised or the representation thereof (*e.g.*, the tensor). In some such implementations, the model performs a function (*e.g.*, reverse diffusion or denoising), on a merged representation that incorporates both the sequence to be denoised and the one or more conditions. An output from the model or a respective layer thereof (*e.g.*, the sequence, or a representation thereof, with all or a portion of noise removed) is thus contextualized relative to the sequence to be denoised, or a representation thereof, that is not so conditioned.

In some embodiments, conditioning with different conditions produces the same or different transformations on the sequence to be denoised or the representation thereof. Alternatively or additionally, in some embodiments, conditioning with different conditions produces one or more transformations on the same or different elements of the sequence to be denoised or the representation thereof (*e.g.*, different positions in the sequence, different residue identities, and/or other chemical or biological characteristics of the sequence). For example, in some implementations, a first condition and a second condition differentially transform a tensor representation of a seed, such that 1) a first denoised sequence generated by the model from a first representation of the seed conditioned by the first condition corresponds to a different nucleic acid or amino acid sequence relative to 2) a second denoised sequence generated by the model from a second representation of the seed conditioned by the second condition. In some embodiments, the model generates 1) a first denoised or partially denoised sequence from a sequence to be denoised, or a representation thereof, that is different from 2) a second denoised or partially denoised sequence generated from the sequence to be denoised, or a representation thereof, where the second denoised or partially denoised sequence is generated by conditioning the sequence to be denoised or the representation using one or more conditions.

In some embodiments, a respective condition is represented as a sequence of tokens. In some embodiments, prior to the conditioning of each respective layer block in the plurality of layer blocks, a respective condition is encoded to produce one or more token embeddings. In some embodiments, the token embeddings are obtained using a transformer model. In some embodiments, the transformer model is a transformer language model (*e.g.*, CLIP Text or BERT). In some embodiments, as illustrated in **FIG. 23B,** the conditions and/or values thereof (*e.g.*, token embeddings) are passed through each respective layer block in the plurality of layer blocks. In some embodiments, as illustrated in **FIG. 23B,** the conditions and/or values thereof (*e.g.*, token embeddings) are passed through each respective downsampling layer block and each respective upsampling layer block in the plurality of layer blocks. See, for example, Alammar, "The Illustrated Stable Diffusion," 2022, available on the Internet at jalammar.github.io/illustrated-stable-diffusion/, which is hereby incorporated herein by reference in its entirety.

Referring again to **FIG. 23A,** in some implementations, the denoising U-Net is used to generate a de novo sequence (*e.g.*, a nucleic acid or amino acid sequence). In some embodiments, the denoising U-Net accepts, as input, a seed for a nucleic acid or amino acid sequence for a biological molecule into an initial state X1 in a plurality of consecutive states XN. For instance, in **FIG. 23A,** the seed inputted into the initial state X1 is illustrated as a "noisy" latent representation of a seed at time step ZT in a plurality of T consecutive states. In some embodiments, the method further includes inputting into the denoising U-Net a plurality of conditions (*e.g.*, target metrics, target sequence, and/or time steps). In some implementations, the method includes obtaining as output from the denoising U-Net a nucleic acid or amino acid sequence for the biological molecule, illustrated in **FIG. 23A** as a denoised latent representation. In some implementations, a latent representation of an input and/or an output to the denoising U-Net (*e.g.*, a latent representation of a seed and/or a nucleic acid or amino acid sequence) is transformed from the latent space. For instance, in some embodiments, an output from the denoising U-Net is converted to a nucleic acid or amino acid sequence (*e.g.*, "de novo gRNA-target design).

Although **FIGS. 23A-B** illustrate a denoising U-Net comprising a symmetric U structure, it will be understood that alternative model architectures are possible and contemplated for use in the present disclosure. In some embodiments, model architectures are determined on a problem-specific basis. In some embodiments, the selection and/or generation of a model architecture for use comprises hyperparameter optimization (*e.g.*, for selection of a number of time steps, layers, *etc.*). For example, in some embodiments, the model comprises a same or different numbers of layer blocks in the downsampling path relative to the upsampling path. In some embodiments, the model comprises a same or different number of layers in a first layer block relative to a second layer block. In some embodiments, the model comprises a same or different spatial resolution in a first layer relative to any other layer in the model. In some embodiments, a downsampling layer and an upsampling layer having the same spatial resolution are not connected by a skip connection. In some embodiments, each respective downsampling layer is connected to a corresponding upsampling layer having the same spatial resolution, and/or each respective upsampling layer is connected to a corresponding downsampling layer having the same spatial resolution.

Non-limiting examples of U-net models are further described, for instance, in Example 1 below.

Referring to block 708, in some embodiments, the transition model is a conditional generator model of a conditional generative adversarial network that generates, for each respective consecutive state $X_n$ in the plurality of consecutive states $X_N$ in the Markov chain following the initial state $X_1$, a corresponding nucleic acid or amino acid sequence predicted by the conditional generator model to confer on the biological molecule the one or more target biological properties approximating the plurality of target metric vales using as input: the seed for the nucleic acid or amino acid sequence, when the respective state $X_n$ is the state immediately following the initial state $X_1$, and a seed based on the corresponding denoised seed sequence from the respective state $X_{n-1}$ in the plurality of consecutive states $X_N$ in the Markov chain that immediately precedes the respective state $X_n$, when the respective state $X_n$ is not the state immediately following the initial state $X_1$; and for each respective consecutive state $X_n$ in the plurality of consecutive states $X_N$ in the Markov chain following the initial state $X_1$, other than a terminal state $X_{n=N}$ in the Markov chain, the diffusion model samples from a posterior distribution of seed values for the corresponding nucleic acid or amino acid sequence to generate the corresponding denoised seed for the nucleic acid or amino acid sequence.

In some embodiments, the conditional generator model uses as input the corresponding denoised seed sequence from the respective state $X_{n-1}$ in the plurality of consecutive states $X_N$ in the Markov chain that immediately precedes the respective state $X_n$, when the respective state $X_n$ is not the state immediately following the initial state $X_1$.

In other embodiments, the transition model uses as input a modified version of the corresponding denoised seed sequence from the respective state $X_{n-1}$ in the plurality of consecutive states $X_N$ in the Markov chain that immediately precedes the respective state $X_n$, when the respective state $X_n$ is not the state immediately following the initial state $X_1$.

In some embodiments, the modification is made in a partial diffusion process for generating the sequence. In some embodiments, a mask of fixed partial sequence at a certain position is generated and at each denoising (e.g., sampling) time step, current denoised sample is modified with that mask. Effectively, the next time step will denoise from this partially fixed previous time step.

In some embodiments, the partial diffusion process includes replacing a denoised representation of one or more nucleotide or amino acid residues prior to one or more rounds of denoising. For example, referring to **FIG. 2E,** the partially denoised seed 202 used as input to the model at time $X'_{t-1}$, is the output from the diffusion transition model following time $X'_t$. However, consider a case where it is known that the presence of an adenine at position 2 confers a target property upon a nucleic acid (e.g., ADAR-mediated or APOBEC-mediated editing efficacy and/or specificity). In a partial diffusion process, the seed representation for position 2 (e.g., [0.75, 0.1, 0.15, 0.05] at time $X'_{t-1}$) of the nucleic acid is replaced with a representation for adenine (e.g., [1, 0, 0, 0]) prior to inputting the seed into the transition model. In some embodiments, the representation for the defined one or more nucleotide or amino acid residues is introduced into the seed prior to every denoising step. In some embodiments, the representation for the defined one or more nucleotide or amino acid residues is introduced into the seed prior to less than every denoising step, e.g., every two, three, four, five, six, seven, eight, nine, ten, or more rounds.

In some embodiments, the biological molecule is a guide RNA and the defined one or more nucleotide residues is a footprint sequence conferring editing efficacy or specificity for a target sequence. In some embodiments, the footprint sequence is a single nucleotide. In some embodiments, the footprint sequence is 2, 3, 4, 5, 6, 7, 8, 9, 10, 11, 12, 13, 14, 15, 16, 17, 18, 19, 20, or more nucleotides in length. In some embodiments, the footprint sequence is at least 2 nucleotides, at least 3 nucleotides, at least 4 nucleotides, at least 5 nucleotides, at least 6 nucleotides, at least 7 nucleotides, at least 8 nucleotides, at least 9 nucleotides, at least 10 nucleotides, at least 11 nucleotides, at least 12 nucleotides, at least 13 nucleotides, at least 14 nucleotides, at least 15 nucleotides, at least 16 nucleotides, at least 17 nucleotides, at least 18 nucleotides, at least 19 nucleotides, at least 20 nucleotides, or more nucleotides in length. In some embodiments, the footprint sequence is no more than 30 nucleotides, no more than 25 nucleotides, no more than 20 nucleotides, no more than 15 nucleotides, no more than 14 nucleotides, no more than 13 nucleotides, no more than 12 nucleotides, no more than 11 nucleotides, no more than 10 nucleotides, no more than 9 nucleotides, no more than 8 nucleotides, no more than 7 nucleotides, no more than 6 nucleotides, no more than 5 nucleotides, or fewer nucleotides in length.

In some embodiments, the footprint is between 1 and 30 nucleotides, between 1 and 25 nucleotides, between 1 and 20 nucleotides, between 1 and 15 nucleotides, between 1 and 14 nucleotides, between 1 and 13 nucleotides, between 1 and 12 nucleotides, between 1 and 11 nucleotides, between 1 and 10 nucleotides, between 1 and 9 nucleotides, between 1 and 8 nucleotides, between 1 and 7 nucleotides, between 1 and 6 nucleotides, or between 1 and 5 nucleotides in length. In some embodiments, the footprint is between 5 and 30 nucleotides, between 5 and 25 nucleotides, between 5 and 20 nucleotides, between 5 and 15 nucleotides, between 5 and 14 nucleotides, between 5 and 13 nucleotides, between 5 and 12 nucleotides, between 5 and 11 nucleotides, between 5 and 10 nucleotides, between 5 and 9 nucleotides, between 5 and 8 nucleotides, between 5 and 7 nucleotides, or between 5 and 6 nucleotides in length. In some embodiments, the footprint is between 10 and 30 nucleotides, between 10 and 25 nucleotides, between 10 and 20 nucleotides, between 10 and 15 nucleotides, between 10 and 14 nucleotides, between 10 and 13 nucleotides, between 10 and 12 nucleotides, or between 10 and 11 nucleotides in length.

In some embodiments, a representation for the defined one or more nucleotide or amino acid residues are introduced into the sequence at the end of the process, also referred to herein as hardcoding the sequence. For example, in some embodiments, the denoising diffusion process is performed without biasing the seed for the nucleic acid or polypeptide and the defined one or more nucleotide or amino acid residues are used to replace residues following projection of the final seed into sequence space.

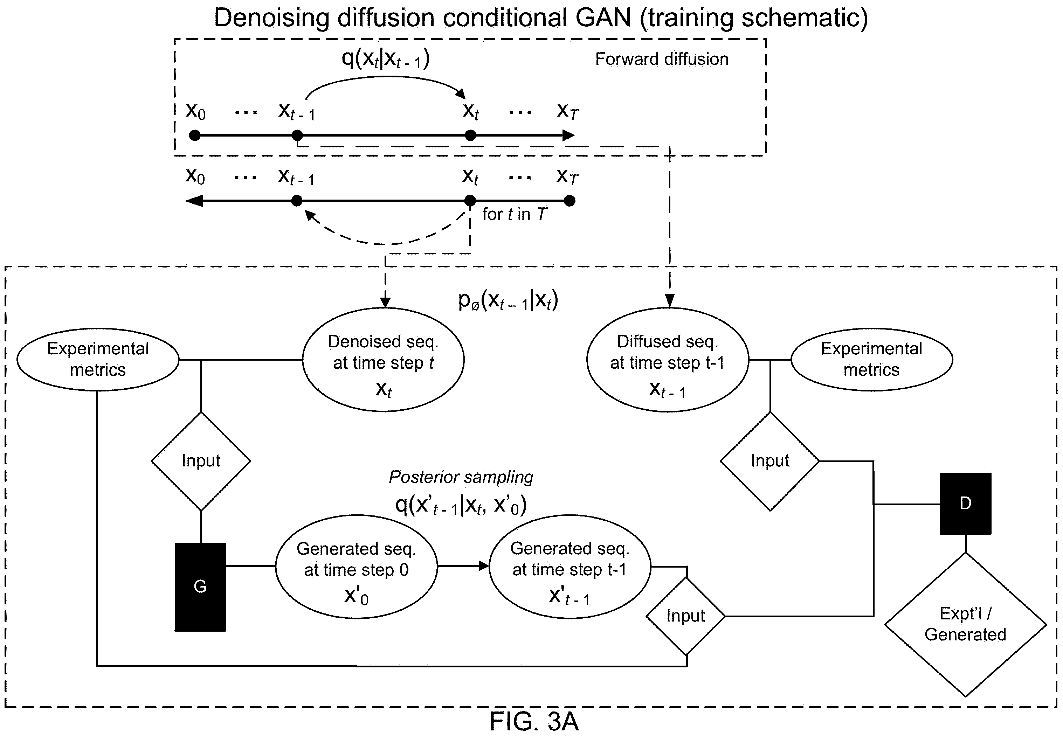

FIGS. 3A-B collectively illustrate an example schematic of a denoising diffusion conditional GAN (conditional ddGAN) model architecture, with reference again to FIGS. 2A-F and FIGS. 1A-E. In particular, FIG. 3A illustrates an example schematic used for training the generator "G" to generate polymer sequences from seeds (e.g., one-hot encoded sequences, seed sequences, fully or partially diffused sequences obtained from experimental sequences, and/or approximations thereof). Using this approach, training samples for input to the discriminator are generated by iteratively denoising (and then posterior sampling) experimental sequences that have been subjected to a diffusion process.

Consider again the "experimental" sequence in the top panel of FIG. 2A. A stepwise forward diffusion process is applied to the experimental sequence over $T$ time steps, e.g., steps $t$-$1$, $t$, $...T$, where 0 indicates the starting time step prior to any addition of noise. The diffusion process gradually adds noise to the sequence; for example, at a given time step $t$ in $T$, additional noise is added to the progressively diffused version of the sequence that was previously obtained at time step $t$-$1$ ($X_{t-1}$), thus generating an even noisier version ($X_t$). Referring again to FIG. 2B, an example of the diffusion process applied to a one hot-encoded sequence is illustrated; note that although the example depicts an experimental $X_0$ sequence, the process can also be applied to a "generated" sequence $X'_0$ (e.g., for posterior sampling, described below).

Referring to the top panel of FIG. 3A, to generate samples for training, each version of the experimental sequence obtained from the forward diffusion process (e.g., for each $t$ in $T$) is inputted into generator G. Particularly, as illustrated in the bottom panel of FIG. 3A, the generator takes as input a diffused experimental sequence at time $t$ ($x_t$), along with target metrics ("input," optionally concatenated in vector format, as described above). The generator G then attempts to output a completely denoised version of what it received as input as a generated sequence, or "$x'_0$." A posterior sampling process is then performed, in which the generated sequence is diffused to time step $t$-$1$, or "$x'_{t-1}$."

Note that the target metrics get passed through the model without manipulation, but in some implementations are referred to by other nodes in each layer of the model. That is, the input to the generator can be a diffused sequence concatenated to target metrics, and the output of the denoising process can be a denoised sequence concatenated to the inputted target metrics.

After posterior sampling, the generated sequence diffused to time step $t$-$1$ ($x'_{t-1}$) is inputted to the discriminator D, along with the target metrics (optionally concatenated, as before). For comparison, the experimental sequence diffused to time step $t$-$1$ ($x_{t-1}$), along with experimental metrics (optionally concatenated), is also inputted to discriminator D.

Here, adversarial training of G and D is performed, as described above for the simple GAN architecture depicted in FIGS. 1A-E, in which the discriminator D attempts to classify each input as experimental or generated. Briefly, when the discriminator successfully identifies experimental and generated sequences, it is rewarded (e.g., via modifications to discriminator parameters) or unaltered (e.g., no changes to discriminator parameters), while the generator is penalized (e.g., via modifications to generator parameters). Alternately, when the discriminator is unable to distinguish between experimental and generated sequences, the generator is rewarded (e.g., via modifications to generator parameters) or unaltered, whereas the discriminator is penalized (e.g., via modifications to discriminator parameters). This process is performed iteratively for each diffused version of the experimental sequence at each time step $t$ in $T$ over the entire forward diffusion process.

Note that the model shown in FIGS. 3A-B is also a conditional model; where G takes into account the target metrics when attempting to generate denoised sequences, and D takes into account both the experimental metrics (with the experimental diffused sequence $x_{t-1}$) and the target metrics (with the generated diffused sequence $x'_{t-1}$) when attempting to discriminate between experimental and generated. An illustrative example of the training process, using a simple case where $T = 2$, is provided in FIGS. 4A-B and described in further detail below.

As described above, after training (to some predetermined performance, e.g., 50%), the discriminator is discarded, and the trained generator is retained for generation of polymer sequences from seeds (in some cases, seed sequences being random and thus, effectively, approximations of diffused input sequences).

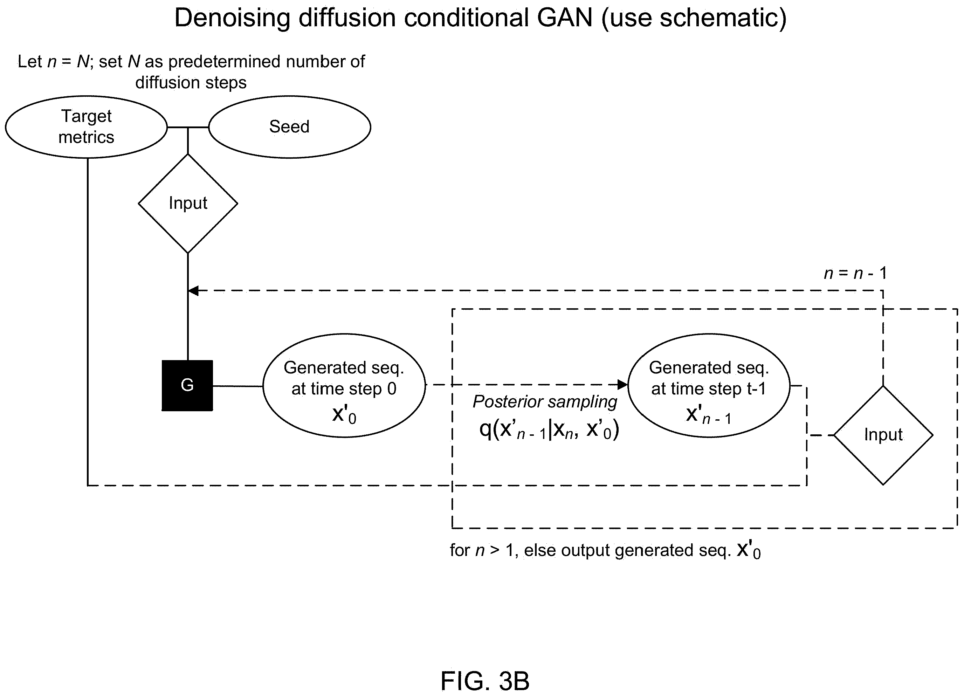

FIG. 3B illustrates a use schematic of a trained conditional ddGAN.

The generative process has multiple denoising steps (for some predetermined number of steps $T$, where $T >$ 1), each of which includes a generation of a fully denoised generated $x'_0$ and posterior sampling to a diffused sequence $x'_{t-1}$ that attempts to approximate a prior state of the input sequence $x_t$ (e.g., an approximation of a single denoising step that is obtained by (i) full denoising prediction + (ii) posterior sampling). This iterative process is performed for each of $T$ iterations, where the first input to G is a seed or diffused sequence, which is deemed to approximate the end product of a diffusion product (hence, $x_t$, where $t$ is initially set as $T$). Target metrics are also inputted into G (optionally concatenated to the seed). G outputs a fully denoised generated $x'_0$ from $x_t$ and posterior sampling is performed to generate a generated diffused sequence $x'_{t-1}$ (or $x'_{t-1}$ after the first iteration). $X'_{t-1}$ is once again concatenated to the target metrics and inputted to G as $x'_t$, allowing the next iteration to produce a sequence $x'_{t-1}$ that is one step further denoised from the $x'_{t-1}$ of the previous iteration.

Thus, the input to the generator in each successive iteration is (i) the output from the prior iteration + posterior sampling, and (ii) an approximation of a prior state of the input sequence of the previous iteration. Thus, each iteration of the generative process accepts, as input, successively "cleaner" or "denoised" sequences that are used to generate the fully denoised generated $x'_0$. Note that each step makes use of the same generator. An illustrative example of the generative process, using a simple case where $T = 2$, is provided in FIG. 4C.

Referring now to FIGS. 4A-B, to illustrate the iterative training of conditional ddGANs detailed above, a simplified training process will now be described, using a forward diffusion process having only two diffusion steps ($t = 1$ and $t = 2$), resulting in experimental sequence versions $x_0$ (original), $x_1$ (1 step of diffusion), and $x_2$ (2 steps of diffusion).

In FIG. 4A, for $t = 2$, experimental diffused sequence $x_2$ and target metrics are inputted into G. G outputs a fully denoised generated sequence $x'_0$. $X'_0$ is then posteriorly sampled to $x'_1$. Generated diffused sequence $x'_1$ and target metrics are inputted, along with experimental diffused sequence $x_1$ and experimental metrics, into D, which attempts to classify the inputs as experimental or generated. In FIG. 4B, for $t = 1$, experimental diffused sequence $x_1$ and target metrics are inputted into G. G outputs a fully denoised generated sequence $x'_0$. Generated diffused sequence $x'_0$ and target metrics are inputted, along with experimental sequence $x_0$ and experimental metrics, into D, which attempts to classify the inputs as experimental or generated.

Methods for updating model parameters during model training are known in the art. In typical embodiments, updating a plurality of parameters is performed by calculating an error for each respective parameter in the plurality of parameters, where the error for each parameter is determined by calculating a loss based on the model output (*e.g.*, the predicted value - generated or experimental) and the input data (*e.g.*, the expected value or true labels - generated or experimental). Parameters are then updated by adjusting the value based on the calculated loss, thereby training the model. Generally, parameters are updated such that the error is minimized (*e.g.*, according to the loss function). In some embodiments, any one of a variety of algorithms and/or methods are contemplated for use in updating parameters, as will be apparent to one skilled in the art. In some embodiments, the loss function is mean square error, quadratic loss, mean absolute error, mean bias error, hinge, multi-class support vector machine, and/or cross-entropy. In some embodiments, the error is computed in accordance with a gradient descent algorithm and/or a minimization function.

Referring now to FIG. 4C, to illustrative the iterative generative process detailed above, a simplified generative process will now be described, using a predetermined $T = 2$ to simulate diffusion steps for posterior sampling.

A seed (*e.g.*, a seed sequence, a fully or partially diffused sequence obtained from an experimental sequence, and/or an approximation thereof) is obtained along with target metrics. For $t = 2$ (top panel), the initial seed is $x_2$ for $t = T$, where $T = 2$. The seed is concatenated with the target metrics and inputted into G. Output from G is a fully denoised generated sequence $x'_0$, along with the target metrics (unmanipulated). A posterior sampling is performed on $x'_0$, thus obtaining $x'_1$. For $t = 1$ (bottom panel), the input to G is the output sequence $x'_1$ from the first iteration, along with the target metrics. Output from G is a fully denoised generated sequence $x'_0$, along with the target metrics (unmanipulated).

Advantageously, in some implementations, ddGANs provide improved polymer sequence generation over traditional GANs. For example, GANs are known to suffer from training instability and mode collapse, some possible reasons for which include the difficulty of directly generating samples from a complex distribution in one-shot, and the overfitting issue when the discriminator only looks at clean samples. In contrast, the ddGAN model breaks the generation process into several conditional denoising diffusion steps in which each step is relatively simple to model, due to the strong conditioning on $x_t$. Moreover, the diffusion process smoothens the data distribution, making the discriminator less likely to overfit. Thus, without being limited to any one theory of operation, the ddGAN model is expected to exhibit better training stability and mode coverage. Moreover, for different consecutive states $t$, $xt$ has different levels of perturbation, and hence using a single network to predict $x_{t-1}$ directly at different $t$ may be difficult. However, in some implementations, using the ddGAN the generator predicts unperturbed $x_0$ and then add back perturbation using $q(x_{t-1} \mid x_t, x_0)$, allowing for a simpler and more accurate prediction target. *See, e.g.,* Xiao et al., "Tackling the Generative Learning Trilemma with Denoising Diffusion GANs," ICLR 2022, each of which is hereby incorporated herein by reference in its entirety.

Referring to block 710, in some embodiments, the plurality of target metrics for the one or more target biological properties of the biological molecule are incorporated into one or more respective layers in the plurality of layers of the transition model.

Referring to block 712, in some embodiments, the plurality of layers of the transition model comprises one or more attention layers that attend to an embedding of the plurality of target metrics for the one or more target biological properties of the biological molecule. In some embodiments, the plurality of layers of the transition model comprises one or more projection layers that project the plurality of target metrics for the one or more target biological properties on an output of a previous layer in the plurality of layers of the transition model using a mapping function between the plurality of target metrics for the one or more target biological properties of the biological molecule and a corresponding set of weights for the projection layer. In some embodiments, the transition model comprises a U-Net neural network having a first plurality of layer blocks and a second plurality of layer blocks, where: each respective layer block in the first plurality of layer blocks comprises a residual network layer and a down-sampling layer; each respective layer block in the second plurality of layer blocks comprises a residual network layer and an up-sampling layer; and at least one respective block layer in the first plurality of block layers or the second plurality of block layers further comprises a respective projection layer in the one or more projection layers. In some embodiments, at least one respective block layer in the first plurality of block layers further comprises a respective projection layer in the one or more projection layers; and at least one respective block layer in the second plurality of block layers further comprises a respective projection layer in the one or more projection layers. In some embodiments, each respective block layer in the first plurality of block layers further comprises a respective projection layer in the one or more projection layers; and each respective block layer in the second plurality of block layers further comprises a respective projection layer in the one or more projection layers.

Referring to block 714, in some embodiments, the generative diffusion model is a bit diffusion model. In some embodiments, the obtaining as output from the generative diffusion model a sequence for the biological molecule comprises obtaining a representation of the nucleic acid or amino acid sequence for the biological molecule that is analog bit encoded, and applying a thresholding operation to the representation of the nucleic acid or amino acid sequence for the biological molecule, thereby obtaining nucleic acid or amino acid sequence for the biological molecule. Bit diffusion models are trained by representing discrete data as binary bits and then training a continuous diffusion model to model these bits as real numbers, referred to as analog bits. To generate samples, the model first generates the analog bits, which are then thresholded to obtain the bits that represent the discrete variables. Analog bits refer to real numbers used to model the bits that represent discrete data (*e.g.*, residue positions, identities, and/or other sequence characteristics). In some implementations, analog bits are modeled by continuous state diffusion models, without requiring a discrete state space or re-formulation of the continuous diffusion process. In some embodiments, as described below, the seed for the nucleic acid or amino acid sequence is further analog bit encoded. For a further description of bit diffusion models see, for example, Chen T. et al., Analog Bits: Generating Discrete Data using Diffusion Models with Self-Conditioning, arXiv:2208.04202 (2023), which is disclosed herein by reference in its entirety. In some embodiments, the analog bits are transformed back to the bits that represent the discrete data (*e.g.*, residue positions, identities, and/or other sequence characteristics) using a thresholding operation. In some embodiments, the thresholding operation is applied to a plurality of values in the analog bit encoded representation of the sequence. In some embodiments, the thresholding operation generates a bimodal and/or binary distribution of the plurality of values.

In some embodiments, the bit diffusion model utilizes a U-Net architecture. As described above, U-Net models typically comprise a U structure with downsampling (contracting) and upsampling (expansive) paths, such that the expansive path is relatively symmetric to the contracting path. Such structure allows the model to propagate information from lower to higher resolution layers. In particular, high resolution features from the downsampling path are combined with upsampled output in the upsampling path to increase the resolution of the output and assemble a more precise output. An exemplary structure of a U-Net is illustrated, for instance, in the denoising U-Net schematic 2302 in FIGS. 23A-B. See, for example, Ronneberger, "U-Net: Convolutional Networks for Biomedical Image Segmentation," 2015, arXiv:1505.04597v1, available on the Internet at arxiv.org/pdf/1505.04597.pdf, which is hereby incorporated herein by reference in its entirety. In some implementations, as illustrated in FIGS. 23A-B, the U-Net architecture comprises a plurality of layer blocks (illustrated in blocks of three), each including at least a residual network layer and a down-sampling or up-sampling layer. In some implementations corresponding residual network layers having the same spatial resolutions (*e.g.*, n x m) are connected by skip connections (dashed gray arrows). Respective layer blocks further include conditioning layers projecting embeddings of corresponding conditions, as illustrated by solid curved arrows (*e.g.*, mRNA target sequence, target metrics, and/or a time step for the reverse diffusion process (*e.g.*, indicating where in the Markov chain the diffusion process is)), to condition the model.

In some embodiments, a nucleotide sequence having *n* nucleotides is represented as *n* discrete bit tokens, *e.g.,* 2-bit tokens, 3-bit tokens, 4-bit tokens, *etc.* Referring to block 716, in some embodiments, the biological molecule is a nucleic acid and the seed of the nucleic acid sequence is one hot encoded into a 2-bit encoding. That is, each character in the 2-bit encoding represents a different nucleotide, *e.g.*, 00 = adenine, 01 = cytosine, 10 = guanine, and 11 = thymine/uridine, or any other assignment of the four nucleic acids to the four characters of a 2-bit encoding.

In some embodiments, the biological molecule is a nucleic acid and the seed of the nucleic acid sequence is encoded (*e.g.,* one hot encoded) into a 4-bit encoding (*e.g.*, binary encoding for each nucleic acid A, T, G, and/or C). In some embodiments, the biological molecule is a polynucleotide and the sequence of the polynucleotide is represented with a 4-bit encoding. For example, in some embodiments, each position of the 4-bit character represents a different nucleotide, *e.g.*, the first position (*e.g.*, character 1000) represents adenine, the second position (*e.g.*, character 0100) represents cytosine, the third position (*e.g.*, character 0010) represents guanine, and the fourth position (*e.g.*, character 0001) represents thymine/uridine, or any other assignment of the four natural nucleic acids to the sixteen characters of a 4-bit encoding. In some embodiments, the encoding of the polynucleotide sequence, *e.g.,* a seed for the sequence of the polynucleotide, is mapped from {0, 1} to {-1, 1}. That is, "0's" in the encoding are mapped to "-1's" and the value is allowed to float between - 1 and 1, rather than between 0 and 1, during denoising.

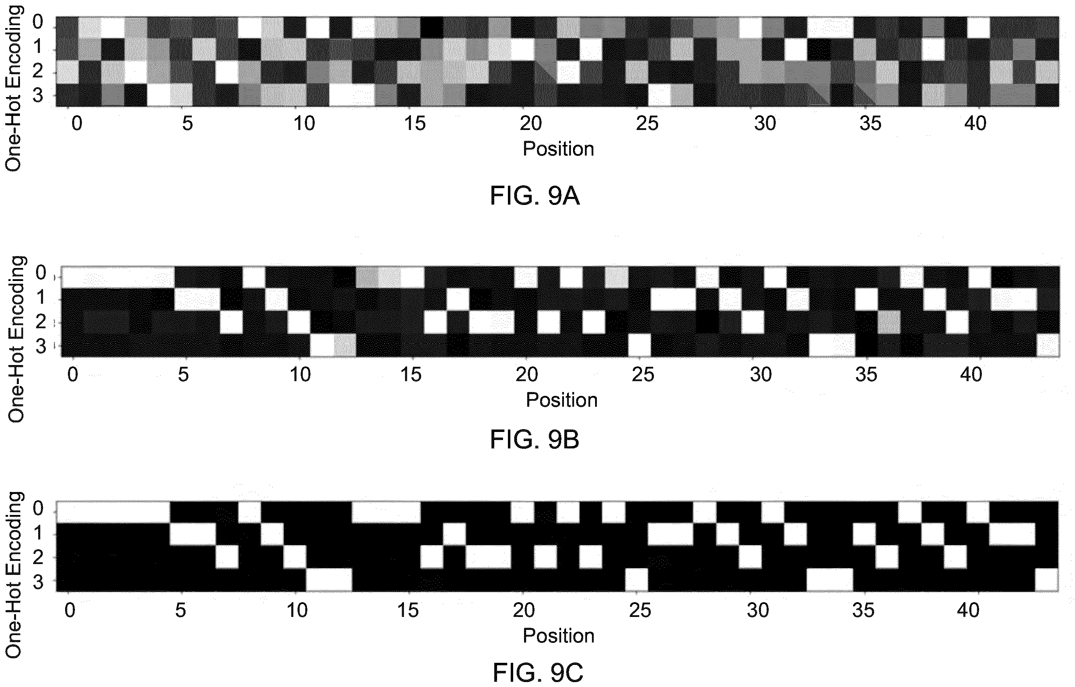

FIGS. 9A-C collectively illustrate an example generation of a 4-bit, one hot encoded nucleic acid sequence from a seed sequence, in accordance with an embodiment of the present disclosure. FIG. 9A illustrates a seed sequence comprising random noise, in which a value is provided (represented as a color and/or an intensity of shading) for each respective nucleic acid identity in a plurality of 4 nucleic acid identities (*e.g.*, A, T, G, and/or C), at each respective position in a plurality of positions in a nucleic acid sequence. (*e.g.,* 45 positions). FIG. 9B illustrates an intermediate stage of denoising, in which the values corresponding to each respective nucleic acid identity, for each respective position in the plurality of positions, are partially denoised and thus less randomly diffused. FIG. 9C illustrates a 4-bit, one hot encoded nucleic acid sequence, in which, at each respective position in the plurality of positions, the identity of the nucleic acid at the respective position is indicated by a 1 or a 0 for each of the 4 possible nucleic acid identities. A 1 indicates where the nucleic acid identity is present, and a 0 indicates where the nucleic acid identity is absent. Alternatively or additionally, in some embodiments, the binary encoding comprises white/black, yes/no, -1/1, and/or any other suitable binary indicators, as will be apparent to one skilled in the art. Accordingly, for a first position (Position 0), the 4 possible nucleic acid identities are represented in the figure as labels "0,", "1," "2," and "3." At position 0, the nucleic acid sequence is one hot encoded such that nucleic acid "0" is present (white) and all other nucleic acids "1," "2," and "3" are absent (black).

In some embodiments, the encoding of the polynucleotide sequence, *e.g.,* a seed for the sequence of the polynucleotide, is mapped from {0, 1} to {-1, 1}. That is, "0's" in the encoding are mapped to "-1's" and the value is allowed to float between -1 and 1, rather than between 0 and 1, during denoising. Referring to block 718, in some embodiments, the 2-bit encoding for the seed of the nucleic acid sequence is mapped from {0, 1} to {-1, 1}.

Referring to block 720, in some embodiments, the transition model generates, for each respective consecutive state $X_n$ in the plurality of consecutive states $X_N$ in the Markov chain following the initial state $X_1$, a corresponding nucleic acid or amino acid sequence predicted by the conditional generator model to confer on the biological molecule the one or more target biological properties approximating the plurality of target metric vales using as input: the seed for the nucleic acid or amino acid sequence, when the respective state $X_n$ is the state immediately following the initial state $X_1$, and the corresponding denoised seed sequence from the respective state $X_{n-1}$ in the plurality of consecutive states $X_N$ in the Markov chain that immediately precedes the respective state $X_n$, when the respective state $X_n$ is not the state immediately following the initial state $X_1$; and for each respective consecutive state $X_n$ in the plurality of consecutive states $X_N$ in the Markov chain following the initial state $X_1$, other than a terminal state $X_{n=N}$ in the Markov chain, the diffusion model samples from a posterior distribution of seed values for the corresponding nucleic acid or amino acid sequence to generate the corresponding denoised seed for the nucleic acid or amino acid sequence.

Referring to block 722, in some embodiments, for a respective consecutive state $X_n$ in the plurality of consecutive states $X_N$ in the Markov chain, the corresponding nucleic acid or amino acid sequence predicted by the conditional generator model to confer on the biological molecule the one or more target biological properties approximating the plurality of target metric vales is self-conditioned on the corresponding nucleic acid or amino acid sequence predicted by the conditional generator model to confer on the biological molecule the one or more target biological properties from the respective state $X_{n-1}$ in the plurality of consecutive states $X_N$ in the Markov chain that immediately precedes the respective state $X_n$.

Referring to block 724, in some embodiments, the biological molecule is a nucleic acid.

Referring to block 726, in some embodiments, the nucleic acid is a transcriptional or translational regulatory element.

Referring to block 728, in some embodiments, the nucleic acid is a guide RNA (gRNA) that facilitates deamination of one or more target adenosines in a target RNA by an Adenosine Deaminases Acting on RNA (ADAR) protein.

Referring to block 730, in some embodiments, the nucleic acid is a gRNA that facilitates deamination of one or more target cytidines in a target RNA by an Apolipoprotein B mRNA Editing Catalytic Polypeptide-like (APOBEC) protein.

Referring to block 732, in some embodiments, the one or more target biological properties comprises a metric for the efficiency of deamination of the one or more target adenosines by a first ADAR protein or the one or more target cytidines by a first APOBEC protein.

Referring to block 734, in some embodiments, the metric for the efficiency of deamination is (i) a prevalence of deamination of the one or more target adenosines or the one or more target cytidines in a plurality of instances of the target mRNA or (ii) a prevalence of the absence of deamination of any nucleotide position in a respective instance of a target mRNA in a plurality of instances of the target mRNA.

Referring to block 736, in some embodiments, the one or more target biological properties comprises a metric for the specificity of deamination of the one or more target adenosines or the one or more target cytidines relative to one or more nucleotide positions, other than the nucleotide positions of the one or more target adenosines or the one or more target cytidines, in a target mRNA by a first ADAR protein or a first APOBEC protein.

Referring to block 738, in some embodiments, the metric for the specificity of deamination of the target nucleotide position relative to one or more nucleotide positions, other than the target nucleotide position, in the target mRNA by the first ADAR protein or the first APOBEC protein is: (i) a comparison of (a) a prevalence of deamination of the target nucleotide position in a plurality of instances of the target mRNA and (b) a prevalence of deamination of at least one nucleotide position, other than the target nucleotide position, in a respective instance of the target mRNA in a plurality of instances of the target mRNA, (ii) a prevalence of deamination of the target nucleotide position, without coincident deamination of one or more nucleotide positions other than the target nucleotide position, in a respective instance of the target mRNA in a plurality of instances of the target mRNA, or (iii) a prevalence of deamination of at least one nucleotide position, other than the target nucleotide position, in a respective instance of the target mRNA in a plurality of instances of the target mRNA.

Referring to block 740, in some embodiments, at the one or more nucleotide positions, other than the target nucleotide position, in the target mRNA, deamination results in a non-synonymous codon edit.

Referring to block 742, in some embodiments, a respective biological property in the one or more target biological properties is normalized by a metric for an efficiency or specificity of deamination of one or more nucleotide positions, other than the target nucleotide position, in a target mRNA by a first ADAR protein or a first APOBEC protein.

Referring to block 744, in some embodiments, the one or more target biological properties comprises a metric for an efficiency or specificity of deamination of one or more nucleotide positions, other than the target nucleotide position, in a target mRNA by a first ADAR protein or first APOBEC protein when facilitated by hybridization of the gRNA to a target mRNA.

Referring to block 746, in some embodiments, the first ADAR protein is human ADAR1 or human ADAR2.

Referring to block 748, in some embodiments, the one or more target biological properties comprises an estimation of a minimum free energy (MFE) for the gRNA.

Referring to block 750, in some embodiments, the one or more target biological properties comprises an estimation of a minimum free energy (MFE) for the guide-target RNA scaffold formed between the guide RNA (gRNA) and the target mRNA.

In some embodiments, a polynucleotide sequence for the target mRNA, encompassing the target nucleotide position and at least a region of the mRNA 5' of the target nucleotide position and a region of the mRNA 3' of the target nucleotide position, is incorporated into one or more respective layers in the plurality of layers of the transition model. In some embodiments, the plurality of layers of the transition model comprises one or more attention layers that attend to an embedding of the polynucleotide sequence for the target mRNA (*e.g.*, where the embedding is generated using a multi-layer perceptron model). In some embodiments, the plurality of layers of the transition model comprises one or more projection layers that project the polynucleotide sequence for the target mRNA on an output of a previous layer in the plurality of layers of the transition model using a mapping function between an embedding of polynucleotide sequence for the target mRNA and a corresponding set of weights for the projection layer. In some embodiments, the transition model comprises a U-Net neural network having a first plurality of layer blocks and a second plurality of layer blocks, where: each respective layer block in the first plurality of layer blocks comprises a residual network layer and a down-sampling layer; each respective layer block in the second plurality of layer blocks comprises a residual network layer and an up-sampling layer; and at least one respective block layer in the first plurality of block layers or the second plurality of block layers further comprises a respective projection layer in the one or more projection layers. In some embodiments, at least one respective block layer in the first plurality of block layers further comprises a respective projection layer in the one or more projection layers; and at least one respective block layer in the second plurality of block layers further comprises a respective projection layer in the one or more projection layers. In some embodiments, each respective block layer in the first plurality of block layers further comprises a respective projection layer in the one or more projection layers; and each respective block layer in the second plurality of block layers further comprises a respective projection layer in the one or more projection layers.

Referring to block 752, in some embodiments, the biological molecule is a polypeptide. In some embodiments, the polypeptide is encoded by an amino acid sequence.

Referring to block 754, in some embodiments, the polypeptide is all or a portion of a capsid protein.

Referring to block 756, in some embodiments, the one or more target biological properties of the polypeptide comprise a measure of specificity of a recombinant Adeno Associated Virus (rAAV) comprising the capsid protein.

Referring to block 758, in some embodiments, the measure of specificity of the rAAV for a respective tissue type in the one or more tissue types is normalized by a measure of specificity of a wild type AAV of the same serotype as the rAAV for the respective tissue type.

Referring to block 760, in some embodiments, the measure of specificity of the rAAV for a respective tissue type in the one or more tissue types is normalized by a measure of specificity of the rAAV for one or more tissue types other than the respective tissue type.

In some embodiments, the methods and systems described herein relate to an amino acid sequence corresponding to amino acids 581-589 of the wild type AAV5 VP1 protein (SEQ ID NO:31) that confers increased CNS tropism to a recombinant AAV when the amino acid sequence is integrated into a capsid protein as compared the CNS tropism of an AAV when amino acids 581-589 of the wild type AAV5 VP1 protein (SEQ ID NO:31) are integrated into the same capsid protein. In some embodiments, the methods and systems described herein relate to an amino acid sequence corresponding to amino acids 581-589 of the wild type AAV5 VP1 protein (SEQ ID NO:31) that confers increased detargeting of a non-CNS tissue (*e.g.*, liver) to a recombinant AAV when the amino acid sequence is integrated into a capsid protein as compared to the detargeting of the non-CNS tissue by an AAV when amino acids 581-589 of the wild type AAV5 VP1 protein (SEQ ID NO:31) are integrated into the same capsid protein. In some embodiments, the methods and systems described herein relate to an amino acid sequence corresponding to amino acids 581-589 of the wild type AAV5 VP1 protein (SEQ ID NO:31) that confers both increased CNS tropism and increased detargeting of a non-CNS tissue (*e.g.*, liver) to a recombinant AAV when the amino acid sequence is integrated into a capsid protein as compared the CNS tropism and detargeting of an AAV when amino acids 581-589 of the wild type AAV5 VP1 protein (SEQ ID NO:31) are integrated into the same capsid protein.

In some embodiments, the seed for the amino acid sequence is selected from those sequences listed in Table 1. In some embodiments, the seed for the amino acid sequence is selected from those sequences listed in Table 1 having the abundance_enriched criteria. In some embodiments, the seed for the amino acid sequence is selected from those sequences listed in Table 1 having the ML_observed criteria. In some embodiments, the seed for the amino acid sequence is selected from those sequences listed in Table 1 having the multiple_nhp criteria. In some embodiments, the seed for the amino acid sequence is selected from those sequences listed in Table 1 having the multiple_samples criteria. In some embodiments, the seed for the amino acid sequence is selected from those sequences listed in Table 1 having the sequence_similarity criteria. In some embodiments, the seed for the amino acid sequence is selected from those sequences listed in Table 1 having the tissue_enriched criteria.

In some embodiments, the seed for the amino acid sequence has at least 70% sequence identity to a sequence listed in Table 1. In some embodiments, the seed for the amino acid sequence has at least 85% sequence identity to a sequence listed in Table 1. In some embodiments, the seed for the amino acid sequence has at least 70% or at least 85% sequence identity to a sequence listed in Table 1 having the abundance_enriched criteria. In some embodiments, the seed for the amino acid sequence has at least 70% or at least 85% sequence identity to a sequence listed in Table 1 having the ML_observed criteria. In some embodiments, the seed for the amino acid sequence has at least 70% or at least 85% sequence identity to a sequence listed in Table 1 having the multiple_nhp criteria. In some embodiments, the seed for the amino acid sequence has at least 70% or at least 85% sequence identity to a sequence listed in Table 1 having the multiple_samples criteria. In some embodiments, the seed for the amino acid sequence has at least 70% or at least 85% sequence identity to a sequence listed in Table 1 having the sequence_similarity criteria. In some embodiments, the seed for the amino acid sequence has at least 70% or at least 85% sequence identity to a sequence listed in Table 1 having the tissue_enriched criteria.

In some embodiments, the seed for the amino acid sequence is generated by modifying an amino acid sequence selected from those sequences listed in Table 1. In some embodiments, the seed for the amino acid sequence is generated by modifying an amino acid sequence selected from those sequences listed in Table 1. In some embodiments, the seed for the amino acid sequence is generated by modifying an amino acid sequence selected from those sequences listed in Table 1 having the ML_observed criteria. In some embodiments, the seed for the amino acid sequence is generated by modifying an amino acid sequence selected from those sequences listed in Table 1 having the multiple_nhp criteria. In some embodiments, the seed for the amino acid sequence is generated by modifying an amino acid sequence selected from those sequences listed in Table 1 having the multiple_samples criteria. In some embodiments, the seed for the amino acid sequence is generated by modifying an amino acid sequence selected from those sequences listed in Table 1 having the sequence_similarity criteria. In some embodiments, the seed for the amino acid sequence is generated by modifying an amino acid sequence selected from those sequences listed in Table 1 having the tissue_enriched criteria.

In some embodiments, the modifying comprises manually inputting a change to the amino acid sequence. In some embodiments, the modifying comprises systematically changing the amino acid sequence. In some embodiments, the modifying comprises randomly changing the amino acid sequence.

In some embodiments, the modifying comprises applying a gaussian noise filter to the amino acid sequence to generate a distribution of probabilities for the identity of one or more amino acid residues in the amino acid sequence.

In some embodiments, the seed for the amino acid sequence comprises, for each respective amino acid position in the amino acid sequence, a corresponding probability, for each respective amino acid in a plurality of amino acids, of the respective amino acid being present at the respective amino acid position.

In some embodiments, the amino acid sequence generated using the model is selected from those sequences listed in Table 1. In some embodiments, the amino acid sequence generated using the model is selected from those sequences listed in Table 1 having the abundance_enriched criteria. In some embodiments, the amino acid sequence generated using the model is selected from those sequences listed in Table 1 having the ML_observed criteria. In some embodiments, the amino acid sequence generated using the model is selected from those sequences listed in Table 1 having the multiple_nhp criteria. In some embodiments, the amino acid sequence generated using the model is selected from those sequences listed in Table 1 having the multiple_samples criteria. In some embodiments, the amino acid sequence generated using the model is selected from those sequences listed in Table 1 having the sequence_similarity criteria. In some embodiments, the amino acid sequence generated using the model is selected from those sequences listed in Table 1 having the tissue_enriched criteria.

In some embodiments, the amino acid sequence generated using the model has at least 70% sequence identity to a sequence listed in Table 1. In some embodiments, the amino acid sequence generated using the model has at least 85% sequence identity to a sequence listed in Table 1. In some embodiments, the amino acid sequence generated using the model has at least 70% or at least 85% sequence identity to a sequence listed in Table 1 having the abundance_enriched criteria. In some embodiments, the amino acid sequence generated using the model has at least 70% or at least 85% sequence identity to a sequence listed in Table 1 having the ML_observed criteria. In some embodiments, the amino acid sequence generated using the model has at least 70% or at least 85% sequence identity to a sequence listed in Table 1 having the multiple_nhp criteria. In some embodiments, the amino acid sequence generated using the model has at least 70% or at least 85% sequence identity to a sequence listed in Table 1 having the multiple_samples criteria. In some embodiments, the amino acid sequence generated using the model has at least 70% or at least 85% sequence identity to a sequence listed in Table 1 having the sequence_similarity criteria. In some embodiments, the amino acid sequence generated using the model has at least 70% or at least 85% sequence identity to a sequence listed in Table 1 having the tissue_enriched criteria.

In some embodiments, the method further comprises assembling a rAAV comprising the respective variant capsid protein having the candidate sequence and a nucleic acid.

In some embodiments, the method further comprises treating a disorder in a subject in need thereof by administering a rAAV comprising the respective variant capsid protein having the candidate sequence and a therapeutic nucleic acid to the subject.

In some embodiments, the biological molecule is an amino acid and the seed of the amino acid sequence is binary encoded. In some embodiments, the seed of the amino acid sequence is one hot encoded. Alternatively or additionally, in some embodiments, the seed of the amino acid sequence is encoded as discrete values. In some embodiments, the seed of the amino acid sequence is encoded using continuous values (*e.g.*, as a probability, ratio, fraction, or likelihood).

In some embodiments, the seed of the amino acid sequence comprises a plurality of positions, each respective position in the plurality of positions corresponding to a respective amino acid identity, and, for each respective position in the plurality of positions, the amino acid sequence comprises a corresponding encoding of the respective amino acid identity. In some embodiments, the encoding is one hot encoding of the respective amino acid identity.

In some implementations, the seed for the amino acid sequence is a tensor, each element in the tensor is a vector corresponding to a different amino acid position in the amino acid sequence, and each vector comprises, for each respective possible amino acid identity in a plurality of possible amino acid identities, a corresponding value for the respective amino acid identity. In some embodiments, the value comprises a one hot encoding of each of the plurality of possible amino acid identities such that the value indicates a presence or absence of the respective amino acid identity at the respective position. In some implementations, for example, the plurality of possible amino acid identities comprises at least 20 amino acid identities. In some embodiments, the plurality of possible amino acid identities comprises at least 22 amino acid identities. In an example embodiment, for a first position in the amino acid sequence, the corresponding vector is [1,0,0,0,0,0,0,0,0,0,0,0,0,0,0,0,0,0,0,0], where 1 indicates a presence for a first amino acid identity and 0 indicates an absence of all other amino acid identities.

In some embodiments, the seed for the amino acid sequence is not one hot encoded, and the seed for the amino acid sequence comprises, for each respective amino acid identity in a plurality of amino acid identities, a corresponding probability of the respective amino acid identity being present at the respective amino acid position.

In some embodiments, the seed for the amino acid sequence is 20-bit or 22-bit encoded.

In some embodiments, the amino acid comprises one or more feature properties and, for each respective feature property in the one or more feature properties, the seed for the amino acid sequence comprises a corresponding encoding of the respective feature property. In some embodiments, the encoding is one hot encoding of the respective feature property.

In some embodiments, the seed for the amino acid sequence is a vector, each element in the vector comprises a corresponding value for a different feature property of the amino acid in one or more feature properties. In some embodiments, the value comprises a one hot encoding of each of the one or more feature properties such that the value indicates that the amino acid comprises or does not comprise the respective feature property. For instance, in an example embodiment, the corresponding vector for a seed for an amino acid sequence is [1,0,0,0,0] for an amino acid comprising five feature properties, where 1 indicates that the amino acid comprises (*e.g.*, is characterized by) a first feature property (*e.g.*, negative charge) and 0 indicates that the amino acid does not comprise all other feature properties (*e.g.*, nonpolar aliphatic, nonpolar aromatic, polar, positive charge). Yet another example encoding for a seed for an amino acid sequence includes [0,0,0,0,1], where 1 indicates that the amino acid comprises the fifth feature property and 0 indicates that the amino acid does not comprise the first, second, third, and fourth feature properties. Various combinations of feature properties that characterize an amino acid are possible, as in the following examples: [01000], [01001], [01100], [01101], [00010], [10010], and so on.

In some embodiments, the seed for the amino acid sequence is not one hot encoded, and the seed for the amino acid sequence comprises, for each respective feature property in one or more feature properties, a corresponding probability that the respective amino acid comprises (*e.g.*, is characterized by) the respective feature property.

In some embodiments, the one or more feature properties comprises at least 3, at least 5, or at least 10 feature properties. In some embodiments, the one or more feature properties comprises at least 1, at least 3, at least 5, at least 10, at least 20, at least 50, at least 100, or at least 500 feature properties. In some embodiments, the one or more feature properties comprises no more than 1000, no more than 500, no more than 100, no more than 50, no more than 20, no more than 10, no more than 5, or no more than 3 feature properties. In some embodiments, the one or more feature properties consists of from 1 to 5, from 3 to 10, from 8 to 30, from 20 to 100, from 50 to 300, or from 200 to 1000 feature properties. In some embodiments, the one or more feature properties falls within another range starting no lower than 1 feature property and ending no higher than 1000 feature properties.

In some embodiments, the one or more feature properties are selected from the group consisting of negative charge, positive charge, nonpolar aliphatic, nonpolar aromatic, and polar.

In some embodiments, the one or more feature properties are selected from the group consisting of high expression, low expression, moderate expression, tissue-specific expression, target tissue expression, and peripheral tissue expression.

In some embodiments, the one or more feature properties are selected from the group consisting of a charge characteristic, a hydropathy value, a solubility value, a phosphorylation characteristic, a flexibility value, a ionic bonding characteristic, a hydrogen bonding characteristic, a hydrophilicity value, a surface accessibility value, a mutability value, a hydrogen bonding donor characteristic, a hydrogen bonding acceptor characteristic, an aggregate hydrogen bonding characteristic, a molecular mass value, a volume value, and a hydrophobicity value.

In some embodiments, charge refers to the electrostatic property of the amino acid side chain as an acid or base, having a positive or negative charge in an aqueous solvent at neutral pH. In some embodiments, phosphorylation refers to whether the functional group of an amino acid residue can have a phosphate group added as a post-translational modification. In some embodiments, ionic bond refers to the capacity of an amino acid residue side chain to participate in electrostatic interactions. In some embodiments, hydrogen bond refers to the capacity of an amino acid residue side chain to participate in hydrogen bond(s), hydrogen bond donor refers to the number of amino acid residue side chain atoms that can donate a hydrogen atom to a hydrogen bond under neutral pH conditions, and hydrogen bond acceptor refers to the number of amino acid residue side chain atoms that can accept a donor hydrogen atom in a hydrogen bond under neutral pH conditions. In some embodiments, aggregate hydrogen bond refers to the number of amino acid residue side chain atoms that can participate in a hydrogen bond. In some embodiments, molecular mass is the predicted molecular weight of an amino acid residue in unit Daltons. In some embodiments, volume refers to the predicted volume of a given amino acid residue in aqueous solution (*see, e.g.,* Zamyatnin, A.A., Protein volume in solution, Prog. Biophys. Mol. Biol., 24:107-123 (1972)). In some embodiments, hydropathy represents the hydrophobic (repels water) or hydrophilic (attracts water) properties of the side chain of a given amino acid residue (*see, e.g.,* Kyte and Doolittle, J. Mol. Biol., 157:105-132 (1982)). In some embodiments, the one or more features is hydrophobicity, where hydrophobicity is measured using Goldman Engelman Steitz, referring to the free energy transfer from amino acid residues in an alpha-helix from non-aqueous condition to water (*see, e.g.,* Engelman et al., Annu. Rev. Biophys. Chem., 15:321-353 (1986)). In some embodiments, flexibility refers to the symmetric or asymmetric distribution of amino acid residues in polypeptides (*see, e.g.,* Bhaskaran, R. & Ponnuswamy, P.R., Int. J. Peptide and Protein Res., 32:4:241-255 (1988)). In some embodiments, mutability refers to the probability that a given amino acid residue would change in across an evolutionary interval, and is calculated by the relative frequency at which a residue is replaced with another (*see, e.g.,* Dayhoff et al., Atlas of Protein Sequence and Structure, Vol. 5, Suppl.3 (1978)).

In some embodiments, the seed for the amino acid sequence is at least 4-bit, at least 5-bit, or at least 6-bit encoded. In some embodiments, the seed for the amino acid sequence is at least 2-bit, at least 3-bit, at least 4-bit, at least 5-bit, at least 6-bit, at least 7-bit, at least 8-bit, at least 10-bit, at least 20-bit, at least 50-bit, at least 100-bit, or at least 500-bit encoded. In some embodiments, the seed for the amino acid sequence is no more than 1000-bit, no more than 500-bit, no more than 100-bit, no more than 50-bit, no more than 20-bit, or no more than 10-bit encoded. In some embodiments, the seed for the amino acid sequence is from 2-bit to 10-bit, from 5-bit to 100-bit, from 50-bit to 500-bit, or from 200-bit to 1000-bit encoded. In some embodiments, the encoding for the seed for the amino acid sequence falls within another range starting no lower than 2-bit and ending no higher than 1000-bit.

In some embodiments, the seed for the amino acid sequence is randomly encoded. For instance, in some embodiments, the seed for the amino acid sequence is randomly bit encoded (binary) and/or randomly encoded as continuous or discrete variables.

In some embodiments, the seed for the amino acid sequence is binary encoded and a scale of each respective value in a plurality of values for the seed is transformed from {0, 1} to {-1, 1}. In some embodiments, the seed for the amino acid sequence is analog bit encoded.

For instance, in an example embodiment, a seed for an amino acid sequence comprises a corresponding vector [0,1,0,0,1] having a scale of {0, 1}, and the transformation transforms the respective vector to [-1, 1, -1, -1, 1].

In some embodiments, an output for a respective model (e.g., a generative and/or diffusion model) is an amino acid sequence that is binary (e.g., bit) encoded, one hot encoded, and/or analog bit encoded. In some embodiments, the method further includes decoding the outputted amino acid sequence from the respective model.

*Conditioning.*

In some embodiments, the inputting a plurality of target metric values for one or more target biological properties of the biological molecule conditions the model to generate, as output, the nucleic acid or amino acid sequence such that the biological molecule is likely to have the one or more target biological properties approximating the plurality of target metrics.

For instance, in some embodiments, an input to a generative model (e.g., a generative conditional adversarial network, a generative conditional diffusion model, and/or a bit diffusion model) further comprises one or more conditions.

In some embodiments, the one or more target biological properties comprise one or more conditions, and the plurality of target metrics for the one or more target biological properties comprise one or more values that approximate the one or more conditions or a metric thereof. In some embodiments, a condition is a property of a sequence, such as a target biological property or a variable of interest. In some embodiments, a target biological property and/or a variable of interest is a performance metric.

In some embodiments, the plurality of target metrics comprises at least 2, at least 3, at least 4, at least 5, at least 6, at least 7, at least 8, at least 9, at least 10, at least 15, at least 20, or at least 30 target metrics. In some embodiments, the plurality of target metrics comprises no more than 50, no more than 30, no more than 20, no more than 10, no more than 5, or no more than 3 target metrics. In some embodiments, the plurality of target metrics consists of from 2 to 10, from 5 to 20, from 12 to 40, or from 20 to 50 target metrics. In some embodiments, the plurality of target metrics falls within another range starting no lower than 2 metrics and ending no higher than 50 metrics.

In some embodiments, the one or more target biological properties comprises at least 1, at least 2, at least 3, at least 4, at least 5, at least 6, at least 7, at least 8, at least 9, at least 10, at least 15, at least 20, or at least 30 target biological properties. In some embodiments, the one or more target biological properties comprises no more than 50, no more than 30, no more than 20, no more than 10, no more than 5, or no more than 2 target biological properties. In some embodiments, the one or more target biological properties consists of from 1 to 10, from 5 to 20, from 12 to 40, or from 20 to 50 target biological properties. In some embodiments, the one or more target biological properties falls within another range starting no lower than 1 property and ending no higher than 50 properties.

In some embodiments, the one or more target biological properties are selected from the group consisting of editing efficiency, editing specificity, normalized editing specificity, ADAR1-specific editing efficiency, ADAR2-specific editing efficiency, and ADAR1/2-specific editing efficiency. In some embodiments, the target biological property is a regression variable.

In some embodiments, a respective target metric in the plurality of target metrics comprises a range of values for a corresponding target biological property. In some embodiments, a respective target metric in the plurality of target metrics comprises a category or label that represents a range of values.

In some implementations, a respective target metric comprises (i) a first label that represents a first range of values for the corresponding target biological property or (ii) a second label that represents a second range of values for the corresponding target biological property. For example, in some embodiments, the first category is determined (e.g., positive) when the first range of values is greater than or equal to a first threshold value, and the second category is determined (e.g., negative) when the second range of values is less than the first threshold value.

In some embodiments, the first threshold value is between 0.5 and 0.9. In some embodiments, the first threshold value is at least 0.4, at least 0.5, at least 0.6, at least 0.7, or at least 0.8. In some embodiments, the first threshold value is no more than 1.1, no more than 1.0, no more than 0.9, no more than 0.8, or no more than 0.7. In some embodiments, the first threshold value is from 0.4 to 0.8, from 0.6 to 0.9, or from 0.7 to 1.1. In some embodiments, the first threshold value falls within another range starting no lower than 0.4 and ending no higher than 1.1.

In some embodiments, the respective target metric is further determined based on a second threshold value that is the same or different from the first threshold value (e.g., a range of values greater than 0.7 and less than 1.1).

In some embodiments, a condition is a target metric, as illustrated in FIGS. 1C, 1E, 2E, and 3B. In some embodiments, a condition is an experimental metric, as illustrated in FIGS. 1B, 2C, and 3A. In some embodiments, a condition is a theoretical metric or a metric that is assigned a predetermined value.

Example embodiments of one or more conditions used for conditioning a model are illustrated in FIGS. 1B-C, 2C, and 2E. For example, the dashed boxes in the illustrated experimental and/or target metrics (e.g., 0.8, 0.4, 0.9) represent optional metrics that can be included as input to the model together with a seed for a nucleic acid or amino acid sequence. In some embodiments, the one or more conditions is a plurality of conditions.

In some embodiments, the plurality of conditions comprises at least 2, at least 3, at least 4, at least 5, at least 6, at least 7, at least 8, at least 9, at least 10, at least 15, at least 20, or at least 30 conditions. In some embodiments, the plurality of conditions comprises no more than 50, no more than 30, no more than 20, no more than 10, no more than 5, or no more than 3 conditions. In some embodiments, the plurality of conditions consists of from 2 to 10, from 5 to 20, from 12 to 40, or from 20 to 50 conditions. In some embodiments, the plurality of conditions falls within another range starting no lower than 2 conditions and ending no higher than 50 conditions.

In some embodiments, the model accepts, as input, a tensor comprising (i) the plurality of target metric values for the one or more target biological properties of the biological molecule and (ii) the seed for a nucleic acid or amino acid sequence for the biological molecule, wherein the plurality of target metric values is concatenated to the seed for the nucleic acid or amino acid sequence. *See,* for example, the experimental and/or target metrics illustrated in FIGS. 1B-C.

In some embodiments, the input to the model is a tensor comprising a plurality of elements, the plurality of elements comprising at least (i) one or more elements corresponding to positions in a nucleic acid or peptide sequence and (ii) one or more elements corresponding to conditions.

In some embodiments, the input tensor comprises at least 5, at least 10, at least 20, at least 50, at least 100, at least 200, or at least 300 elements. In some embodiments, the input tensor comprises no more than 500, no more than 300, no more than 200, no more than 100, no more than 50, or no more than 10 elements. In some embodiments, the input tensor consists of from 5 to 50, from 20 to 100, from 80 to 300, or from 100 to 500 elements. In some embodiments, the input tensor falls within another range starting no lower than 5 elements and ending no higher than 500 elements.

In some embodiments, a first input has the same or different length as a second input. In other words, in some implementations, the model can accommodate inputs of different lengths, depending on the length of the seed for the nucleic acid or amino acid sequence and/or the number of conditions to be included in the input.

In some embodiments, for each respective condition, the model accepts, as input, a corresponding conditioning value. In some implementations, a corresponding conditioning value is a value between 0 and 100. In some implementations, a corresponding conditioning value is an integer value or a decimal value.

In some embodiments, the one or more conditions are incorporated into the model during training. For instance, as described above with reference to FIGS. 1A-E, training a generative model comprises inputting into the model (i) a seed for a nucleic acid or amino acid sequence ("seed") and (ii) target performance metrics ("target metrics"). The model outputs a "generated" sequence. The addition of target metrics is referred to as "conditioning" and applies additional constraints on the generative model beyond simply generating sequences.

In some embodiments, training the model further comprises generating predicted metrics for the generated sequence. For instance, in some implementations, the predicted metrics are obtained using experimental validation, high-throughput screening, and/or by inputting the generated sequence into a trained model that predicts performance metrics based on input sequences (*e.g.,* a CNN or XGBoost model). In some embodiments, training the model further includes adjusting one or more parameters of the generative model based on a difference between the conditions (*e.g.,* the "true" or target properties) and the predicted metrics predicted for the generated sequence. Methods for training generative models, including adjusting one or more parameters, suitable for use in the present disclosure are known in the art, as described above.

Alternatively or additionally, in some embodiments, the model is a diffusion conditional generative adversarial network (GAN), and the model is trained by comparing a denoising output to a diffusion input, where the denoising output includes (i) a sequence generated by denoising of a diffused sequence from time step T to a time step t-1 and (ii) one or more conditions, and where the diffusion input includes (i) a sequence generated by diffusion of an undiffused sequence from time step 0 to time step t-1 and (ii) the one or more conditions. Generally, the one or more conditions are not altered during the training process. In other words, in some implementations, the conditioning values allow the model to "learn" how to construct a denoised sequence, given respective values for the one or more conditions. In some embodiments, the training is performed adversarially using a discriminator, as described above with reference to FIGS. 1A and 3A.

In some embodiments, the method further comprises evaluating the nucleic acid or amino acid sequence for the biological molecule that is outputted by the model using a validation model that generates, as output from the validation model, a corresponding predicted metric for the nucleic acid or amino acid sequence. In some embodiments, the validation model is a CNN or XGBoost model for predicting one or more performance metrics.

In some embodiments, the performance metric is editing, selectivity, normalized selectivity, ADAR1-specific activity, ADAR2-specific activity, ADAR1/2-specific activity, and/or minimum free energy.

In some embodiments, the corresponding performance metric for the generated nucleic acid or amino acid sequence is selected based on a condition used to condition the model that generated the nucleic acid or amino acid sequence.

In some embodiments, the corresponding predicted metric is selected from the plurality of target metric values for the one or more target biological properties, and the method further includes comparing the corresponding predicted metric to a corresponding target metric value in the plurality of target metric values.

*Regulatory Sequences.*

In some embodiments, the nucleic acid sequence is a regulatory element or regulatory sequence. In some embodiments, the regulatory element or regulatory sequence is an enhancer or a repressor. In some embodiments, an enhancer is paired with a core promoter to generate a promoter, in which the enhancer enhances transcription of downstream nucleic acid sequence. In some embodiments, a repressor is paired with a core promoter to generate a promoter, in which the repressor represses transcription of a downstream nucleic acid sequence. In some embodiments, an insulator is paired with a core promoter and/or enhancer, in which the insulator modifies trans-activation of the enhancer sequence with the core promoter. In some embodiments, the nucleic acid sequence is all or a portion of a promoter sequence.

In some embodiments, a regulatory element comprises nucleotide sequences, such as promoters, enhancers, terminators, polyadenylation sequences, introns, *etc.,* that provide for the expression of a coding sequence in a cell. In some embodiments, a promoter (alternatively "promoter element") comprises a DNA regulatory element that coordinates expression of a coding sequence (*e.g.,* RNA transcription). Generally, promoter elements are located 5' of the translation start site of a gene. In some embodiments, a promoter element is constitutively active (*e.g.,* Jet, CMV, or minCMV) such that it drives effectively constant expression of the coding sequence. In other embodiments, a promoter element is inducible, such that expression of the coding sequence is driven only in the presence of a particular element or condition (either an endogenous or exogenous), such as doxycycline activating of the Tet promoter. In some embodiments, promoters coordinate transcription as part of a cellular genome or as an exogenous element (*e.g.,* a plasmid). In some embodiments, a CMV, CAG, JeT, EF1alpha, TetOn, PGK, MND, or minCMV promoter is used. In some embodiments, a CMV, CAG, JeT, EF1alpha, TetOn, PGK, MND, or minCMV promoter is used to drive expression of a protein coding gene. In some embodiments, a mU7, hU1, or hU6 promoter is used to drive expression of a gRNA.

In some embodiments, the regulatory element comprises a transcriptional termination signal. In some embodiments, a transcriptional termination signal occurs following an open reading frame sequence or other transcriptionally active nucleotide sequence and directs termination of transcription. Optionally, the element recruits other cellular proteins (e.g., poly A polymerase) to the site. This element initiates the process of releasing the newly synthesized RNA from the transcription machinery. Non-exhaustive examples of such elements include an SV40 polyadenylation signal, a bovine growth hormone (BGH) polyadenylation signal, a rabbit beta globin (rbGlob) polyadenylation signal, and a herpes simplex virus type 1 thymidine kinase (HSV TK) polyadenylation signal. The choice of transcriptional termination signals can depend upon the type of cells being used for the screening assay. For example, in some embodiments, prokaryotic cells use rho-dependent and rho-independent transcriptional termination mechanisms, the former of which relies upon formation of GC-rich hairpin in the RNA transcript. In some embodiments, eukaryotic cells also use different transcriptional termination mechanisms, dependent upon the RNA polymerase. For example, Polymerase II, which is primarily responsible for mRNA and miRNA transcription, relies on the recruitment of termination factors resulting in cleavage of the nascent RNA at a cleavage signal positioned between a polyadenylation signal and a GU-rich sequence. These elements are commonly referred to together as a polyadenylation sequence. Polymerase III, which is primarily responsible for expression of tRNA and other short RNA, relies on a specific sequence and RNA secondary structure to induce transcript cleavage, similar to the Rho-independent termination found in prokaryotes.

In some embodiments, the regulatory element comprises a promoter. In some implementations, the promoter directs the activation or inhibition of sequence expression. In some embodiments, the regulatory element comprises an enhancer. In some implementations, enhancers provide transcriptional signals to the promoter (*e.g.,* dependent on context such as cell type and cell state). Enhancers can function through clusters of transcription factor motifs; for instance, in some embodiments, enhancers use varying complexities with respect to the sequence (*e.g.,* vocabulary), organization, and combination (*e.g.,* syntax) of transcription factor motifs. Such complex "grammar" can be difficult to predict. Advantageously, the present disclosure utilizes machine learning approaches to identify enhancer syntax and vocabulary.

In some implementations, the model is a bit diffusion model, and the generated nucleic acid sequence is an enhancer sequence that is conditioned to confer upon the generated enhancer sequence improved performance over endogenous enhancers. In some embodiments, the plurality of target metrics for the one or more target biological properties comprises one or more conditions for the respective enhancer sequence.

In some embodiments, the one or more target biological properties are selected from the group consisting of gene expression activity, cellular activity, relative activity of a first cell type compared to a second cell type, presence or absence of one or more enriched motifs, and/or presence or absence of one or more k-mers. For instance, in some embodiments, the one or more target biological properties includes neuron activity, liver cell activity, and/or cancer cell activity (*e.g.* mouse primary neuron activity, HepG2 activity, *etc.,* or in a specific cell type (*e.g.,* hepatocyte, neuron, *etc.*) after *in vivo* administration, *e.g.*, administration to a mouse or non-human primate). In some embodiments, the plurality of target metrics for the one or more target biological properties comprises a metric determined based on the one or more target biological properties (*e.g.,* a fold change, a log fold change, a mean, a median, and/or a difference between an activity of a first cell type and an activity of a second cell type).

*Retraining.*

In some embodiments, the model is trained by a procedure comprising training the model on a first training dataset, and retraining the model on a second training dataset other than the first training dataset.

For example, in some embodiments, the model is a bit diffusion model that is trained on a first training dataset and retrained on a second training dataset. In some embodiments, the first training dataset is a tissue agnostic library and the second training dataset is a biology-informed tissue-specific library. In some embodiments, the first training dataset is a tandem repeat library.

In some embodiments, the method further comprises evaluating a generated enhancer sequence using a validation model that generates, as output from the validation model, a corresponding performance metric for the generated enhancer sequence (*e.g.,* a CNN ensemble and/or an XGBoost model for predicting one or more performance metrics). In some embodiments, the corresponding performance metric for the generated enhancer sequence is selected based on a condition used in generating the generated enhancer sequence (*e.g.,* a cellular activity and/or a relative activity of a first cell type compared to a second cell type (*e.g.,* increased activity in the first cell type and/or decreased activity in the second cell type)).

## 7.5. Additional Embodiments

Another aspect of the present disclosure provides a computer system including one or more processors and a non-transitory computer-readable medium including computer-executable instructions that, when executed by the one or more processors, cause the processors to perform any of the methods and/or embodiments disclosed herein.

Yet another aspect of the present disclosure provides a non-transitory computer-readable storage medium having stored thereon program code instructions that, when executed by a processor, cause the processor to perform any of the methods and/or embodiments disclosed herein.

## 7.6. Exemplary Embodiments

Embodiment 1 - A method for generating a polymer sequence for a biological molecule having one or more target biological properties, the method comprising: at a computer system comprising at least one processor and a memory storing at least one program for execution by the at least one processor: inputting (i) a plurality of target metrics for one or more target biological properties of a biological molecule and (ii) a seed for a nucleic acid or amino acid sequence for the biological molecule into an initial state $X_1$ in a plurality of consecutive states $X_N$ in a Markov chain of a generative diffusion model to obtain as output from the generative diffusion model a nucleic acid or amino acid sequence for the biological molecule that is predicted by the generative diffusion model to confer on the biological molecule the one or more target biological properties approximating the plurality of target metrics, wherein: for each respective consecutive state $X_n$ in the plurality of consecutive states $X_N$ in the Markov chain following the initial state $X_1$, the diffusion model generates a corresponding denoised seed for the nucleic acid or amino acid sequence for the biological molecule using a transition model, wherein the transition model comprises a plurality of layers, that accounts for the plurality of target metrics for the one or more target biological properties using as input: the seed for the nucleic acid or amino acid sequence, when the respective state $X_n$ is the state immediately following the initial state $X_1$, and a corresponding updated seed for the nucleic acid or amino acid sequence based on the corresponding denoised seed for the nucleic acid or amino acid sequence from the respective state $X_{n-1}$ in the plurality of consecutive states $X_N$ in the Markov chain that immediately precedes the respective state $X_n$, when the respective state $X_n$ is not the state immediately following the initial state $X_1$.

Embodiment 2 - A method for generating a polymer sequence for a biological molecule having one or more target biological properties, the method comprising: at a computer system comprising at least one processor and a memory storing at least one program for execution by the at least one processor: inputting (i) a plurality of target metric values for one or more target biological properties of a biological molecule and (ii) a seed for a nucleic acid or amino acid sequence for the biological molecule into a conditional generator model of a conditional generative adversarial network to obtain as output from the conditional generator model a nucleic acid or amino acid sequence for the biological molecule that is predicted by the conditional generator model to confer on the biological molecule the one or more target biological properties approximating the plurality of target metric values.

Embodiment 3 - The method of embodiment 1, wherein: an indication of the position, in the Markov chain, of the transition from the state that immediately precedes the respective consecutive state $X_n$ is incorporated into one or more respective layers in the plurality of layers of the transition model; and the generative diffusion model generates, for each respective consecutive state $X_n$ in the plurality of consecutive states $X_N$ in the Markov chain following the initial state $X_1$, the corresponding denoised seed for the nucleic acid or amino acid sequence for the biological molecule.

Embodiment 4 - The method of embodiment 3, wherein the plurality of layers of the transition model comprises one or more attention layers that attend to an embedding of the indication of the position, in the Markov chain, of the transition.

Embodiment 5 - The method of embodiment 3, wherein the plurality of layers of the transition model comprises one or more temporal projection layers that project the indication of the position, in the Markov chain, of the transition on an output of a previous layer in the plurality of layers of the transition model using a mapping function between the indication of the position, in the Markov chain, of the transition and a corresponding set of weights for the temporal projection layer.

Embodiment 6 - The method of any one of embodiments 1-5, wherein the transition model comprises a U-Net neural network.

Embodiment 7 - The method of embodiment 1, wherein: the transition model is a conditional generator model of a conditional generative adversarial network that generates, for each respective consecutive state $X_n$ in the plurality of consecutive states $X_N$ in the Markov chain following the initial state $X_1$, a corresponding nucleic acid or amino acid sequence predicted by the conditional generator model to confer on the biological molecule the one or more target biological properties approximating the plurality of target metric vales using as input: the seed for the nucleic acid or amino acid sequence, when the respective state $X_n$ is the state immediately following the initial state $X_1$, and a corresponding updated seed for the nucleic acid or amino acid sequence based on the corresponding denoised seed for the nucleic acid or amino acid sequence from the respective state $X_{n-1}$ in the plurality of consecutive states $X_N$ in the Markov chain that immediately precedes the respective state $X_n$, when the respective state $X_n$ is not the state immediately following the initial state $X_1$; and for each respective consecutive state $X_n$ in the plurality of consecutive states $X_N$ in the Markov chain following the initial state $X_1$, other than a terminal state $X_{n=N}$ in the Markov chain, the diffusion model samples from a posterior distribution of seed values for the corresponding nucleic acid or amino acid sequence to generate the corresponding denoised seed for the nucleic acid or amino acid sequence.

Embodiment 8 - The method of any one of embodiments 1-7, wherein the plurality of target metrics for the one or more target biological properties of the biological molecule are incorporated into one or more respective layers in the plurality of layers of the transition model.

Embodiment 9 - The method of embodiment 8, wherein the plurality of layers of the transition model comprises one or more attention layers that attend to an embedding of the plurality of target metrics for the one or more target biological properties of the biological molecule.

Embodiment 10 - The method of embodiment 8, wherein the plurality of layers of the transition model comprises one or more projection layers that project the plurality of target metrics for the one or more target biological properties on an output of a previous layer in the plurality of layers of the transition model using a mapping function between the plurality of target metrics for the one or more target biological properties of the biological molecule and a corresponding set of weights for the projection layer.

Embodiment 11 - The method of embodiment 10, wherein the transition model comprises a U-Net neural network having a first plurality of layer blocks and a second plurality of layer blocks, wherein: each respective layer block in the first plurality of layer blocks comprises a residual network layer and a down-sampling layer; each respective layer block in the second plurality of layer blocks comprises a residual network layer and an up-sampling layer; and at least one respective block layer in the first plurality of block layers or the second plurality of block layers further comprises a respective projection layer in the one or more projection layers.

Embodiment 12 - The method of embodiment 10, wherein: at least one respective block layer in the first plurality of block layers further comprises a respective projection layer in the one or more projection layers; and at least one respective block layer in the second plurality of block layers further comprises a respective projection layer in the one or more projection layers.

Embodiment 13 - The method of embodiment 10, wherein: each respective block layer in the first plurality of block layers further comprises a respective projection layer in the one or more projection layers; and each respective block layer in the second plurality of block layers further comprises a respective projection layer in the one or more projection layers.

Embodiment 14 - The method of any one of embodiments 1-13, wherein the generative diffusion model is a bit diffusion model, and wherein: the obtaining as output from the generative diffusion model a nucleic acid sequence for the biological molecule comprises: obtaining a representation of the nucleic acid or amino acid sequence for the biological molecule that is analog bit encoded, and applying a thresholding operation to the representation of the nucleic acid or amino acid sequence for the biological molecule, thereby obtaining nucleic acid or amino acid sequence for the biological molecule.

Embodiment 15 - The method of embodiment 14, wherein the biological molecule is a nucleic acid and the seed of the nucleic acid sequence is one hot encoded into a 2-bit encoding.

Embodiment 16 - The method of embodiment 15, wherein the 2-bit encoding for the seed of the nucleic acid sequence is mapped from {0, 1} to {-1, 1}.

Embodiment 17 - The method of any one of embodiments 14-16, wherein: the transition model generates, for each respective consecutive state $X_n$ in the plurality of consecutive states $X_N$ in the Markov chain following the initial state $X_1$, a corresponding nucleic acid or amino acid sequence predicted by the conditional generator model to confer on the biological molecule the one or more target biological properties approximating the plurality of target metric values using as input: the seed for the nucleic acid or amino acid sequence, when the respective state $X_n$ is the state immediately following the initial state $X_1$, and the corresponding denoised seed sequence from the respective state $X_{n-1}$ in the plurality of consecutive states $X_N$ in the Markov chain that immediately precedes the respective state $X_n$, when the respective state $X_n$ is not the state immediately following the initial state $X_1$; and for each respective consecutive state $X_n$ in the plurality of consecutive states $X_N$ in the Markov chain following the initial state $X_1$, other than a terminal state $X_{n=N}$ in the Markov chain, the diffusion model samples from a posterior distribution of seed values for the corresponding nucleic acid or amino acid sequence to generate the corresponding denoised seed for the nucleic acid or amino acid sequence.

Embodiment 18 - The method of embodiment 17, wherein, for a respective consecutive state $X_n$ in the plurality of consecutive states $X_N$ in the Markov chain, the corresponding nucleic acid or amino acid sequence predicted by the conditional generator model to confer on the biological molecule the one or more target biological properties approximating the plurality of target metric values is self-conditioned on the corresponding nucleic acid or amino acid sequence predicted by the conditional generator model to confer on the biological molecule the one or more target biological properties from the respective state $X_{n-1}$ in the plurality of consecutive states $X_N$ in the Markov chain that immediately precedes the respective state $X_n$.

Embodiment 19 - The method of any one of embodiments 1-18, wherein the biological molecule is a nucleic acid.

Embodiment 20 - The method of embodiment 19, wherein the nucleic acid is DNA and the model allows for adenine (A), cytosine (C), guanine (G), or thymine (T) at each position of the nucleic acid sequence.

Embodiment 21 - The method of embodiment 19, wherein the nucleic acid is RNA and the model allows for adenine (A), cytosine (C), guanine (G), or uracil (U) at each position of the nucleic acid sequence.

Embodiment 22 - The method of embodiment 20 or 21, wherein the model further allows for a modified nucleotide at one or more position of the nucleic acid sequence.

Embodiment 23 - The method of any one of embodiments 19-22, wherein the nucleic acid is a transcriptional or translational regulatory element.

Embodiment 24 - The method of any one of embodiments 19-22, wherein the nucleic acid is a guide RNA (gRNA) that facilitates deamination of one or more target adenosines in a target RNA by an Adenosine Deaminases Acting on RNA (ADAR) protein.

Embodiment 25 - The method of any one of embodiments 19-22, wherein the nucleic acid is a gRNA that facilitates deamination of one or more target cytidines in a target RNA by an Apolipoprotein B mRNA Editing Catalytic Polypeptide-like (APOBEC) protein.

Embodiment 26 - The method of embodiment 24 or 25, wherein the one or more target biological properties comprises a metric for the efficiency of deamination of the one or more target adenosines by a first ADAR protein or the one or more target cytidines by a first APOBEC protein.

Embodiment 27 - The method of embodiment 26, wherein the metric for the efficiency of deamination is (i) a prevalence of deamination of the one or more target adenosines or the one or more target cytidines in a plurality of instances of the target mRNA or (ii) a prevalence of the absence of deamination of any nucleotide position in a respective instance of a target mRNA in a plurality of instances of the target mRNA.

Embodiment 28 - The method of any one of embodiments 24-27, wherein the one or more target biological properties comprises a metric for the specificity of deamination of the one or more target adenosines or the one or more target cytidines relative to one or more nucleotide positions, other than the nucleotide positions of the one or more target adenosines or the one or more target cytidines, in a target mRNA by a first ADAR protein or a first APOBEC protein.

Embodiment 29 - The method of embodiment 28, wherein the metric for the specificity of deamination of the target nucleotide position relative to one or more nucleotide positions, other than the target nucleotide position, in the target mRNA by the first ADAR protein or the first APOBEC protein is: (i) a comparison of (a) a prevalence of deamination of the target nucleotide position in a plurality of instances of the target mRNA and (b) a prevalence of deamination of at least one nucleotide position, other than the target nucleotide position, in a respective instance of the target mRNA in a plurality of instances of the target mRNA, (ii) a prevalence of deamination of the target nucleotide position, without coincident deamination of one or more nucleotide positions other than the target nucleotide position, in a respective instance of the target mRNA in a plurality of instances of the target mRNA, or (iii) a prevalence of deamination of at least one nucleotide position, other than the target nucleotide position, in a respective instance of the target mRNA in a plurality of instances of the target mRNA.

Embodiment 30 - The method of embodiment 29, wherein, at the one or more nucleotide positions, other than the target nucleotide position, in the target mRNA, deamination results in a non-synonymous codon edit.

Embodiment 31 - The method of any one of embodiments 24-30, wherein a respective biological property in the one or more target biological properties is normalized by a metric for an efficiency or specificity of deamination of one or more nucleotide positions, other than the target nucleotide position, in a target mRNA by a first ADAR protein or a first APOBEC protein.

Embodiment 32 - The method of any one of embodiments 24-31, wherein the one or more target biological properties comprises a metric for an efficiency or specificity of deamination of one or more nucleotide positions, other than the target nucleotide position, in a target mRNA by a first ADAR protein or first APOBEC protein when facilitated by hybridization of the gRNA to a target mRNA.

Embodiment 33 - The method of any one of embodiments 24-32, wherein the first ADAR protein is human ADAR1 or human ADAR2.

Embodiment 34 - The method of any one of embodiments 24-33, wherein the one or more target biological properties comprises an estimation of a minimum free energy (MFE) for the gRNA.

Embodiment 35 - The method of any one of embodiments 24-34, wherein the one or more target biological properties comprises an estimation of a minimum free energy (MFE) for the guide-target RNA scaffold formed between the guide RNA (gRNA) and the target mRNA.

Embodiment 36 - The method of any one of embodiments 24-35, wherein a polynucleotide sequence for the target mRNA, encompassing the target nucleotide position and at least a region of the mRNA 5' of the target nucleotide position and a region of the mRNA 3' of the target nucleotide position, is incorporated into one or more respective layers in the plurality of layers of the transition model.

Embodiment 37 - The method of embodiment 36, wherein the plurality of layers of the transition model comprises one or more attention layers that attend to an embedding of the polynucleotide sequence for the target mRNA.

Embodiment 38 - The method of embodiment 36, wherein the plurality of layers of the transition model comprises one or more projection layers that project the polynucleotide sequence for the target mRNA on an output of a previous layer in the plurality of layers of the transition model using a mapping function between an embedding of polynucleotide sequence for the target mRNA and a corresponding set of weights for the projection layer.

Embodiment 39 - The method of embodiment 38, wherein the transition model comprises a U-Net neural network having a first plurality of layer blocks and a second plurality of layer blocks, wherein: each respective layer block in the first plurality of layer blocks comprises a residual network layer and a down-sampling layer; each respective layer block in the second plurality of layer blocks comprises a residual network layer and an up-sampling layer; and at least one respective block layer in the first plurality of block layers or the second plurality of block layers further comprises a respective projection layer in the one or more projection layers.

Embodiment 40 - The method of embodiment 38, wherein: at least one respective block layer in the first plurality of block layers further comprises a respective projection layer in the one or more projection layers; and at least one respective block layer in the second plurality of block layers further comprises a respective projection layer in the one or more projection layers.

Embodiment 41 - The method of embodiment 38, wherein: each respective block layer in the first plurality of block layers further comprises a respective projection layer in the one or more projection layers; and each respective block layer in the second plurality of block layers further comprises a respective projection layer in the one or more projection layers.

Embodiment 42 - The method of any one of embodiments 1-18, wherein the biological molecule is a polypeptide.

Embodiment 43 - The method of embodiment 42, wherein the model allows for any one of the twenty standard amino acids in the eukaryotic genetic code at each position of the amino acid sequence.

Embodiment 44 - The method of embodiment 43, wherein the model further allows for a non-standard amino acid at one or more positions of the amino acid sequence.

Embodiment 45 - The method of embodiment 42, wherein the polypeptide is all or a portion of a capsid protein.

Embodiment 46 - The method of embodiment 45, wherein the one or more target biological properties of the polypeptide comprise a measure of specificity of a recombinant Adeno Associated Virus (rAAV) comprising the capsid protein.

Embodiment 47 - The method of embodiment 46, wherein the measure of specificity of the rAAV for a respective tissue type in the one or more tissue types is normalized by a measure of specificity of a wild type AAV of the same serotype as the rAAV for the respective tissue type.

Embodiment 48 - The method of embodiment 46 or 47, wherein the measure of specificity of the rAAV for a respective tissue type in the one or more tissue types is normalized by a measure of specificity of the rAAV for one or more tissue types other than the respective tissue type.

Embodiment 49 - The method of any one of embodiments 42-48, wherein the seed for the amino acid sequence is selected from those sequences listed in Table 1.

Embodiment 50 - The method of any one of embodiments 42-48, wherein the seed for the amino acid sequence has at least 85% sequence identity to a sequence listed in Table 1.

Embodiment 51 - The method of any one of embodiments 42-48, wherein the seed for the amino acid sequence is generated by modifying an amino acid sequence selected from those sequences listed in Table 1.

Embodiment 52 - The method of embodiment 51, wherein the modifying comprises manually inputting a change to the amino acid sequence.

Embodiment 53 - The method of embodiment 51, wherein the modifying comprises systematically changing the amino acid sequence.

Embodiment 54 - The method of embodiment 51, wherein the modifying comprises randomly changing the amino acid sequence.

Embodiment 55 - The method of embodiment 51, wherein the modifying comprises applying a gaussian noise filter to the amino acid sequence to generate a distribution of probabilities for the identity of one or more amino acid residues in the amino acid sequence.

Embodiment 56 - The method of any one of embodiments 42-55, wherein the seed for the amino acid sequence comprises, for each respective amino acid position in the amino acid sequence, a corresponding probability, for each respective amino acid in a plurality of amino acids, of the respective amino acid being present at the respective amino acid position.

Embodiment 57 - The method of any one of embodiments 42-56, wherein the amino acid sequence generated using the model comprises a sequence listed in Table 1.

Embodiment 58 - The method of any one of embodiments 42-56, wherein the amino acid sequence generated using the model comprises a sequence having at least 85% sequence identity to a sequence listed in Table 1.

Embodiment 59 - The method of any one of embodiments 1-58, wherein the biological molecule is a polypeptide and the seed for the amino acid sequence of the polypeptide is binary encoded.

Embodiment 60 - The method of embodiment 59, wherein the seed for the amino acid sequence of the polypeptide is one hot encoded.

Embodiment 61 - The method of any one of embodiments 1-58, wherein the biological molecule is a polypeptide and the seed for the amino acid sequence of the polypeptide is encoded using continuous values.

Embodiment 62 - The method of any one of embodiments 59-61, wherein, for each residue of the amino acid sequence, the seed comprises a vector or bit-character and each respective position in the vector or bit-character corresponds to a respective amino acid identity in a plurality of amino acid identities.

Embodiment 63 - The method of embodiment 62, wherein the seed for the amino acid sequence is 20-bit or 22-bit encoded.

Embodiment 64 - The method of any one of embodiments 59-63, wherein the polypeptide comprises one or more feature properties and, for each respective feature property in the one or more feature properties, the seed for the amino acid sequence of the polypeptide comprises a corresponding encoding of the respective feature property.

Embodiment 65 - The method of embodiment 64, wherein the one or more feature properties comprises at least 3, at least 5, or at least 10 feature properties.

Embodiment 66 - The method of embodiment 64 or 65, wherein the one or more feature properties are selected from the group consisting of negative charge, positive charge, nonpolar aliphatic, nonpolar aromatic, and polar.

Embodiment 67 - The method of embodiment 64 or 65, wherein the one or more feature properties are selected from the group consisting of a charge characteristic, a hydropathy value, a solubility value, a phosphorylation characteristic, a flexibility value, a ionic bonding characteristic, a hydrogen bonding characteristic, a hydrophilicity value, a surface accessibility value, a mutability value, a hydrogen bonding donor characteristic, a hydrogen bonding acceptor characteristic, an aggregate hydrogen bonding characteristic, a molecular mass value, a volume value, and a hydrophobicity value.

Embodiment 68 - The method of any one of embodiments 59-67, wherein the seed for the amino acid sequence is at least 4-bit, at least 5-bit, or at least 6-bit encoded.

Embodiment 69 - The method of any one of embodiments 1-68, wherein the seed for the amino acid sequence is randomly encoded.

Embodiment 70 - The method of any one of embodiments 1-69, wherein the seed for the amino acid sequence is binary encoded and a scale of each respective value in a plurality of values for the seed is transformed from {0, 1} to {-1, 1}.

Embodiment 71 - The method of embodiment 70, wherein the seed for the amino acid sequence is analog bit encoded.

Embodiment 72 - The method of embodiment 14, wherein the biological molecule is a nucleic acid and the seed for the nucleic acid sequence is represented with 4-bit encoding.

Embodiment 73 - The method of embodiment 72, wherein the 4-bit encoding for the seed for the nucleic acid sequence is scaled from {0, 1} to {-1, 1}.

Embodiment 74 - The method of embodiment 73, wherein the seed for the nucleic acid sequence is analog bit encoded.

Embodiment 75 - The method of any one of embodiments 72-74, wherein the 4-bit encoding comprises, for each respective position in a plurality of positions, a corresponding representation of a different nucleotide in a plurality of nucleotides.

Embodiment 76 - The method of any one of embodiments 1-75, wherein the inputting a plurality of target metric values for one or more target biological properties of the biological molecule conditions the model to generate, as output, the nucleic acid or amino acid sequence such that the biological molecule is predicted by the model to have the one or more target biological properties approximating the plurality of target metrics.

Embodiment 77 - The method of embodiment 76, wherein the one or more target biological properties comprise one or more conditions, and the plurality of target metrics for the one or more target biological properties comprise one or more values that approximate the one or more conditions or a metric thereof.

Embodiment 78 - The method of embodiment 76 or 77, wherein the one or more target biological properties are selected from the group consisting of editing, specificity, normalized specificity, ADAR1-specific activity, ADAR2-specific activity, and ADAR1/2-specific activity.

Embodiment 79 - The method of any one of embodiments 76-78, wherein a respective target metric in the plurality of target metrics comprises a range of values for a corresponding target biological property.

Embodiment 80 - The method of embodiment 79, wherein a respective target metric comprises (i) a first label that represents a first range of values for the corresponding target biological property or (ii) a second label that represents a second range of values for the corresponding target biological property.

Embodiment 81 - The method of any one of embodiments 76-80, wherein the model accepts, as input, a tensor comprising (i) the plurality of target metric values for the one or more target biological properties of the biological molecule and (ii) the seed for a nucleic acid or amino acid sequence for the biological molecule, wherein the plurality of target metric values is concatenated to the seed for the nucleic acid or amino acid sequence.

Embodiment 82 - The method of any one of embodiments 76-81, further comprising evaluating the nucleic acid or amino acid sequence for the biological molecule that is outputted by the model using a validation model that generates, as output from the validation model, a corresponding predicted metric for the nucleic acid or amino acid sequence.

Embodiment 83 - The method of embodiment 82, wherein the corresponding predicted metric is selected from the plurality of target metric values for the one or more target biological properties, further comprising comparing the corresponding predicted metric to a corresponding target metric value in the plurality of target metric values.

Embodiment 84 - The method of any one of embodiments 1-83, wherein the nucleic acid sequence is a regulatory sequence.

Embodiment 85 - The method of embodiment 84, wherein the regulatory sequence is an enhancer or a repressor.

Embodiment 86 - The method of any one of embodiments 1-85, wherein the nucleic acid sequence is all or a portion of a promoter sequence.

Embodiment 87 - The method of any one of embodiments 84-86, wherein the one or more target biological properties are selected from the group consisting of gene expression activity, cellular activity, relative activity of a first cell type compared to a second cell type, presence or absence of one or more enriched motifs, and presence or absence of one or more k-mers.

Embodiment 88 - The method of any one of embodiments 1-87, wherein the corresponding updated seed for the nucleic acid or amino acid sequence is the corresponding denoised seed for the nucleic acid or amino acid sequence from the respective state $X_{n-1}$ in the plurality of consecutive states $X_N$ in the Markov chain that immediately precedes the respective state $X_n$.

Embodiment 89 - The method of any one of embodiments 1-87, wherein the corresponding updated seed for the nucleic acid or amino acid sequence is a version of the corresponding denoised seed for the nucleic acid or amino acid sequence from the respective state $X_{n-1}$ in the plurality of consecutive states $X_N$ in the Markov chain that immediately precedes the respective state $X_n$, that is modified to replace a denoised representation of one or more nucleotide or amino acid residues with a representation for a defined one or more nucleotide or amino acid residues.

Embodiment 90 - The method of embodiment 89, wherein the biological molecule is a guide RNA and the defined one or more nucleotide residues is a footprint sequence conferring editing efficacy or specificity for a target sequence.

Embodiment 91 - The method of any one of embodiments 1-87, wherein the model is trained by a procedure comprising: training the model on a first training dataset, and retraining the model on a second training dataset other than the first training dataset.

Embodiment 92 - A system comprising: a processor; and a memory storing instructions, when executed by the processor, cause the processor to perform steps comprising any of the methods recited in embodiments 1-90.

Embodiment 93 - A non-transitory computer-readable medium storing computer code comprising instructions, when executed by one or more processors, causing the processors to perform any of the methods recited in embodiments 1-90.

7.7. Examples

The following examples are illustrative of the disclosure and should not be construed as limiting in any way the general nature of the disclosure of the description throughout this specification.

*Example 1 - ADAR1, ADAR2, and ADAR1/2-specific performance of gRNAs generated by target-agnostic bit diffusion models*. Three generative bit-diffusion models were evaluated for ADAR1, ADAR2, and ADAR 1 and ADAR 2 (ADAR1/2)-specific performance of sequences generated from seeds conditioned with ADAR1 and/or ADAR2-specific target metrics. Bit diffusion models utilizing a U-Net architecture as the transition model for the reverse diffusion process were trained to output nucleotide sequences for a gRNA, given conditioning for the target sequence and target editing metrics. The U-Net architecture included a plurality of layer blocks, each including a residual network layer and either a down-sampling or up-sampling layer. Corresponding residual network layers having the same spatial resolutions were connected by skip connections. Respective layer block further included conditioning layers projecting embeddings of the corresponding mRNA target sequence, target metrics, and a time step for the reverse diffusion process (e.g., indicating where in the Markov chain the diffusion process is), to condition the model. In some embodiments, the embeddings are generated using a multi-layer perceptron model. See, for example, the example bit diffusion models disclosed with reference to block 714 and **FIGS. 23A-B.**

The training of Denoising diffusion probabilistic models (DDPMs) involves gradually adding noise to an input X (for example, an image) and then training a denoising neural network (e.g., a U-net) to iteratively remove the added noise. This training process leads to a model that generates realistic samples from initial random noise. Importantly, conditional DDPMs can perform guided sampling by appending an embedding of conditioning values with the input X during training. Here, we built on a variant of DDPM, bit diffusion, which works with samples of discrete nature, making it ideal for generative modeling of biological sequences. Specifically, a bit diffusion model was trained using a poly-target library, conditioning on the target sequences and editing outcomes. After training, this model can rapidly sample gRNAs for a given target sequence with desired editing efficiency and specificity for both ADAR1 and ADAR2.

Training was performed against data for a set of 50,253 training gRNAs targeting 5,643 different adenosines across mRNA for 1,898 different genes, with a median of 9 gRNA designs per target adenosine, split into 80% training, 10% testing, and 10% validation sets. Editing data for each training gRNA was obtained using in vitro editing assays measuring ADAR1-specific editing efficiency for the target adenosine, ADAR1-specific editing specificity of the target adenosine, ADAR2-specific editing efficiency for the target adenosine, and ADAR2-specific editing specificity of the target adenosine.

Briefly, the nucleotide sequence of each training gRNA in the training set was one hot encoded using 4-bit encoding, where each position of the encoded characters represented a different nucleotide identity. As an example, the encoding can be visualized as a 1-dimensional vector, each position in the vector comprising a corresponding element representing a position in the sequence. In some embodiments, each element in the vector comprises a corresponding plurality of channels, which can in turn be visualized as a tensor of possible element identities (*e.g.,* nucleic acid identities). Each respective channel comprises a value that indicates an "intensity" or weight of the respective identity at the respective position in the sequence (*e.g.,* a probability that the nucleic acid at the respective position has the respective identity). In some implementations, mixtures of identities are possible (*e.g.,* where the sum of "intensities" or probabilities is 1, and where the respective intensity of one or more possible identities is less than 1). Other methods of representing nucleotide sequences are possible, as will be apparent to one skilled in the art, such as 2-dimensional vectors comprising, for each respective position in the sequence, a first entry indicating the position of the nucleic acid in the sequence and a second entry indicating the identity (or one or more weights thereof) of the nucleic acid in the sequence. In some embodiments, each respective nucleotide sequence of each training gRNA in the training set is the same length. In some embodiments, at least a first nucleotide sequence for a first training gRNA in the training set is a different length from a second nucleotide sequence for a second training gRNA in the training set. In some embodiments, the method further comprises padding and/or truncating one or more nucleotide sequences or encodings thereof. In some such embodiments, the padding and/or truncating adjusts a length of a nucleotide sequence or an encoding thereof to a target length $l$. In some embodiments, the padding and/or truncating adjusts a length of a nucleotide sequence or an encoding thereof to a multiple of a target length $l$, *e.g.*, $p \times l$. In some embodiments, $l$ is a positive integer from 2 to 100. In some embodiments, $p$ is a positive integer from 2 to 1000. In some implementations, an encoded value for a position in a sequence is zero (*e.g.,* where the position results from a padding of a nucleotide sequence or an encoding thereof).

The encoded characters were mapped from {1,0} to {1, -1 }. The encoded sequence was then diffused in a forward diffusion process by systematically introducing Gaussian noise into the 4-bit encoding in a forward diffusion process over a series of iterations, resulting in a corresponding diffused seed for each respective training gRNA in the training set. The generative U-Net architecture was then trained by inputting the diffused seeds to remove noise from the diffused seed over a series of iterations having a time schedule. In some embodiments, the time schedule is a defined time schedule having a defined plurality of time steps. In some embodiments, the same plurality of time steps is used for forward diffusion (*e.g.,* adding noise) as for denoising the diffused seed over the plurality of iterations. In some embodiments, the denoising uses a different plurality of time steps from a plurality of time steps used for forward diffusion (*e.g.,* adding noise). After the first iteration, the denoised sequence from the previous iteration was used as the input into the U-Net. During each iteration, for each training gRNA, a one-hot encoding of the corresponding target mRNA sequence, the editing metrics measured for the training gRNA using the in vitro assays (ADAR1 metrics only, ADAR2 metrics only, or ADAR1 metrics and ADAR2 metrics), and an indication of the time step in the series of iterations (*e.g.,* a time stamp for the denoising process) were embedded into the conditioning layers of the model. The conditioning signals for the target sequence, target metrics, and time step were each embedded independently. The time step was embedded using a respective multi-layer perceptron (MLP) model, which comprised a plurality of updatable parameters (*e.g.,* weights) and was then fed into each layer block (*e.g.,* ResNetBlockClassConditioned object). In each block (*e.g.,* each object block) the time signal was embedded again and then used to perform a scale and shift operation on the input signal (*e.g.,* the denoised sequence from the previous iteration). The target metrics were also separately embedded and then added to the time signal before it was inputted to the blocks. The target sequences were embedded in an MLP housed in each of the layer blocks (*e.g.,* the ResNetBlockClassConditioned object) and then concatenated to the input signal. For the target sequence, the conditioning included applying linear projections and GELU functions to the target sequence signal. For the target metrics, sinusoidal position embedding, linear projections, and GELU was applied to the target metric signal. For the time step, the conditioning comprised applying linear projections and GELU to the time step.

Weights and biases for the U-Net were adjusted based on differences between the generative output and known guide sequence according to known methods.

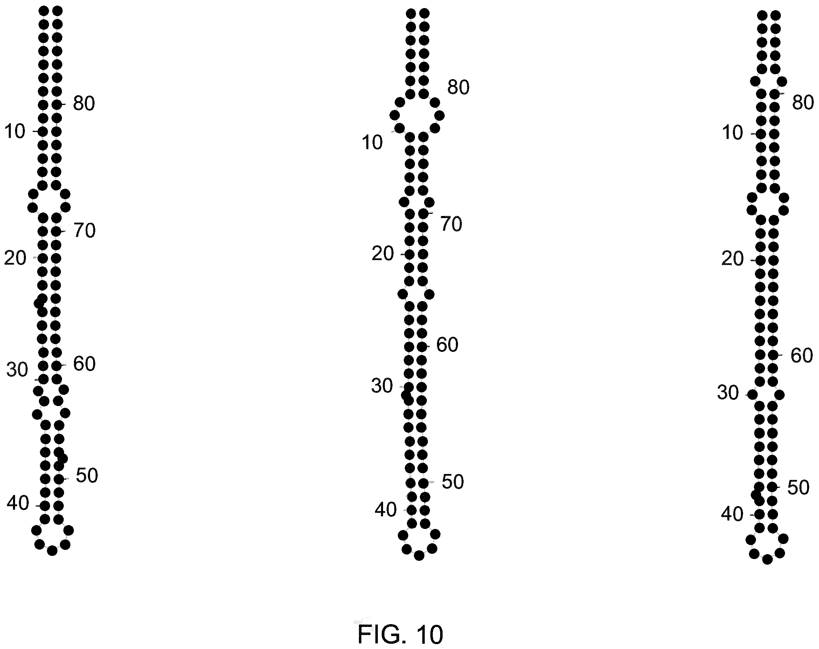

A first model was obtained, comprising a target-agnostic bit diffusion model trained on gRNA-target scaffold sequences conditioned with ADAR1-specific performance (editing, specificity). Nucleic acid sequences for a gRNA-target scaffold were generated by denoising random seeds for a gRNA sequence targeting an unseen mRNA sequence (an mRNA sequence for which none of the training guides targeted) using the trained ADAR1-specific U-Net by conditioning the model with the target mRNA sequence and target ADAR1-specific editing metrics. A range of editing metrics was used to generate guide sequences having a range of predicted editing efficiencies and specificities. Examples of predicted gRNA-target scaffold structures for generated guides using conditioning with high target ADAR1-specific editing efficiencies and specificities are shown in **FIG. 10.** Notably, the sequences displayed known nano-footprints, asymmetry, and in general appeared as reasonable designs, with on-target position at position 16.

The performance of the generated scaffold sequences was validated using trained, target-agnostic XGBoost models that produced, as output, predicted performance metrics upon receiving a generated scaffold sequence as input.

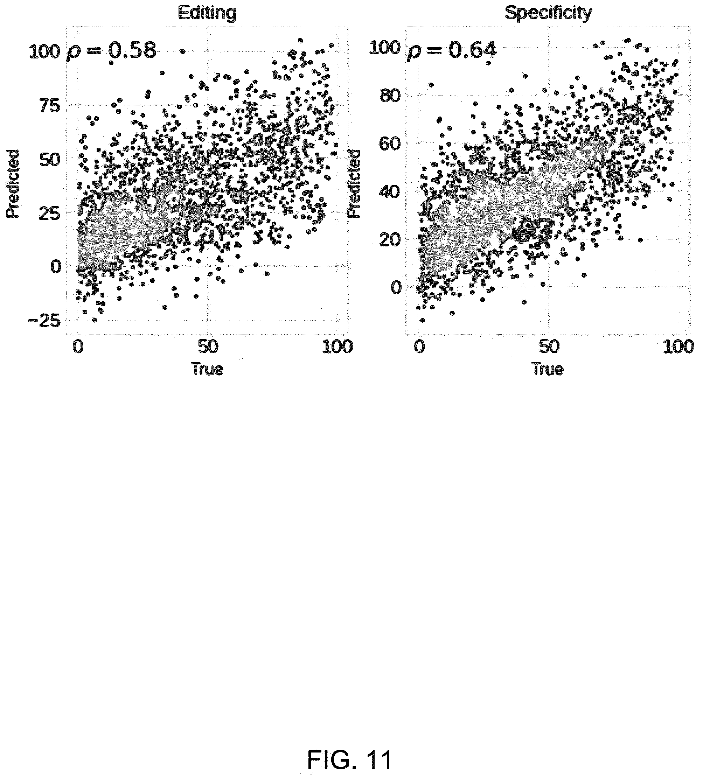

**FIG. 11** illustrates the predicted performance metrics plotted against "true" labels, where the "true" labels are the performance metrics used for conditioning. The sampling range for editing and specificity was constrained by the minimum and maximum values seen for each target. In particular, subsampling was performed by enforcing symmetry between desired ADAR1 and ADAR2 editing and specificity values, thereby selecting a subset of conditioning values from a plurality of randomly chosen conditioning values. In this way, the subsampling removed conditioning values that were harder to achieve (*e.g.,* a large difference between ADAR1 and ADAR2 editing) and resulted in better agreement between the models. After subsampling, the models performed reasonably well and were used to benchmark the generative designs obtained from bit diffusion.

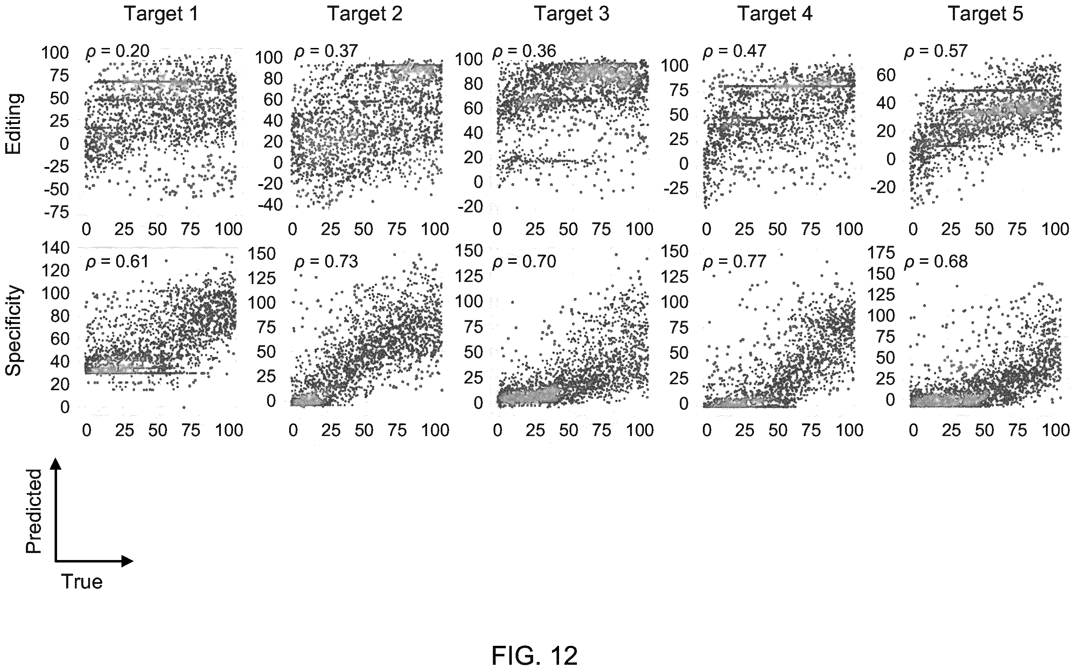

**FIG. 12** further illustrates ADAR1-specific performance of generative designs obtained from bit diffusion, for each of 5 targets previously unseen by the bit diffusion model. Random conditioning values between 0 and 100 were selected for both editing and specificity to validate the model. The figure shows moderate concordance between predicted and true editing performance and relatively robust concordance between predicted and true specificity for ADAR1. These results illustrate the bit diffusion model's ability to produce reasonably accurate gRNA sequences even for unseen targets.

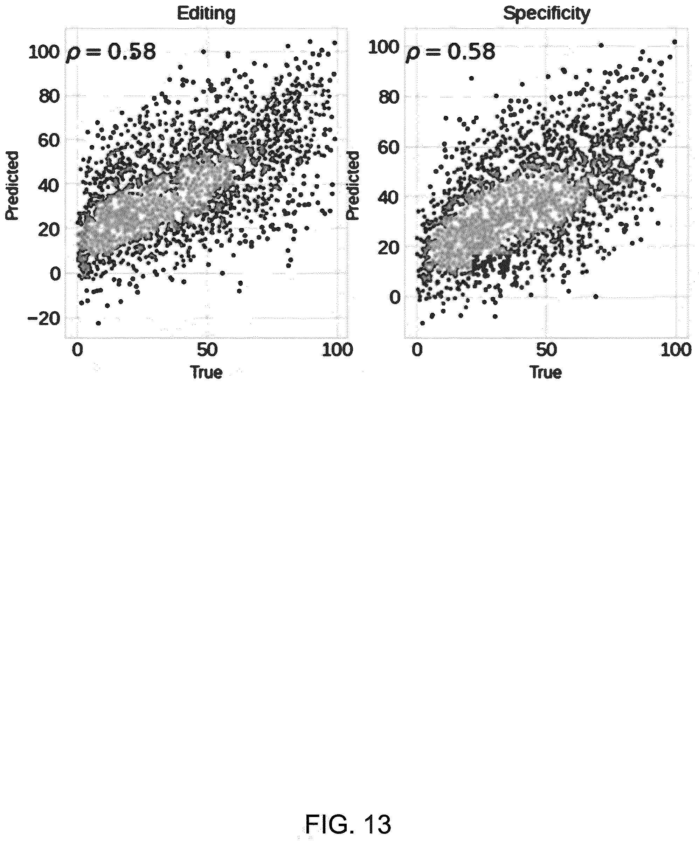

Similar results were obtained for a second model comprising a target-agnostic bit diffusion model trained on gRNA-target scaffold sequences conditioned with ADAR2-specific performance (editing, specificity), as illustrated in **FIG. 13.**

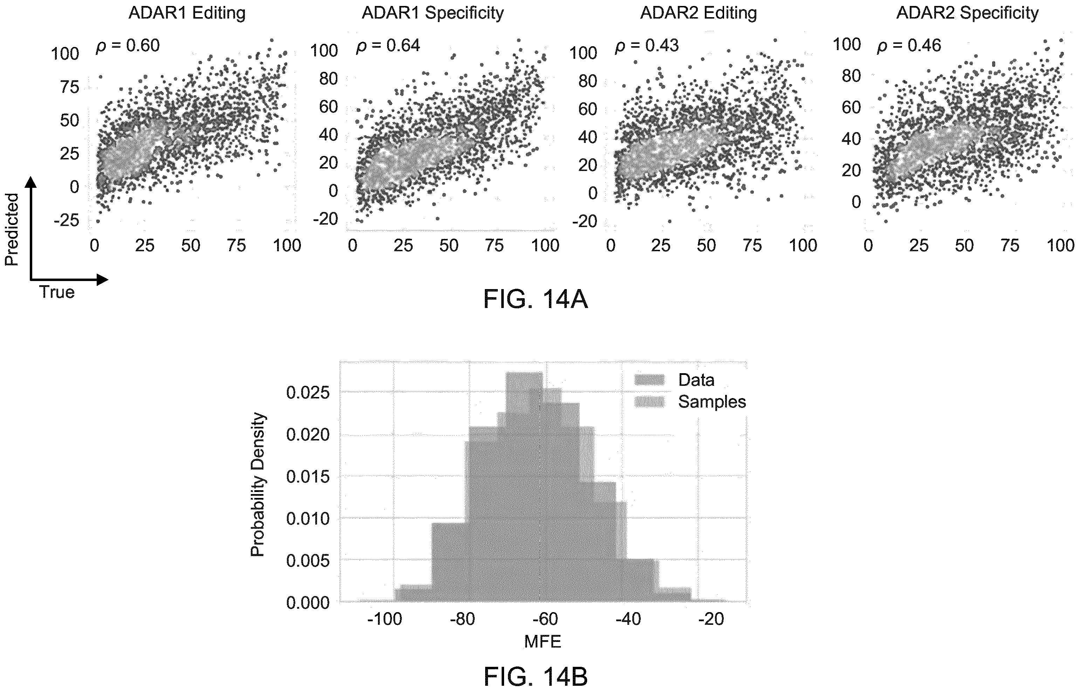

Additionally, further results were obtained for a third model comprising a target-agnostic bit diffusion model trained on gRNA-target scaffold sequences conditioned with both ADAR1 and ADAR2-specific performance (ADAR1 editing, ADAR1 specificity, ADAR2 editing, and ADAR2 specificity), as illustrated in **FIG. 14A.**

The histogram in **FIG. 14B** illustrates the probability densities of the minimum free energy calculated for both the training dataset ("Data") and the bit diffusion model-generated sequences ("Samples"). The plot shows strong similarity between the MFEs of each of the datasets, further illustrating the concordance of the generated sequences with expected outcomes.

*Example 2 - Comparative performance of designs generated by target-specific and target-agnostic models: input optimization, bit diffusion, and structural sampling.*

Three approaches for generating sequences were compared, including an input optimization approach, a bit diffusion model, and a structural sampling scoring process (e.g., of guides designed using existing algorithms). Briefly, an input optimization approach comprises a model including a plurality of (e.g., at least 100,000) parameters and an input layer configured to accept the data structure, where the model is configured to output predicted values for each condition in a plurality of conditions (e.g., target biological properties). The input optimization operation comprises i) responsive to inputting the data structure comprising a seed for a nucleic acid or amino acid sequence, obtaining a set of calculated values for the plurality of conditions, and ii) back-propagating through the model, while holding the plurality of parameters fixed, a difference between the set of calculated values and a set of target values to modify the seed for the nucleic acid or amino acid sequence responsive to the difference, thereby generating a polymer sequence.

The structural sampling approach was performed by generating a large number of candidate guide sequences by introducing nucleotide substitutions, insertions, and/or deletions into a guide sequence with perfect complementarity to the target sequence and then scoring each guide sequence using a predictive model trained against ADAR-mediated editing data. A trained model ensemble, in some embodiments, is used to score and rank the list. For example, given the target number and lengths of mutations with regard to perfectly complementary target and guide strands (perfect duplex) in an engineered gRNA, an algorithm generates candidate engineered gRNAs, e.g., by introducing nucleotide substitutions, insertions, and/or deletions into a guide sequence with perfect complementarity to the target sequence. These candidate sequences are evaluated using a predictive model (e.g., an ensemble CNN or boosted trees algorithm) trained on existing engineered gRNA data to predict the candidates' biological properties (e.g., target edit score and specificity score when edited by ADAR1 and/or ADAR2) and/or the minimum free energy of the folded structure. These mutated sequences are then ranked by their predicted scores, effectively eliminating poorly performed sequences and narrowing down the vast sequence space to be tested experimentally.

For each of the three approaches, three different models were trained to produce sequences active on each of three endpoints: ADAR1-specific, ADAR2-specific, or ADAR1/2-specific activity (e.g., 9 different models). Moreover, inputs to each model were conditioned on values (e.g., concatenated with input sequences) to yield positive, negative, ADAR1-specific, and/or ADAR2-specific guides. Positive guide sequences were defined as having editing and/or normalized specificity of greater than or equal to 0.7 and less than or equal to 1.1. Negative guide sequences were defined as having editing and/or normalized specificity of less than 0.7. Training datasets included the following numbers of selected training sequences per category: 1500 positives, 150 negatives, 750 ADAR1-specific, and 750 ADAR2-specific.

Selection of libraries for model training employed a combination of randomness and heuristics. For example, library selection included both weighted random selection of training sequences and selection via applied thresholds for both minimum and maximum target property values. For each approach, a target-specific (MAPT) library and a target-agnostic (ML) library (as described in Example 1) were used in training the corresponding models to evaluate the ability of the models to generate gRNA sequences under target-specific or generalized conditions (e.g., 18 different models).

Sequences were generated using each of the trained models as follows:

- Input optimization with ADAR1 endpoint (MAPT library or ML library),

- Input optimization with ADAR2 endpoint (MAPT library or ML library),

- Input optimization with ADAR1/2 endpoint (MAPT library or ML library),

- Bit diffusion with ADAR1 endpoint (MAPT library or ML library),

- Bit diffusion with ADAR2 endpoint (MAPT library or ML library),

- Bit diffusion with ADAR1/2 endpoint (MAPT library or ML library),

- Structural sampling with ADAR1 endpoint (MAPT library or ML library),

- Structural sampling with ADAR2 endpoint (MAPT library or ML library), and

- Structural sampling with ADAR1/2 endpoint (MAPT library or ML library).

For each of the 18 models, inputs to the respective model were conditioned on positive, negative, ADAR1-specific, or ADAR2-specific values.

Performance of generated sequences was validated using XGBoost models trained to produce, as output, predicted performance metrics (ADAR1-specific editing, ADAR1-specific normalized specificity, ADAR2-specific editing, and ADAR2-specific normalized specificity) upon receiving a generated sequence as input. These were labeled as "Prediction: ADAR1" and "Prediction: ADAR2."

Predicted performance metrics are shown as plots with predicted editing on the x-axis and predicted normalized specificity on the y-axis, for each respective approach (e.g., input optimization, bit diffusion, or structural sampling), for each respective training library (e.g., MAPT or ML), and for each respective endpoint (*e.g.,* ADAR1-specific, ADAR2-specific, or ADAR1/2-specific). Moreover, for each permutation, the plots were further stratified by conditioning (*e.g.,* positive vs. negative or ADAR1-specific vs. ADAR2-specific) and further by prediction type (ADAR1 editing or normalized specificity vs. ADAR2 editing or normalized specificity).

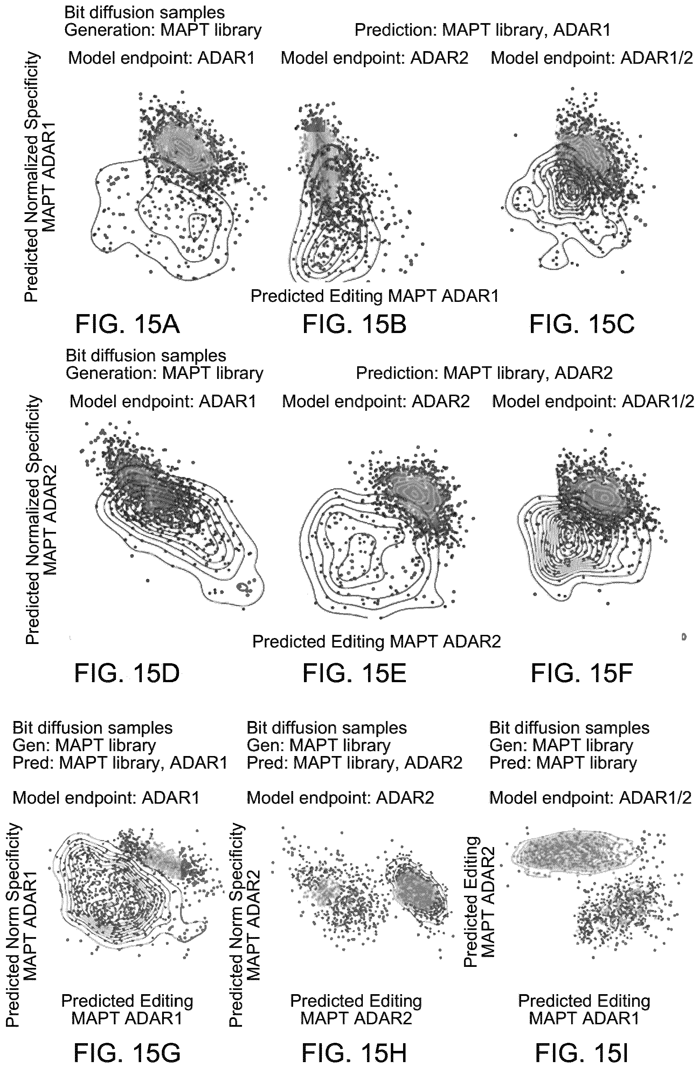

For example, **FIGS. 15A-I** collectively illustrate the predicted performance of sequences generated by bit diffusion using models trained on a target-specific library (MAPT). The top left panel **(FIGS. 15A-C)** illustrates a comparison of ADAR1-specific predicted metrics (ADAR1 predicted editing and ADAR1 predicted normalized specificity) produced by each of the bit diffusion models (ADAR1 endpoint, ADAR2 endpoint, and ADAR1/2 endpoint), while the bottom left panel **(FIGS. 15D-F)** illustrates a comparison of ADAR2-specific predicted metrics (ADAR2 predicted editing and ADAR2 predicted normalized specificity). **FIGS. 15A** and **15D** correspond to sequences generated using an ADAR1-specific bit diffusion model (ADAR1 endpoint), **FIGS. 15B** and **15E** correspond to sequences generated using an ADAR2-specific bit diffusion model (ADAR2 endpoint), and **FIGS. 15C** and **15F** correspond to sequences generated using an ADAR1/2-specific bit diffusion model (ADAR1/2 endpoint).

The shaded density contour plot illustrates the distribution of performance metrics for sequences generated using "positive" conditioning, while the unshaded density contour plot illustrates the distribution of performance metrics for sequences generated using "negative" conditioning, as described above. Notably, all three endpoint models produce sequences under positive conditioning that segregate well from sequences produced under negative conditioning as measured by both ADAR1 and ADAR2-specific predicted editing and specificity.

**FIGS. 15G-I** collectively illustrate the predicted performance of sequences generated by an ADAR1/2-specific bit diffusion model (ADAR1/2 endpoint), where predicted performance is stratified by ADAR1-specific vs. ADAR2-specific conditioning. **FIG. 15G** illustrates a comparison of ADAR1-specific predicted metrics (ADAR1 predicted editing and ADAR1 predicted normalized specificity), **FIG. 15H** illustrates a comparison of ADAR2-specific predicted metrics (ADAR2 predicted editing and ADAR2 predicted normalized specificity), and **FIG. 15I** illustrates a comparison of ADAR1-specific predicted editing vs. ADAR2-specific predicted editing.

The shaded density contour plot illustrates the distribution of performance metrics for sequences generated using ADAR1-specific conditioning, while the unshaded density contour plot illustrates the distribution of performance metrics for sequences generated using ADAR2-specific conditioning. Notably, sequences generated using ADAR1 conditioning are predicted to have higher ADAR1-specific performance metrics (**FIG. 15G**) while sequences generated using ADAR2 conditioning are predicted to have higher ADAR2-specific performance metrics (**FIG. 15H**). Similarly, a comparison of predicted ADAR1- and ADAR2-specific editing shows good discrimination between sequences generated using ADAR1 conditioning compared to sequences generated using ADAR2 conditioning (**FIG. 15I**).

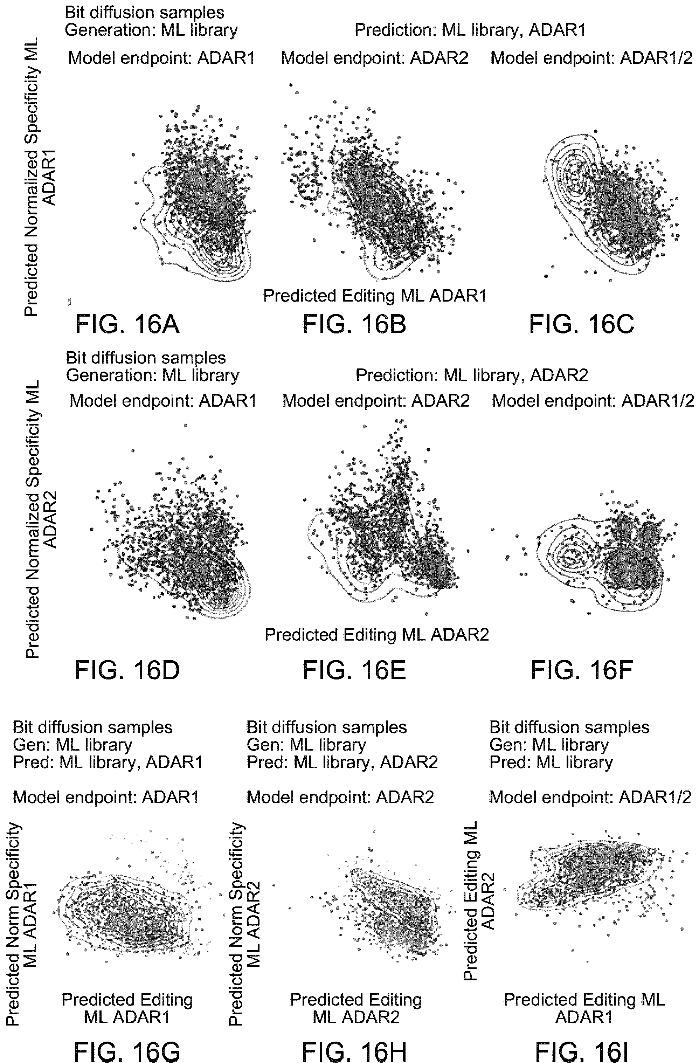

**FIGS. 16A-I** collectively illustrate the predicted performance of sequences generated by bit diffusion models trained on a target-agnostic library (ML). Moderate segregation between positive and negative conditioning was observed when evaluating sequences obtained from an ADAR1-specific model (ADAR1 endpoint) and an ADAR1/2-specific model (ADAR1/2 endpoint) using ADAR1-specific predicted performance metrics (**FIGS. 16A** and **16C**). Moreover, moderate segregation was observed between ADAR1 and ADAR2 conditioning when evaluating sequences obtained from an ADAR1/2-specific model (ADAR1/2 endpoint) using ADAR2-specific predicted metrics (**FIG. 16H**).

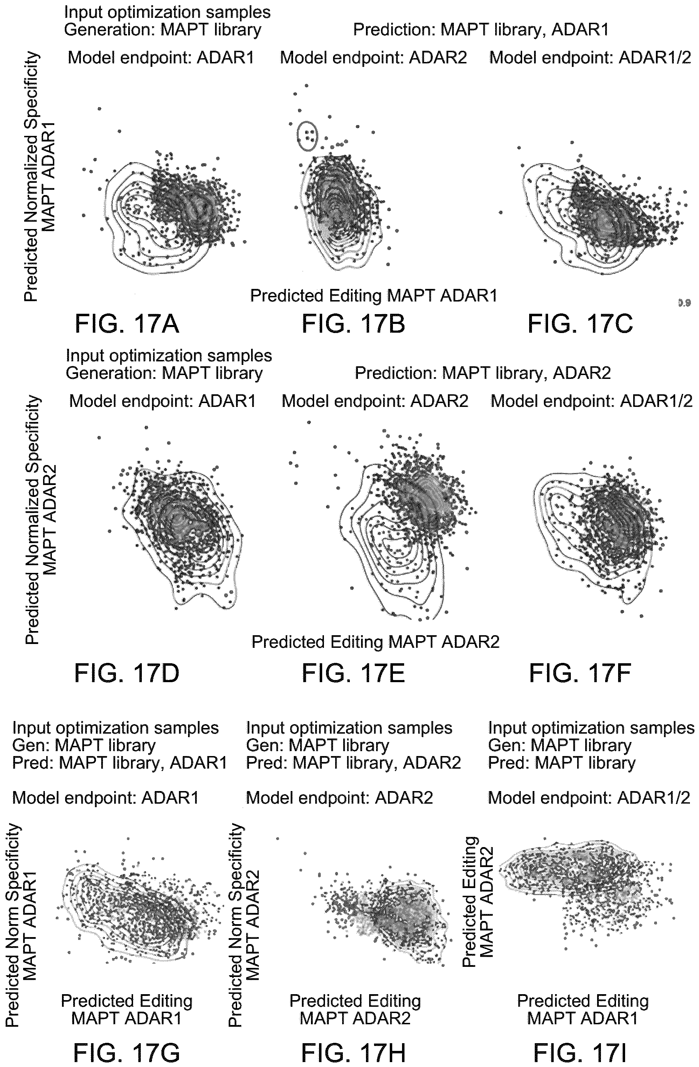

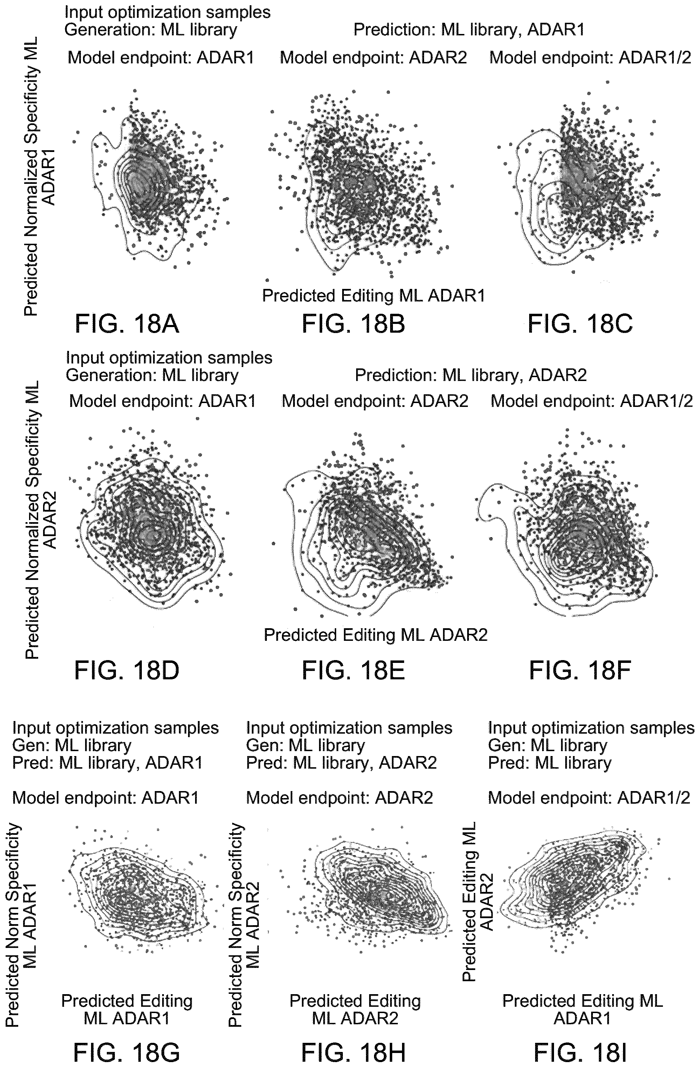

Predicted performance metrics for generative designs obtained using the input optimization approach are shown for models trained on a target-specific library (**FIGS. 17A-I**) and a target-agnostic library (**FIGS. 18A-I**). As described above, the effect of conditioning on sequence generation was enhanced when models were trained using target-specific libraries compared to target-agnostic libraries, although to a lesser extent than observed when using bit diffusion models.

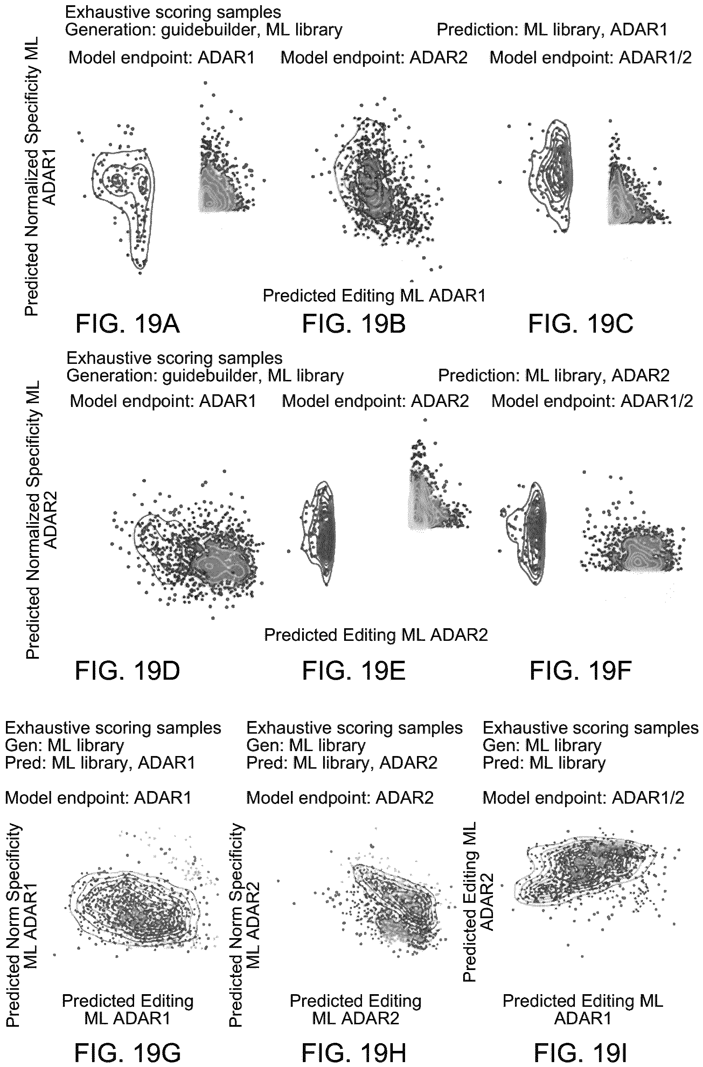

Predicted performance metrics for guide sequences generated using structural sampling are also shown for models trained on a target-agnostic library (**FIGS. 19A-I**)**. Notably, sequences obtained using positive and negative conditioning were strongly segregated with respect to predicted editing but weakly segregated with respect to specificity in most cases.

***Example 3** - **Training a bit diffusion model on intravitreal data for AAV5 variants with 581-589 region mutations.***

A bit diffusion model comprising an architecture as illustrated in **FIGS. 23A-B** was trained on intravitreal data for AAV5 variants having mutations in the 581-589 region ("581-589 AAV5 variants"). First, a dataset of intravitreal 581-589 AAV5 variant data was obtained. For each 581-589 AAV5 variant amino acid sequence, each of 20 amino acids (e.g., amino acid identities) were encoded using 5-bit encoding as follows:

- "A":[-1,-1,-1,-1,1],

- "R":[1,-1,-1,-1,-1],

- "N":[-1,1,-1,-1,-1],

- "D":[-1,1,-1,-1,1],

- "C":[-1,-1,1,-1,-1],

- "Q":[-1,1,1,-1,-1],

- "E":[-1,1,1,-1,1],

- "G":[-1,-1,-1,1,-1],

- "H":[1,-1,-1,1,-1],

- "I":[1,-1,1,-1,-1],

- "L": [1, -1, 1, -1,1],

- "K": [1, -1, -1, -1,1],

- "M":[1,-1,1,1,-1],

- "F":[1,1,-1,-1,-1],

- "P":[-1,-1,-1,1,1],

- "S":[-1,-1,1,1,-1],

- "T":[-1,-1,1,1,1],

- "W":[1,1,-1,-1,1],

- "Y":[1,1,-1,1,-1], and

- "V":[1,-1,1,1,1].

581-589 AAV5 variant sequences were categorized as "positive" and "negative" according to their tissue specificity and such labels were used as conditioning for each corresponding sequence in training the model (*e.g.,* as 1s and 0s). The number of positive samples included in the library was 99269 and the number of negative samples included was 58903. Referring again to **FIGS. 23A-B,** a U-Net denoising model was used to remove the noise in the training data. The diffusion model was trained on the entire training dataset without train-test or train-test-validate splits. During the training process, the length of time in the forward diffusion process, $t$, was allowed to vary. A reconstruction loss was used to update the parameters in the model during training. The denoising model then attempted to reconstruct the original sequence.

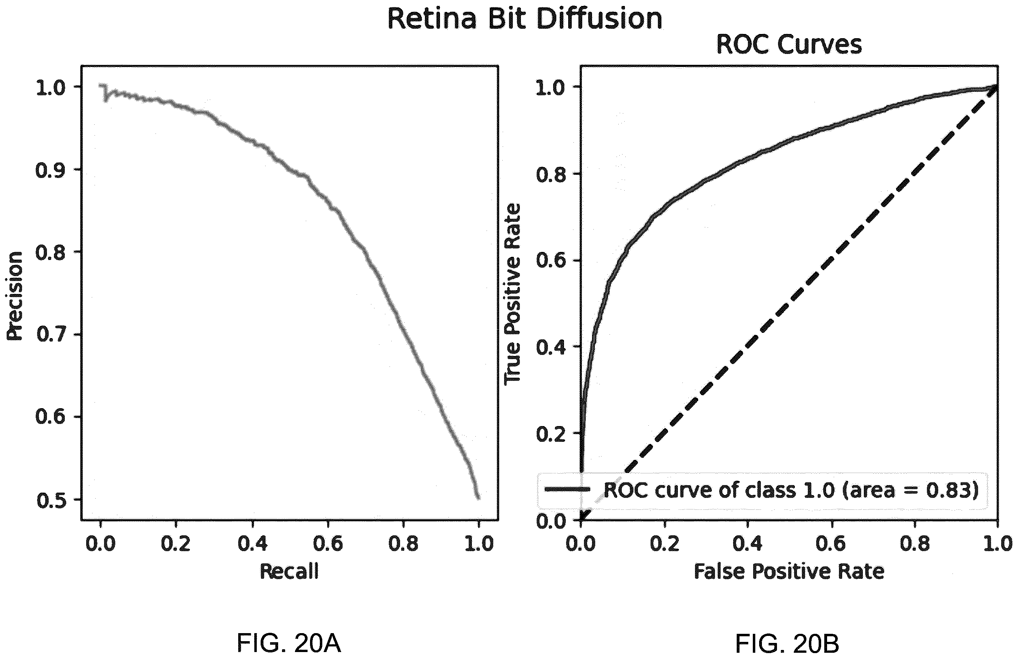

Performance of 581-589 AAV5 variant sequences generated by the bit diffusion model was validated using a trained, retina-specific XGBoost model that produced, as output, predicted labels upon receiving a generated 581-589 AAV5 variant sequence as input. The output of the bit diffusion model was further evaluated based on a comparison of the predicted labels with the "true" labels used for conditioning. **FIGS. 20A-B** illustrate the precision and recall curve and the receiver operating characteristic (ROC) curve obtained from the evaluation. The plots show relatively high precision and recall and relatively high true positive rates relative to false positive rates for the bit diffusion model-generated 581-589 AAV5 variant sequences.

The trained bit diffusion model was then used to generate amino acid sequences from seed sequences, optionally conditioned with tissue-specific attributes (e.g., "present in only retina" or "not present in retina").

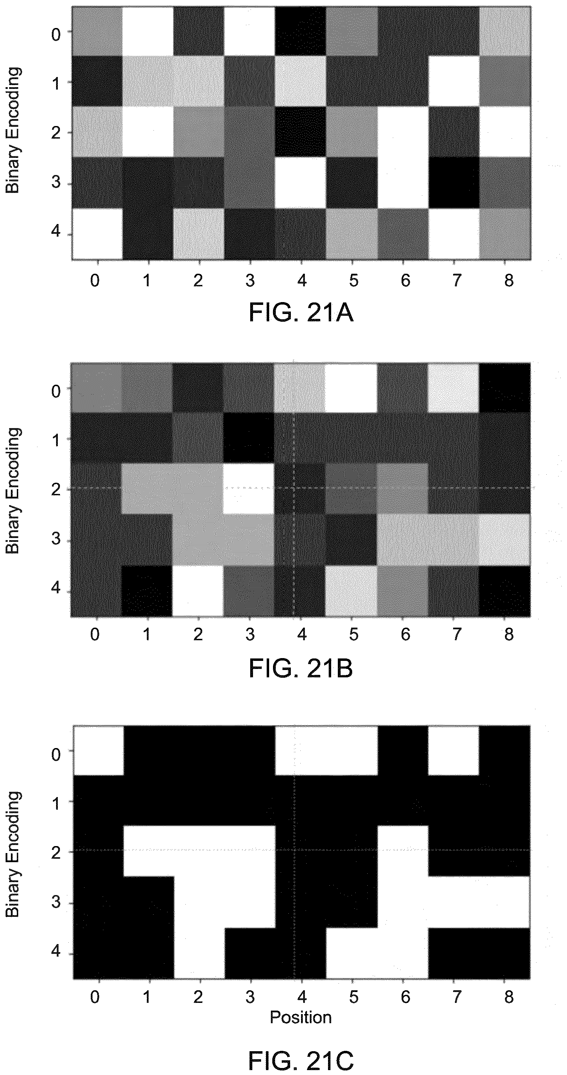

**FIGS. 21A-C** collectively illustrate an example generation of a 5-bit encoded amino acid sequence from a seed sequence, in accordance with an embodiment of the present disclosure. **FIG. 21A** illustrates a seed sequence comprising random noise, in which, for each respective position in a plurality of positions in an amino acid sequence (9 positions), for each respective encoding character in a sequence of characters (5 characters), a corresponding value is provided at the respective character (represented as a color and/or an intensity of shading). **FIG. 21B** illustrates an intermediate stage of denoising, in which the values corresponding to each respective encoding character, for each respective position in the plurality of positions, are partially denoised and thus less randomly diffused. **FIG. 21C** illustrates a 5-bit, one hot encoded amino acid sequence, in which, at each respective position in the plurality of positions, the identity of the amino acid at the respective position is indicated by a sequence of 1's or 0's (*e.g.*, a 1 or a 0 for each of the 5 encoding characters in the sequence of characters).

### Example 4 - Performance of retrained bit diffusion model for conditional generation of enhancer sequences.

In some instances, training libraries for target-specific sequences include reverse-complement designs, which can lead to design dropout during barcode association and pseudo-count adjustment and further result in potential loss of discoveries. In some such embodiments, generative design models trained on such libraries are subject to bias.

Accordingly, CNN ensemble and bit diffusion models were trained to generate enhancer sequences conditioned on mouse primary neuron (MPN) activity, liver cancer cell (HepG2) activity, and/or a metric comprising log fold change of MPN vs HepG2 activity. The bit diffusion model included a U-Net model comprising an architecture as illustrated in **FIGS. 23A-B.**

The model was trained on an unbiased tandem repeat library and then retrained on a tissue-specific CNS library. Model training was performed as described in Example 1. In particular, the models were first trained using the tandem repeat library. Updated parameters (*e.g.*, weights) obtained from the tandem repeat library training was used as the initialization point to retain using the CNS library. For CNN ensemble models, hyperparameter tuning was performed prior to the initial training on the tandem repeat library. Training inputs to the model were the enhancer sequences from the training libraries and were further conditioned using measured activities for three target properties: mouse primary neuron (MPN) activity, liver cancer cell (HepG2) activity, and/or a metric comprising log fold change of MPN vs HepG2 activity. Conditioning was further performed as described above in Example 1, with each of the target properties embedded as the actual value * 100.

Using the trained model, 25,000 enhancer sequences were generated conditioned on mouse primary neuron (MPN) activity, 25,000 enhancer sequences were generated conditioned on HepG2 activity, and 25,000 enhancer sequences were generated conditioned on MPN vs HepG2 activity log fold change. After subsampling the generated enhancer sequences, a corresponding 2000 enhancer sequences for MPN activity, 200 enhancer sequences for HepG2 activity, and 500 enhancer sequences for MPN vs. HepG2 activity log fold change remained. The generated sequences could be further synthesized for experimental validation.

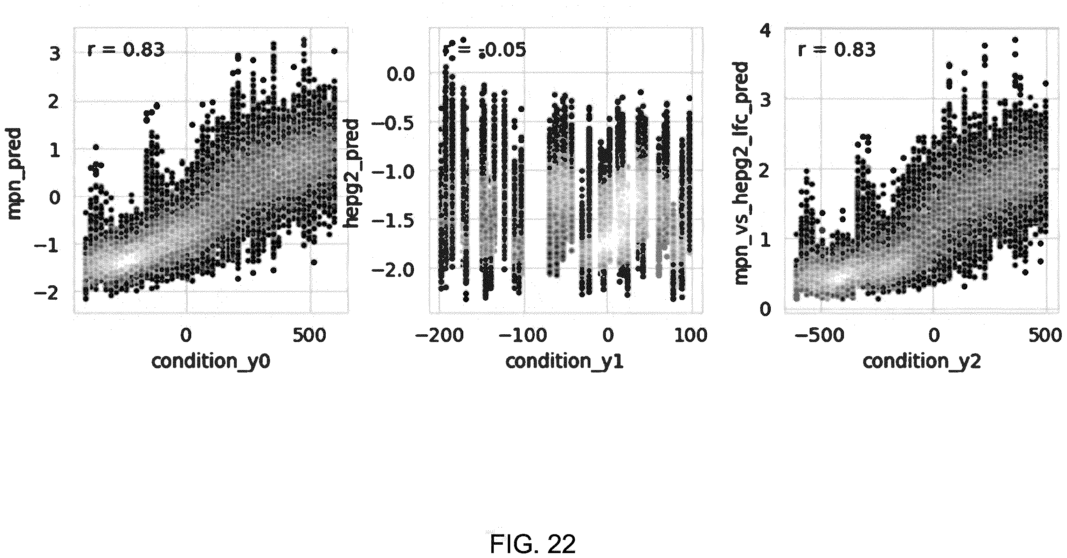

A proof-of-concept assay was performed to assess the ability of the retrained bit diffusion model to generate enhancer sequences conditioned on a particular target property for MPN activity. Performance of the generated enhancer sequences generated by the retrained bit diffusion model was validated using a trained CNN ensemble model that produced, as output, predicted labels according to each of three performance criteria (MPN activity, HepG2 activity, and/or log fold change of MPN vs HepG2 activity) upon receiving a generated enhancer sequence as input. The output of the bit diffusion model was further evaluated based on a comparison of the predicted labels (y-axis) with the "true" labels used for conditioning (x-axis). **FIG. 22** illustrates the results of this evaluation, in which good concordance is observed for MPN activity (r = 0.83) and MPN vs. HepG2 activity log fold change (r = 0.83), but poor concordance is observed for HepG2 activity. These results illustrate the bit diffusion model's ability to generate enhancer sequences that are active for a target property (MPN activity) but not active for non-target properties (HepG2 activity). In general, activity (*e.g.*, MPN and HepG2 activity) for enhancer sequences generated by the bit diffusion model were positively correlated with conditioning values. However, the enhancer sequences generated by the bit diffusion model did not outperform the top candidates identified through screening in the initial proof-of-concept study.

### Example 5 - Comparison of machine learning-aided in silico guide RNA screening and generative design.

This example demonstrates comparison of three machine learning-aided methodologies for identifying efficient ADAR-mediated guide RNA (gRNA) for editing novel mRNA targets that were not used in the training of the respective models. The three methodologies tested were *in silico* screening of a large number of putative guide sequences designed according to general editing schema using a target-agnostic, distributed gradient-boosted decision tree ensemble (the "XGBoost model"), input optimization of gRNA sequences using a target-agnostic ensemble of convolutional neural networks (the "CNN model"), and generative sequence design using a target-agnostic bit diffusion model, where all three models were trained against the same set of ADAR1-mediated and ADAR2-mediated *in vitro* editing data for a multi-target library of 50,253 gRNAs targeting 5,643 diverse targets (a single target adenosine target for each guide) across 1,898 genes.

The XGBoost model was iteratively trained against the editing data for the multi-target library of 50,253 gRNA, where the training was completed when the loss function was sufficiently stable (*e.g.*, the machine learning model converged), after a predetermined number of rounds for a particular set of training samples, and/or after a performance of the respective model satisfied a validation threshold (*e.g.*, using the validation set of validation gRNAs). Briefly, the nucleotide sequence of the guide-target scaffold for each guide in the multi-target library was one-hot encoded as a vector having four values (representing A, U, G, and C, respectively), where 1 was used to represent the nucleotide present at a position and 0 was used to represent the absence of that nucleotide at the position. For example, a vector [1, 0, 0, 0] for position Y in the nucleotide sequence can represent an A at position Y of the sequence. Models were trained using the one-hot encoded sequence for each of the library guides as independent variables and the corresponding empirically-derived ADAR1 editing efficiencies (defined as the percentage of target nucleotides edited in the *in vitro* assay by ADAR1), the empirically-derived ADAR2 editing efficiencies (defined as the percentage of target nucleotides edited in the *in vitro* assay by ADAR2), the empirically-derived ADAR1 editing normalized specificity (defined as 1 - the proportion of reads with any off-target nucleotides edited in the *in vitro* assay by ADAR1), the empirically-derived ADAR2 editing normalized specificity (defined as 1 - the proportion of reads with any off-target nucleotides edited in the *in vitro* assay by ADAR2), and minimum free energy (MFE) predictions for the gRNA-target scaffolds prepared using the ViennaRNA python package (Lorenz, R. et al., ViennaRNA Package 2.0, Algorithms for Molecular Biology, 6:1 26, 2011, doi:10.1186/1748-7188-6-26). The model was validated against a set of gRNA held out of the training. The model results had a Spearman correlation coefficient of 0.79 for ADAR1-mediated editing efficacy, 0.78 for ADAR1-mediated editing specificity, 0.73 for ADAR2-mediated editing efficacy, and 0.77 for ADAR2-mediated editing specificity, highlighting the strong concordance between the observed and predicted metrics.

To train the CNN model, a hyperparameter space for component CNN models was searched and the top twenty designs were selected to represent the ensemble. Each component CNN model was initialized with randomized weights and refined against the editing data for the multi-target library of 50,253 gRNA. Briefly, the nucleotide sequence of the guide-target scaffold for each guide in the multi-target library was one-hot encoded as a vector having four values (representing A, U, G, and C, respectively), where 1 was used to represent the nucleotide present at a position and 0 was used to represent the absence of that nucleotide at the position. For example, a vector [1, 0, 0, 0] for position Y in the nucleotide sequence can represent an A at position Y of the sequence. Models were trained using the one-hot encoded sequence for each of the library guides as independent variables and the corresponding empirically-derived ADAR1 editing efficiencies (defined as the percentage of target nucleotides edited in the *in vitro* assay by ADAR1), the empirically-derived ADAR2 editing efficiencies (defined as the percentage of target nucleotides edited in the *in vitro* assay by ADAR2), the empirically-derived ADAR1 editing specificity (defined as the ratio of the number of target nucleotides edited in the *in vitro* assay by ADAR1 to the number of off-target nucleotides edited in the *in vitro* assay by ADAR1), the empirically-derived ADAR2 editing specificity (defined as the ratio of the number of target nucleotides edited in the *in vitro* assay by ADAR1 to the number of off-target nucleotides edited in the *in vitro* assay by ADAR1), and minimum free energy (MFE) predictions for the gRNA-target scaffolds prepared using the ViennaRNA python package (Lorenz, R. et al., ViennaRNA Package 2.0, Algorithms for Molecular Biology, 6:1 26, 2011, doi:10.1186/1748-7188-6-26). The model was validated against a set of gRNA held out of the training. The model results had a Spearman correlation coefficient of 0.73 or greater for each of the efficiency and specificity metrics, highlighting the strong concordance between the observed and predicted metrics.

The bit diffusion model was trained, as described in **Example 1,** conditioning the model against the editing data for the multi-target library of 50,253 gRNA. A U-Net convolutional neural network was used as the transition model for the diffusion process. Briefly, the U-Net architecture is composed of a contraction and expansion path. The contraction path decreases the resolution of the signal while increasing the number of feature channels through convolution and pooling. The expansion path increases resolution and decreases the number of feature channels through up-sampling and convolution over the concatenated signal with the contraction path. The training parameters for the model include time steps, learning rate, epochs, noise mode, learning rate schedule, and batch size. The nucleotide sequence of the guide-target scaffold for each guide in the multi-target library was one-hot encoded as a vector having four values (representing A, U, G, and C, respectively), where 1 was used to represent the nucleotide present at a position and 0 was used to represent the absence of that nucleotide at the position. For example, a vector [1, 0, 0, 0] for position Y in the nucleotide sequence can represent an A at position Y of the sequence. The encoded characters were mapped from {1,0} to {1,-1}.

In the realm of guide RNA (gRNA) generation, a method akin to one-hot encoding was applied, where each sequence entry assumes a value between -1 and 1. Operating in a continuous space, the model accepts discrete values as input and thresholds its outputs back into the discrete domain. The conditional diffusion model operates within the gRNA space, with the conditioning signal containing the one-hot encoded target sequence, and scalar values describing the editing and specificity.

During training, the forward diffusion process iteratively applies Gaussian noise to the sequence's one-hot encoding over a predetermined number of time steps. In the reverse diffusion process, a denoising U-net model then refines these noised gRNAs, aiming to match the final iteration's output with the initial input. This process enables the model to learn the distribution defining the interaction between the gRNA and target, as the experimentally measured editing and specificity. In each iteration, the gRNA is refined to align with the underlying distribution that governs its complementarity with the target sequence, along with the desired editing and specificity levels. When performing inference, the model samples Gaussian noise and takes as input the set of desired conditioning values before applying the previously described reverse diffusion process to obtain a sample. This novel application of diffusion models to gRNA generation provides a method to efficiently sample from regions of interest, e.g., gRNAs with high editing and specificity.

The dataset was partitioned into training, validation, and test subsets, comprising 60%, 20%, and 20% of the data, respectively. To prevent data leakage, the splits were stratified based on the targets ensuring that the model needed to find solutions for unseen targets. The CNN was trained using a stochastic gradient optimizer, while the conditional diffusion model employed an Adam optimizer. The latter was trained for 100 epochs with a learning rate of 1e-4. To stabilize the model and mitigate epoch-to-epoch fluctuations, an exponential moving average was applied with a beta of 0.995. Additionally, the conditional diffusion model utilized a linear beta learning rate scheduler with a beta value starting at 0.0001 and ending at 0.2.

The three models were then used to identify gRNA sequences against 42 novel mRNA targets, from 35 different genes, that were not present in the 5,643 diverse targets used to train the models. For the XGBoost model, a set of 1000 gRNA sequences were generated, using a heuristic that successively adds secondary structure to a perfectly formed duplex formed between the target sequence and the guide RNA, against each of the 42 novel targets. The ADAR1 and ADAR2 efficiency and specificity of each gRNA sequence was then scored by the XGBoost model. The top 140 guides screened for each target were selected for further evaluation based on a weighting based on predicted editing efficacy and a predicted specificity score.

The CNN model was used for input optimization to generate 300 gRNA sequences against each of the 42 novel targets in a first batch and 430 gRNA sequences against each of the 42 novel targets in a second batch. Briefly, the parameters of the CNN component networks were frozen along with the portion of the input attributed to the target and a stochastic gradient descent optimization procedure was used to generate gRNA sequences starting from seeds. Briefly, for each guide, the input optimization procedure was seeded with a random representation of a guide sequence, where each nucleotide is encoded as a vector having four values (representing A, U, G, and C, respectively) ranging between 0 and 1 that collectively sum to 1 and the target sequence one-hot encoded for the identity of the nucleotide at each position of the target. 1000 total optimization iterations were performed using the stochastic gradient descent optimization procedure to minimize a loss function $(Loss = |\hat{Y} - Y_d|$ where $\hat{Y}$ are predicted values for on-target editing efficiency, editing specificity, and MFE) using target metrics for on-target editing efficiency and editing specificity weighted evenly. MFE was weighted as 0 during the optimization. Two constraints were strictly enforced during the input optimization: no nucleotide values were allowed to fall below zero or exceed one, and the nucleotide values in each vector were required to sum to one. During the optimization procedure, the iterative gRNA solution often ventured outside the feasible space. To combat this, the updated seed for the gRNA sequence being refined was projected back to meet the constraints every 25 steps. After a predetermined number of steps, the iterative solution was projected to the nearest one-hot encoding of a sequence, which was considered the final solution for a given initialization.

The bit diffusion model was used to generate 50,000 gRNA sequences against each of the 42 novel targets. Briefly, for each initialization of the generative bit diffusion process, an 4 x n matrix seed was generated for the concatenation of a gRNA sequence, a short linker, and the target sequence, where each of the 4 rows of the matrix corresponded to a different nucleotide and each of the n columns in the matrix corresponded to a position in the concatenated guide-linker-target sequence. The columns in the matrix corresponding to the linker and the target sequence were one-hot encoded with the corresponding sequences, with a value of 1 corresponding to the presence of a particular nucleotide at the position and a value of -1 corresponding to the absence of the particular nucleotide at the position. The columns in the matrix corresponding to the gRNA sequence were initialized with a value between -1 and 1 for each nucleotide (each row) sampled from a normal distribution. Reverse diffusion of the seed was then performed over 100 epochs. At each epoch, the model was conditioned at one or more blocks with target performance metrics for ADAR1-mediated editing efficacy, ADAR1-mediated editing specificity, ADAR2-mediated editing efficacy, and ADAR2-mediated editing specificity.

The gRNA sequences selected from the structural sampling with the XGBoost model, the gRNA sequences generated by input optimization, and the gRNA sequences generated by the reverse diffusion process were used to synthesize single-stranded guide-target hairpins and evaluated for ADAR1-mediated and ADAR2-mediated editing of the target nucleotide in each target sequence using a cell-free *in vitro* screening assay as described in PCT International Patent Application Publication No. WO 2022/119975, the disclosure of which is incorporated herein by reference in its entirety for all purposes. Briefly, each candidate guide RNA was synthesized in a single RNA with the target sequence bridged through a nucleotide linker capable of forming a hairpin, such that the guide folds over and forms an intramolecular guide-target scaffold. The candidate guide/target hairpins were incubated in vitro with purified ADAR1 and/or ADAR2. After incubation, the RNAs are reverse transcribed and sequenced. ADAR-mediated editing at the target position and at each off-target adenosine are scored on a unique-read by unique-read basis. Each gRNA sequence was then scored as either a hit or not a hit based on whether a threshold percentage of target sequences were edited at only the target nucleotide in the assay.

**FIG. 24A** shows a representative plot of the percentage of hits obtained using each of the three techniques as a function of the threshold, from 0.2 to 0.8, for 1 of the 42 novel targets. In **FIG. 24A,** trace 2402 represents the percentage of hits obtained by generative bit diffusion as the threshold is relaxed, trace 2404 represents the percentage of hits obtained by structural sampling with the XGBoost model, and trace 2406 represents the percentage of hits obtained from input optimization. As the Figure shows, generative bit diffusion provides a significantly higher percentage of hits across a range of thresholds. The threshold was then fixed at 40% editing of the target nucleotide only and the results for all 42 novel targets were evaluated for ADAR1-mediated editing and ADAR2-mediated editing. **FIG. 24B** illustrates the results of this analysis in a box and whisker plot, where the median percentage is represented as the center bar in the box, the upper quartile (top 25th percentile) by the upper boundary of the box, the lower quartile (bottom 25th percentile) by the lower boundary of the box, the upper extreme (top 10th percentile) by the upper whisker, and the lower extreme (bottom 10th percentile) by the lower whisker. As shown, the generative bit diffusion process resulted in a significantly higher percentage of hits across the set of 42 novel targets and provided significantly more hits for the highest performing targets than did the structural sampling and input optimization. **FIG. 24C** illustrates the hit rate for all 42 novel targets individually across the three techniques using both ADAR enzymes, with lines connecting results for the same target across all three techniques. As shown, bit diffusion provided a higher number of hits for most targets and about an equivalent number of hits for the other novel targets.

Taken together, these results suggest that generative diffusion models, such as bit diffusion, outperform other models for gRNA guide design / selection for novel targets (target sequences that were not used in the training of generalized models). Moreover, the bit diffusion process was approximately 10,000 times faster at generating new sequences than the input optimization procedure.

### *Example 6 - Comparison of diffusion, hardcoded diffusion, and partial diffusion models for generative guide RNA design.*

This example demonstrates comparison of three denoising diffusion techniques for generative guide RNA design: denoising diffusion, denoising diffusion with a hardcoded core footprint, and partial denoising diffusion using a mask to update the core footprint at each step of the denoising.

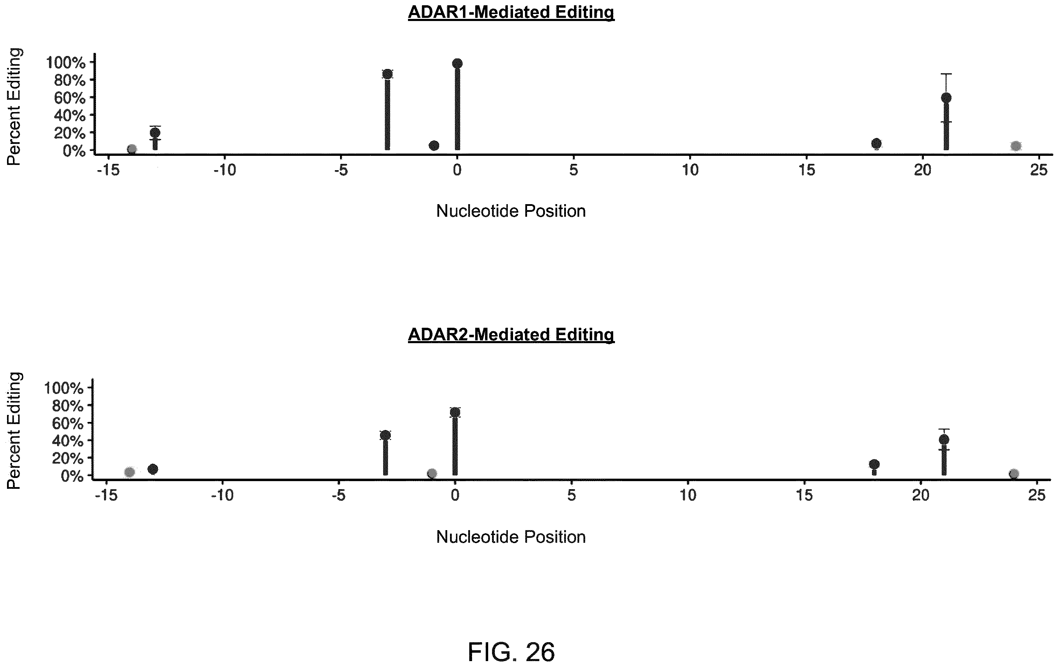

An 11 nucleotide core footprint for guide RNAs directing ADAR-mediated editing of a target site in a target mRNA was identified, spanning from the -4 position (4 nucleotides upstream of the target site) to the +7 position (7 nucleotides downstream of the target site), excluding the target nucleotide. While this footprint promoted robust editing of the target nucleotide, significant off-target editing was observed at the -3 and -13 positions in guides containing this footprint. For instance, **FIG. 26** illustrates ADAR1 and ADAR2 editing of the target mRNA mediated by a guide RNA containing the core footprint sequence, in which nucleotide position 0 corresponds to the target nucleotide for ADAR editing. It was proposed that denoising diffusion could be used to identify sequences upstream and downstream of the core footprint that reduced the off-target editing.

Three techniques were compared: denoising diffusion, denoising diffusion with a hardcoded core footprint, and partial denoising diffusion using a mask to update the core footprint at each step of the denoising. For all three techniques, a bit diffusion model trained as described in **Example 1** was used. As described above, the transitional model for the denoising diffusion process was a U-Net convolutional neural network conditioned against in vitro editing data for a multi-target library of 50,253 gRNAs. For the standard diffusion process, the sequence of the guide was denoised over 50 epochs as described above, without biasing against the core footprint. For the hardcoded process, the sequence of the guide was denoised over 50 epochs as described above, without biasing against the core footprint, and then modified by replacing part of the denoised sequence with the sequence of the core footprint. For the partial diffusion process, the sequence of the guide was denoised over 50 epochs. However, after every epoch, the partially denoised seed for the sequence was replaced with the sequence of the core footprint.

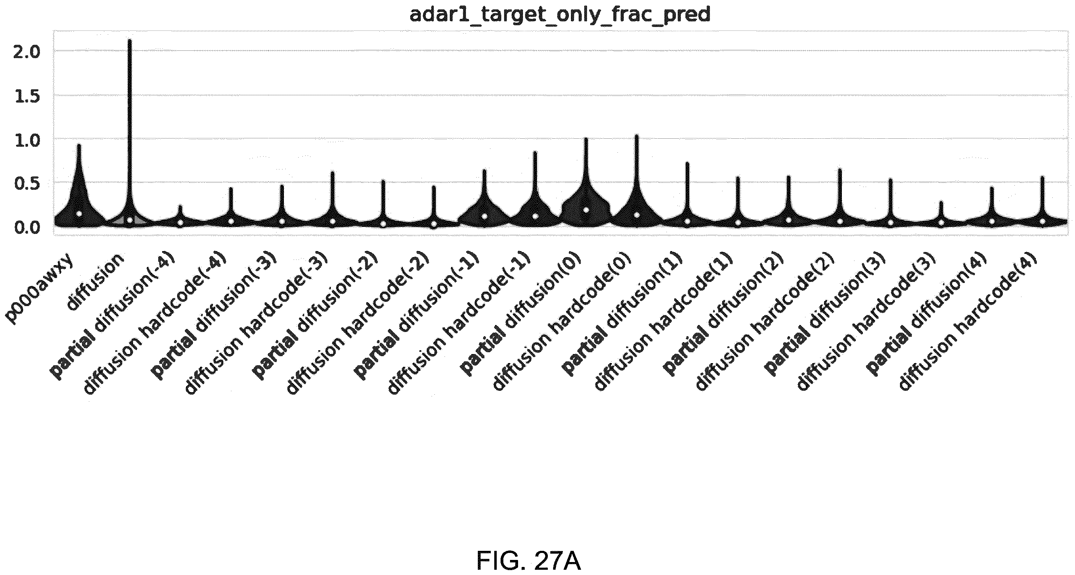

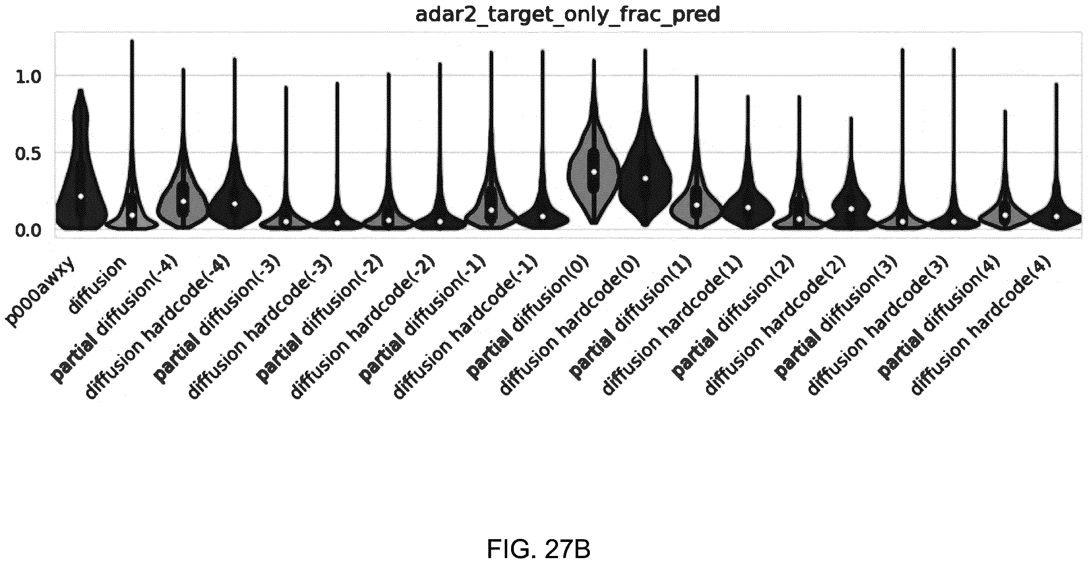

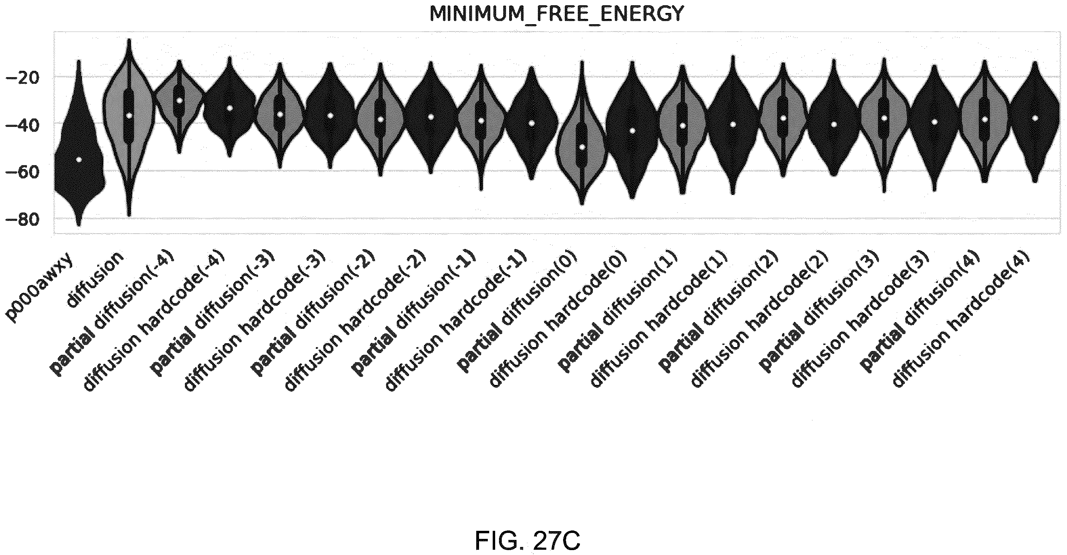

The standard bit diffusion process was performed 24,000 times, generating 24,000 candidate guide RNA sequences. The hardcoded and partial denoising processes were performed 2000 times each, generating 2000 candidate guide RNA sequences for each process. Then, the position of the core footprint was shifted 1, 2, 3, and 4 positions upstream ((-1), (-2), (-3), and (-4), as indicated in **FIGS. 27A, 27B,** and **27C)** and 1, 2, 3, and 4 positions downstream ((1), (2), (3), and (4), as indicated in **FIGS. 27A, 27B,** and **27C),** and the hardcoded and partial denoising processes were performed 2000 times for each process at each register shift, generating 16,000 additional candidate guide sequences for each process. Sequences generated without register shifting with core footprint are indicated as (0) in **FIGS. 27A, 27B, and 27C.** Finally, the core footprint was trimmed to the central 9, 7, and 5 nucleotides and the hardcoded and partial denoising processes were performed 2000 times for each process at each level of trimming, generating 6000 additional candidate guide sequences for each process. In total 24,000 candidate guide sequences were generated using each of the three diffusion techniques.

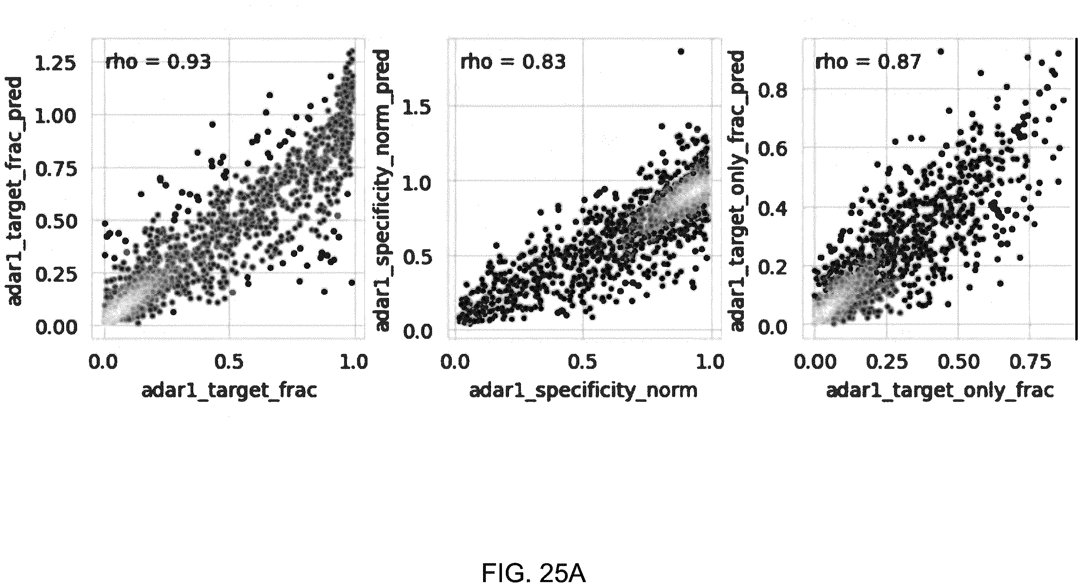

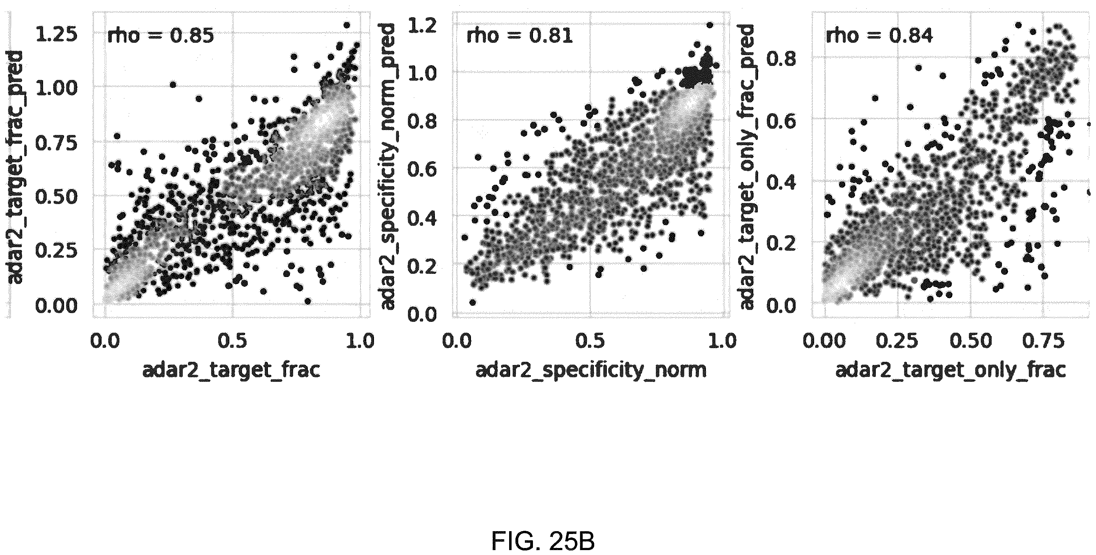

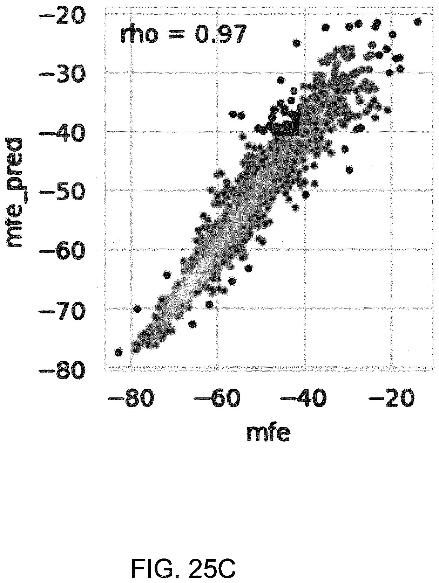

To score the guide RNAs generated above, separate gradient boosted ensemble models were trained using XGBoost, against in vitro editing data for a library of 4233 guide RNAs for the target site in the target mRNA using a 1/3 training/test split, for predicting (i) ADAR1 target fraction (defined as the percentage of reads with the target nucleotide edited in the *in vitro* assay by ADAR1), (ii) normalized ADAR1 specificity (defined as 1 - the proportion of reads with any off-target nucleotides edited in the *in vitro* assay by ADAR1), (iii) ADAR1 target fraction only (defined as the percentage of reads in which only the target nucleotide is edited in the *in vitro* assay by ADAR1), (iv) ADAR2 target fraction (defined as the percentage of reads with the target nucleotide edited in the *in vitro* assay by ADAR2), (v) normalized ADAR2 specificity (defined as 1 - the proportion of reads with any off-target nucleotides edited in the *in vitro* assay by ADAR2), (vi) ADAR2 target fraction only (defined as the percentage of reads in which only the target nucleotide is edited in the *in vitro* assay by ADAR2), and (vii) minimum free energy (MFE) as predicted using using the ViennaRNA python package (Lorenz, R. et al., ViennaRNA Package 2.0, Algorithms for Molecular Biology, 6:126, 2011, doi:10.1186/1748-7188-6-26). Performance of the models against the test split of the training data is illustrated in **FIGs. 25A-25C.**

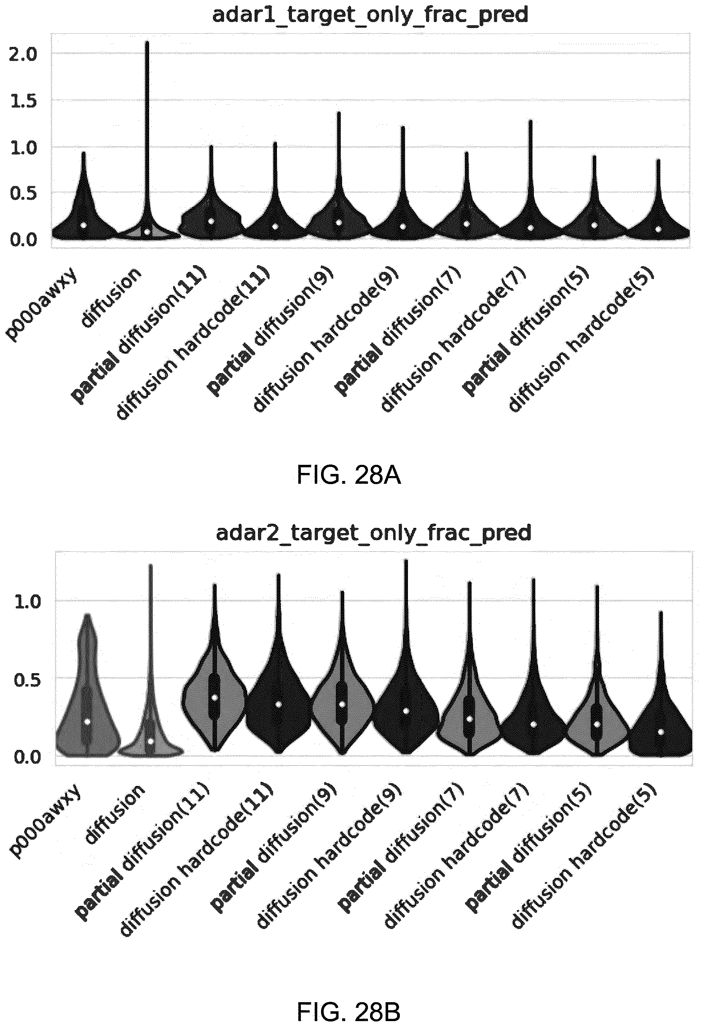

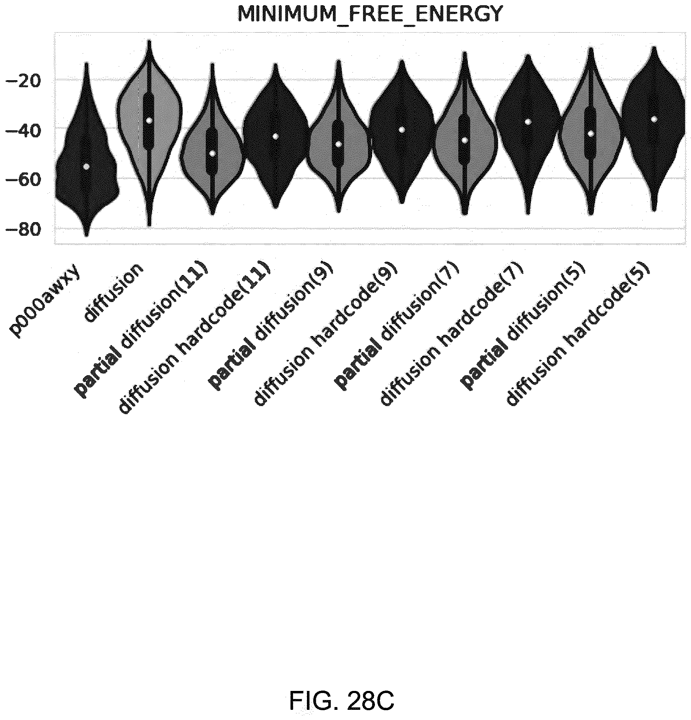

Each of these models was then used to score each of the 24,000 candidate guide sequences generated using each of the three diffusion techniques above. Violin plots illustrating the ADAR1 target fraction only, ADAR2 target fraction only, and MFE metrics predicted for each group of guides generated by standard diffusion (diffusion), partial diffusion incorporating the entire core footprint sequence (partial diffusion), and diffusion where the core footprint sequence was hardcoded into the guide (diffusion hardcode), are shown in **FIGS. 27A, 27B,** and **27C,** respectively. The violin plot labeled as p000awxy corresponded to the set of training guides used to train the XGBoost prediction models. Violin plots illustrating the same three metrics for each group of guides generated by partial diffusion or hardcoded diffusion incorporating different lengths of the core footprint sequence are illustrated in **FIGS. 28A, 28B,** and **28C,** respectively.

As shown in **FIGS. 27A-27C,** when placed right across the targeted positions (0), the core footprint sequence introduced by partial diffusion is predicted to perform better than hardcoded and normal diffusion. Moreover, partial diffusion with centered footprints also leads to lowest overall MFE for generative samples. Further, as shown in **FIGS. 28A-28C,** editing performance decreases as the length of the core footprint sequence is decreased and MFE increases.

## 7.8. Additional Considerations

All references cited herein are incorporated by reference to the same extent as if each individual publication, database entry (e.g., Genbank sequences or GeneID entries), patent application, or patent, was specifically and individually indicated to be incorporated by reference in its entirety, for all purposes. This statement of incorporation by reference is intended by Applicants, pursuant to 37 C.F.R. §1.57(b)(1), to relate to each and every individual publication, database entry (e.g., Genbank sequences or GeneID entries), patent application, or patent, each of which is clearly identified in compliance with 37 C.F.R. §1.57(b)(2), even if such citation is not immediately adjacent to a dedicated statement of incorporation by reference. The inclusion of dedicated statements of incorporation by reference, if any, within the specification does not in any way weaken this general statement of incorporation by reference. Citation of the references herein is not intended as an admission that the reference is pertinent prior art, nor does it constitute any admission as to the contents or date of these publications or documents.

The foregoing description of the embodiments has been presented for the purpose of illustration; it is not intended to be exhaustive or to limit the patent rights to the precise forms disclosed. Persons skilled in the relevant art will appreciate that many modifications and variations are possible in light of the above disclosure.

Any feature mentioned in one claim category, e.g., method, can be claimed in another claim category, e.g., computer program product, system, storage medium, as well. The dependencies or references back in the attached claims are chosen for formal reasons only. However, any subject matter resulting from a deliberate reference back to any previous claims (in particular multiple dependencies) can be claimed as well, so that any combination of claims and the features thereof is disclosed and can be claimed regardless of the dependencies chosen in the attached claims. The subject matter will be understood to include not only the combinations of features as set out in the disclosed embodiments but also any other combination of features from different embodiments. Various features mentioned in the different embodiments can be combined with explicit mentioning of such combination or arrangement in an example embodiment or without any explicit mentioning. Furthermore, any of the embodiments and features described or depicted herein can be claimed in a separate claim and/or in any combination with any embodiment or feature described or depicted herein or with any of the features.

Some portions of this description describe the embodiments in terms of algorithms and symbolic representations of operations on information. These operations and algorithmic descriptions, while described functionally, computationally, or logically, are understood to be implemented by computer programs or equivalent electrical circuits, microcode, or the like. Furthermore, it has also proven convenient at times, to refer to these arrangements of operations as engines, without loss of generality. The described operations and their associated engines are, in some embodiments, embodied in software, firmware, hardware, or any combinations thereof.

Any of the steps, operations, or processes described herein, in some embodiments, are performed or implemented with one or more hardware or software engines, alone or in combination with other devices. In one embodiment, a software engine is implemented with a computer program product comprising a computer-readable medium containing computer program code, which can be executed by a computer processor for performing any or all of the steps, operations, or processes described. The term "steps" does not mandate or imply a particular order. For example, while this disclosure describes, in some embodiments, a process that includes multiple steps sequentially with arrows present in a flowchart, the steps in the process do not need to be performed by the specific order claimed or described in the disclosure. In some implementations, some steps are performed before others even though the other steps are claimed or described first in this disclosure. Likewise, any use of (i), (ii), (iii), *etc.,* or (a), (b), (c), *etc.,* in the specification or in the claims, unless specified, is used to better enumerate items or steps and also does not mandate a particular order.

Throughout this specification, plural instances may implement components, operations, or structures described as a single instance. Although individual operations of one or more methods are illustrated and described as separate operations, in some implementations one or more of the individual operations are performed concurrently, and nothing requires that the operations be performed in the order illustrated. Structures and functionality presented as separate components in example configurations are, in some embodiments, implemented as a combined structure or component. Similarly, in some embodiments, structures and functionality presented as a single component are implemented as separate components. These and other variations, modifications, additions, and improvements fall within the scope of the subject matter herein. In addition, the term "each" used in the specification and claims does not imply that every or all elements in a group need to fit the description associated with the term "each." For example, "each member is associated with element A" does not imply that all members are associated with an element A. Instead, the term "each" only implies that a member (of some of the members), in a singular form, is associated with an element A. In claims, in some instances, the use of a singular form of a noun implies at least one element even though a plural form is not used.

Finally, the language used in the specification has been principally selected for readability and instructional purposes, rather than selected to delineate or circumscribe the patent rights. It is therefore intended that the scope of the patent rights be limited not by this detailed description, but rather by any claims that issue on an application based hereon. Accordingly, the disclosure of the embodiments is intended to be illustrative, but not limiting, of the scope of the patent rights.

Although inventions have been particularly shown and described with reference to a preferred embodiment and various alternate embodiments, it will be understood by persons skilled in the relevant art that various changes in form and details can be made therein without departing from the spirit and scope of the invention.

## Claims

1. A method for generating a polymer sequence for a biological molecule having one or more target biological properties, the method comprising:

   at a computer system comprising at least one processor and a memory storing at least one program for execution by the at least one processor:

   inputting (i) a plurality of target metrics for one or more target biological properties of a biological molecule and (ii) a seed for a nucleic acid or amino acid sequence for the biological molecule into an initial state $X_1$ in a plurality of consecutive states $X_N$ in a Markov chain of a generative diffusion model to obtain as output from the generative diffusion model a nucleic acid or amino acid sequence for the biological molecule that is predicted by the generative diffusion model to confer on the biological molecule the one or more target biological properties approximating the plurality of target metrics, wherein:

   for each respective consecutive state $X_n$ in the plurality of consecutive states $X_N$ in the Markov chain following the initial state $X_1$, the diffusion model generates a corresponding denoised seed for the nucleic acid or amino acid sequence for the biological molecule using a transition model, wherein the transition model comprises a plurality of layers, that accounts for the plurality of target metrics for the one or more target biological properties using as input:

   the seed for the nucleic acid or amino acid sequence, when the respective state $X_n$ is the state immediately following the initial state $X_1$, and

   a corresponding updated seed for the nucleic acid or amino acid sequence based on the corresponding denoised seed for the nucleic acid or amino acid sequence from the respective state $X_{n-1}$ in the plurality of consecutive states $X_N$ in the Markov chain that immediately precedes the respective state $X_n$, when the respective state $X_n$ is not the state immediately following the initial state $X_1$.

2. The method of claim 1, wherein:

   an indication of the position, in the Markov chain, of the transition from the state that immediately precedes the respective consecutive state $X_n$ is incorporated into one or more respective layers in the plurality of layers of the transition model; and

the generative diffusion model generates, for each respective consecutive state $X_n$ in the plurality of consecutive states $X_N$ in the Markov chain following the initial state $X_1$, the corresponding denoised seed for the nucleic acid or amino acid sequence for the biological molecule.

3. The method of claim 2, wherein:

the plurality of layers of the transition model comprises one or more attention layers that attend to an embedding of the indication of the position, in the Markov chain, of the transition, or
the plurality of layers of the transition model comprises one or more temporal projection layers that project the indication of the position, in the Markov chain, of the transition on an output of a previous layer in the plurality of layers of the transition model using a mapping function between the indication of the position, in the Markov chain, of the transition and a corresponding set of weights for the temporal projection layer.

4. The method of any one of claims 1-3, wherein the transition model comprises a U-Net neural network.

5. The method of any one of claims 1-4, wherein the plurality of target metrics for the one or more target biological properties of the biological molecule are incorporated into one or more respective layers in the plurality of layers of the transition model.

6. The method of claim 5, wherein:

the plurality of layers of the transition model comprises one or more attention layers that attend to an embedding of the plurality of target metrics for the one or more target biological properties of the biological molecule, or
the plurality of layers of the transition model comprises one or more projection layers that project the plurality of target metrics for the one or more target biological properties on an output of a previous layer in the plurality of layers of the transition model using a mapping function between the plurality of target metrics for the one or more target biological properties of the biological molecule and a corresponding set of weights for the projection layer.

7. The method of any one of claims 1-6, wherein the generative diffusion model is a bit diffusion model, and wherein: the obtaining as output from the generative diffusion model a nucleic acid sequence for the biological molecule comprises:

obtaining a representation of the nucleic acid or amino acid sequence for the biological molecule that is analog bit encoded, and
applying a thresholding operation to the representation of the nucleic acid or amino acid sequence for the biological molecule, thereby obtaining nucleic acid or amino acid sequence for the biological molecule.

8. The method of claim 7, wherein the biological molecule is a nucleic acid and the seed of the nucleic acid sequence is one hot encoded into a 2-bit encoding, optionally wherein the 2-bit encoding for the seed of the nucleic acid sequence is mapped from {0, 1} to {-1, 1}.

9. The method of any one of claims 1-8, wherein the biological molecule is a nucleic acid.

10. The method of claim 9, wherein the nucleic acid is a guide RNA (gRNA) that facilitates deamination of one or more target adenosines in a target RNA by an Adenosine Deaminases Acting on RNA (ADAR) protein.

11. The method of claim 10, wherein a polynucleotide sequence for the target mRNA, encompassing the target nucleotide position and at least a region of the mRNA 5' of the target nucleotide position and a region of the mRNA 3' of the target nucleotide position, is incorporated into one or more respective layers in the plurality of layers of the transition model.

12. The method of claim 11, wherein:

the plurality of layers of the transition model comprises one or more attention layers that attend to an embedding of the polynucleotide sequence for the target mRNA, or
the plurality of layers of the transition model comprises one or more projection layers that project the polynucleotide sequence for the target mRNA on an output of a previous layer in the plurality of layers of the transition model using a mapping function between an embedding of polynucleotide sequence for the target mRNA and a corresponding set of weights for the projection layer.

13. The method of any one of claims 1-8, wherein the biological molecule is a polypeptide, optionally wherein the polypeptide is all or a portion of a capsid protein.

14. The method of any one of claims 1-13, wherein the corresponding updated seed for the nucleic acid or amino acid sequence is the corresponding denoised seed for the nucleic acid or amino acid sequence from the respective state $X_{n-1}$ in the plurality of consecutive states $X_N$ in the Markov chain that immediately precedes the respective state $X_n$.

15. The method of any one of claims 1-13, wherein the corresponding updated seed for the nucleic acid or amino acid sequence is a version of the corresponding denoised seed for the nucleic acid or amino acid sequence from the respective state $X_{n-1}$ in the plurality of consecutive states $X_N$ in the Markov chain that immediately precedes the respective state $X_n$, that is modified to replace a denoised representation of one or more nucleotide or amino acid residues with a representation for a defined one or more nucleotide or amino acid residues.

16. A system comprising:

    a processor; and
    a memory storing instructions, when executed by the processor, cause the processor to perform steps comprising any of the methods recited in claims 1-15.

17. A non-transitory computer-readable medium storing computer code comprising instructions, when executed by one or more processors, causing the processors to perform any of the methods recited in claims 1-15.

GAN (training schematic)Conditional

Experimental sequence

Experimental metrics

Input

Seed

Input

Generator "G"

Generated sequence

Input

Discriminator "D"

Expt'l/ Generated

Target metrics

FIG. 1A

EP 4 432 289 A1

# Conditional GAN Training (example inputs/outputs)

FIG. 1B

EP 4 432 289 A1

# Conditional GAN Training (example inputs/outputs) cont'd

Seed — [0.2, 0.4, 0.15, 0.25], [0.1, 0.35, 0.2, 0.35], [0.6, 0.15, 0.05, 0.2],...[0.35, 0.1, 0.25, 0.3]

Target metrics — **[ 0.2, 0.8, 0.4, ... 0.9 ]**

Input — [0.2, 0.4, 0.15, 0.25], [0.1, 0.35, 0.2, 0.35], [0.6, 0.15, 0.05, 0.2],...[0.35, 0.1, 0.25, 0.3], **[ 0.2, 0.8, 0.4, ... 0.9 ]**

|  | A | A | T | ⋅ ⋅ ⋅ | C |
|---|---|---|---|---|---|
| Generated Sequence | [1, 0, 0, 0], | [1, 0, 0, 0], | [0, 1, 0, 0], | ⋅ ⋅ ⋅ | [0, 0, 1, 0] |

FIG. 1C

EP 4 432 289 A1

# Conditional GAN (use schematic)

Seed

Target
metrics

Input

Generator
"G"

Generated
sequence

FIG. 1D

EP 4 432 289 A1

# Conditional GAN (example inputs/outputs)

Seed: [0.2, 0.4, 0.15, 0.25], [0.1, 0.35, 0.2, 0.35], [0.6, 0.15, 0.05, 0.2],...[0.35, 0.1, 0.25, 0.3]

Target metrics: **[ 0.2, 0.8, 0.4, ... 0.9 ]**

Concat. input: [0.2, 0.4, 0.15, 0.25], [0.1, 0.35, 0.2, 0.35], [0.6, 0.15, 0.05, 0.2],...[0.35, 0.1, 0.25, 0.3], **[ 0.2, 0.8, 0.4, ... 0.9 ]**

Generated Sequence:

A      A      T    · · ·    C

[1, 0, 0, 0],    [1, 0, 0, 0],    [0, 1, 0, 0],   · · ·   [0, 0, 1, 0]

FIG. 1E

EP 4 432 289 A1

Diffusion model (training schematic)

1. Forward diffusion

$$q(x_t|x_{t-1})$$

Experimental sequence

$x_0$ $\cdots$ $x_{t-1}$ $x_t$ $\cdots$ $x_T$

Diffused sequence @ $x_T$

Diffused sequence @ $x_T$

Experimental sequence

$x_0$ $\cdots$ $x_{t-1}$ $x_t$ $\cdots$ $x_T$

Experimental metrics

$$p_\emptyset(x_{t-1}|x_t)$$

2. Reverse diffusion (de-noising)

3. Maximize likelihood of $p_\emptyset(x_0)$; adjust model parameters $\emptyset$

FIG. 2A

EP 4 432 289 A1

# Diffusion Model Training (example inputs/intermediates/outputs)

Position: 1     2     3   · · ·   N

$x_0$ — Experimental sequence

[0, 1, 0, 0],    [1, 0, 0, 0],    [0, 0, 1, 0],   · · ·   [0, 0, 0, 1]

T     A     C   · · ·   G

$x_{t-1}$ — Diffused sequence @ $x_{t-1}$

[0.05, 0.8, 0.05, 0.1], [0.85, 0.07, 0.08, 0],   · · ·   [0.07, 0.08, 0.1, 0.75]

$x_t$ — Diffused sequence @ $x_t$

[0.08, 0.65, 0.1, 0.17], [0.75, 0.1, 0.15, 0.05],   · · ·   [0.09, 0.11, 0.2, 0.6]

$x_T$ — Diffused sequence @ $x_T$

[0.2, 0.4, 0.15, 0.25], [0.1, 0.35, 0.2, 0.35],   · · ·   [0.1, 0.35, 0.2, 0.35]

Forward diffusion

FIG. 2B

EP 4 432 289 A1

Diffusion Model Training (example inputs/intermediates/outputs) cont'd

FIG. 2C

# Diffusion model (use schematic)

Reverse diffusion (de-noising)

$$p_\phi(x_{t-1}|x_t)$$

Generated sequence

$x'_0$ ... $x'_{t-1}$ $x'_t$ ... $x'_T$

Seed sequence

Target metrics

FIG. 2D

## Diffusion model (example inputs/outputs)

Position:   1      2      3    $\cdots$    N

**Seed:** [0.2, 0.4, 0.15, 0.25], [0.1, 0.35, 0.2, 0.35], $\cdots$ [0.1, 0.35, 0.2, 0.35]

**Target metrics:** [ 0.2, 0.8, 0.4, ... 0.9 ]

**Input:** [0.2, 0.4, 0.15, 0.25], [0.1, 0.35, 0.2, 0.35], ... [0.1, 0.35, 0.2, 0.35], [ 0.2, 0.8, 0.4, ... 0.9 ]

—202

$X'_T$

$\vdots$

$X'_t$

$\vdots$

$X'_{t-1}$

$\vdots$

$X'_0$

Generative (de-noising)

**De-noised seed @ $X'_{t-1}$:** [0.08, 0.65, 0.1, 0.17], [0.75, 0.1, 0.15, 0.05], $\cdots$ [0.09, 0.11, 0.2, 0.6]

**Target metrics:** [ 0.2, 0.8, 0.4, ... 0.9 ]

**Input:** [0.08, 0.65, 0.1, 0.17], [0.75, 0.1, 0.15, 0.05], ... [0.09, 0.11, 0.2, 0.6], [ 0.2, 0.8, 0.4, ... 0.9 ]

FIG. 2E

EP 4 432 289 A1

# Diffusion model (example inputs/outputs)

Position:  1             2             3      · · ·      N

Generative (de-noising)

$x'_T$

⋮

$x'_0$

De-noised seed @ $x'_0$   [0.01, 0.94, 0.03, 0.02],   [0.92, 0.05, 0.01, 0.02],   · · ·   [0.01, 0.03, 0.02, 0.94]

Projection

Generated sequence   [0, 1, 0, 0],   [1, 0, 0, 0],   [0, 0, 0, 1],   · · ·   [0, 0, 0, 1]

T             A             G      · · ·      G

FIG. 2F

# Denoising diffusion conditional GAN (training schematic)

$q(x_t|x_{t-1})$  Forward diffusion

$x_0$ ... $x_{t-1}$    $x_t$ ... $x_T$

$x_0$ ... $x_{t-1}$    $x_t$ ... $x_T$    for $t$ in $T$

$p_\varnothing(x_{t-1}|x_t)$

Experimental metrics

Denoised seq. at time step $t$   $x_t$

Diffused seq. at time step t-1   $x_{t-1}$

Experimental metrics

Input

Input

*Posterior sampling*
$q(x'_{t-1}|x_t, x'_0)$

G

Generated seq. at time step 0   $x'_0$

Generated seq. at time step t-1   $x'_{t-1}$

Input

D

Expt'l / Generated

FIG. 3A

EP 4 432 289 A1

# Denoising diffusion conditional GAN (use schematic)

Let $n = N$; set $N$ as predetermined number of diffusion steps

Target metrics — Seed

Input

$n = n - 1$

G — Generated seq. at time step 0 $x'_0$

Posterior sampling $q(x'_{n-1}|x_n, x'_0)$

Generated seq. at time step t-1 $x'_{n-1}$

Input

for $n > 1$, else output generated seq. $x'_0$

FIG. 3B

EP 4 432 289 A1

Denoising conditional GAN (training schematic) – Example for T = 2 @ $t = 2$

FIG. 4A

EP 4 432 289 A1

EP 4 432 289 A1

Denoising conditional GAN (training schematic) – Example for T = 2 @ *t* = 1

$q(x_1|x_0)$

$X_0$     $X_1$     $X_2$

Forward diffusion

*t* = 1

FIG. 4B

## Denoising conditional GAN (use schematic) – Example for T = 2

*t* = 2

Seed / Diffused seq. $X_2$

Target metrics

Concat. input

Generated seq. at time step 0 $x'_0$

*Posterior sampling* $q(x'_1|x_2, x'_0)$

Generated seq. at time step 1 $x'_1$

Target metrics

*t* = 1

Generated seq. at time step 1 $x'_1$

Target metrics

Concat. input

Generated seq. at time step 0 $x'_0$

Target metrics

FIG. 4C

EP 4 432 289 A1

500

| | |
|---|---|
| Operating system | 520 |
| Network communications module | 522 |
| Information data store | 530 |
| Model construct store | 540 |
| Output data store | 570 |
| ⋮ | |

512

518

Network
506

CPU
502

Power
supply
514

User interface 506

Display — 508
Input — 510

Network
interface
504

FIG. 5A

| Information data store 530 | | |
|---|---|---|
| | Biological molecule 1 532-1 | |
| | | Target metric value 1 534-1-1 |
| | | ⋮ |
| | | Target metric value L 534-1-L |
| | | Seed for biological molecule 1 536-1 |
| | ⋮ | |
| | Biological molecule K 532-K | |
| Model construct store 540-1 | | |
| | Conditional generator model 541 | |
| Output data store 570 | | |
| | Output sequence 572 | |

## FIG. 5B

Information data store 530

> Biological molecule 1 532-1
>
> > Target metric value 1 534-1-1
> >
> > ⋮
> >
> > Target metric value L 534-1-L
> >
> > Seed for biological molecule 1 536-1
>
> ⋮
>
> Biological molecule K 532-K

Model construct store 540-2

> Generative diffusion model 542
>
> > Initial state 1 544-1
> >
> > State 2 544-2
> >
> > > Denoised seed for state 2 546-2
> >
> > ⋮
> >
> > State N 544-N
> >
> > > Denoised seed for state N 546-N
> >
> > Transition model 548
> >
> > > Layer 1 550-1
> > >
> > > ⋮
> > >
> > > Layer P 550-P

Output data store 570

> Output sequence 572

# FIG. 5C

600

A method for generating a polymer sequence for a biological molecule having one or more target biological properties. In some embodiments, the method is performed at a computer system comprising at least one processor and a memory storing at least one program for execution by the at least one processor.

602

Input (i) a plurality of target metric values for one or more target biological properties of a biological molecule and (ii) a seed for a nucleic acid or amino acid sequence for the biological molecule into a conditional generator model from a conditional generative adversarial network to obtain as output from the conditional generator model a nucleic acid or amino acid sequence for the biological molecule that is predicted by the conditional generator model to confer on the biological molecule the one or more target biological properties approximating the plurality of target metric values.

FIG. 6

—700

A method for generating a polymer sequence for a biological molecule having one or more target biological properties. In some embodiments, the method is performed at a computer system comprising at least one processor and a memory storing at least one program for execution by the at least one processor.

—702

Input (i) a plurality of target metrics for one or more target biological properties of a biological molecule and (ii) a seed for a nucleic acid or amino acid sequence for the biological molecule into an initial state X1 in a plurality of consecutive states XN in a Markov chain of a generative diffusion model to obtain as output from the generative diffusion model a nucleic acid or amino acid sequence for the biological molecule that is predicted by the generative diffusion model to confer on the biological molecule the one or more target biological properties approximating the plurality of target metrics.

For each respective consecutive state Xn in the plurality of consecutive states XN in the Markov chain following the initial state X1, the diffusion model generates a corresponding denoised seed for the nucleic acid or amino acid sequence for the biological molecule using a transition model, where the transitional model comprises a plurality of layers, that accounts for the plurality of target metrics for the one or more target biological properties using as input: the seed for the nucleic acid or amino acid sequence, when the respective state Xn is the state immediately following the initial state X1, and the corresponding denoised seed for the nucleic acid or amino acid sequence based on the respective state Xn-1 in the plurality of consecutive states XN in the Markov chain that immediately precedes the respective state Xn, when the respective state Xn is not the state immediately following the initial state X1.

—704

An indication of the position, in the Markov chain, of the transition from the state that immediately precedes the respective consecutive state Xn is embedded into one or more respective layers in the plurality of layers of the transition model; and the generative diffusion model generates, for each respective consecutive state Xn in the plurality of consecutive states XN in the Markov chain following the initial state X1, the corresponding denoised seed for the nucleic acid or amino acid sequence for the biological molecule.

—706

The transition model comprises a U-Net neural network.

(A)

FIG. 7A

A

702 (Cont.)

708

The transition model is a conditional generator model from a conditional generative adversarial network that generates, for each respective consecutive state Xn in the plurality of consecutive states XN in the Markov chain following the initial state X1, a corresponding nucleic acid or amino acid sequence predicted by the conditional generator model to confer on the biological molecule the one or more target biological properties approximating the plurality of target metric values using as input: the seed for the nucleic acid or amino acid sequence, when the respective state Xn is the state immediately following the initial state X1, and the corresponding denoised seed sequence from the respective state Xn-1 in the plurality of consecutive states XN in the Markov chain that immediately precedes the respective state Xn, when the respective state Xn is not the state immediately following the initial state X1; and for each respective consecutive state Xn in the plurality of consecutive states XN in the Markov chain following the initial state X1, other than a terminal state Xn=N in the Markov chain, the diffusion model samples from a posterior distribution of seed values for the corresponding nucleic acid or amino acid sequence to generate the corresponding denoised seed for the nucleic acid or amino acid sequence.

B

FIG. 7B

B

702 (Cont.)

710

The plurality of target metrics for the one or more target biological properties of the biological molecule are embedded into one or more respective layers in the plurality of layers of the transition model.

712

The plurality of layers of the transition model comprises one or more attention layers that attend to an embedding of the plurality of target metrics for the one or more target biological properties of the biological molecule.

714

The generative diffusion model is a bit diffusion model.

716

The biological molecule is a nucleic acid and the seed of the nucleic acid sequence is one hot encoded into a 2-bit encoding.

718

The 2-bit encoding for the seed of the nucleic acid sequence is scaled from {0, 1} to {-1, 1}.

C

FIG. 7C

C

702 (Cont.)

714 (Cont.)

716

720

The transition model generates, for each respective consecutive state Xn in the plurality of consecutive states XN in the Markov chain following the initial state X1, a corresponding nucleic acid or amino acid sequence predicted by the conditional generator model to confer on the biological molecule the one or more target biological properties approximating the plurality of target metric values using as input: the seed for the nucleic acid or amino acid sequence, when the respective state Xn is the state immediately following the initial state X1, and the corresponding denoised seed sequence from the respective state Xn-1 in the plurality of consecutive states XN in the Markov chain that immediately precedes the respective state Xn, when the respective state Xn is not the state immediately following the initial state X1; and for each respective consecutive state Xn in the plurality of consecutive states XN in the Markov chain following the initial state X1, other than a terminal state Xn=N in the Markov chain, the diffusion model samples from a posterior distribution of seed values for the corresponding nucleic acid or amino acid sequence to generate the corresponding denoised seed for the nucleic acid or amino acid sequence.

722

For a respective consecutive state Xn in the plurality of consecutive states XN in the Markov chain, the corresponding nucleic acid or amino acid sequence predicted by the conditional generator model to confer on the biological molecule the one or more target biological properties approximating the plurality of target metric values is self-conditioned on the corresponding nucleic acid or amino acid sequence predicted by the conditional generator model to confer on the biological molecule the one or more target biological properties from the respective state Xn-1 in the plurality of consecutive states XN in the Markov chain that immediately precedes the respective state Xn.

D

FIG. 7D

D

702 (Cont.)

724

The biological molecule is a nucleic acid.

726

The nucleic acid is a transcriptional or translational regulatory element.

728

The nucleic acid is a guide RNA (gRNA) that facilitates deamination of one or more target adenosines in a target RNA by an Adenosine Deaminases Acting on RNA (ADAR) protein.

730

The nucleic acid is a gRNA that facilitates deamination of one or more target cytidines in a target RNA by an Apolipoprotein B mRNA Editing Catalytic Polypeptide-like (APOBEC) protein.

732

The one or more target biological properties comprises a metric for the efficiency of deamination of the one or more target adenosines by a first ADAR protein or the one or more target cytidines by a first APOBEC protein.

734

The metric for the efficiency of deamination is (i) a prevalence of deamination of the one or more target adenosines or the one or more target cytidines in a plurality of instances of the target mRNA or (ii) a prevalence of the absence of deamination of any nucleotide position in a respective instance of a target mRNA in a plurality of instances of the target mRNA.

E

FIG. 7E

E

702 (Cont.)

724 (Cont.)

730

736

The one or more target biological properties comprises a metric for the specificity of deamination of the one or more target adenosines or the one or more target cytidines relative to one or more nucleotide positions, other than the nucleotide positions of the one or more target adenosines or the one or more target cytidines, in a target mRNA by a first ADAR protein or a first APOBEC protein.

738

The metric for the specificity of deamination of the target nucleotide position relative to one or more nucleotide positions, other than the target nucleotide position, in the target mRNA by the first ADAR protein or the first APOBEC protein is: (i) a comparison of (a) a prevalence of deamination of the target nucleotide position in a plurality of instances of the target mRNA and (b) a prevalence of deamination of at least one nucleotide position, other than the target nucleotide position, in a respective instance of the target mRNA in a plurality of instances of the target mRNA, (ii) a prevalence of deamination of the target nucleotide position, without coincident deamination of one or more nucleotide positions other than the target nucleotide position, in a respective instance of the target mRNA in a plurality of instances of the target mRNA, or (iii) a prevalence of deamination of at least one nucleotide position, other than the target nucleotide position, in a respective instance of the target mRNA in a plurality of instances of the target mRNA.

740

At the one or more nucleotide positions, other than the target nucleotide position, in the target mRNA, deamination results in a non-synonymous codon edit.

F

FIG. 7F

F

702 (Cont.)

724 (Cont.)

730

742

A respective biological property in the one or more target biological properties is normalized by a metric for an efficiency or specificity of deamination of one or more nucleotide positions, other than the target nucleotide position, in a target mRNA by a first ADAR protein or a first APOBEC protein.

744

The one or more target biological properties comprises a metric for an efficiency or specificity of deamination of one or more nucleotide positions, other than the target nucleotide position, in a target mRNA by a first ADAR protein or first APOBEC protein when facilitated by hybridization of the gRNA to a target mRNA.

746

The first ADAR protein is human ADAR1 or human ADAR2.

748

The one or more target biological properties comprises an estimation of a minimum free energy (MFE) for the gRNA.

750

The one or more target biological properties comprises an estimation of a minimum free energy (MFE) for the guide-target RNA scaffold formed between the guide RNA (gRNA) and the target mRNA.

G

FIG. 7G

G

702 (Cont.)

752

The biological molecule is a polypeptide.

754

The polypeptide is all or a portion of a capsid protein.

756

The one or more target biological properties of the polypeptide comprise a measure of specificity of a recombinant Adeno Associated Virus (rAAV) comprising the capsid protein.

758

The measure of specificity of the rAAV for a respective tissue type in the one or more tissue types is normalized by a measure of specificity of a wild type AAV of the same serotype as the rAAV for the respective tissue type.

760

The measure of specificity of the rAAV for a respective tissue type in the one or more tissue types is normalized by a measure of specificity of the rAAV for one or more tissue types other than the respective tissue type.

FIG. 7H

FIG. 8A

FIG. 8B

FIG. 9A

FIG. 9B

FIG. 9C

EP 4 432 289 A1

FIG. 10

FIG. 11

FIG. 12

FIG. 13

FIG. 14A

FIG. 14B

Bit diffusion samples
Generation: MAPT library          Prediction: MAPT library, ADAR1

Model endpoint: ADAR1     Model endpoint: ADAR2     Model endpoint: ADAR1/2

Predicted Normalized Specificity MAPT ADAR1

Predicted Editing MAPT ADAR1

FIG. 15A              FIG. 15B              FIG. 15C

Bit diffusion samples
Generation: MAPT library          Prediction: MAPT library, ADAR2

Model endpoint: ADAR1     Model endpoint: ADAR2     Model endpoint: ADAR1/2

Predicted Normalized Specificity MAPT ADAR2

Predicted Editing MAPT ADAR2

FIG. 15D              FIG. 15E              FIG. 15F

Bit diffusion samples          Bit diffusion samples          Bit diffusion samples
Gen: MAPT library              Gen: MAPT library              Gen: MAPT library
Pred: MAPT library, ADAR1      Pred: MAPT library, ADAR2      Pred: MAPT library

Model endpoint: ADAR1          Model endpoint: ADAR2          Model endpoint: ADAR1/2

Predicted Norm Specificity MAPT ADAR1

Predicted Norm Specificity MAPT ADAR2

Predicted Editing MAPT ADAR2

Predicted Editing          Predicted Editing          Predicted Editing
MAPT ADAR1                 MAPT ADAR2                 MAPT ADAR1

FIG. 15G              FIG. 15H              FIG. 15I

Bit diffusion samples
Generation: ML library          Prediction: ML library, ADAR1
Model endpoint: ADAR1    Model endpoint: ADAR2    Model endpoint: ADAR1/2

Predicted Normalized Specificity ML ADAR1

Predicted Editing ML ADAR1

FIG. 16A          FIG. 16B          FIG. 16C

Bit diffusion samples
Generation: ML library          Prediction: ML library, ADAR2
Model endpoint: ADAR1    Model endpoint: ADAR2    Model endpoint: ADAR1/2

Predicted Normalized Specificity ML ADAR2

Predicted Editing ML ADAR2

FIG. 16D          FIG. 16E          FIG. 16F

Bit diffusion samples          Bit diffusion samples          Bit diffusion samples
Gen: ML library               Gen: ML library               Gen: ML library
Pred: ML library, ADAR1       Pred: ML library, ADAR2       Pred: ML library

Model endpoint: ADAR1         Model endpoint: ADAR2         Model endpoint: ADAR1/2

Predicted Norm Specificity ML ADAR1

Predicted Norm Specificity ML ADAR2

Predicted Editing ML ADAR2

Predicted Editing
ML ADAR1

Predicted Editing
ML ADAR2

Predicted Editing ML
ADAR1

FIG. 16G          FIG. 16H          FIG. 16I

EP 4 432 289 A1

Input optimization samples
Generation: MAPT library

Prediction: MAPT library, ADAR1

Model endpoint: ADAR1    Model endpoint: ADAR2    Model endpoint: ADAR1/2

Predicted Normalized Specificity MAPT ADAR1

Predicted Editing MAPT ADAR1

0.9

FIG. 17A          FIG. 17B          FIG. 17C

Input optimization samples
Generation: MAPT library

Prediction: MAPT library, ADAR2

Model endpoint: ADAR1    Model endpoint: ADAR2    Model endpoint: ADAR1/2

Predicted Normalized Specificity MAPT ADAR2

Predicted Editing MAPT ADAR2

FIG. 17D          FIG. 17E          FIG. 17F

Input optimization samples
Gen: MAPT library
Pred: MAPT library, ADAR1

Input optimization samples
Gen: MAPT library
Pred: MAPT library, ADAR2

Input optimization samples
Gen: MAPT library
Pred: MAPT library

Model endpoint: ADAR1

Model endpoint: ADAR2

Model endpoint: ADAR1/2

Predicted Norm Specificity MAPT ADAR1

Predicted Norm Specificity MAPT ADAR2

Predicted Editing MAPT ADAR2

Predicted Editing
MAPT ADAR1

Predicted Editing
MAPT ADAR2

Predicted Editing
MAPT ADAR1

FIG. 17G          FIG. 17H          FIG. 17I

Input optimization samples
Generation: ML library    Prediction: ML library, ADAR1

Model endpoint: ADAR1    Model endpoint: ADAR2    Model endpoint: ADAR1/2

Predicted Normalized Specificity ML ADAR1

Predicted Editing ML ADAR1

FIG. 18A          FIG. 18B          FIG. 18C

Input optimization samples
Generation: ML library    Prediction: ML library, ADAR2

Model endpoint: ADAR1    Model endpoint: ADAR2    Model endpoint: ADAR1/2

Predicted Normalized Specificity ML ADAR2

Predicted Editing ML ADAR2

FIG. 18D          FIG. 18E          FIG. 18F

Input optimization samples
Gen: ML library
Pred: ML library, ADAR1

Model endpoint: ADAR1

Input optimization samples
Gen: ML library
Pred: ML library, ADAR2

Model endpoint: ADAR2

Input optimization samples
Gen: ML library
Pred: ML library

Model endpoint: ADAR1/2

Predicted Norm Specificity ML ADAR1

Predicted Editing ML ADAR1

Predicted Norm Specificity ML ADAR2

Predicted Editing ML ADAR2

Predicted Editing ML ADAR2

Predicted Editing ML ADAR1

FIG. 18G          FIG. 18H          FIG. 18I

Exhaustive scoring samples
Generation: guidebuilder, ML library          Prediction: ML library, ADAR1
Model endpoint: ADAR1     Model endpoint: ADAR2   Model endpoint: ADAR1/2

Predicted Normalized Specificity ML ADAR1

Predicted Editing ML ADAR1

FIG. 19A          FIG. 19B          FIG. 19C

Exhaustive scoring samples
Generation: guidebuilder, ML library          Prediction: ML library, ADAR2
Model endpoint: ADAR1     Model endpoint: ADAR2   Model endpoint: ADAR1/2

Predicted Normalized Specificity ML ADAR2

Predicted Editing ML ADAR2

FIG. 19D          FIG. 19E          FIG. 19F

Exhaustive scoring samples       Exhaustive scoring samples       Exhaustive scoring samples
Gen: ML library                  Gen: ML library                  Gen: ML library
Pred: ML library, ADAR1          Pred: ML library, ADAR2          Pred: ML library

Model endpoint: ADAR1            Model endpoint: ADAR2            Model endpoint: ADAR1/2

Predicted Norm Specificity ML ADAR1

Predicted Editing ML ADAR1

Predicted Norm Specificity ML ADAR2

Predicted Editing ML ADAR2

Predicted Editing ML ADAR2

Predicted Editing ML ADAR1

FIG. 19G          FIG. 19H          FIG. 19I

Retina Bit Diffusion

ROC Curves

FIG. 20B

FIG. 20A

FIG. 21A

FIG. 21B

FIG. 21C

FIG. 22

FIG. 23A

FIG. 23B

FIG. 24A

FIG. 24B

ADAR1　　　　　　　　　　ADAR2

FIG. 24C

FIG. 25A

FIG. 25B

FIG. 25C

ADAR1-Mediated Editing

ADAR2-Mediated Editing

FIG. 26

EP 4 432 289 A1

FIG. 27A

FIG. 27B

FIG. 27C

## adar1_target_only_frac_pred

FIG. 28A

## adar2_target_only_frac_pred

FIG. 28B

MINIMUM_FREE_ENERGY

FIG. 28C

Europäisches
Patentamt

European
Patent Office

Office européen
des brevets

# EUROPEAN SEARCH REPORT

**Application Number**

EP 24 16 3981

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SHENGCHAO LIU ET AL: "A Text-guided Protein Design Framework", ARXIV.ORG, CORNELL UNIVERSITY LIBRARY, 201 OLIN LIBRARY CORNELL UNIVERSITY ITHACA, NY 14853, 9 February 2023 (2023-02-09), XP091434166, * the whole document * | 1-9, 14-17 | INV. G16B20/00 G16B40/20 |
| X | Ingraham John ET AL: "Illuminating protein space with a programmable generative model", bioRxiv, 2 December 2022 (2022-12-02), XP093183353, DOI: 10.1101/2022.12.01.518682 Retrieved from the Internet: URL:https://www.biorxiv.org/content/10.1101/2022.12.01.518682v1.full.pdf * the whole document * | 1-9, 14-17 | |
| A | ZHIYE GUO ET AL: "Diffusion Models in Bioinformatics: A New Wave of Deep Learning Revolution in Action", ARXIV.ORG, CORNELL UNIVERSITY LIBRARY, 201 OLIN LIBRARY CORNELL UNIVERSITY ITHACA, NY 14853, 13 February 2023 (2023-02-13), XP091443646, * the whole document * | 1-17 | **TECHNICAL FIELDS SEARCHED (IPC)** G16B |
| A | HANQUN CAO ET AL: "A Survey on Generative Diffusion Model", ARXIV.ORG, CORNELL UNIVERSITY LIBRARY, 201 OLIN LIBRARY CORNELL UNIVERSITY ITHACA, NY 14853, 8 October 2022 (2022-10-08), XP091337586, * the whole document * | 1-17 | |

The present search report has been drawn up for all claims

| Place of search | Date of completion of the search | Examiner |
|---|---|---|
| Munich | 8 July 2024 | Díaz de Lezana, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 63490435
- US 63506770
- US 63509254
- US 635011138 A
- US 63585119
- US 2015025175 W
- US 2014050423 W
- US 2016067353 W
- US 2018041503 W
- US 18041509 W
- US 2004011786 W
- US 2004011833 W
- US 10988763 B
- US 10941402 B
- EP 3507366 A
- US 20200199586 A
- EP 3712269 A
- WO 2021071858 A
- WO 2021243023 A
- EP 2852668 A
- EP 3103872 A
- US 9340784 B
- US 9796976 B
- WO 2022119975 A

### Non-patent literature cited in the description

- **ROSENTHAL.** *J Exp Biol,* June 2015, vol. 218 (12), 1812-1821
- **LIU et al.** Learning cis-regulatory principles of ADAR-based RNA editing from CRISPR-mediated mutagenesis. *Nat Commun.,* 2021, vol. 12 (1), 2165
- **AQUINO-JARQUIN.** Novel engineered programmable systems for ADAR-mediated RNA editing. *Mol. Ther. Nucleic Acids,* 2020, vol. 19, 1065-72
- **EGGINGTON et al.** Predicting sites of ADAR editing in double-stranded RNA. *Nat. Commun,* 2011, vol. 2 (1), 319
- **KIM et al.** RNA editing at a limited number of sites is sufficient to prevent MDA5 activation in the mouse brain. *PLOS Genetics,* 2021, vol. 17 (5), e1009516
- **ALTSCHUL et al.** *J. Mol. Biol.,* 1990, vol. 215, 403-410
- Imagenet classification with deep convolutional neural networks. **KRIZHEVSKY et al.** Advances in Neural Information Processing Systems 2. Curran Associates, Inc, 2012, 1097-1105
- **ZEILER.** ADADELTA: an adaptive learning rate method. *CoRR,* 2012, vol. abs/1212.5701
- Neurocomputing: Foundations of research. **RUMELHART et al.** ch. Learning Representations by Back-propagating Errors. MIT Press, 1988, 696-699
- **VINCENT et al.** Stacked denoising autoencoders: Learning useful representations in a deep network with a local denoising criterion. *J Mach Learn Res,* 2010, vol. 11, 3371-3408
- **LAROCHELLE et al.** Exploring strategies for training deep neural networks. *J Mach Learn Res,* 2009, vol. 10, 1-40
- **HASSOUN.** Fundamentals of Artificial Neural Networks. *Massachusetts Institute of Technology,* 1995
- **DUDA et al.** Pattern Classification. John Wiley & Sons, Inc, 2001
- **HASTIE et al.** The Elements of Statistical Learning. Springer-Verlag, 2001
- **DRAGHICI.** Data Analysis Tools for DNA Microarrays. Chapman & Hall/CRC, 2003
- **MOUNT.** Bioinformatics: sequence and genome analysis. Cold Spring Harbor Laboratory Press, 2001
- **CRISTIANINI ; SHAWE-TAYLOR.** An Introduction to Support Vector Machines. Cambridge University Press, 2000
- A training algorithm for optimal margin classifiers. **BOSER et al.** Proceedings of the 5th Annual ACM Workshop on Computational Learning Theory. ACM Press, 1992, 142-152
- **VAPNIK.** Statistical Learning Theory. Wiley, 1998
- **DUDA.** Pattern Classification. John Wiley & Sons, Inc, 2001, 259, , 262-265
- **HASTIE.** The Elements of Statistical Learning. Springer, 2001
- **FUREY et al.** *Bioinformatics,* 2000, vol. 16, 906-914

- **NG et al.** On discriminative vs. generative classifiers: A comparison of logistic regression and naive Bayes. *Advances in Neural Information Processing Systems,* 2002, vol. 14
- **HASTIE et al.** The elements of statistical learning: data mining, inference, and prediction. Springer, 2001
- **DUDA.** Pattern Classification. John Wiley & Sons, Inc, 2001
- **DUDA et al.** Pattern Classification. John Wiley & Sons, 2001
- **DUDA.** Pattern Classification. John Wiley & Sons, Inc, 2001, 395-396
- **DUDA.** Pattern Classification. John Wiley & Sons, Inc, 2001, 396-408
- **BREIMAN.** Random Forests--Random Features. *Technical Report 567, Statistics Department, U.C. Berkeley,* September 1999
- **AGRESTI.** An Introduction to Categorical Data Analysis. John Wiley & Son, 1996, 103-144
- **MCLACHLAN et al.** *Bioinformatics,* 2002, vol. 18 (3), 413-422
- **SCHLIEP et al.** *Bioinformatics,* 2003, vol. 19 (1), i255-i263
- **DUDA ; HART.** Pattern Classification and Scene Analysis. John Wiley & Sons, Inc, 1973
- **FERNANDES et al.** Transfer Learning with Partial Observability Applied to Cervical Cancer Screening. *Pattern Recognition and Image Analysis: 8th Iberian Conference Proceedings,* 2017, 243-250
- **BROWNLEE.** *A Gentle Introduction to Generative Adversarial Networks (GANs),* 2019
- **ZAMYATNIN, A.A.** Protein volume in solution. *Prog. Biophys. Mol. Biol.,* 1972, vol. 24, 107-123
- **KYTE ; DOOLITTLE.** *J. Mol. Biol.,* 1982, vol. 157, 105-132
- **ENGELMAN et al.** *, Annu. Rev. Biophys. Chem.,* 1986, vol. 15, 321-353
- **BHASKARAN, R. ; PONNUSWAMY, P.R.** *Int. J. Peptide and Protein Res,* 1988, vol. 32 (4), 241-255
- **DAYHOFF et al.** *Atlas of Protein Sequence and Structure,* 1978, vol. 5 (3
- **O'CONNOR.** *Introduction to Diffusion Models for Machine Learning,* 2022
- **XIAO et al.** Tackling the Generative Learning Trilemma with Denoising Diffusion GANs. *ICLR 2022*
- **RONNEBERGER.** U-Net: Convolutional Networks for Biomedical Image Segmentation. *arXiv:1505.04597v1,* 2015
- **CRISTINA.** The Attention Mechanism from Scratch. *Machine Learning Mastery,* 2022
- **MOKHTARANI.** *Embeddings in Machine Learning: Everything You Need to Know,* 2021
- **ALAMMAR.** *The Illustrated Stable Diffusion,* 2022
- **CHEN T. et al.** Analog Bits: Generating Discrete Data using Diffusion Models with Self-Conditioning,. *arXiv:2208.04202,* 2023
- **ENGELMAN et al.** *Annu. Rev. Biophys. Chem.,* 1986, vol. 15, 321-353
- **BHASKARAN, R ; PONNUSWAMY, P.R.** *Int. J. Peptide and Protein Res,* 1988, vol. 32 (4), 241-255
- **DAYHOFF et al.** *, Atlas of Protein Sequence and Structure,* 1978, vol. 5 (3
- **LORENZ, R. et al.** *ViennaRNA Package 2.0, Algorithms for Molecular Biology,* 2011, vol. 6 (1), 26
- **LORENZ, R et al.** ViennaRNA Package 2.0. *Algorithms for Molecular Biology,* 2011, vol. 6 (1), 26
- **LORENZ, R. et al.** ViennaRNA Package 2.0. *Algorithms for Molecular Biology,* 2011, vol. 6 (1), 26